# EUROPEAN PATENT APPLICATION

(11) **EP 2 607 357 A1**
(43) Date of publication of application: **26.06.2013**
(21) Application number: 13157532.6
(22) Date of filing: 08.10.2008
(51) Int. Cl.: C07D 277/82, C07D 417/12, C07D 417/14, C07D 491/08, A61K 31/428, A61P 3/00

(54) **BENZOTHIAZOLES AS GHRELIN RECEPTOR MODULATORS**

(30) Priority: 10.10.2007 US 978889 P
(62) Divisional of application: 08806736.8
(71) Applicant: Astrazeneca AB, SE-151 85 Södertälje (SE)
(72) Inventor: Allen, Jack, Mcqueen, Macclesfield, Cheshire SK10 4TG (GB); Butlin, Roger, John, Macclesfield, Cheshire SK10 4TG (GB); Green, Clive, Macclesfield, Cheshire SK10 4TG (GB); McCoull, William, Macclesfield, Cheshire SK10 4TG (GB); Robb, Graeme, Richard, Macclesfield, Cheshire SK10 4TG (GB); Wood, James, Matthew, Macclesfield, Cheshire SK10 4TG (GB)
(74) Representative: Storm, Jan Peter

(57) **Abstract**

A compound of formula I 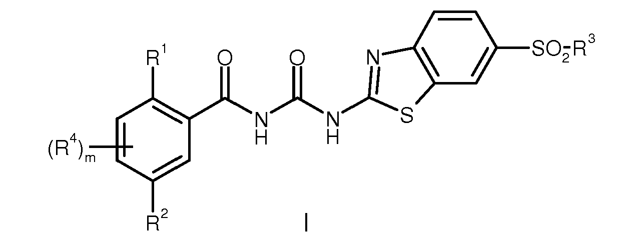 or a pharmaceutically acceptable salt thereof in which R¹, R², R³, R⁴ and m are as described in the specification for use in the treatment of obesity and/or diabetes.

## Description

### Field of invention

The present invention relates to N-aroyl-N'-(6-(optionally substituted) alkylsulfonyl benzothiazol-2-yl)ureas, to their use as Ghrelin receptor modulators that are useful in regulating food intake, to pharmaceutical formulations containing them and to processes for their preparation.

### Background of the invention

Ghrelin, a circulating hormone produced predominantly by endocrine cells in the stomach and intestines the stomach, is the endogenous ligand for the Growth Hormone Secretagogue-Receptor (GHS-R). It has been shown to act at the hypothalamus to increase food consumption. Circulating levels of this hormone rise prior to feeding, and drop rapidly following food intake. Hence it may act as a physiological meal-initiation signal. Circulating levels fall in obesity but rise with weight loss, indicative of a role in the long-term control of energy balance. The Growth Hormone Secretagogue receptor is the only known ghrelin receptor. Antagonists (or partial agonists or inverse agonists) at this receptor may block meal initiation, thus decreasing food intake and/or block the adaptive increase in GHS activation expected to result from increased circulating ghrelin with weight loss. On the other hand agonists at this receptor may be useful in stimulating food intake and thus be useful in treating eating disorders, for example anorexia nervosa, or in treating cachexia resulting from cancer or AIDS. The GHS-R is a seven transmembrane G-protein coupled receptor (GPCR). In cells overexpressing the cloned receptor, GHS-R has been shown to couple to calcium signalling, in particular requiring the presence of Gαq11. This class of calcium-coupled GPCR is particularly well suited for screening using the FLIPR assay. This area has recently been reviewed in Expert Opin. Ther. Patent 2002, 12(11) 1599-1618.

An increasing body of evidence suggests that ghrelin may have a role in the control of glucose homeostasis, and that GHS-R1 antagonists might prove useful in the treatment of diabetes. Ghrelin and GHS-R1 are expressed in pancreatic Islets of Langerhans, and ghrelin alters insulin secretion both in vitro and in vivo. Ghrelin and GHSR-/- mice show improved glucose tolerance in glucose tolerance tests, potentially due to improvements in both sensitivity to- and secretion of insulin. Ablation of ghrelin also improves the diabetic phenotype of ob/ob mice. Peptide and small molecule ghrelin antagonists are reported to decrease the glucose excursion in rodent glucose tolerance tests. This area has been recently reviewed in Neuroendocrinology 2007, (Epub ahead of print) (DOI: 10.1159/000109094), 86, 215-228.

Ghrelin has a putative role in the regulation of gastrointestinal function. It induces a specific motor pattern in the fasted state and acts postprandially to accelerate gastric emptying. Applications in post-operative ileus and gastroparesis have been explored. This area has been recently reviewed in Current Opinion in Pharmacology 2006 6(6) 553-558. Diaminopyrimidine derivatives are disclosed as having GHS-R antagonism in US2005/0171131 and US2005/0070712.

2-Benzothiazolylurea derivatives are disclosed as having protein kinase inhibitory activity in WO01/57008 and as having ubiquitin ligase inhibitory activity in WO2005/037845.
2-Chloro-N-[[6-(2-trifluoromethylsulfonyl)benzothiazol-2-yl]carbamoyl]benzamide and related compounds are disclosed as having activity as insecticides in EP198 244.

There remains a need for potent compounds with a low incidence of side-effects that can be used to treat obesity and/ or diabetes.

### Description of the invention

The present invention provides a compound of formula I 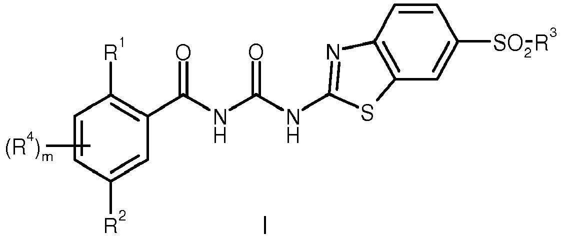 or a pharmaceutically acceptable salt thereof in which
**R¹** represents halo, nitro, a C₁₋₆alkyl group optionally substituted by one, two or three fluoro, a C₂₋₆alkenyl group, a C₃₋₆cycloalkyl group, phenyl, phenoxy, a phenylC₁₋₄alkyl group, a phenoxyC₁₋₄alkyl group, pyrrolyl, a group R^{a}S(O)ₙ(O)ₒ in which R^{a} represents phenyl or a C₁₋₄alkyl optionally substituted by one or more fluoro, n is 0, 1 or 2 and o is 0 except that when n is 2 then o is 0 or 1; wherein any aromatic ring in a substituent R¹ is optionally substituted by one or more of the following: halo, a C₁₋₃alkyl group and a C₁₋₃alkoxy group;
**R²** represents H, halo, a C₁₋₆alkyl group optionally substituted by one, two or three fluoro, a C₂₋₆alkynyl group, a C₂₋₆alkenyl group, a C₁₋₆alkylSO₂O group, a C₃₋₆cycloalkyl group, a C₃₋₆cycloalkyl C₁₋₆alkyl group, a C₃₋₆cycloalkoxy group, nitro, sulfamoyl, a group R^{b}R^{c}N(CH₂)ₚ- in which R^{b} and R^{c} independently represent H, a C₁₋₆alkyl group, a C₁₋₆alkoxycarbonyl group or a C₃₋₆cycloalkyl group or R^{b} and R^{c} together with the nitrogen atom to which they are attached represent a saturated or partially unsaturated 3 to 10 membered heterocyclic ring optionally containing an additional oxygen, nitrogen, S or SO₂ wherein the heterocyclic ring is optionally substituted by one or more of the following: a C₁₋₆alkyl group, hydroxy, a C₁₋₆alkoxycarbonyl group or a group -NR⁵R⁶ in which R⁵ and R⁶ independently represent H, a C₁₋₆alkyl group, a C₁₋₆alkoxycarbonyl group or a C₃₋₆cycloalkyl group or R⁵ and R⁶ together with the nitrogen atom to which they are attached represent a saturated or partially unsaturated 3 to 7 membered heterocyclic ring; and p = 0, 1, 2, 3, 4, 5 or 6, or **R²** represents a C₁₋₆alkoxy group optionally substituted by a group -NR^{b}R^{c} in which R^{b} and R^{c} are as defined above; or **R²** represents a five or six membered heteroaryl ring each of which is optionally substituted by one or more C₁₋₄alkyl groups or by one or more amino groups of formula -NR^{b}R^{c} in which R^{b} and R^{c} are as defined above; or **R²** represents a group (O)ᵤR⁷ in which u is 0 or 1 and R⁷ represents a carbon linked saturated or partially unsaturated 3 to 10 membered heterocyclic group containing one or more N, S or O, wherein the S may be in its oxidised form of SO or SO₂, which is optionally bicyclic including bridged and/or optionally fused to a benzene ring and any ring is optionally substituted by one or more of the following: hydroxy, oxo, carboxy, C₁-₆alkoxycarbonyl, a C₁₋₆alkoxy group optionally substituted by one or more hydroxy and/or C₁₋₆alkoxy, C₁₋₆alkanoyl, benzoyl, amino, C₁₋₃alkylamino, di(C₁₋₃ alkyl)amino or a C₁₋₆alkyl, C₃₋₆cycloalkyl or C₃₋₆cycloalkylC₁₋₄alkyl wherein each of the last three groups is optionally substituted by one or more hydroxy and/or C₁₋₆alkoxy;
**R³** represents a C₁₋₆alkyl group, a hydroxyC₁₋₆alkyl group, a chloroC₁₋₆alkyl group, a C₁₋₄alkoxyC₁₋₄alkyl group, a C₃₋₁₀cycloalkylC₁₋₄alkyl group or a group -(CH₂)_{q}-NR^{f}R^{g} in which q is 0, 1, 2 , 3, 4, 5 or 6 and the alkylene chain is optionally substituted by 1, 2, 3 or 4 C₁₋₄alkyl groups and R^{f} and R^{g} independently represent H, a C₁₋₆alkyl group optionally substituted by one or more fluoro, a C₃₋₁₀cycloalkyl group (optionally substituted by one or more fluoro and/or by or more C₁₋₄alkyl groups), a C₃₋₁₀cycloalkylC₁₋₄alkyl group, a phenylC₁₋₄alkyl group, a group (CH₂)ᵥ-R^{L} wherein v is 0, 1, 2 or 3 and R^{L} is a carbon linked saturated or partially unsaturated 3 to 10 membered heterocyclic group containing one or more N, S or O, wherein the S may be in its oxidised form of SO or SO₂, which is optionally bicyclic including bridged and/or optionally fused to a benzene ring and any ring is optionally substituted by one or more of the following: hydroxy, oxo, carboxy, a C₁₋₆alkoxy group optionally substituted by one or more hydroxy and/or C₁₋₆alkoxy, C₁₋₄alkanoyl, benzoyl, amino, C₁₋₃alkylamino, di(C₁₋₃ alkyl)amino or a C₁₋₆alkyl, C₃₋₁₀cycloalkyl or C₃₋₁₀cycloalkylC₁₋₄alkyl wherein each of the last three groups is optionally substituted by one or more hydroxy and/or C₁₋₆alkoxy;
or R^{f} and R^{g} together with the nitrogen atom to which they are attached represent a saturated or partially unsaturated 3 to 10 membered heterocyclic ring optionally containing an additional oxygen, nitrogen, S or SO₂ wherein the heterocyclic ring is optionally substituted by one or more of the following: fluoro, C₁₋₄alkyl optionally substituted by cyano, C₃₋₆cycloalkyl, hydroxy, C₁₋₄alkanoyl, C₁₋₄alkoxyC₁₋₄alkyl, C₁₋₆alkoxycarbonyl, C₁₋₄alkylsulfonyl or a group -NR^{h}Rⁱ and R^{h} and R¹ independently represent H or a C₁₋₄alkyl group;
**or R³** represents a group -(CH₂)ᵣ-A-(CH₂)ₛ-R^{j} in which r is 2 , 3 or 4 s is 2, 3 or 4, A is N(R^{k})-, O, S, SO or SO₂ and either alkylene chain is optionally substituted by 1, 2, 3 or 4 C₁₋₄alkyl groups, and R^{j} is hydroxyl, a C₁₋₄alkoxy group, carboxy, a group -CO₂C₁₋₄alkyl a group -CONR¹²R¹³ in which R¹² and R¹³ independently represent H or a C₁₋₄alkyl group and R^{k} is H, a C₁₋₄alkyl group or a C₃₋₆cycloalkyl group;
**or R³** represents a group -(CH₂)ᵣ-A-(CH₂)ₛ-NR^{m}Rⁿ in which r is 2 or 3, s is 2 or 3, A is N(R^{k})-, O, S, SO or SO₂ and either alkylene chain is optionally substituted by 1, 2, 3 or 4 C₁₋₄alkyl groups, and R^{m} and Rⁿ independently represent H or a C₁₋₄alkyl group, and R^{k} is H, a C₁₋₄alkyl group or a C₃₋₆cycloalkyl group;
**or R³** represents a group (CH₂)ₜ-R^{o} wherein t is 0, 1, 2 or 3 and R° is a carbon linked saturated or partially unsaturated 3 to 10 membered heterocyclic group containing one or more N, S or O, wherein the S may be in its oxidised form of SO or SO₂, which is optionally bicyclic including bridged and/or optionally fused to a benzene ring and any ring is optionally substituted by one or more of the following: hydroxy, oxo, carboxy, C₁₋₆alkoxycarbonyl, a C₁₋₆alkoxy group optionally substituted by one or more hydroxy or C₁₋₆alkoxy, C₁₋₄alkanoyl, benzoyl, amino, C₁₋₃alkylamino, di(C₁₋₃ alkyl)amino or a C₁₋₆alkyl, C₃₋₆cycloalkyl or C₃₋₆cycloalkylC₁₋₄alkyl wherein each of the last three groups is optionally substituted by one or more hydroxy or C₁₋₆alkoxy; or R^{o} represents an aromatic 5 or 6 membered heterocyclic group containing one or more N, S or O, optionally substituted by one or more of the following: halo, a C₁₋₃alkyl group or a C₁₋₃alkoxy group;
**or R³** represents a C₃₋₁₀cycloalkyl group (optionally substituted by one or more groups of formula -NR^{p} R^{q} in which R^{p} and R^{q} independently represent H, C₁₋₄alkyl, C₁₋₆alkoxycarbonyl, C₁₋₄alkanoyl, C₁₋₄alkylsulfonyl or C₁₋₄alkoxyC₁₋₄alkyl group);
wherein any available aliphatic carbon atom in a group R³ is optionally substituted by hydroxy, a C₁₋₃alkyl or C₁₋₃alkoxy provided that no more than six positions are substituted in this manner;
and any available aromatic carbon atom in a group R³ is optionally substituted by halo, hydroxy, a C₁₋₃alkyl, or C₁₋₃alkoxy provided that no more than four positions are substituted in this manner;
**R⁴** represents halo, a C₁₋₄alkyl , C₁₋₄alkoxy, nitro, or independently a group R^{a}S(O)ₙ(O)ₒ as defined above, a saturated or partially unsaturated 3 to 10 membered heterocyclic group containing one or more N, S or O, wherein the S may be in its oxidised form of SO or SO₂, which is optionally substituted by one or more of the following: hydroxy, oxo, carboxy, a C₁₋₆alkoxy group optionally substituted by one or more hydroxy or C₁₋₆alkoxy, C₁₋₄alkanoyl, benzoyl, amino, C₁₋₃alkylamino, di(C₁₋₃ alkyl)amino or a C₁₋₆alkyl, C₃₋₆cycloalkyl or C₃₋₆cycloalkylC₁₋₄alkyl wherein each of the last three groups is optionally substituted by one or more hydroxy and/or C₁₋₆alkoxy; or R⁴ represents pyrrolyl or pyrazolyl optionally substituted by one or more C₁₋₆alkyl groups;
and m is 0, 1, 2 or 3.

The present invention provides a compound of formula I 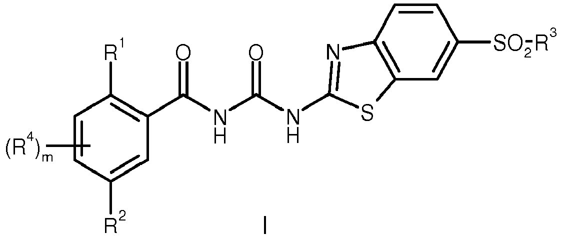 or a pharmaceutically acceptable salt thereof in which
**R¹** represents halo, nitro, a C₁₋₆alkyl group optionally substituted by one two or three fluoro, a C₂₋₆alkenyl group, a C₃₋₆cycloalkyl group, phenyl, phenoxy, a phenylC₁₋₄alkyl group, a phenoxyC₁₋₄alkyl group, pyrrolyl, pyridyl, a group R^{a}S(O)ₙ(O)ₒ in which R^{a} represents phenyl or a C₁₋₄alkyl optionally substituted by one or more fluoro, n is 0, 1 or 2 and o is 0 except that when n is 2 then o is 0 or 1; wherein any aromatic ring in a substituent R¹ is optionally substituted by one or more of the following: halo, a C₁₋₃alkyl group or a C₁₋₃alkoxy group;
**R²** represents H, halo, a C₁₋₆alkyl group optionally substituted by one, two or three fluoro, a C₂₋₆alkynyl group, a C₂₋₆alkenyl group, a C₁₋₆alkylSO₂O group, a C₃₋₆cycloalkyl group, a C₃₋₆cycloalkyl C₁₋₆alkyl group, a C₃₋₆cycloalkoxy group, nitro, a group R^{b}R^{c}N(CH₂)ₚ- in which R^{b} and R^{c} independently represent H, a C₁₋₆alkyl group or a C₃₋₆cycloalkyl group or R^{b} and R^{c} together with the nitrogen atom to which they are attached represent a saturated or partially unsaturated 3 to 10 membered heterocyclic ring optionally containing an additional oxygen, nitrogen, S or SO₂ wherein the heterocyclic ring is optionally substituted by one or more of the following: a C₁₋₆alkyl group or a group -NR^{b}R^{c} in which R^{b} and R^{c} are as defined above; and p = 0, 1, 2, 3, 4, 5 or 6 or **R²** represents a C₁₋₆alkoxy group optionally substituted by a group -NR^{b}R^{c} in which R^{b} and R^{c} are as defined above or R² represents a group SO₂NR^{d}R^{e} in which R^{d} and R^{e} independently represent H or a C₁₋₆alkyl group or **R²** represents a five or six membered heteroaryl ring each of which is optionally substituted by one or more C₁₋₄alkyl groups or by one or more amino groups of formula -NR^{b}R^{c} in which R^{b} and R^{c} are as defined above; or R² represents a carbon linked saturated or partially unsaturated 3 to 10 membered heterocyclic group containing one or more N, S or O, wherein the S may be in its oxidised form of SO or SO₂, which is optionally bicyclic including bridged and/or optionally fused to a benzene ring and any ring is optionally substituted by one or more of the following: hydroxy, oxo, carboxy, a C₁₋₆alkoxy group optionally substituted by one or more hydroxy or C₁₋₆alkoxy, C₁₋₄alkanoyl, benzoyl, amino, C₁₋₃alkylamino, di(C₁₋₃ alkyl)amino or a C₁₋₆alkyl, C₃₋₆cycloalkyl or C₃₋₆cycloalkylC₁₋₄alkyl wherein each of the last three groups is optionally substituted by one or more hydroxy or C₁₋₆alkoxy;
**R³** represents a C₁₋₆alkyl group, a hydroxyC₁₋₆alkyl group, a chloroC₁₋₆alkyl group, a C₁₋₄alkoxyC₁₋₄alkyl group, a C₃₋₁₀cycloalkylC₁₋₄alkyl group or a group-(CH₂)_{q} NR^{f}R^{g} in which q is 0, 1, 2 , 3, 4, 5 or 6 and the alkylene chain is optionally substituted by 1, 2, 3 or 4 C₁₋₄alkyl groups and R^{f} and R^{g} independently represent H, a C₁₋₆alkyl group optionally substituted by one or more fluoro, a C₃₋₁₀cycloalkyl group (optionally substituted by one or more fluoro and/or by or more C₁₋₄alkyl groups), a C₃₋₁₀cycloalkylC₁₋₄alkyl group, a phenylC₁₋₄alkyl group, a group (CH₂)ₜ-R¹ wherein t is 0, 1, 2 or 3 and R¹ is a carbon linked saturated or partially unsaturated 3 to 10 membered heterocyclic group containing one or more N, S or O, wherein the S may be in its oxidised form of SO or SO₂, which is optionally bicyclic including bridged and/or optionally fused to a benzene ring and any ring is optionally substituted by one or more of the following: hydroxy, oxo, carboxy, a C₁₋₆alkoxy group optionally substituted by one or more hydroxy or C₁₋₆alkoxy, C₁₋₄alkanoyl, benzoyl, amino, C₁₋₃alkylamino, di(C₁₋₃ alkyl)amino or a C₁₋₆alkyl, C₃₋₁₀cycloalkyl or C₃₋₁₀cycloalkylC₁₋₄alkyl wherein each of the last three groups is optionally substituted by one or more hydroxy or C₁₋₆alkoxy;
or R^{f} and R^{g} together with the nitrogen atom to which they are attached represent a saturated or partially unsaturated 3 to 10 membered heterocyclic ring optionally containing an additional oxygen, nitrogen, S or SO₂ wherein the heterocyclic ring is optionally substituted by one or more of the following: fluoro, C₁₋₄alkyl optionally substituted by cyano, C₃₋₆cycloalkyl, hydroxy, C₁₋₄alkanoyl, C₁₋₄alkoxyC₁₋₄alkyl, C₁₋₆alkoxycarbonyl, C₁₋₄alkylsulfonyl or a group -NR^{h}Rⁱ and R^{h} and Rⁱ independently represent H or a C₁₋₄alkyl group;
**or R³** represents a group -(CH₂)ᵣ-A-(CH₂)ₛ-Rⁱ in which r is 2, 3 or 4 s is 2, 3 or 4, A is N(R^{k})-, O, S, SO or SO₂ and either alkylene chain is optionally substituted by 1, 2, 3 or 4 C₁₋₄alkyl groups, and R^{j} is hydroxyl, a C₁₋₄alkoxy group, carboxy, a group -CO₂C₁₋₄alkyl a group -CONR¹²R¹³ in which R¹² and R¹³ independently represent H or a C₁₋₄alkyl group and R^{k} is H, a C₁₋₄alkyl group or a C₃₋₆cycloalkyl group;
**or R³** represents a group -(CH₂)ᵣ-A-(CH₂)ₛ-NR^{m}Rⁿ in which r is 2 or 3, s is 2 or 3, A is N(R^{k})-, O, S, SO or SO₂ and either alkylene chain is optionally substituted by 1, 2, 3 or 4 C₁₋₄alkyl groups, and R^{m} and Rⁿ independently represent H or a C₁₋₄alkyl group, and R^{k} is H, a C₁₋₄alkyl group or a C₃₋₆cycloalkyl group;
**or R³** represents a group (CH₂)ₜ-R° wherein t is 0, 1, 2 or 3 and R° is a carbon linked saturated or partially unsaturated 3 to 10 membered heterocyclic group containing one or more N, S or O, wherein the S may be in its oxidised form of SO or SO₂, which is optionally bicyclic including bridged and/or optionally fused to a benzene ring and any ring is optionally substituted by one or more of the following: hydroxy, oxo, carboxy, a C₁₋₆alkoxy group optionally substituted by one or more hydroxy or C₁₋₆alkoxy, C₁₋₄alkanoyl, benzoyl, amino, C₁₋₃alkylamino, di(C₁₋₃ alkyl)amino or a C₁₋₆alkyl, C₃₋₆cycloalkyl or C₃₋₆cycloalkylC₁₋₄alkyl wherein each of the last three groups is optionally substituted by one or more hydroxy or C₁₋₆alkoxy; or R° represents an aromatic 5 or 6 membered heterocyclic group containing one or more N, S or O, optionally substituted by one or more of the following: halo, a C₁₋₃alkyl group or a C₁₋₃alkoxy group;
**or R³** represents a C₃₋₁₀cycloalkyl group ( optionally substituted by one or more of the following groups: NR^{p} R^{q} in which R^{p} and R^{q} independently represent H, C₁₋₄alkyl, C₁₋₆alkoxycarbonyl, C₁₋₄alkanoyl, C₁₋₄alkylsulfonyl or C₁₋₄alkoxyC₁₋₄alkyl group);
wherein any available aliphatic carbon atom in a group R³ is optionally substituted by hydroxy, a C₁₋₃alkyl or C₁₋₃alkoxy provided that no more than six positions are substituted in this manner;
and any available aromatic carbon atom in a group R³ is optionally substituted by halo, hydroxy, a C₁₋₃alkyl, or C₁₋₃alkoxy provided that no more than four positions are substituted in this manner;
**R⁴** represents halo, a C₁₋₄alkyl, C₁₋₄alkoxy, nitro, a group R^{a}S(O)ₙ(O)ₒ as defined above, a saturated or partially unsaturated 3 to 10 membered heterocyclic group containing one or more N, S or O, wherein the S may be in its oxidised form of SO or SO₂, which is optionally substituted by one or more of the following: hydroxy, oxo, carboxy, a C₁₋₆alkoxy group optionally substituted by one or more hydroxy or C₁₋₆alkoxy, C₁₋₄alkanoyl, benzoyl, amino, C₁₋₃alkylamino, di(C₁₋₃ alkyl)amino or a C₁₋6alkyl, C₃₋₆cycloalkyl or C₃₋₆cycloalkylC₁₋₄alkyl wherein each of the last three groups is optionally substituted by one or more hydroxy or C₁₋₆alkoxy; or R⁴ represents pyrrolyl or pyrazolyl optionally substituted by one or more C₁₋₆alkyl groups
and m is 0, 1, 2 or 3.

It will be understood that when m is 2 or 3 then the substituents R⁴ are independently selected and may be the same or different.

In one group of compounds of formula I, m is 0.

In another group of compounds of formula I, **R²** represents a C₂₋₄alkynyl group, a C₁₋₄alkylSO₂O, a C₃₋₆cycloalkyl group, a C₃₋₆cycloalkoxy group, nitro, a group R^{b}R^{c}N(CH₂)ₚ- in which p is 0 or 1 and R^{b} and R^{c} together with the nitrogen atom to which they are attached represent a saturated or partially unsaturated 4 to 6 membered heterocyclic ring optionally containing an additional oxygen or nitrogen wherein the heterocyclic ring is optionally substituted by one or more of the following: a C₁₋₄alkyl group or a group -NR^{d}R^{e} in which R^{d} and R^{e} independently represent H or a C₁₋₄alkyl group; a C₁₋₄alkoxy group (optionally substituted by a group NR^{d}R^{e} in which R^{d} and R^{e} independently represent H or a C₁₋₄alkyl group); or **R²** represents pyrrolyl, pyrazolyl, imidazolyl, triazolyl, thiazolyl, pyrimidinyl or pyridinyl each of which is optionally substituted by one or more C₁₋₄alkyl groups.

In another aspect the present invention provides a compound of formula I as represented by formula IA 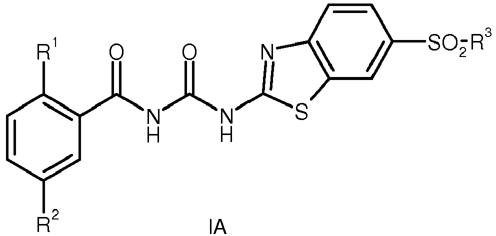 or a pharmaceutically acceptable salt thereof in which
**R¹** represents halo;
**R²** represents a C₂₋₄alkynyl group, a C₁₋₄alkylSO₂O, a C₃₋₆cycloalkyl group, a C₃₋₆cycloalkoxy group, nitro, a group R^{b}R^{c}N(CH₂)ₚ- in which p is 0 or 1 and R^{b} and R^{c} together with the nitrogen atom to which they are attached represent a saturated or partially unsaturated 4 to 6 membered heterocyclic ring optionally containing an additional oxygen or nitrogen wherein the heterocyclic ring is optionally substituted by one or more of the following: a C₁₋₄alkyl group or a group -NR^{d}R^{e} in which R^{d} and R^{e} independently represent H or a C₁₋₄alkyl group; a C₁₋₄alkoxy group (optionally substituted by a group NR^{d}R^{e} in which R^{d} and R^{e} independently represent H or a C₁₋₄alkyl group); or **R²** represents pyrrolyl, pyrazolyl, imidazolyl, triazolyl, thiazolyl, pyrimidinyl or pyridinyl each of which is optionally substituted by one or more C₁₋₄alkyl groups;
**R³** represents a C₁₋₄alkyl group or a group-(CH₂)_{q} NR^{f}R^{g} in which q is 2 or 3 and R^{f} and R^{g} independently represent H, a C₁₋₄alkyl group, a C₃₋₆cycloalkyl group, a C₃₋₆cycloalkyl C₁₋₄alkyl group, or R^{f} and R^{g} together with the nitrogen atom to which they are attached represent a saturated or partially unsaturated 4 to 6 membered heterocyclic ring optionally containing an additional oxygen or nitrogen wherein the heterocyclic ring is optionally substituted by one or more C₁₋₄alkyl groups, **or R³** represents a group -(CH₂)ᵣ-NH-(CH₂)ₛ-R^{j} in which r is 2 or 3, s is 2 or 3, and R^{j} is a C₁₋₄alkoxy group;
**or R³** represents a carbon linked saturated 4 to 6 membered heterocyclic group containing one N optionally substituted by one or more C₁₋₄alkyl groups.

Preferred values of each variable group R¹, R², R³, R⁴, and m are as follows. Such values may be used where appropriate with any of the values, definitions, claims, aspects or embodiments defined hereinbefore or hereinafter. In particular, each may be used as an individual limitation on the broadest definition of formula (I). Further, each of the following values may be used in combination with one or more of the other following values to limit the broadest defintion of formula (I).

In one group of compounds of formula I, m is 0.

In another group of compounds of formula I, **R²** represents a C₂₋₄alkynyl group, a C₁₋₄alkylSO₂O, a C₃₋₆cycloalkyl group, a C₃₋₆cycloalkoxy group, nitro, a group R^{b}R^{c}N(CH₂)ₚ- in which p is 0 or 1 and R^{b} and R^{c} together with the nitrogen atom to which they are attached represent a saturated or partially unsaturated 4 to 6 membered heterocyclic ring optionally containing an additional oxygen or nitrogen wherein the heterocyclic ring is optionally substituted by one or more of the following: a C₁₋₄alkyl group or a group -NR^{d}R^{e} in which R^{d} and R^{e} independently represent H or a C₁₋₄alkyl group; a C₁₋₄alkoxy group (optionally substituted by a group NR^{d}R^{e} in which R^{d} and R^{e} independently represent H or a C₁₋₄alkyl group); or **R²** represents pyrrolyl, pyrazolyl, imidazolyl, triazolyl, thiazolyl, pyrimidinyl or pyridinyl each of which is optionally substituted by one or more C₁₋₄alkyl groups.

In a still further group of compounds of formula I, **R³** represents a C₁₋₄alkyl group or a group-(CH₂)_{q} NR^{f}R^{g} in which q is 2 or 3 and R^{f} and R^{g} independently represent H, a C₁₋₄alkyl group, a C₃₋₆cycloalkyl group, a C₃₋₆cycloalkyl C₁₋₄alkyl group, or R^{f} and R^{g} together with the nitrogen atom to which they are attached represent a saturated or partially unsaturated 4 to 6 membered heterocyclic ring optionally containing an additional oxygen or nitrogen wherein the heterocyclic ring is optionally substituted by one or more C₁₋₄alkyl groups,
**or R³** represents a group -(CH₂)ᵣ-NH-(CH₂)ₛ-R^{j} in which r is 2 or 3, s is 2 or 3, and Rⁱ is a C₁₋₄alkoxy group;
**or R³** represents a carbon linked saturated 4 to 6 membered heterocyclic group containing one N optionally substituted by one or more C₁₋₄alkyl groups.

In one group of compounds of formula I or of formula IA, R¹ represents bromo, chloro and iodo.

In a second group of compounds of formula I or of formula IA, R¹ represents chloro.

In a third group of compounds of formula I or of formula IA, R² represents cyclopropyl, cyclopentyl, ethoxy, ethynyl, cyclopentyloxy, nitro, pyrrol-1-yl, pyridin-2-yl, pyrimidin-2-yl, pyrazol-1-yl, imidazol-1-yl, thiazol-5-yl, [1,2,3]-triazol-1-yl, [1,2,4]-triazol-1-yl, 1-pyrrolidinyl, 2,5-dihydro-pyrrol-1-yl, morpholin-4-yl, pyrrolidin-1-ylmethyl, 2-(dimethylamino)ethoxy, methylsulfonyloxy, 3-methylpyrazol-1-yl, 5-methylpyrazol-1-yl, 4-methylpiperazin-1-yl or 3-dimethylaminopyrrolidin-1-yl.

In a fourth group of compounds of formula I or of formula IA, R³ represents 2-morpholin-4-ylethyl, 2-(2-methoxyethylamino)ethyl, 2-(dimethylamino)ethyl, 2-methylaminoethyl, 2-(3-hydroxypyrrolidin-1-yl)ethyl, 2-(pyrrolidin-1-yl)ethyl, 2-(diethylamino)ethyl, 2-(N-(2-methoxyethyl) -N'-methyl)amino)ethyl, 2-[(N-2-hydroxyethyl-N'-methyl)amino] ethyl, 2-(butan-2-ylamino)ethyl, 2-(azetidin-1-yl)ethyl, 2-(2-hydroxyethylamino)ethyl, 2-(N-ethyl-N'-(2-methoxyethyl)amino)ethyl, 2-[(3S)-3-fluoropyrrolidin-1-yl]ethyl, 2-(cyclopentylamino)ethyl, 2-(2,5-dimethylpyrrolidin-1-yl)ethyl, 2-(2,6-dimethylmorpholin-4-yl)ethyl, 2-(1-phenylethylamino)ethyl, 2-(3,3-difluoropyrrolidin-1-yl)ethyl, 2-(oxolan-2-ylmethylamino)ethyl, 2-(4-methylpiperazin-1-yl)ethyl, 2-(3,5-dimethylpiperazin-1-yl)ethyl, 2-(cyclobutylamino)ethyl, 2-(N-methyl-N'-(oxolan-2-ylmethyl)amino)ethyl, 2-(2-methyl-1-piperidyl)ethyl, 2-(2-methylpropylamino)ethyl, 2-(4-ethylpiperazin-1-yl)ethyl, 2-(1,4-oxazepan-4-yl)ethyl, 2-(2-fluoroethylamino)ethyl, 2-[(3R)-3-fluoropyrrolidin-1-yl]ethyl, 2-(1-piperidyl)ethyl, 2-(cyclopropylmethylamino)ethyl, 2-(norbornan-2-ylamino)ethyl, 2-[2-(methoxymethyl)pyrrolidin-1-yl]ethyl, [2-[(1S,4S)-3-oxa-6-azabicyclo[2.2.1]hept-6-yl]ethyl, 2-(2-propan-2-yloxyethylamino)ethyl, 2-[(1-methylcyclopropyl)amino]ethyl, 2-methoxyethyl, 2-hydroxyethyl, 2-(2-methoxyethoxy)ethyl, 2-(2-dimethylaminoethoxy)ethyl, pyrrolidin-3-yl, methyl, pyrrolidin-3-yl, (3S)-pyrrolidin-3-yl, (3R)-pyrrolidin-3-yl, 2-(isopropylamino)ethyl, 3-(isopropylamino)propyl, 3-(diethylamino)propyl, 3-(cyclopropylmethylamino)propyl, 3-(piperazin-1-yl)propyl, 2-(azetidin-1-yl)ethyl, 3-(azetidin-1-yl)propyl, 2-(propan-2-ylamino)ethyl, 3-(propan-2-ylamino)propyl, 2-piperazin-1-ylethyl, 3-(4-methylpiperazin-1-yl)propyl, 2-(2-methoxyethylamino)ethyl, 3-(2-methoxyethylamino)propyl or 1-methyl-4 piperidinyl, 2-(carbamoylmethoxy)ethyl, 2-(2-hydroxyethoxy)ethyl, 2-(2-carboxyethoxy)ethyl, ethenyl, 3-piperidyl, 1-(propan-2-yl)-3-piperidyl, 1-ethyl-3-piperidyl, 1-(cyclopropylmethyl)-3-piperidyl, 1-(cyclopropylmethyl)pyrrolidin-3-yl, 3-morpholin-4-ylpropyl, 3-chloropropyl, 3-pyrrolidin-1-ylpropyl, 3-(1,1-dioxo-1,4-thiazinan-4-yl)propyl, 3-(cyclopropylmethylamino)propyl, 3-(1-piperidyl)propyl, 3-diethylaminopropyl, 3-(3,3-difluoropyrrolidin-1-yl)propyl, [(2R)-1-(cyclopropylmethyl)pyrrolidin-2-yl]methyl, 3-(3-fluoropyrrolidin-1-yl)propyl, 3-(4,4-difluoro-1-piperidyl)propyl, 3-(2-hydroxyethylamino)propyl, 3-(3-hydroxypyrrolidin-1-yl)propyl, 1-propan-2-ylpyrrolidin-3-yl, 1-ethylpyrrolidin-3-yl, (2R)-1-ethylpyrrolidin-2-yl]methyl, [(2R)-pyrrolidin-2-yl]methyl, 1-methyl-3-piperidyl, 1-methylpyrrolidin-3-yl, [(2R)-1-propan-2-ylpyrrolidin-2-yl]methyl, [(2R)-1-methylpyrrolidin-2-yl]methyl, 1-ethyl-4-piperidyl, 1-(propan-2-yl)-4-piperidyl, 1-(cyclopropylmethyl)azetidin-3-yl, 4-piperidyl, pyrid-2-ylmethyl, 1-(cyclopropylmethyl)-4-piperidyl, azetidin-3-yl, 1-ethylazetidin-3-yl, 1-propan-2-ylazetidin-3-yl, pyrid-3-ylmethyl, 5-methyl-1,2-oxazol-3-yl)methyl, 1H-imidazol-2-ylmethyl, 2-(pyrid-2-yl)ethyl, 2-methyl-1,3-thiazol-4-yl)methyl, 3-methoxypropyl, 3-imidazol-1-ylpropyl, 3-[(3S,5R)-3,5-dimethylpiperazin-1-yl]propyl, 3-(4-ethylpiperazin-1-yl)propyl, 3-(4-acetylpiperazin-1-yl)propyl, 3-(4-propan-2-ylpiperazin-1-yl)propyl, 3-[4-(2-methoxyethyl)piperazin-1-yl]propyl, 3-(4-dimethylamino-1-piperidyl)propyl, 3-dimethylaminopropyl, 3-(2-methoxyethylamino)propyl, 3-(4-dimethylamino-1-piperidyl)propyl, 3-dimethylaminopropyl, 3-(2-methoxyethylamino)propyl, 3-(4-methyl-1,4-diazepan-1-yl)propyl, 3-(4-*tert*-butoxycarbonylpiperazin1-yl)propyl, 3-[4-(2-cyanoethyl)piperazin-1-yl]propyl, 3-(4-methylsulfonylpiperazin-1-yl)propyl, 3-(*tert-*butyloxycarbonylamino)cyclobut-1-yl, 3-aminocyclobutyl, 3-methylaminocyclobutyl, 3-dimethylaminocyclobutyl, 1-(-*tert*-butoxycarbonyl)piperidin-4-ylmethyl , 2-(1-(*-tert-*butoxycarbonyl)piperidin-1-yl)ethyl, 4-piperidylmethyl, 2-(4-piperidyl)ethyl, 3-(2-methoxyethylamino)cyclobutyl or 3-acetamidocyclobutyl.

In a fifth group of compounds of formula I or of formula IA, R³ represents 2-morpholin-4-ylethyl, 2-(2-methoxyethylamino)ethyl, 2-(dimethylamino)ethyl, 2-methylaminoethyl, 2-(3-hydroxypyrrolidin-1-yl)ethyl, 2-(pyrrolidin-1-yl)ethyl, 2-(diethylamino)ethyl, 2-(N-(2-methoxyethyl) -N'-methyl)amino)ethyl, 2-[(N-2-hydroxyethyl-N'- methyl)amino] ethyl, 2-(butan-2-ylamino)ethyl, 2-(azetidin-1-yl)ethyl, 2-(2-hydroxyethylamino)ethyl, 2-(N-ethyl-N'- (2-methoxyethyl)amino)ethyl, 2-[(3S)-3-fluoropyrrolidin-1-yl]ethyl, 2-(cyclopentylamino)ethyl, 2-(2,5-dimethylpyrrolidin-1-yl)ethyl, 2-(2,6-dimethylmorpholin-4-yl)ethyl, 2-(1-phenylethylamino)ethyl, 2-(3,3-difluoropyrrolidin-1-yl)ethyl, 2-(oxolan-2-ylmethylamino)ethyl, 2-(4-methylpiperazin-1-yl)ethyl, 2-(3,5-dimethylpiperazin-1-yl)ethyl, 2-(cyclobutylamino)ethyl, 2-(N-methyl-N'-(oxolan-2-ylmethyl)amino)ethyl, 2-(2-methyl-1-piperidyl)ethyl, 2-(2-methylpropylamino)ethyl, 2-(4-ethylpiperazin-1-yl)ethyl, 2-(1,4-oxazepan-4-yl)ethyl, 2-(2-fluoroethylamino)ethyl, 2-[(3R)-3-fluoropyrrolidin-1-yl]ethyl, 2-(1-piperidyl)ethyl, 2-(cyclopropylmethylamino)ethyl, 2-(norbornan-2-ylamino)ethyl, 2-[2-(methoxymethyl)pyrrolidin-1-yl]ethyl, [2-[(1S,4S)-3-oxa-6-azabicyclo[2.2.1]hept-6-yl]ethyl, 2-(2-propan-2-yloxyethylamino)ethyl, 2-[(1-methylcyclopropyl)amino]ethyl, 2-methoxyethyl, 2-hydroxyethyl, 2-(2-methoxyethoxy)ethyl, 2-(2-dimethylaminoethoxy)ethyl, pyrrolidin-3-yl.

In a sixth group of compounds of formula I or of formula IA, R³ represents methyl, pyrrolidin-3-yl, (3S)-pyrrolidin-3-yl, (3R)-pyrrolidin-3-yl, 2-(isopropylamino)ethyl, 3-(isopropylamino)propyl, 3-(diethylamino)propyl, 3-(cyclopropylmethylamino)propyl, 3-(piperazin-1-yl)propyl, 2-(azetidin-1-yl)ethyl, 3-(azetidin-1-yl)propyl, 2-(propan-2-ylamino)ethyl, 3-(propan-2-ylamino)propyl, 2-piperazin-1-ylethyl, 3-(4-methylpiperazin-1-yl)propyl, 2-(2-methoxyethylamino)ethyl, 3-(2-methoxyethylamino)propyl or 1-methyl-4 piperidinyl.

In a seventh group of compounds of formula I or of formula IA, R³ represents 2-(carbamoylmethoxy)ethyl, 2-(2-hydroxyethoxy)ethyl, 2-(2-carboxyethoxy)ethyl, ethenyl, 3-piperidyl, 1-(propan-2-yl)-3-piperidyl, 1-ethyl-3-piperidyl, 1-(cyclopropylmethyl)-3-piperidyl, 1-(cyclopropylmethyl)pyrrolidin-3-yl, 3-morpholin-4-ylpropyl, 3-chloropropyl, 3-pyrrolidin-1-ylpropyl, 3-(1,1-dioxo-1,4-thiazinan-4-yl)propyl, 3-(cyclopropylmethylamino)propyl, 3-(1-piperidyl)propyl, 3-diethylaminopropyl, 3-(3,3-difluoropyrrolidin-1-yl)propyl, [(2R)-1-(cyclopropylmethyl)pyrrolidin-2-yl]methyl, 3-(3-fluoropyrrolidin-1-yl)propyl, 3-(4,4-difluoro-1-piperidyl)propyl, 3-(2-hydroxyethylamino)propyl, 3-(3-hydroxypyrrolidin-1-yl)propyl, 1-propan-2-ylpyrrolidin-3-yl, 1-ethylpyrrolidin-3-yl, (2R)-1-ethylpyrrolidin-2-yl]methyl, [(2R)-pyrrolidin-2-yl]methyl, 1-methyl-3-piperidyl, 1-methylpyrrolidin-3-yl, [(2R)-1-propan-2-ylpyrrolidin-2-yl]methyl, [(2R)-1-methylpyrrolidin-2-yl]methyl, 1-ethyl-4-piperidyl, 1-(propan-2-yl)-4-piperidyl, 1-(cyclopropylmethyl)azetidin-3-yl, 4-piperidyl, pyrid-2-ylmethyl, 1-(cyclopropylmethyl)-4-piperidyl, azetidin-3-yl,1-ethylazetidin-3-yl, 1-propan-2-ylazetidin-3-yl, pyrid-3-ylmethyl, 5-methyl-1,2-oxazol-3-yl)methyl, 1H-imidazol-2-ylmethyl, 2-(pyrid-2-yl)ethyl, 2-methyl-1,3-thiazol-4-yl)methyl, 3-methoxypropyl, 3-imidazol-1-ylpropyl, 3-[(3S,SR)-3,5-dimethylpiperazin-1-yl]propyl, 3-(4-ethylpiperazin-1-yl)propyl, 3-(4-acetylpiperazin-1-yl)propyl, 3-(4-propan-2-ylpiperazin-1-yl)propyl, 3-[4-(2-methoxyethyl)piperazin-1-yl]propyl, 3-(4-dimethylamino-1-piperidyl)propyl, 3-dimethylaminopropyl, 3-(2-methoxyethylamino)propyl, 3-(4-dimethylamino-1-piperidyl)propyl, 3-dimethylaminopropyl, 3-(2-methoxyethylamino)propyl, 3-(4-methyl-1,4-diazepan-1-yl)propyl, 3-(4-*tert*-butoxycarbonylpiperazin1-yl)propyl, 3-[4-(2-cyanoethyl)piperazin-1-yl]propyl, 3-(4-methylsulfonylpiperazin-1-yl)propyl, 3-(*tert-*butyloxycarbonylamino)cyclobut-1-yl, 3-aminocyclobutyl, 3-methylaminocyclobutyl, 3-dimethylaminocyclobutyl, 1-(-*tert*-butoxycarbonyl)piperidin-4-ylmethyl, 2-(1-(-*tert-*butoxycarbonyl)piperidin-1-yl)ethyl, 4-piperidylmethyl, 2-(4-piperidyl)ethyl, 3-(2-methoxyethylamino)cyclobutyl or 3-acetamidocyclobutyl.

In a eighth group of compounds of formula I, m is 0,1 or 2 and R⁴ represents chloro, fluoro, methyl, methoxy, methylsulfonyl, morpholino, pyrazol-1-yl, piperidino, 2,5-dimethylpyrrol-1-yl, nitro or 3-methylpyrazoly-yl.

In a ninth group of compounds of formula I or formula IA, R² represents H, ethyl, ethoxy, 1-acetylpyrrolidin-3-yloxy, (1-isopropylpiperidin-3-yl)methoxy, 3-(dimethylamino)pyrrolidin-1-yl, (R)-3-(dimethylamino)pyrrolidin-1-yl, (S)-3-(dimethylamino)pyrrolidin-1-yl, 4-tert-butoxycarbonylpiperazin-1-yl, 4-(dimethylamino)piperidin-1-yl, 3,5-dimethyl-1H-pyrazol-1-yl, 3-(diethylamino)pyrrolidin-1-yl, 1H-pyrazol-1-yl, 1-tert-butoxycarbonylpiperidin-4-yloxy, 1-methylpiperidin-4-yloxy, morpholino, 3-(dimethylamino)pyridin-2-yl, 1-tert-butoxycarbonylpyrrolidin-3-yloxy, pyrrolidin-3-yloxy, 1-methylpyrrolidin-3-yloxy, 6-(dimethylamino)pyridin-2-yl, 1-tert-butoxycarbonylazetidin-3-yloxy, azetidin-3-yloxy, 1-methylazetidin-3-yloxy, 5-methyl-1H-pyrazol-1-yl, (dimethylamino)methyl or iodo.

In a tenth group of compounds of formula I or formula IA, R² represents ethyl, ethoxy, 1-acetylpyrrolidin-3-yloxy, (1-isopropylpiperidin-3-yl)methoxy, 3-(dimethylamino)pyrrolidin-1-yl, (R)-3-(dimethylamino)pyrrolidin-1-yl, (S)-3-(dimethylamino)pyrrolidin-1-yl, 4-tert-butoxycarbonylpiperazin-1-yl, 4-(dimethylamino)piperidin-1-yl, 3,5-dimethyl-1H-pyrazol-1-yl, 3-(diethylamino)pyrrolidin-1-yl, 1H-pyrazol-1-yl, 1-tert-butoxycarbonylpiperidin-4-yloxy, 1-methylpiperidin-4-yloxy, morpholino, 3-(dimethylamino)pyridin-2-yl, 1-tert-butoxycarbonylpyrrolidin-3-yloxy, pyrrolidin-3-yloxy, 1-methylpyrrolidin-3-yloxy, 6-(dimethylamino)pyridin-2-yl, 1-tert-butoxycarbonylazetidin-3-yloxy, azetidin-3-yloxy, 1-methylazetidin-3-yloxy, 5-methyl-1H-pyrazol-1-yl, (dimethylamino)methyl or iodo.

In an eleventh group of compounds of formula I or formula IA, R³ represents amino, methylamino, dimethylamino, isopropylamino, 2-hydroxyethylamino, 1-(isopropylamino)-2-methylpropan-2-yl, 3-(4-methylpiperazin-1-yl)propyl, 3-(4-methylpiperazin-1-yl)propyl, 3-(4-methyl-1,4-diazepan-1-yl)propyl, 2-methyl-1-(pyrrolidin-1-yl)propan-2-yl, 1-methylpiperidin-4-yl, 1-tert-butoxycarbonylpiperidin-4-yl or piperidin-4-yl.

In a twelfth group of compounds of formula I, m is 1 and R⁴ represents 4-chloro, 4-methoxy, 4-ethoxy, 4-isopropoxy, 4-methyl, 3-(1H-pyrazol-1-yl) or 4-fluoro.

In a thirteenth group of compounds of formula I, m is 0 or 1 and R⁴ represents 4-chloro, 4-methoxy, 4-ethoxy, 4-isopropoxy, 4-methyl, 3-(1H-pyrazol-1-yl) or 4-fluoro.

In another aspect the present invention provides a compound of formula I as represented by formula IB 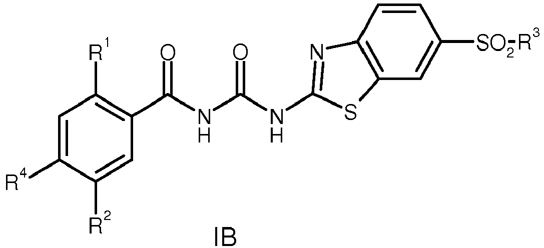 or a pharmaceutically acceptable salt thereof in which
**R¹** represents halo;
**R²** represents a C₁₋₄alkyl group, a C₁₋₄alkoxy group a C₂₋₄alkynyl group, morpholino, pyrazolyl optionally substituted by a C₁₋₄alkyl group or pyrrolidino optionally substituted by a group N⁵R⁶ in which R⁵ and R⁶ independently represent H or a C₁₋₃alkyl group; **R³** represents a C₁₋₄alkyl group or a group-(CH₂)_{q} NR^{f}R^{g} in which q is 2 or 3 and the alkylene chain is optionally substituted by one or two C₁₋₂alkyl groups and R^{f} and R^{g} together with the nitrogen atom to which they are attached represent pyrrolidino or piperazino optionally substituted by a C₁₋₄alkyl group or **R³** represents piperdinyl optionally substituted by one or two C₁₋₂alkyl groups; and
**R⁴** represents H , fluoro or a C₁₋₃alkoxy group.

Particularly in compounds of formula IB R¹ is chloro. Particularly in compounds of formula IB R⁴ is H, fluoro or methoxy.

"Pharmaceutically acceptable salt", where such salts are possible, includes both pharmaceutically acceptable acid and base addition salts. A suitable pharmaceutically acceptable salt of a compound of formula I is, for example, an acid-addition salt of a compound of formula I which is sufficiently basic, for example an acid-addition salt with an inorganic or organic acid such as hydrochloric, hydrobromic, sulphuric, trifluoroacetic, citric or maleic acid; or, for example a base-addition salt of a compound of formula I which is sufficiently acidic, for example an alkali or alkaline earth metal salt such as a sodium, calcium or magnesium salt, or an ammonium salt, or a salt with an organic base such as methylamine, dimethylamine, trimethylamine, piperidine, morpholine or tris-(2-hydroxyethyl)amine. Such salts may be prepared by methods known to those skilled in the art.

Throughout the specification and the appended claims, a given chemical formula or name shall encompass all stereoisomers including optical isomers and racemates thereof as well as mixtures in different proportions of the separate enantiomers, where such stereoisomers and enantiomers exist, as well as pharmaceutically acceptable salts thereof and solvates thereof such as for instance hydrates including solvates of the free compounds or solvates of a salt of the compound. Enantiomers may be isolated by separation of a racemate for example by resolution or chiral HPLC. Diastereomers may be isolated by separation of diastereomeric mixtures for instance by fractional crystallisation, HPLC or flash chromatography. Alternatively the stereoisomers may be made by chiral synthesis from chiral starting materials under conditions that will not cause racemisation or epimerisation, or by derivatisation, with a chiral reagent. All stereoisomers are included within the scope of the invention. All tautomers, where possible, are included within the scope of the invention. The present invention also encompasses compounds containing one or more isotopes for example ¹⁴C, ¹¹C or ¹⁹F and their use as isotopically labelled compounds for pharmacological and metabolic studies.

Compounds of Formula (I) may form salts which are within the ambit of the invention. Pharmaceutically-acceptable salts are preferred although other salts may be useful in, for example, isolating or purifying compounds. In another aspect, the invention relates to compounds of formula (I) as hereinabove defined or to a pharmaceutically-acceptable salt.

In another aspect, the invention relates to compounds of formula (I) as hereinabove defined or to a pro-drug thereof. Suitable examples of pro-drugs of compounds of formula (I) are in-vivo hydrolysable esters of compounds of formula (I). Therefore in another aspect, the invention relates to compounds of formula (I) as hereinabove defined or to an in-vivo hydrolysable ester thereof.

In this specification the generic term "alkyl" includes both straight-chain and branched-chain alkyl groups. However references to individual alkyl groups such as "propyl" are specific for the straight chain version only and references to individual branched-chain alkyl groups such as *t*-butyl are specific for the branched chain version only. An analogous convention applies to other generic terms.

Examples of a C₁₋₆alkyl group include methyl, ethyl, propyl, isopropyl, butyl, tert-butyl, pentyl and hexyl; examples of a C₁₋₆alkoxy group include methoxy, ethoxy, propoxy, isopropoxy and *tert*-butoxy; examples of a C₃₋₁₀cycloalkyl group include cyclopropyl, cyclobutyl, cyclopentyl, cyclohexyl, norbornanyl and adamantyl, and also include bicyclic, bridged or spiro groups examples of halo include fluoro, chloro, bromo and iodo; examples of hydroxy C₁₋₆alkyl include hydroxymethyl, 1-hydroxyethyl, 2-hydroxyethyl, 2-hydroxypropyl, 3-hydroxypropyl, 1-hydroxyisopropyl and 4-hydroxybutyl; examples of C₁₋₄alkoxyC₁₋₄alkyl include methoxymethyl, ethoxymethyl, tert-butoxymethyl, 2-methoxyethyl, 2-ethoxyethyl, methoxypropyl, 2-methoxypropyl and methoxybutyl; examples of C₁₋₆alkylamino include methylamino, ethylamino, propylamino, isopropylamino, butylamino and tert-butylamino; examples of di (C₁₋₆alkyl)amino include dimethylamino, methyl(ethyl)amino, diethylamino, dipropylamino, di-isopropylamino and dibutylamino.

The term "a carbon linked saturated or partially saturated 4 to 10 membered heterocyclic group containing containing one or more N, S or O, wherein the S may be in its oxidised form of SO or SO₂, which is optionally fused to a benzene ring or a heteroaryl ring "includes oxetanyl, tetrahydrofuranyl, tetrahydropyranyl, 2,3-dihydro-1,3-thiazolyl, 1,3-thiazolidinyl, 1,3-oxazolidinyl, oxepanyl, oxolanyl, azetidinyl, pyrrolinyl, pyrrolidinyl, morpholinyl, thiamorpholinyl (perhydro-1,4-thiazinyl), (8-oxa-3-azabicyclo[3.2.1]octyl), (7-oxa-3-azabicyclo[3.1.1]heptyl), 3-oxa-6-azabicyclo[2.2.1]hept-6-yl, perhydroazepinyl, perhydrooxazepinyl, tetrahydro-1,4-thiazinyl, 1-oxotetrahydrothienyl, 1,1-dioxotetrahydro-1,4-thiazinyl, piperidinyl, homopiperidinyl, piperazinyl, homopiperazinyl, dihydropyridinyl, tetrahydropyridinyl, dihydropyrimidinyl,tetrahydropyrimidinyl, or tetrahydroquinolyl each of which may be optionally substituted as previously described.

When two substituents on an amine together with the nitrogen atom to which they are attached represent a saturated or partially unsaturated 3 to 10 membered heterocyclic ring optionally containing an additional oxygen, sulphur, SO, SO₂ or nitrogen (and/or optionally fused to a benzene ring) then this group also includes bicyclic, bridged or spiro groups for example azetidino, pyrrolidino, morpholino, piperidino, imidazolidinyl, imidazolinyl, piperazino, thiamorpholino (perhydro-1,4-thiazinyl), homopiperazino, perhydroazepino, perhydrooxazepino, (2,3-dihydro-1,3-thiazolyl, 1,3-thiazolidinyl, 1,3-oxazolidinyl, oxepanyl , oxazepanyl, dihydropyrimidinyl, tetrahydropyrimidinyl, homopiperidinyl and 3-oxa-6-azabicyclo[2.2.1]heptyl each of which is optionally substituted as previously described.

The term a five or six membered heteroaryl ring includes aromatic 5- or 6-membered monocyclic ring with up to five ring heteroatoms selected from oxygen, nitrogen and sulfur, which may, unless otherwise specified be carbon or nitrogen linked. The term "five or six membered heteroaryl ring" includes pyrrolyl, thienyl, furyl, pyrazolyl, imidazolyl, oxazolyl, isoxazolyl, thiazolyl, isothiazolyl, thiadiazolyl, 1,3,4-oxadiazolyl, 1,2,4-oxadiazolyl, triazolyl, furazanyl, tetrazolyl, pyridyl, pyrimidyl, pyrazinyl, pyridazinyl and 1,3,5-triazinyl.

In one embodiment of the invention are provided compounds of formula (I), in an alternative embodiment are provided pharmaceutically-acceptable salts of compounds of formula (I), in a further alternative embodiment are provided in-vivo hydrolysable esters of compounds of formula (I), and in a further alternative embodiment are provided pharmaceutically-acceptable salts of in-vivo hydrolysable esters of compounds of formula (I).

Specific compounds of the invention include one or more of the following, that is any number of the compounds below from 1 to 270 inclusive in any permutation:
2-chloro-N-[[6-(2-morpholin-4-ylethylsulfonyl)benzothiazol-2-yl]carbamoyl]benzamide;
2-chloro-N-[[6-[2-(2-methoxyethylamino)ethylsulfonyl]benzothiazol-2-yl]carbamoyl]-benzamide;
2-chloro-N-[[6-(2-dimethylaminoethylsulfonyl)benzothiazol-2-yl]carbamoyl]benzamide;
2-chloro-N-[[6-(2-methylaminoethylsulfonyl)benzothiazol-2-yl]carbamoyl]benzamide;
2-chloro-N-[[6-[2-(3-hydroxypyrrolidin-1-yl)ethylsulfonyl]benzothiazol-2-yl]carbamoyl]benzamide;
2-chloro-N-[[6-(2-pyrrolidin-1-ylethylsulfonyl)benzothiazol-2-yl]carbamoyl]benzamide;
2-chloro-N-[[6-(2-diethylaminoethylsulfonyl)benzothiazol-2-yl]carbamoyl]benzamide;
2-chloro-N-[[6-(2-piperazin-1-ylethylsulfonyl)benzothiazol-2-yl]carbamoyl]benzamide;
2-chloro-N-[[6-[2-(2-methoxyethyl-methyl-amino)ethylsulfonyl]benzothiazol-2-yl]carbamoyl]benzamide;
2-chloro-N-[[6-[2-(propan-2-ylamino)ethylsulfonyl]benzothiazol-2-yl]carbamoyl]benzamide;
2-chloro-N-[[6-[2-(2-hydroxyethyl-methyl-amino)ethylsulfonyl]benzothiazol-2-yl]carbamoyl]benzamide;
N-[[6-[2-(butan-2-ylamino)ethylsulfonyl]benzothiazol-2-yl]carbamoyl]-2-chlorobenzamide;
N-[[6-[2-(azetidin-1-yl)ethylsulfonyl]benzothiazol-2-yl]carbamoyl]-2-chlorobenzamide;
2-chloro-N-[[6-[2-(2-hydroxyethylamino)ethylsulfonyl]benzothiazol-2-yl]carbamoyl]-benzamide;
2-chloro-N-[[6-[2-(ethyl-(2-methoxyethyl)amino)ethylsulfonyl]benzothiazol-2-yl]carbamoyl]benzamide;
2-chloro-N-[[6-[2-[(3S)-3-fluoropyrrolidin-1-yl]ethylsulfonyl]benzothiazol-2-yl]carbamoyl]benzamide;
2-chloro-N-[[6-[2-(cyclopentylamino)ethylsulfonyl]benzothiazol-2-yl]carbamoyl]benzamide;
2-chloro-N-[[6-[2-(2,5-dimethylpyrrolidin-1-yl)ethylsulfonyl]benzothiazol-2-yl]carbamoyl]benzamide;
2-chloro-N-[[6-[2-(2,6-dimethylmorpholin-4-yl)ethylsulfonyl]benzothiazol-2-yl]carbamoyl]benzamide;
2-chloro-N-[[6-[2-(1-phenylethylamino)ethylsulfonyl]benzothiazol-2-yl]carbamoyl]benzamide;
2-chloro-N-[[6-[2-(3,3-difluoropyrrolidin-1-yl)ethylsulfonyl]benzothiazol-2-yl]carbamoyl]benzamide;
2-chloro-N-[[6-[2-(oxolan-2-ylmethylamino)ethylsulfonyl]benzothiazol-2-yl]carbamoyl]benzamide;
2-chloro-N-[[6-[2-(4-methylpiperazin-1-yl)ethylsulfonyl]benzothiazol-2-yl]carbamoyl]benzamide;
2-chloro-N-[[6-[2-(3,5-dimethylpiperazin- I -yl)ethylsulfonyl]benzothiazol-2-yl]carbamoyl]benzamide;
2-chloro-N-[[6-[2-(cyclobutylamino)ethylsulfonyl]benzothiazol-2-yl]carbamoyl]benzamide;
2-chloro-N-[[6-[2-(methyl-(oxolan-2-ylmethyl)amino)ethylsulfonyl]benzothiazol-2-yl]carbamoyl]benzamide;
2-chloro-N-[[6-[2-(2-methyl-1-piperidyl)ethylsulfonyl]benzothiazol-2-yl]carbamoyl]benzamide;
2-chloro-N-[[6-[2-(2-methylpropylamino)ethylsulfonyl]benzothiazol-2-yl]carbamoyl]benzamide;
2-chloro-N-[[6-[2-(4-ethylpiperazin-1-yl)ethylsulfonyl]benzothiazol-2-yl]carbamoyl]benzamide;
2-chloro-N-[[6-[2-(1,4-oxazepan-4-yl)ethylsulfonyl]benzothiazol-2-yl]carbamoyl]benzamide;
2-chloro-N-[[6-[2-(2-fluoroethylamino)ethylsulfonyl]benzothiazol-2-yl]carbamoyl]benzamide;
2-chloro-N-[[6-[2-[(3R)-3-fluoropyrrolidin-1-yl]ethylsulfonyl]benzothiazol-2-yl]carbamoyl]benzamide;
2-chloro-N-[[6-[2-(1-piperidyl)ethylsulfonyl]benzothiazol-2-yl]carbamoyl]benzamide;
2-chloro-N-[[6-[2-(cyclopropylmethylamino)ethylsulfonyl]benzothiazol-2-yl]carbamoyl]benzamide;
2-chloro-N-[[6-[2-(norbornan-2-ylamino)ethylsulfonyl]benzothiazol-2-yl]carbamoyl]benzamide;
2-chloro-N-[[6-[2-[2-(methoxymethyl)pyrrolidin-1-yl]ethylsulfonyl]benzothiazol-2-yl]carbamoyl]benzamide;
2-chloro-N-[[6-[2-[(1S,4S)-3-oxa-6-azabicyclo[2.2.1]hept-6-yl]ethylsulfonyl]benzothiazol-2-yl] carbamoyl]benzamide;
2-chloro-N-[[6-[2-(2-propan-2-yloxyethylamino)ethylsulfonyl]benzothiazol-2-yl]carbamoyl]benzamide;
2-chloro-N-[[6-[2-[(1-methylcyclopropyl)amino]ethylsulfonyl]benzothiazol-2-yl]carbamoyl]benzamide;
2-chloro-N-[[6-(2-methoxyethylsulfonyl)benzothiazol-2-yl]carbamoyl]benzamide;
2-chloro-N-[[6-(2-hydroxyethylsulfonyl)benzothiazol-2-yl]carbamoyl]benzamide;
2-chloro-N-[[6-[2-(2-methoxyethoxy)ethylsulfonyl]benzothiazol-2-yl]carbamoyl]-benzamide;
2-chloro-N-[[6-[2-(2-dimethylaminoethoxy)ethylsulfonyl]benzothiazol-2-yl]carbamoyl]benzamide;
2-chloro-N-[(6-pyrrolidin-3-ylsulfonylbenzothiazol-2-yl)carbamoyl]benzamide;
2-chloro-5-(pyridin-2-yl)-N-(6-(methylsulfonyl)benzothiazol-2-ylcarbamoyl)benzamide;
2-chloro-5-(pyrazol-1-yl)-N-(6-(methylsulfonyl)benzothiazol-2-ylcarbamoyl)benzamide;
2-chloro-5-[1,2,3]triazol-1-yl-N-(6-(methylsulfonyl)benzothiazol-2-ylcarbamoyl)benzamide;
2-chloro-5-(1-pyrrolidinyl)-N-(6-(methylsulfonyl)benzothiazol-2-ylcarbamoyl)benzamide;
2-chloro-5-cyclopropyl-N-(6-(methylsulfonyl)benzothiazol-2-ylcarbamoyl)benzamide;
2-chloro-5-(2,5-dihydro-pyrrol-1-yl)-N-(6-(methylsulfonyl)benzothiazol-2-ylcarbamoyl)benzamide;
2-chloro-5-cyclopentyloxy-N-(6-(methylsulfonyl)benzothiazol-2-ylcarbamoyl)benzamide;
2-chloro-5-cyclopentyl-N-(6-(methylsulfonyl)benzothiazol-2-ylcarbamoyl)benzamide;
2-chloro-5-ethoxy-N-(6-(methylsulfonyl)benzothiazol-2-ylcarbamoyl)benzamide;
2-chloro-N-(6-(methylsulfonyl)benzothiazol-2-ylcarbamoyl)-5-morpholin-4-yl benzamide;
2-chloro-5-(5-methyl-1H-pyrazol-1-yl)-N-(6-(methylsulfonyl)benzothiazol-2-ylcarbamoyl)benzamide;
2-chloro-5-(3-methyl-1H-pyrazol-l-yl)-N-(6-(methylsulfonyl)benzothiazol-2-ylcarbamoyl)benzamide;
2-chloro-N-(6-(methylsulfonyl)benzothiazol-2-ylcarbamoyl)-5-(pyrimidin-2-yl)benzamide;
4-chloro-3-(6-(methylsulfonyl)benzothiazol-2-ylcarbamoylcarbamoyl)phenyl methanesulfonate;
2-chloro-5-(4-methylpiperazin-1-yl)-N-(6-(methylsulfonyl)benzothiazol-2-ylcarbamoyl)benzamide;
2-chloro-5-(3-(dimethylamino)pyrrolidin-1-yl)-N-(6-(methylsulfonyl)benzothiazol-2-ylcarbamoyl)benzamide;
2-chloro-5-ethynyl-N-(6-(methylsulfonyl)benzothiazol-2-ylcarbamoyl)benzamide; 2-chloro-5-pyrrolidin-1-ylmethyl-N-(6-(methylsulfonyl)benzothiazol-2-ylcarbamoyl)benzamide;
2-chloro-5-(2-(dimethylamino)ethoxy)-N-(6-(methylsulfonyl)benzothiazol-2-ylcarbamoyl)benzamide;
2,5-chloro-N-[(6-methylsulfonylbenzothiazol-2-yl)carbamoyl]benzamide;
2-chloro-5-methyl-N-(6-(methylsulfonyl)benzothiazol-2-ylcarbamoyl)benzamide;
2-chloro-N-[(6-methylsulfonylbenzothiazol-2-yl)carbamoyl]-5-pyrrol-1-yl-benzamide;
2-chloro-N-(6-(methylsulfonyl)benzothiazol-2-ylcarbamoyl)-5-(1H-1,2,4-triazol-1-yl)benzamide;
2-chloro-N-(6-(methylsulfonyl)benzothiazol-2-ylcarbamoyl)-5-(thiazol-5-yl)benzamide;
2-chloro-5-(1H-imidazol-1-yl)-N-(6-(methylsulfonyl)benzothiazol-2-ylcarbamoyl)benzamide;
2-chloro-N-(6-(methylsulfonyl)benzothiazol-2-ylcarbamoyl)-5-nitrobenzamide; 2-chloro-N-[[6-(3-diethylaminopropylsulfonyl)benzothiazol-2-yl]carbamoyl]-5-pyrrol-1-yl-benzamide;
2-chloro-N-[[6-[3-(cyclopropylmethylamino)propylsulfonyl]benzothiazol-2-yl]carbamoyl]-5-pyrrol-1-yl-benzamide;
2-chloro-N-[[6-(3-piperazin-1-ylpropylsulfonyl)benzothiazol-2-yl]carbamoyl]-5-pyrrol-1-yl-benzamide;
N-[[6-[3-(azetidin-1-yl)propylsulfonyl]benzothiazol-2-yl]carbamoyl]-2-chloro-5-pyrrol-1-yl-benzamide;
2-chloro-N-[[6-[3-(propan-2-ylamino)propylsulfonyl]benzothiazol-2-yl]carbamoyl]-5-pyrrol-1-yl-benzamide;
2-chloro-N-[[6-[3-(2-methoxyethylamino)propylsulfonyl]benzothiazol-2-yl]carbamoyl]-5-pyrrol-1-yl-benzamide;
2-chloro-N-[[6-[(3S)-pyrrolidin-3-yl]sulfonylbenzothiazol-2-yl]carbamoyl]-5-pyrrol-1-yl-benzamide;
2-chloro-N-[[6-[(3R)-pyrrolidin-3-yl]sulfonylbenzothiazol-2-yl]carbamoyl]-5-pyrrol-1-yl-benzamide;
2-chloro-N-[[6-(2-piperazin-1-ylethylsulfonyl)benzothiazol-2-yl]carbamoyl]-5-pyrrol-1-yl-benzamide;
2-chloro-N-[[6-[2-(2-methoxyethylamino)ethylsulfonyl]benzothiazol-2-yl]carbamoyl]-5-pyrrol-1-yl-benzamide;
N-[[6-[2-(azetidin-1-yl)ethylsulfonyl]benzothiazol-2-yl]carbamoyl]-2-chloro-5-pyrrol-1-yl-benzamide;
2-chloro-N-[[6-[2-(propan-2-ylamino)ethylsulfonyl]benzothiazol-2-yl]carbamoyl]-5-pyrrol-1-yl-benzamide;
2-bromo-N-(6-(2-(isopropylamino)ethylsulfonyl)benzothiazol-2-ylcarbamoyl)-5-(1 H-pyrrol-1-yl)benzamide;
2-iodo-N-(6-(2-(isopropylamino)ethylsulfonyl)benzothiazol-2-ylcarbamoyl)-5-(1 H-pyrrol-1-yl)benzamide;
2-chloro-N-(6-(3-(4-methylpiperazin-1-yl)propylsulfonyl)benzothiazol-2-ylcarbamoyl)-5-(1H-pyrrol-1-yl)benzamide;
2-chloro-N-(6-(3-(4-methylpiperazin-1-yl)propylsulfonyl)benzothiazol-2-ylcarbamoyl)-5-(pyridin-2-yl)benzamide;
2-chloro-N-(6-(3-(4-methylpiperazin-1-yl)propylsulfonyl)benzothiazol-2-ylcarbamoyl)-5-(pyrrolidin-1-yl)benzamide;
2-chloro-N-(6-(3-(4-methylpiperazin-1-yl)propylsulfonyl)benzothiazol-2-ylcarbamoyl)-5-(1H-pyrazol-1-yl)benzamide;
2-chloro-N-(6-(3-(4-methylpiperazin-1-yl)propylsulfonyl)benzothiazol-2-ylcarbamoyl)-5-morpholinobenzamide;
2-chloro-5-(5-methyl-1H-pyrazol-1-yl)-N-(6-(3-(4-methylpiperazin-1-yl)propylsulfonyl)benzothiazol-2-ylcarbamoyl)benzamide;
2-chloro-5-(3-methyl-1H-pyrazol-1-yl)-N-(6-(3-(4-methylpiperazin-1-yl)propylsulfonyl)benzothiazol-2-ylcarbamoyl)benzamide;
4-chloro-3-(6-(3-(4-methylpiperazin-1-yl)propylsulfonyl)benzothiazol-2-ylcarbamoylcarbamoyl)phenyl methanesulfonate;
2-chloro-5-ethoxy-N-(6-(3-(4-methylpiperazin-1-yl)propylsulfonyl)benzothiazol-2-ylcarbamoyl)benzamide;
2-chloro-5-cyclopropyl-N-(6-(3-(4-methylpiperazin-1-yl)propylsulfonyl)benzothiazol-2-ylcarbamoyl)benzamide;
2-chloro-N-(6-(3-(4-methylpiperazin-1-yl)propylsulfonyl)benzothiazol-2-ylcarbamoyl)-5-(1H-1,2,4-triazol-1-yl)benzamide;
2-chloro-N-(6-(3-(4-methylpiperazin-1-yl)propylsulfonyl)benzothiazol-2-ylcarbamoyl)-5-(thiazol-5-yl)benzamide;
2-chloro-N-(6-(3-(diethylamino)propylsulfonyl)benzothiazol-2-ylcarbamoyl)-5-morpholinobenzamide;
2-chloro-N-(6-(3-(diethylamino)propylsulfonyl)benzothiazol-2-ylcarbamoyl)-5-(1 H-pyrazol-1-yl)benzamide;
2-chloro-N-(6-(1-methylpiperidin-4-ylsulfonyl)benzothiazol-2-ylcarbamoyl)-5-morpholinobenzamide;
2-chloro-N-(6-(1-methylpiperidin-4-ylsulfonyl)benzothiazol-2-ylcarbamoyl)-5-(1H-pyrazol-1-yl)benzamide;
2,6-Dichloro-N-[(6-methylsulfonylbenzothiazol-2-yl)carbamoyl]benzamide;
2-Chloro-N-[(6-methylsulfonylbenzothiazol-2-yl)carbamoyl]benzamide;
2-Bromo-N-[(6-methylsulfonylbenzothiazol-2-yl)carbamoyl]benzamide;
N-[(6-Methylsulfonylbenzothiazol-2-yl)carbamoyl]-2-nitro-benzamide;
N-[(6-Methylsulfonylbenzothiazol-2-yl)carbamoyl]-2-phenyl-benzamide;
2-Chloro-6-fluoro-N-[(6-methylsulfonylbenzothiazol-2-yl)carbamoyl]benzamide;
2-Chloro-4-fluoro-N-[(6-methylsulfonylbenzothiazol-2-yl)carbamoyl]benzamide;
2-Fluoro-N-[(6-methylsulfonylbenzothiazol-2-yl)carbamoyl]benzamide;
2-Chloro-3-fluoro-N-[(6-methylsulfonylbenzothiazol-2-yl)carbamoyl]benzamide
2-Chloro-3,4-dimethoxy-N-[(6-methylsulfonylbenzothiazol-2-yl)carbamoyl]benzamide;
2,6-Difluoro-N-[(6-methylsulfonylbenzothiazol-2-yl)carbamoyl]benzamide;
2-Chloro-4-methylsulfonyl-N-[(6-methylsulfonylbenzothiazol-2-yl)carbamoyl]benzamide;
2-Chloro-N-[(6-methylsulfonylbenzothiazol-2-yl)carbamoyl]-4-morpholin-4-yl-benzamide;
2-Chloro-N-[(6-methylsulfonylbenzothiazol-2-yl)carbamoyl]-4-pyrazol-1-yl-benzamide;
2-Chloro-N-[(6-methylsulfonylbenzothiazol-2-yl)carbamoyl]-4-pyrrolidin-1-yl-benzamide;
2-Iodo-N-[(6-methylsulfonylbenzothiazol-2-yl)carbamoyl]benzamide;
2-Chloro-4-(2,5-dimethylpyrrol-1-yl)-N-[(6-methylsulfonylbenzothiazol-2-yl)carbamoyl]benzamide;
N-[[6-[2-(Carbamoylmethoxy)ethylsulfonyl]benzothiazol-2-yl]carbamoyl]-2-chlorobenzamide;
2-Chloro-N-[[6-[2-(2-hydroxyethoxy)ethylsulfonyl]benzothiazol-2-yl]carbamoyl]benzamide;
2-[2-[2-[(2-Chlorobenzoyl)carbamoylamino]benzothiazol-6-yl]sulfonylethoxy]acetic acid;
2-(4-Fluorophenyl)-4-methoxy-N-[(6-methylsulfonylbenzothiazol-2-yl)carbamoyl]benzamide;
2-(4-Methoxyphenyl)-N-[(6-methylsulfonylbenzothiazol-2-yl)carbamoyl]benzamide;
2-(2-Fluorophenyl)-N-[(6-methylsulfonylbenzothiazol-2-yl)carbamoyl]benzamide;
2-(4-Fluorophenyl)-N-[(6-methylsulfonylbenzothiazol-2-yl)carbamoyl]benzamide;
N-[(6-Methylsulfonylbenzothiazol-2-yl)carbamoyl]-2-phenoxy-benzamide;
2-Methyl-N-[(6-methylsulfonylbenzothiazol-2-yl)carbamoyl]benzamide;
2-Chloro-N-[(6-ethenylsulfonylbenzothiazol-2-yl)carbamoyl]benzamide;
2-Ethylsulfanyl-N-[(6-methylsulfonylbenzothiazol-2-yl)carbamoyl]benzamide;
N-[(6-Methylsulfonylbenzothiazol-2-yl)carbamoyl]-2-(phenoxymethyl)benzamide;
2-Methylsulfanyl-N-[(6-methylsulfonylbenzothiazol-2-yl)carbamoyl]benzamide;
N-[(6-Methylsulfonylbenzothiazol-2-yl)carbamoyl]-2-phenylsulfanyl-benzamide;
N-[(6-Methylsulfonylbenzothiazol-2-yl)carbamoyl]-2-pyrrol-1-yl-benzamide;
2-Ethylsulfonyl-N-[(6-methylsulfonylbenzothiazol-2-yl)carbamoyl]benzamide;
N-[(6-Methylsulfonylbenzothiazol-2-yl)carbamoyl]-2-propan-2-yl-benzamide;
N-[(6-Methylsulfonylbenzothiazol-2-yl)carbamoyl]-2-(trifluoromethylsulfonyloxy)-benzamide;
2-Chloro-N-[(6-methylsulfonylbenzothiazol-2-yl)carbamoyl]-5-sulfamoyl-benzamide;
2-Chloro-N-[[6-(3-piperidylsulfonyl)benzothiazol-2-yl]carbamoyl]benzamide;
2-Chloro-N-[[6-[(1-propan-2-yl-3-piperidyl)sulfonyl]benzothiazol-2-yl]carbamoyl]benzamide;
2-Ethyl-N-[(6-methylsulfonylbenzothiazol-2-yl)carbamoyl]benzamide;
2-Chloro-N-[[6-[(1-ethyl-3-piperidyl)sulfonyl]benzothiazol-2-yl]carbamoyl]benzamide;
2-Chloro-N- [[6-[[1-(cyclopropylmethyl)-3-piperidyl]sulfonyl]benzothiazol-2-yl]carbamoyl]benzamide;
2-Chloro-N-[[6-[1-(cyclopropylmethyl)pyrrolidin-3-yl]sulfonylbenzothiazol-2-yl]carbamoyl]benzamide;
2-Chloro-N-[[6-[3-(propan-2-ylamino)propylsulfonyl]benzothiazol-2-yl]carbamoyl]benzamide;
2-Chloro-N-[[6-(3-morpholin-4-ylpropylsulfonyl)benzothiazol-2-yl]carbamoyl]benzamide;
N-[[6-[3-(Azetidin-1-yl)propylsulfonyl]benzothiazol-2-yl]carbamoyl]-2-chloro-benzamide;
2-Chloro-N-[[6-(3-chloropropylsulfonyl)benzothiazol-2-yl]carbamoyl]benzamide;
2-Chloro-N-[[6-(3-pyrrolidin-1-ylpropylsulfonyl)benzothiazol-2-yl]carbamoyl]benzamide;
2-Chloro-N-[[6-[3-(1,1-dioxo-1,4-thiazinan-4-yl)propylsulfonyl]benzothiazol-2-yl]carbamoyl]benzamide;
2-Chloro-N-[[6-[3-(cyclopropylmethylamino)propylsulfonyl]benzothiazol-2-yl]carbamoyl]benzamide;
2-Chloro-N-[[6-[3-(1-piperidyl)propylsulfonyl]benzothiazol-2-yl]carbamoyl]benzamide;
2-Chloro-N-[[6-(3-diethylaminopropylsulfonyl)benzothiazol-2-yl]carbamoyl]benzamide;
2-Chloro-N-[[6-[3-(3,3-difluoropyrrolidin-1-yl)propylsulfonyl]benzothiazol-2-yl]carbamoyl]benzamide;
2-Chloro-N-[[6-[3-(4-methylpiperazin-1-yl)propylsulfonyl]benzothiazol-2-yl]carbamoyl]benzamide;
2-Chloro-N-[[6-[[(2R)-1-(cyclopropylmethyl)pyrrolidin-2-yl]methylsulfonyl]benzothiazol-2-yl] carbamoyl]benzamide;
2-Chloro-N-[[6-[3-(3-fluoropyrrolidin-1-yl)propylsulfonyl]benzothiazol-2-yl]carbamoyl]benzamide;
2-Chloro-N-[[6-[3-(4,4-difluoro-1-piperidyl)propylsulfonyl]benzothiazol-2-yl]carbamoyl]benzamide;
2-Chloro-N-[[6-[3-(2-hydroxyethylamino)propylsulfonyl]benzothiazol-2-yl]carbamoyl]benzamide;
2-Chloro-N-[[6-[3-(3-hydroxypyrrolidin-1-yl)propylsulfonyl]benzothiazol-2-yl]carbamoyl]benzamide;
5-Bromo-2-chloro-N-[(6-methylsulfonylbenzothiazol-2-yl)carbamoyl]benzamide;
2-Chloro-N-[[6-(1-propan-2-ylpyrrolidin-3-yl)sulfonylbenzothiazol-2-yl]carbamoyl]benzamide;
2-Chloro-N-[[6-(1-ethylpyrrolidin-3-yl)sulfonylbenzothiazol-2-yl]carbamoyl]benzamide;
2-Chloro-N-[[6-[[(2R)-1-ethylpyrrolidin-2-yl]methylsulfonyl]benzothiazol-2-yl]carbamoyl]benzamide;
2-Chloro-N-[[6-[[(2R)-pyrrolidin-2-yl]methylsulfonyl]benzothiazol-2-yl]carbamoyl]benzamide;
2-Chloro-N-[[6-[(1-methyl-3-piperidyl)sulfonyl]benzothiazol-2-yl]carbamoyl]benzamide;
2-Chloro-N-[[6-(1-methylpyrrolidin-3-yl)sulfonylbenzothiazol-2-yl]carbamoyl]benzamide;
2-Chloro-N-[[6-[[(2R)-1-propan-2-ylpyrrolidin-2-yl]methylsulfonyl]benzothiazol-2-yl]carbamoyl]benzamide;
2-Chloro-N-[[6-[[(2R)-1-methylpyrrolidin-2-yl]methylsulfonyl]benzothiazol-2-yl]carbamoyl]benzamide;
N-[(6-Methylsulfonylbenzothiazol-2-yl)carbamoyl]-2-(trifluoromethyl)benzamide;
2-Chloro-N-[[6-[(3S)-pyrrolidin-3-yl]sulfonylbenzothiazol-2-yl]carbamoyl]benzamide;
2-Chloro-N-[[6-[(3R)-pyrrolidin-3-yl]sulfonylbenzothiazol-2-yl]carbamoyl]benzamide;
2-Chloro-N-[[6-[(1-ethyl-4-piperidyl)sulfonyl]benzothiazol-2-yl]carbamoyl]benzamide;
2-Chloro-N-[[6-[(1-propan-2-yl-4-piperidyl)sulfonyl]benzothiazol-2-yl]carbamoyl]benzamide;
2-Chloro-N-[[6-[1-(cyclopropylmethyl)azetidin-3-yl]sulfonylbenzothiazol-2-yl]carbamoyl]benzamide;
2-Chloro-N-[[6-(4-piperidylsulfonyl)benzothiazol-2-yl]carbamoyl]benzamide;
2-Chloro-4,5-difluoro-N-[(6-methylsulfonylbenzothiazol-2-yl)carbamoyl]benzamide;
2-Chloro-N-[[6-(pyridin-2-ylmethylsulfonyl)benzothiazol-2-yl]carbamoyl]benzamide;
2,4-Dichloro-N-[(6-methylsulfonylbenzothiazol-2-yl)carbamoyl]benzamide;
2-Chloro-N-[(6-methylsulfonylbenzothiazol-2-yl)carbamoyl]-4-nitro-benzamide;
2-Chloro-N-[(6-methylsulfonylbenzothiazol-2-yl)carbamoyl]-5-(trifluoromethyl)benzamide;
2-Chloro-N-[[6-[[1-(cyclopropylmethyl)-4-piperidyl]sulfonyl]benzothiazol-2-yl]carbamoyl]benzamide;
N-[[6-(Azetidin-3-ylsulfonyl)benzothiazol-2-yl]carbamoyl]-2-chloro-benzamide;
2-Chloro-N-[[6-[(1-methyl-4-piperidyl)sulfonyl]benzothiazol-2-yl]carbamoyl]benzamide;
2-Chloro-N-[[6-(1-ethylazetidin-3-yl)sulfonylbenzothiazol-2-yl]carbamoyl]benzamide;
2-Chloro-N-[[6-(1-propan-2-ylazetidin-3-yl)sulfonylbenzothiazol-2-yl]carbamoyl]benzamide;
2-Chloro-N-[[6-(pyridin-3-ylmethylsulfonyl)benzothiazol-2-yl]carbamoyl]benzamide;
2-Chloro-N-[[6-[(5-methyl-1,2-oxazol-3-yl)methylsulfonyl]benzothiazol-2-yl]carbamoyl]benzamide;
2-Chloro-N-[[6-(1H-imidazol-2-ylmethylsulfonyl)benzothiazol-2-yl]carbamoyl]benzamide;
2-Chloro-N-[[6-(2-pyridin-2-ylethylsulfonyl)benzothiazol-2-yl]carbamoyl]benzamide;
2-Chloro-N-[[6-[(2-methyl-1,3-thiazol-4-yl)methylsulfonyl]benzothiazol-2-yl]carbamoyl]benzamide;
2-Chloro-N-[[6-(3-methoxypropylsulfonyl)benzothiazol-2-yl]carbamoyl]benzamide;
2-Chloro-N-[[6-(3-imidazol-1-ylpropylsulfonyl)benzothiazol-2-yl]carbamoyl]benzamide;
2-Chloro-6-fluoro-3-methyl-N-[(6-methylsulfonylbenzothiazol-2-yl)carbamoyl]benzamide;
6-Chloro-2-fluoro-3-methyl-N-[(6-methylsulfonylbenzothiazol-2-yl)carbamoyl]benzamide;
2-Chloro-N-[[6-[3-[(3S,5R)-3,5-dimethylpiperazin-1-yl]propylsulfonyl]benzothiazol-2-yl]carbamoyl]benzamide;
2-Chloro-N-[[6-[3-(4-ethylpiperazin-1-yl)propylsulfonyl]benzothiazol-2-yl]carbamoyl]benzamide;
N-[[6-[3-(4-Acetylpiperazin-1-yl)propylsulfonyl]benzothiazol-2-yl]carbamoyl]-2-chlorobenzamide;
2-Chloro-N-[[6-[3-(4-propan-2-ylpiperazin-1-yl)propylsulfonyl]benzothiazol-2-yl]carbamoyl]benzamide;
2-Chloro-N-[[6-[3-[4-(2-methoxyethyl)piperazin-1-yl]propylsulfonyl]benzothiazol-2-yl]carbamoyl]benzamide;
2-Chloro-N-[[6-[3-(4-dimethylamino-1-piperidyl)propylsulfonyl]benzothiazol-2-yl]carbamoyl]benzamide;
2-Chloro-N-[[6-(3-dimethylaminopropylsulfonyl)benzothiazol-2-yl]carbamoyl]benzamide;
2-Chloro-N-[[6-[3-(2-methoxyethylamino)propylsulfonyl]benzothiazol-2-yl]carbamoyl]benzamide;
2-Chloro-N-[[6-[3-(4-methyl-1,4-diazepan-1-yl)propylsulfonyl]benzothiazol-2-yl]carbamoyl]benzamide;
*tert*-Butyl 4-[3-[2-[(2-chlorobenzoyl)carbamoylamino]benzothiazol-6-yl]sulfonylpropyl]piperazine-1-carboxylate;
2-Chloro-N-[[6-(3-piperazin-1-ylpropylsulfonyl)benzothiazol-2-yl]carbamoyl]benzamide;
2-Chloro-N-[[6-[3-[4-(2-cyanoethyl)piperazin-1-yl]propylsulfonyl]benzothiazol-2-yl]carbamoyl]benzamide;
2-Chloro-N-[[6-[3-(4-methylsulfonylpiperazin-1-yl)propylsulfonyl]benzothiazol-2-yl]carbamoyl]benzamide;
2-Chloro-N-[[6-[3-(3-methylpiperazin-1-yl)propylsulfonyl]benzothiazol-2-yl]carbamoyl]benzamide;
2-Chloro-N-[[6-[4-(4-methylpiperazin-1-yl)butylsulfonyl]benzothiazol-2-yl]carbamoyl]benzamide;
2-Chloro-N-[[6-(4-diethylaminobutylsulfonyl)benzothiazol-2-yl]carbamoyl]benzamide;
2-Chloro-N-[[6-[3-(4-methylpiperazin-1-yl)propylsulfonyl]benzothiazol-2-yl]carbamoyl]-4-(3-methylpyrazol-1-yl)benzamide;
*tert*-Butyl N-[3-[2-[(2-chlorobenzoyl)carbamoylamino]benzothiazol-6-yl]sulfonylcyclobutyl]carbamate;
N-[[6-(3-Aminocyclobutyl)sulfonylbenzothiazol-2-yl]carbamoyl]-2-chloro-benzamide;
2-Chloro-N-[[6-(3-methylaminocyclobutyl)sulfonylbenzothiazol-2-yl]carbamoyl]benzamide;
2-Chloro-N-[[6-(3-dimethylaminocyclobutyl)sulfonylbenzothiazol-2-yl]carbamoyl]benzamide;
*tert*-Butyl 4-[[2-[(2-chlorobenzoyl)carbamoylamino]benzothiazol-6-yl]sulfonylmethyl]piperidine-1-carboxylate;
*tert*-Butyl 4-[2-[2-[(2-chlorobenzoyl)carbamoylamino]benzothiazol-6-yl]sulfonylethyl]piperidine-1-carboxylate;
2-Chloro-N-[[6-(4-piperidylmethylsulfonyl)benzothiazol-2-yl]carbamoyl]benzamide;
2-Chloro-5-ethynyl-N-[[6-[3-(4-methylpiperazin-1-yl)propylsulfonyl]benzothiazol-2-yl]carbamoyl]benzamide;
2-Chloro-N-[[6-[2-(4-piperidyl)ethylsulfonyl]benzothiazol-2-yl]carbamoyl]benzamide;
2-Chloro-N-[[6-[3-(2-methoxyethylamino)cyclobutyl]sulfonylbenzothiazol-2-yl]carbamoyl]benzamide;
N-[[6-(3-Acetamidocyclobutyl)sulfonylbenzothiazol-2-yl]carbamoyl]-2-chloro-benzamide;
2-chloro-N-[[6-(isopropylsulfamoyl)-1,3-benzothiazol-2-yl]carbamoyl]benzamide;
2-chloro-N-(6-(1-(isopropylamino)-2-methylpropan-2-ylsulfonyl)benzo[d]thiazol-2-ylcarbamoyl)benzamide;
2-chloro-5-ethyl-N-(6-(3-(4-methylpiperazin-1-yl)propylsulfonyl)benzo[d]thiazol-2-ylcarbamoyl)benzamide;
5-(1-acetylpyrrolidin-3-yloxy)-2-chloro-N-(6-(methylsulfonyl)benzo[d]thiazol-2-ylcarbamoyl)benzamide;
(S)-2-chloro-5-((1-isopropylpiperidin-3-yl)methoxy)-N-(6-(methylsulfonyl)benzo[d]-thiazol-2-ylcarbamoyl)benzamide;
2-chloro-N-[(6-sulfamoyl-1,3-benzothiazol-2-yl)carbamoyl]benzamide;
2-chloro-N-[[6-(methylsulfamoyl)-1,3-benzothiazol-2-yl]carbamoyl]benzamide;
2-chloro-N-[[6-(2-hydroxyethylsulfamoyl)-1,3-benzothiazol-2-yl]carbamoyl]benzamide;
2-chloro-N-[[6-(dimethylsulfamoyl)-1,3-benzothiazol-2-yl]carbamoyl]benzamide;
(R)-2-chloro-5-(3-(dimethylamino)pyrrolidin-1-yl)-N-(6-(methylsulfonyl)benzo[d]thiazol-2-ylcarbamoyl)benzamide;
tert-butyl 4-(4-chloro-3-(6-(methylsulfonyl)benzo[d]thiazol-2-ylcarbamoylcarbamoyl)phenyl)piperazine-1-carboxylate;
(S)-2-chloro-5-(3-(dimethylamino)pyrrolidin-1-yl)-N-(6-(methylsulfonyl)benzo[d]thiazol-2-ylcarbamoyl)benzamide;
2-chloro-5-(4-(dimethylamino)piperidin-1-yl)-N-(6-(methylsulfonyl)benzo[d]thiazol-2-ylcarbamoyl)benzamide;
2-chloro-5-(3,5-dimethyl-1H-pyrazol-1-yl)-N-(6-(3-(4-methylpiperazin-1-yl)propylsulfonyl)benzo[d]thiazol-2-ylcarbamoyl)benzamide;
2-chloro-5-iodo-N-(6-(3-(4-methyl-1,4-diazepan-1-yl)propylsulfonyl)benzo[d]thiazol-2-ylcarbamoyl)benzamide;
2-chloro-5-(3-(diethylamino)pyrrolidin-1-yl)-N-(6-(methylsulfonyl)benzo[d]thiazol-2-ylcarbamoyl)benzamide;
2-chloro-N-(6-(3-(4-methylpiperazin-1-yl)propylsulfonyl)benzo[d]thiazol-2-ylcarbamoyl)-3-(1H-pyrazol-1-yl)benzamide;
2,4-dichloro-N-(6-(3-(4-methylpiperazin-1-yl)propylsulfonyl)benzo[d]thiazol-2-ylcarbamoyl)-5-(1H-pyrazol-1-yl)benzamide;
2-chloro-4-methoxy-N-(6-(3-(4-methylpiperazin-1-yl)propylsulfonyl)benzo[d]thiazol-2-ylcarbamoyl)-5-(1H-pyrazol-1-yl)benzamide;
tert-butyl 4-(4-chloro-3-(6-(methylsulfonyl)benzo[d]thiazol-2-ylcarbamoylcarbamoyl)phenoxy)piperidine-1-carboxylate;
2-chloro-5-(1-methylpiperidin-4-yloxy)-N-(6-(methylsulfonyl)benzo[d]thiazol-2-ylcarbamoyl)benzamide;
2-chloro-N-(6-(2-methyl-1-(pyrrolidin-1-yl)propan-2-ylsulfonyl)benzo[d]thiazol-2-ylcarbamoyl)-5-morpholinobenzamide;
2-chloro-N-(6-(1-(isopropylamino)-2-methylpropan-2-ylsulfonyl)benzo[d]thiazol-2-ylcarbamoyl)-5-morpholinobenzamide;
2-chloro-5-(3-(dimethylamino)pyridin-2-yl)-N-(6-(methylsulfonyl)benzo[d]thiazol-2-ylcarbamoyl)benzamide;
tert-butyl 3-(4-chloro-3-(6-(methylsulfonyl)benzo[d]thiazol-2-ylcarbamoylcarbamoyl)phenoxy)pyrrolidine-1-carboxylate;
2-chloro-N-(6-(methylsulfonyl)benzo[d]thiazol-2-ylcarbamoyl)-5-(pyrrolidin-3-yloxy)benzamide;
2-chloro-5-(1-methylpyrrolidin-3-yloxy)-N-(6-(methylsulfonyl)benzo[d]thiazol-2-ylcarbamoyl)benzamide;
2-chloro-5-(6-(dimethylamino)pyridin-2-yl)-N-(6-(methylsulfonyl)benzo[d]thiazol-2-ylcarbamoyl)benzamide;
tert-butyl 3-(4-chloro-3-(6-(methylsulfonyl)benzo[d]thiazol-2-ylcarbamoylcarbamoyl)phenoxy)azetidine-1-carboxylate;
5-(azetidin-3-yloxy)-2-chloro-N-(6-(methylsulfonyl)benzo[d]thiazol-2-ylcarbamoyl)benzamide;
2-chloro-4-fluoro-N-(6-(1-methylpiperidin-4-ylsulfonyl)benzo[d]thiazol-2-ylcarbamoyl)-5-(1H-pyrazol-1-yl)benzamide;
2-chloro-4-methoxy-N-(6-(1-methylpiperidin-4-ylsulfonyl)benzo[d]thiazol-2-ylcarbamoyl)-5-morpholinobenzamide;
2-chloro-5-ethoxy-4-methoxy-N-(6-(1-methylpiperidin-4-ylsulfonyl)benzo[d]thiazol-2-ylcarbamoyl)benzamide;
2-chloro-5-(1-methylazetidin-3-yloxy)-N-(6-(methylsulfonyl)benzo[d]thiazol-2-ylcarbamoyl)benzamide;
2,4-dichloro-N-(6-(1-methylpiperidin-4-ylsulfonyl)benzo[d]thiazol-2-ylcarbamoyl)-5-morpholinobenzamide;
2,4-dichloro-N-(6-(1-methylpiperidin-4-ylsulfonyl)benzo[d]thiazol-2-ylcarbamoyl)-5-(1H-pyrazol-1-yl)benzamide;
2-chloro-4-methyl-N-(6-(1-methylpiperidin-4-ylsulfonyl)benzo[d]thiazol-2-ylcarbamoyl)-5-(1H-pyrazol-1-yl)benzamide;
2-chloro-4-ethoxy-N-(6-(1-methylpiperidin-4-ylsulfonyl)benzo[d]thiazol-2-ylcarbamoyl)-5-(1H-pyrazol-1-yl)benzamide;
tert-butyl 4-(2-(3-(2-chloro-4-methoxy-5-(1H-pyrazol-1-yl)benzoyl)ureido)benzo[d]-thiazol-6-ylsulfonyl)piperidine-1-carboxylate;
2-chloro-4-methoxy-N-(6-(piperidin-4-ylsulfonyl)benzo[d]thiazol-2-ylcarbamoyl)-5-(1H-pyrazol-1-yl)benzamide;
2-chloro-4-methoxy-N-(6-(1-methylpiperidin-4-ylsulfonyl)benzo[d]thiazol-2-ylcarbamoyl)-5-(1H-pyrazol-1-yl)benzamide;
2-chloro-4-methoxy-5-(5-methyl-1H-pyrazol-1-yl)-N-(6-(1-methylpiperidin-4-ylsulfonyl)benzo[d]thiazol-2-ylcarbamoyl)benzamide;
2-chloro-5-((dimethylamino)methyl)-N-(6-(methylsulfonyl)benzo[d]thiazol-2-ylcarbamoyl)benzamide;
2-chloro-4-isopropoxy-N-(6-(1-methylpiperidin-4-ylsulfonyl)benzo [d]thiazol-2-ylcarbamoyl)-5-(1H-pyrazol-1-yl)benzamide;
2-Chloro-5-(3-dimethylamino-1-piperidyl)-N-[(6-methylsulfonyl-1,3-benzothiazol-2-yl)carbamoyl]benzamide; or
2-Chloro-5-(3-dimethylaminocyclobutoxy)-N-[(6-methylsulfonyl-1,3-benzothiazol-2-yl)carbamoyl]benzamide
or a pharmaceutically acceptable salt thereof.

In another aspect the present invention provides a compound selected from one of the following or any number from 2 to 11 of the following compounds:
2-Chloro-5-(3-(dimethylamino)pyrrolidin-1-yl)-N-(6-(methylsulfonyl)benzothiazol-2-ylcarbamoyl)benzamide;
2-Chloro-N-(6-(3-(4-methylpiperazin-1-yl)propylsulfonyl)benzothiazol-2-ylcarbamoyl)-5-(pyrrolidin-1-yl)benzamide;
2-Chloro-N-(6-(3-(4-methylpiperazin-1-yl)propylsulfonyl)benzothiazol-2-ylcarbamoyl)-5-morpholinobenzamide;
2-Chloro-5-(5-methyl-1H-pyrazol-1-yl)-N-(6-(3-(4-methylpiperazin-1-yl)propylsulfonyl)benzothiazol-2-ylcarbamoyl)benzamide;
2-Chloro-5-ethoxy-N-(6-(3-(4-methylpiperazin-1-yl)propylsulfonyl)benzothiazol-2-ylcarbamoyl)benzamide;
2-Chloro-N-(6-(1-methylpiperidin-4-ylsulfonyl)benzothiazol-2-ylcarbamoyl)-5-morpholinobenzamide;
2-Chloro-5-ethynyl-N-[[6-[3-(4-methylpiperazin-1-yl)propylsulfonyl]benzothiazol-2-yl]carbamoyl]benzamide;
2-chloro-5-ethyl-N-(6-(3-(4-methylpiperazin-1-yl)propylsulfonyl)benzo[d]thiazol-2-ylcarbamoyl)benzamide;
2-chloro-N-(6-(2-methyl-1-(pyrrolidin-1-yl)propan-2-ylsulfonyl)benzo[d]thiazol-2-ylcarbamoyl)-5-morpholinobenzamide;
2-chloro-4-fluoro-N-(6-(1-methylpiperidin-4-ylsulfonyl)benzo[d]thiazol-2-ylcarbamoyl)-5-(1H-pyrazol-1-yl)benzamide;
2-chloro-4-methoxy-N-(6-(1-methylpiperidin-4-ylsulfonyl)benzo[d]thiazol-2-ylcarbamoyl)-5-morpholinobenzamide;
or a pharmaceutically acceptable salt thereof.

### Methods of preparation

A compound of the invention, or a salt thereof, may be prepared by any process known to be applicable to the preparation of such compounds or structurally related compounds. Functional groups may be protected and deprotected using conventional methods. For examples of protecting groups such as amino and carboxylic acid protecting groups (as well as means of formation and eventual deprotection), see T.W. Greene and P.G.M. Wuts, "Protective Groups in Organic Synthesis", Second Edition, John Wiley & Sons, New York, 1991.

Processes for the synthesis of compounds of formula (I) are provided as a further feature of the invention. Thus, according to a further aspect of the invention there is provided a process for the preparation of a compound of formula (I).

Compounds of formula (I) may be prepared by:
a) reacting a benzamide of formula (II): 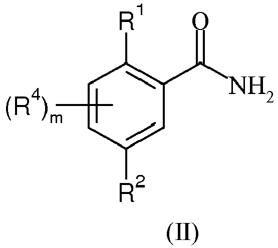 in which R¹, R², R⁴ and m are as previously defined with an amine of formula (III) 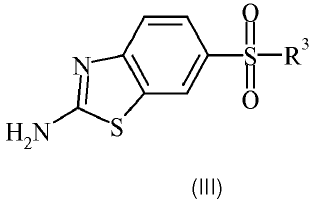 in which R³ is as previously defined in the presence of oxalyl chloride, optionally in the presence of an inert solvent, for example THF, optionally in the presence of a base, for example DIPEA, optionally in the presence of a Lewis acid, for example trimethylaluminium, at a temperature in the range of 80 - 150 °C to give a compound of formula (I); or
b) reacting a compound of formula (IV): 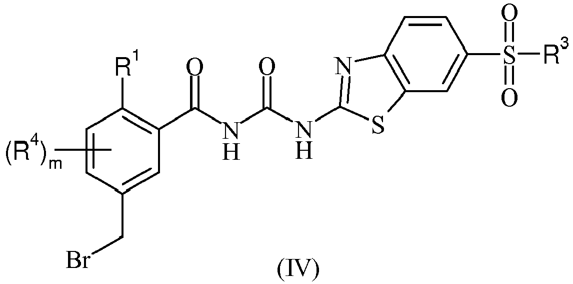 in which R¹, R³, R⁴ and m are as previously defined with an amine, optionally in the presence of an inert solvent, for example MeCN, at a temperature in the range between ambient temperature and the boiling point of the solvent to give a compound of formula (I); or
c) reacting a compound of formula (V): 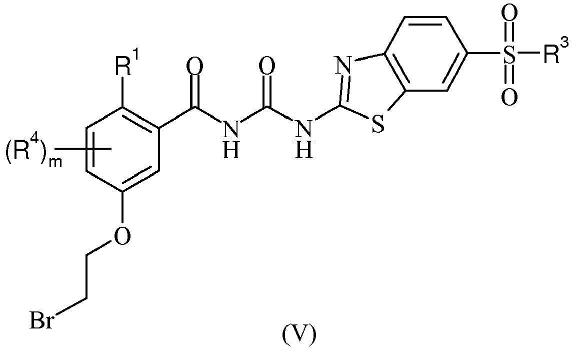 in which R¹, R³, R⁴ and m are as previously defined with an amine, optionally in the presence of an inert solvent, for example MeCN, at a temperature in the range of 80 - 150°C to give a compound of formula (I); or
d) reacting a carbamate of formula (VI): 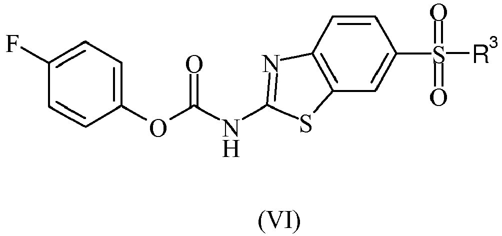 in which R³ is as previously defined with a benzamide of formula (II), optionally in the presence of an inert solvent, for example THF, in the presence of a base, for example potassium *tert*-butoxide, at a temperature in the range between ambient temperature and 150°C to give a compound of formula (I); or
e) reacting a compound of formula (VII): 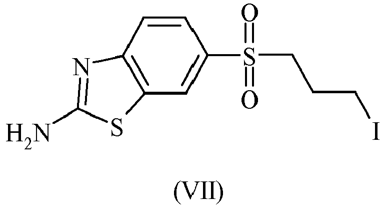 with a benzamide of formula (II) in the presence of oxalyl chloride and then an amine, optionally in the presence of an inert solvent, for example THF, at a temperature in the range of 50 - 150°C to give a compound of formula (I); or
f) reacting a compound of formula (VIII): 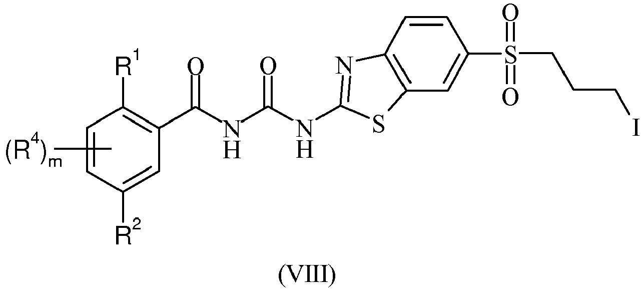 in which R¹, R², R⁴ and m are as previously defined with an amine, optionally in the presence of an inert solvent, for example THF, at a temperature in the range between ambient temperature and 150 °C to give a compound of formula (I); or
g) reacting a compound of formula (IX): 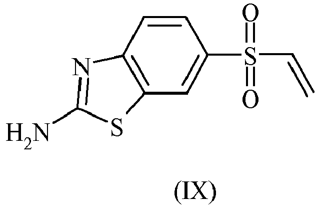 with a benzamide of formula (II) in the presence of oxalyl chloride and then an amine, optionally in the presence of an inert solvent, for example THF, at a temperature in the range of 80 - 150 °C to give a compound of formula (I); or
h) reacting a compound of formula (X): 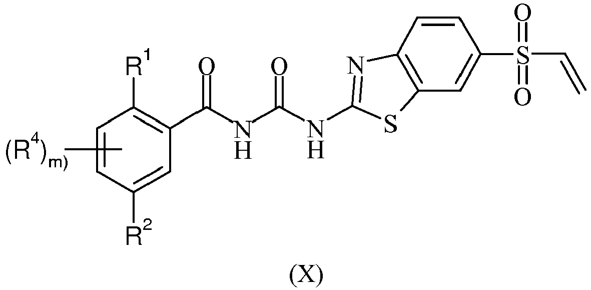 in which R¹, R², R⁴ and m are as previously defined with an amine of formula (XI) 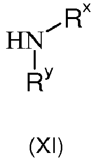 in which R^{x} and R^{y} are as previously defined optionally in the presence of an inert solvent, for example THF, at a temperature in the range of 80 - 150 °C to give a compound of formula (I) I in which R³ represents a group -(CH₂)₂-NR^{x}R^{y}, and R¹, R², R⁴, m, R^{x} and R^{y} are as previously defined; or
i) reacting a compound of formula (X) with an alcohol or an alkoxide salt thereof, optionally in the presence of an inert solvent, for example THF, in the presence of a base when the alcohol is used, for example sodium hydride, at a temperature in the range between 0 °C and the boiling point of the solvent to give a compound of formula (I); or
j) reacting a compound of formula (X) with a base (or a hydrolysing agent), for example Triton B, optionally in the presence of an inert solvent, for example THF, at a temperature in the range between ambient temperature and 150 °C to give a compound of formula (I); or
k) reacting a compound of formula (XII): 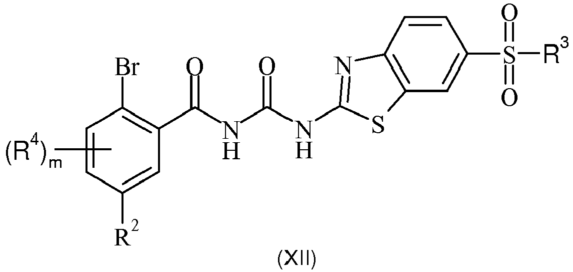
in which R², R³, R⁴ and m are as previously defined with copper(I) iodide, and a ligand, for example N,N'-dimethylethylenediamine, optionally in the presence of an additive, for example sodium iodide, optionally in the presence of an inert solvent, for example dioxane, at a temperature in the range of 80 - 150 °C to give a compound of formula (I).

In process (a), a nucleophile of formula (II) can be reacted with an appropriate electrophile such as oxalyl chloride and then with an appropriate amine of formula (III). The reaction is generally carried out in an appropriate organic solvent such as THF, at an appropriate temperature, generally between 80 °C and 150 °C, optionally in a microwave reactor. The reaction is normally continued until LCMS analysis indicates that reaction is complete. Typical reaction times are between 2 and 30 minutes. In one embodiment, the solvent is THF and the reaction temperature is 120 °C.

Compounds of formula (II) may be commercially available or may be prepared by reaction of a benzoic acid with an appropriate electrophile such as isopropyl chloroformate and then an appropriate nucleophile such as ammonia. Benzoic acids may be commercially available or may be prepared by reaction of a benzoate with an appropriate nucleophile such as potassium hydroxide. Benzoates may be commercially available or may be prepared by reaction of an aryl or hetaryl halide with an approriate coupling partner such as a boronic acid, boronate, stannane, alkene or terminal alkyne. Aryl or hetaryl halides may be commercially available or may be prepared using methods that are well-known in the literature. Typical processes that may be used to prepare compounds of formula (II) are illustrated in the following scheme:

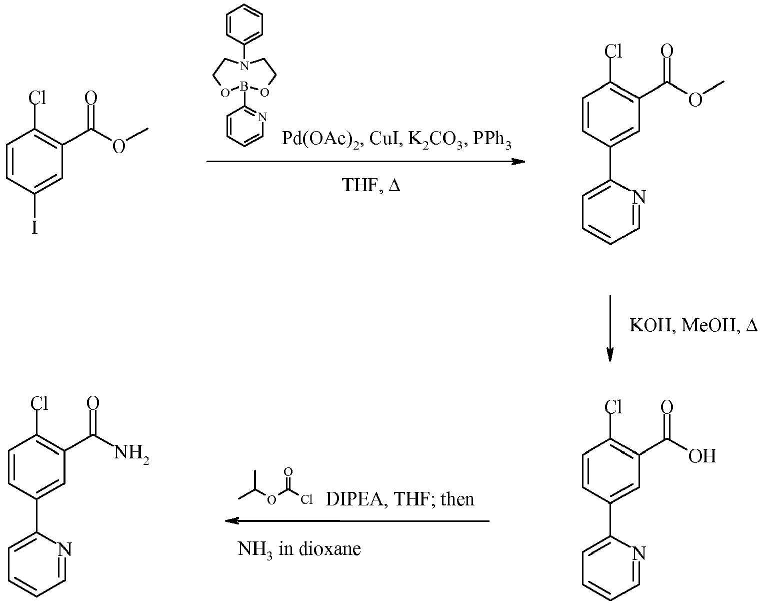

Compounds of formula (II) may also be prepared by reaction of an aryl fluoride with an appropriate nucleophile such as pyrazole. A typical process that may be used to prepare compounds of formula (II) is illustrated in the following scheme:

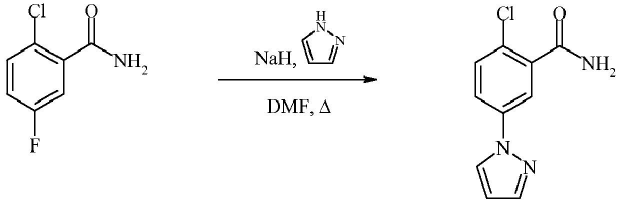

Compounds of formula (II) may also be prepared by reaction of an aryl boronic acid with an appropriate nucleophile such as imidazole. A typical process that may be used to prepare compounds of formula (II) is illustrated in the following scheme:

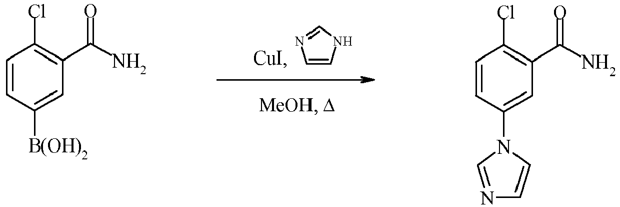

Compounds of formula (II) may also be prepared by reaction of a nucleophile such as a phenol with an appropriate electrophile such as an alkyl bromide. Phenol-benzamides may be prepared by reaction of a benzoate with a nucleophile such as ammonia. Benzoates may be commercially available or may be prepared using methods that are well-known in the literature. Typical processes that may be used to prepare compounds of formula (II) are illustrated in the following scheme:

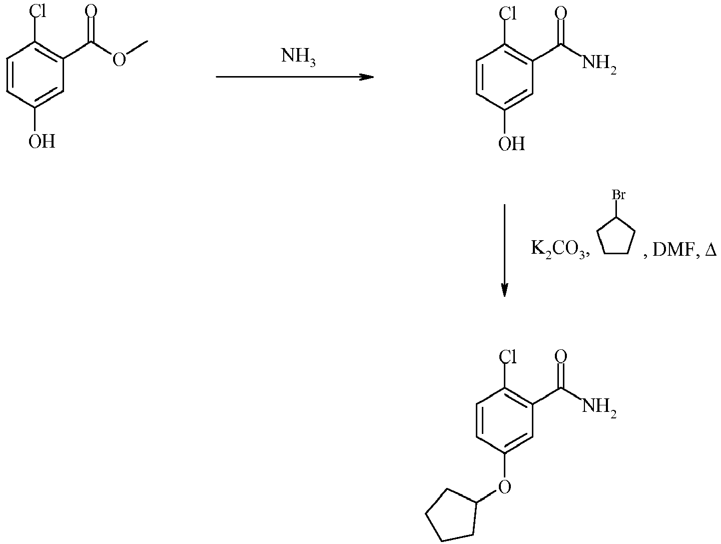

Compounds of formula (II) may also be prepared by hydrogenation of an appropriate alkene such as cyclopentene. A typical process that may be used to prepare compounds of formula (II) is illustrated in the following scheme:

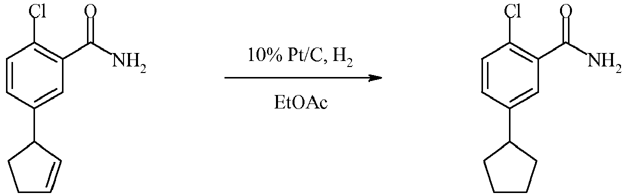

Benzoates may also be prepared by reaction of a nucleophile such as an aryl hydrazine with an appropriate electrophile such as acetylacetaldehyde dimethyl acetal. Aryl hydrazines may be commercially available or may be prepared using methods that are well-known in the literature. A typical process that may be used to prepare benzoates is illustrated in the following scheme:

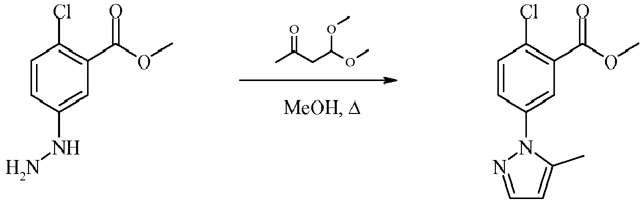

Benzoic acids may also be prepared by reaction of a nucleophile such as an aniline with an appropriate electrophile such as dimethoxy tetrahydrofuran. Anilines may be commercially available or may be prepared using methods that are well-known in the literature. A typical process that may be used to prepare benzoic acids is illustrated in the following scheme:

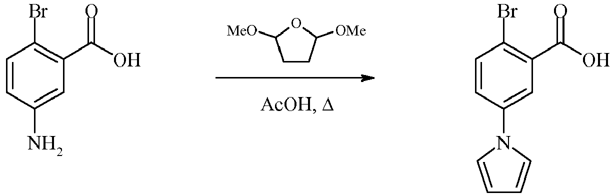

Compounds of formula (III) may be commercially available or may be prepared by oxidation of the corresponding sulfides with an appropriate oxidant such as mCPBA. The sulfides may be prepared by S-alkylation of a thiol with an appropriate electrophile such as a sulfonate. Thiols may be commercially available or may be prepared using methods that are well-known in the literature. Typical processes that may be used to prepare compounds of formula (III) are illustrated in the following scheme:

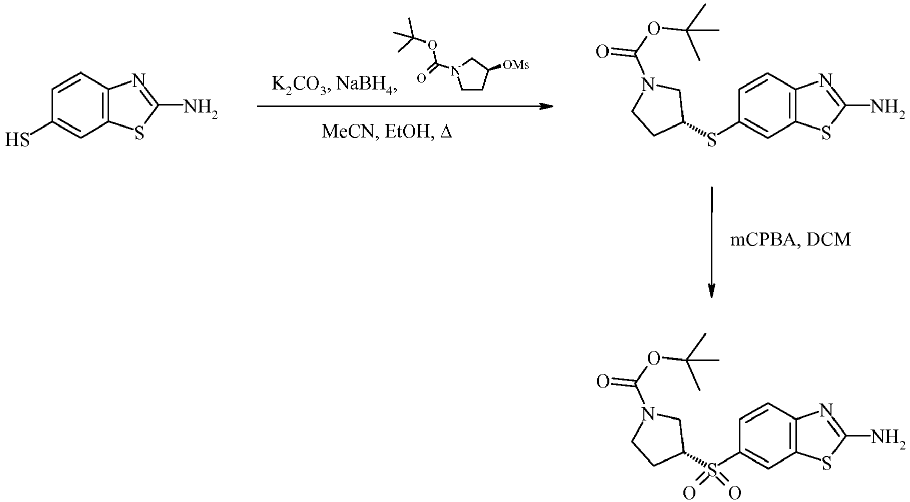

In process (b), an electrophile of formula (IV) can be reacted with an amine. The reaction is generally carried out in an appropriate organic solvent such as MeCN, and at an appropriate temperature, generally between ambient temperature and the boiling point of the solvent. The reaction is normally continued until LCMS analysis indicates that reaction is complete. Typical reaction times are between 2 and 30 minutes. In one embodiment, the solvent is MeCN and the reaction temperature is ambient temperature.

Compounds of formula (IV) may be prepared by the method of process (a) by reaction of a nucleophile such as a benzamide with an appropriate electrophile such as oxalyl chloride to generate an acyl isocyanate, then reaction with a nucleophile such as an amine. The bromo-benzamide may be prepared by reaction of the tolyl-benzamide with a brominating reagent such as NBS. Typical processes that may be used to prepare compounds of formula (IV) are illustrated in the following scheme:

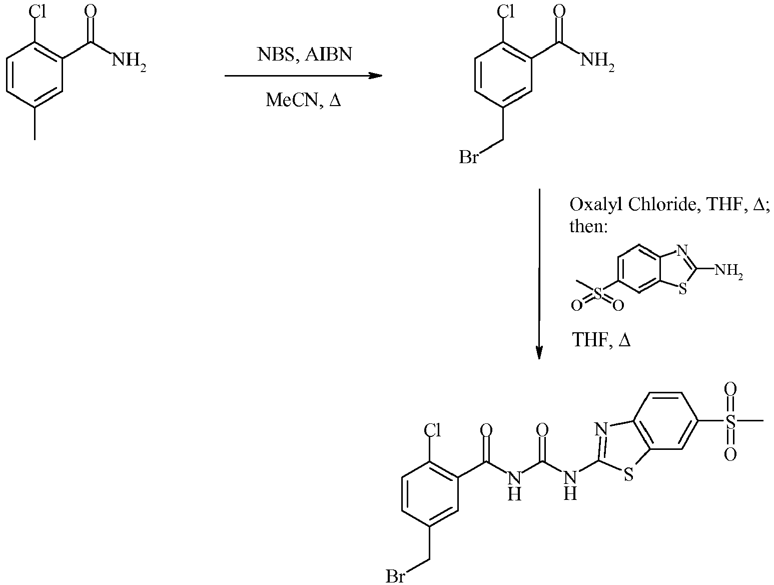

Amines may be commercially available or may be prepared using methods that are well-known in the literature.

In process (c), an electrophile of formula (V) can be reacted with an amine. The reaction is generally carried out in an appropriate organic solvent such as MeCN, and at an appropriate temperature, generally between 80 °C and 150 °C, optionally in a microwave reactor. The reaction is normally continued until LCMS analysis indicates that reaction is complete. Typical reaction times are between 2 and 30 minutes. In one embodiment, the solvent is MeCN and the reaction temperature is ambient temperature.

Compounds of formula (V) may be prepared by the method of process (a) by reaction of a nucleophile such as a benzamide with an appropriate electrophile such as oxalyl chloride to generate an acyl isocyanate, then reaction with a nucleophile such as an amine. The bromo-benzamide may be prepared by O-alkylation of the phenol-benzamide with an appropriate electrophile such as an alkoxyphosphonium salt. Phenol-benzamides may be prepared by reaction of a benzoate with a nucleophile such as ammonia. Typical processes that may be used to prepare compounds of formula (V) are illustrated in the following scheme:

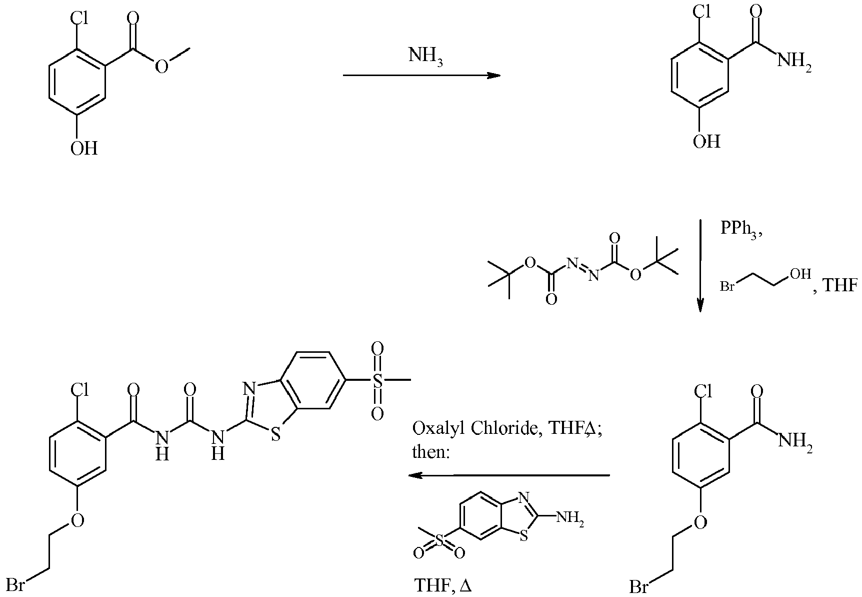

In process (d) a carbamate of formula (VI) can be reacted with a nucleophile of formula (II). The reaction is generally carried out in an appropriate organic solvent such as THF, and in the presence of an appropriate base, such as potassium *tert*-butoxide, at an appropriate temperature, generally between ambient temperature and 150 °C, optionally in a microwave reactor. The reaction is normally continued until LCMS analysis indicates that reaction is complete. Typical reaction times are between 5 minutes and 24 hours. In one embodiment, the solvent is THF, the base is potassium *tert*-butoxide and the reaction temperature is 65 °C.

Compounds of formula (VI) may be prepared by reaction of an amine with an appropriate electrophile such as a chloroformate. A typical process that may be used to prepare compounds of formula (VI) is illustrated in the following scheme:

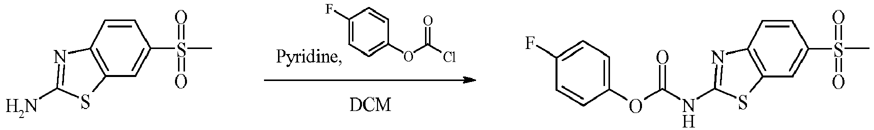

In process (e) a nucleophile of formula (II) can be reacted with an appropriate electrophile such as oxalyl chloride to generate an acyl isocyanate. The acyl isocyanate is reacted with an amine of formula (VII) and then another amine. The reaction is generally carried out in an appropriate organic solvent such as THF, at an appropriate temperature, generally between 50 °C and 150 °C. The reaction is normally continued until LCMS analysis indicates that reaction is complete. Typical reaction times are between 2 minutes and 5 hours. In one embodiment, the solvent is THF and the reaction temperature is 120 °C.

A compound of formula (VII) may be prepared by iodination of the corresponding chloride with an appropriate iodine nucleophile such as sodium iodide. The chloride may be prepared by oxidation of the corresponding sulphide with an appropriate oxidant such as mCPBA. The sulphide may be prepared by S-alkylation of a commercially available thiol with an appropriate electrophile such as an alkyl iodide. Typical processes that may be used to prepare a compound of formula (VII) are illustrated in the following scheme:

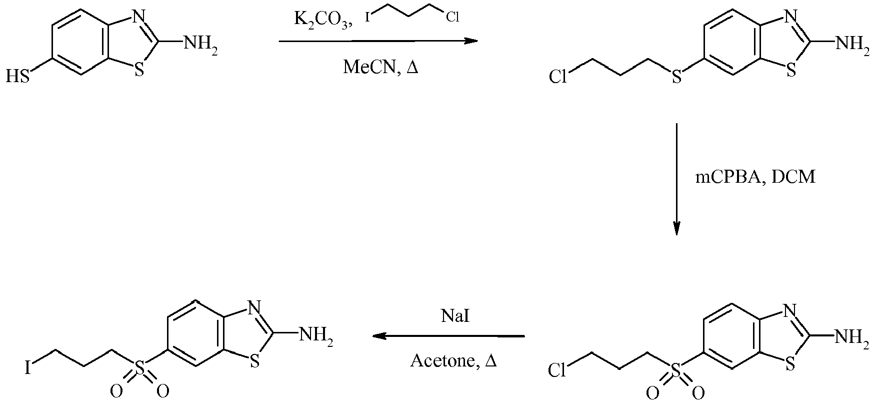

In process (f) a compound of formula (VIII) can be reacted with an amine. The reaction is generally carried out in an appropriate organic solvent such as THF, at an appropriate temperature, generally between ambient temperature and 150 °C, optionally in a microwave reactor. The reaction is normally continued until LCMS analysis indicates that reaction is complete. Typical reaction times are between 2 minutes and 20 hours. In one embodiment, the solvent is THF and the reaction temperature is 120 °C.
Compounds of formula (VIII) may be prepared by the method of process (a) by reaction of a nucleophile of formula (II) with an appropriate electrophile such as oxalyl chloride to generate an acyl isocyanate, then reaction with a nucleophile such as an amine of formula (VII). A typical process used to prepare compounds of formula (VIII) is illustrated in the following scheme:

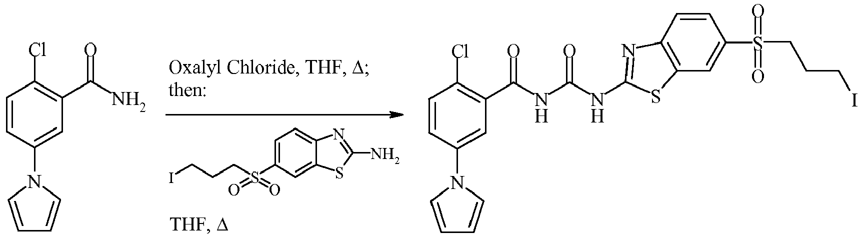

In process (g) a nucleophile of formula (II) can be reacted with an appropriate electrophile such as oxalyl chloride to generate an acyl isocyanate. The acyl isocyanate is reacted with an amine of formula (IX) and then another amine. The reaction is generally carried out in an appropriate organic solvent such as THF, at an appropriate temperature, generally between 80 °C and 150 °C, optionally in a microwave reactor. The reaction is normally continued until LCMS analysis indicates that reaction is complete. Typical reaction times are between 2 and 30 minutes. In one embodiment, the solvent is THF and the reaction temperature is 120 °C.

A compound of formula (IX) may be prepared using methods that are well-known in the literature (WO2002057370).

In process (h) a compound of formula (X) can be reacted with an appropriate nucleophile such as an amine. The reaction is generally carried out in an appropriate organic solvent such as THF, at an appropriate temperature, generally between 80 °C and 150 °C, optionally in a microwave reactor. The reaction is normally continued until LCMS analysis indicates that reaction is complete. Typical reaction times are between 2 and 30 minutes. In one embodiment, the solvent is THF and the reaction temperature is 120 °C. Compounds of formula (X) may be prepared by the method of process (a) by reaction of a nucleophile of formula (II) with an appropriate electrophile such as oxalyl chloride to generate an acyl isocyanate, then reaction with a nucleophile such as an amine of formula (IX). A typical process used to prepare compounds of formula (X) is illustrated in the following scheme:

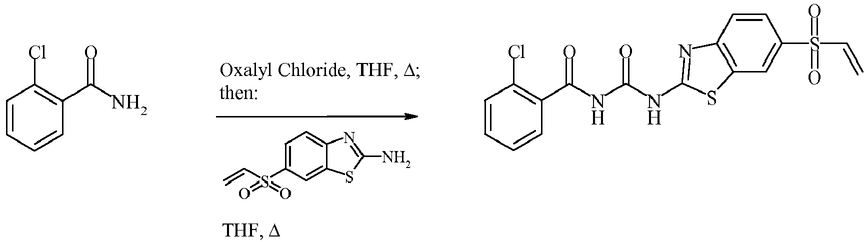

In process (i) an electrophile of formula (X) can be reacted with an alcohol or an alkoxide salt thereof, optionally in the presence of a base. The reaction is generally carried out in an appropriate organic solvent such as THF, at an appropriate temperature, generally between 0 °C and the boiling point of the solvent. Bases that may be used include inorganic bases such as sodium hydride. The reaction is normally continued until LCMS analysis indicates that reaction is complete. Typical reaction times are between 30 minutes and 24 hours. In one embodiment, the solvent is THF, the reaction temperature is 20 °C, and sodium hydride is used as the base.

Alcohols or alkoxide salts thereof are either commercially available or may be prepared using methods that are well-known in the literature.

In process (j) an electrophile of formula (X) can be reacted with a base (or a hydrolysing agent). The reaction is generally carried out in an appropriate organic solvent such as THF and at an appropriate temperature, generally between ambient temperature and 150 °C. The reaction is normally continued until LCMS analysis indicates that reaction is complete. Typical reaction times are between 2 and 20 hours. In one embodiment, the base (or hydrolysing agent) is Triton B, the solvent is THF and the reaction temperature is ambient temperature.

Bases (or a hydrolysing agents) are either commercially available or may be prepared using methods that are well-known in the literature.

In process (k) a compound of formula (XII) can be reacted with copper(I) iodide and a ligand, optionally in the presence of an additive. The reaction is generally carried out in an appropriate organic solvent such as dioxane and at an appropriate temperature, generally between 80 and 150 °C. The reaction is normally continued until LCMS analysis indicates that reaction is complete. Typical reaction times are between 2 and 24 hours. In one embodiment, the ligand is N,N'-dimethylethylenediamine, the additive is sodium iodide, the solvent is dioxane and the reaction temperature is 100 °C.

Compounds of formula (XI) are commercially available or may be prepared by methods known to those skilled in the art.

Compounds of formula (XII) may be prepared by the method of process (g) by reaction of a nucleophile of formula (II) with an appropriate electrophile such as oxalyl chloride to generate an acyl isocyanate. The acyl isocyanate is reacted with an amine of formula (IX) and then another amine. A typical process used to prepare compounds of formula (XII) is illustrated in the following scheme:

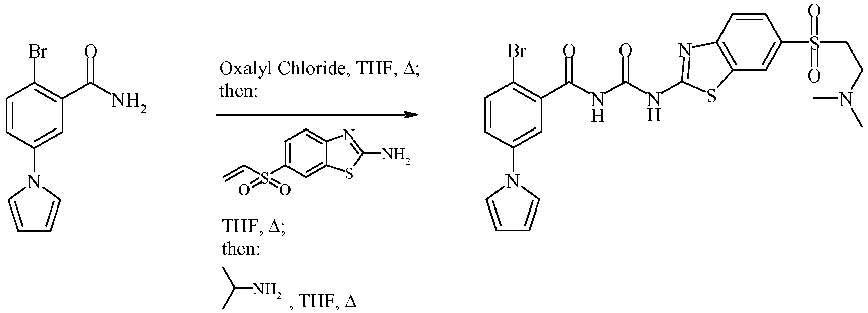

It will be appreciated by those skilled in the art that the above reactions may be performed in a different sequence if so desired and that certain reagents may require that certain substituents are protected before a particular process is carried out and then deprotected at a suitable subsequent stage, particularly amine groups. It will also be appreciated by those skilled in the art that certain compounds of formula I may be prepared by reacting another compound of formula I by functional group modifications known to those skilled in the art. For example amines may be acylated to give amides or amides may be hydrolysed to give amines. Primary and secondary amines may be alkylated to give secondary and tertiary amines, respectively, for example using reductive alkylation. A further example is the reduction of alkyne groups to alkanes.

Certain compounds of formulae II, II, IV, V, VI, VII, VIII, X and XII are believed to be novel and are claimed herein as another aspect of the present invention.

### Pharmaceutical preparations

The compounds of the invention will normally be administered via the oral, parenteral, intravenous, intramuscular, subcutaneous or in other injectable ways, buccal, rectal, vaginal, transdermal and/or nasal route and/or via inhalation, in the form of pharmaceutical preparations comprising the active ingredient or a pharmaceutically acceptable addition salt, in a pharmaceutically acceptable dosage form. Depending upon the disorder and patient to be treated and the route of administration, the compositions may be administered at varying doses.

Suitable daily doses of the compounds of the invention in the therapeutic treatment of humans are about 0.001-10 mg/kg body weight, preferably 0.01-1 mg/kg body weight. Oral formulations are preferred particularly tablets or capsules which may be formulated by methods known to those skilled in the art to provide doses of the active compound in the range of 0.5mg to 500mg for example 1 mg, 3 mg, 5 mg, 10 mg, 25mg, 50mg, 100mg and 250mg.

According to a further aspect of the invention there is also provided a pharmaceutical formulation including any of the compounds of the invention, or pharmaceutically acceptable derivatives thereof, in admixture with pharmaceutically acceptable adjuvants, diluents and/or carriers.

According to a further aspect there is provided a pharmaceutical formulation comprising a compound of formula I or pharmaceutically acceptable salt thereof, in admixture with pharmaceutically acceptable adjuvants, diluents and/or carriers for use in the treatment of obesity or type 2 diabetes.

### Pharmacological properties

The compounds of formula (I) are Ghrelin receptor modulators, including agonists, antagonists and partial agonists. In another aspect the present invention provides a compound of formula I as previously defined for use as a medicament, and in particular a medicament for regulating food intake, body weight or energy homeostasis. In a further aspect the present invention provides a method of regulating food intake comprising administering a compound of formula I to a mammal, particularly a human, in need thereof.

In particular the compounds of formula (I) are useful for the treatment of obesity or being overweight, for the prevention of weight gain, for the modulation of appetite and/or satiety, eating disorders, for the treatment of diabetes, for the treatment of metabolic syndrome, for the treatment of the Prader-Willi syndrome, for the treatment of cachexia resulting from cancer or congestive heart failure, for the treatment of wasting due to ageing, AIDS, chronic liver failure or chronic obstructive pulmonary disease (COPD). Compounds of formula (I) are particularly useful for the treatment of obesity or diabetes, particularly type 2 diabetes. In another aspect the present invention provides a compound of formula I or a pharmaceutically acceptable salt thereof for the treatment of obesity or type 2 diabetes. In another aspect the present invention provides a compound of formula I or a pharmaceutically acceptable salt thereof for the treatment obesity or being overweight, prevention of weight gain, for modulation of appetite and/or satiety, eating disorders and the treatment of diabetes mellitus.

In addition the compounds of formula (I) may also be useful for the treatment of inflammatory conditions, cardiac dysfunction, Alzheimer's disease, post-operative ileus and gastroparesis.

The term eating disorders includes amongst others binge eating, anorexia, bulimia and compulsive eating disorders.

The compounds of formula (I) that are Ghrelin receptor antagonists are useful for the treatment of obesity or being overweight, (e.g., promotion of weight loss and maintenance of weight loss), for the prevention of weight gain (e.g., medication-induced or subsequent to cessation of smoking), for modulation of appetite and/or satiety, for treating eating disorders (e.g. binge eating, bulimia and compulsive eating) and for the treatment of diabetes mellitus.

In a further aspect the present invention provides the use of a Ghrelin receptor antagonist of formula I in the preparation of a medicament for the treatment or prophylaxis of obesity or being overweight, (e.g., promotion of weight loss and maintenance of weight loss), prevention of weight gain (e.g., medication-induced or subsequent to cessation of smoking), for modulation of appetite and/or satiety, eating disorders (e.g. binge eating, anorexia, and compulsive eating) or type 2 diabetes.

In a still further aspect the present invention provides a method of treating obesity or being overweight, (e.g., promotion of weight loss and maintenance of weight loss), prevention of weight gain (e.g., medication-induced or subsequent to cessation of smoking), for modulation of appetite and/or satiety, eating disorders (e.g. binge eating, bulimia and compulsive eating) comprising administering a pharmacologically effective amount of a Ghrelin receptor antagonist of formula I to a patient in need thereof.

In a further aspect the present invention provides the use of a Ghrelin receptor agonist of formula I for the treatment of cachexia resulting from for example: cancer; congestive heart failure; chronic renal failure; infection or autoimmune disease, for the treatment of wasting due to ageing, AIDS, chronic liver failure or chronic obstructive pulmonary disease (COPD).

In a still further aspect the present invention provides a method of treating cachexia resulting from cancer or congestive heart failure, for the treatment of wasting due to ageing, AIDS, chronic liver failure or chronic obstructive pulmonary disease (COPD). comprising administering a pharmacologically effective amount of a Ghrelin receptor agonist of formula I to a patient in need thereof.

### Combination Therapy

A compound of the invention may be combined with another therapeutic agent that is useful in the treatment of obesity and/ or diabetes such as other anti-obesity drugs, that affect energy expenditure, glycolysis, gluconeogenesis, glucogenolysis, lipolysis, lipogenesis, fat absorption, fat storage, fat excretion, hunger and/or satiety and/or craving mechanisms, appetite/motivation, food intake, or G-I motility.

A compound of the invention may be combined with another therapeutic agent that is useful in the treatment of disorders associated with obesity such as hypertension, hyperlipidaemias, dyslipidaemias, diabetes, sleep apnea, asthma, heart disorders, atherosclerosis, macro and micro vascular diseases, liver steatosis, cancer, joint disorders, and gallbladder disorders. For example, a compound of the present invention may be used in combination with another therapeutic agent that lowers blood pressure or that decreases the ratio of LDL:HDL or an agent that causes a decrease in circulating levels of LDL-cholesterol. In patients with diabetes mellitus the compounds of the invention may also be combined with therapeutic agents used to treat complications related to micro-angiopathies.

A compound of the invention may be used alongside other therapies for the treatment of obesity and its associated complications, the metabolic syndrome and type 2 diabetes. These include biguanide drugs (for example Metformin), insulin (synthetic insulin analogues) oral antihyperglycemics (these are divided into prandial glucose regulators and alpha-glucosidase inhibitors) and sulfonylureas for example: glimepiride, glibenclamide (glyburide), gliclazide, glipizide, gliquidone, chloropropamide, tolbutamide, acetohexamide, glycopyramide, carbutamide, glibonuride, glisoxepid, glybuthiazole, glibuzole, glyhexamide, glymidine, glypinamide, phenbutamide, tolcylamide and tolazamide. Preferably the sulfonylurea is glimepiride or glibenclamide (glyburide). More preferably the sulfonylurea is glimepiride.

In another aspect of the invention, the compound of formula I, or a pharmaceutically acceptable salt thereof may be administered in association with a PPAR modulating agent for example pioglitazone or rosiglitazone. PPAR modulating agents include but are not limited to a PPAR alpha and/or gamma agonist, or pharmaceutically acceptable salts, solvates, solvates of such salts or prodrugs thereof. Suitable PPAR alpha and/or gamma agonists, pharmaceutically acceptable salts, solvates, solvates of such salts or prodrugs thereof are well known in the art.

In addition the combination of the invention may be used in conjunction with a sulfonylurea. The present invention also includes a compound of the present invention in combination with a cholesterol-lowering agent. The cholesterol-lowering agents referred to in this application include but are not limited to inhibitors of HMG-CoA reductase (3-hydroxy-3-methylglutaryl coenzyme A reductase). Suitably the HMG-CoA reductase inhibitor is a statin.

In the present application, the term "cholesterol-lowering agent" also includes chemical modifications of the HMG-CoA reductase inhibitors, such as esters, prodrugs and metabolites, whether active or inactive.

The present invention also includes a compound of the present invention in combination with an inhibitor of the ileal bile acid transport system (IBAT inhibitor). The present invention also includes a compound of the present invention in combination with a bile acid binding resin.

The present invention also includes a compound of the present invention in combination with a bile acid sequestering agent, for example colestipol or cholestyramine or cholestagel.

According to an additional further aspect of the present invention there is provided a combination treatment comprising the administration of an effective amount of a compound of the formula I, or a pharmaceutically acceptable salt thereof, optionally together with a pharmaceutically acceptable diluent or carrier, with the simultaneous, sequential or separate administration one or more of the following agents selected from:
a CETP (cholesteryl ester transfer protein) inhibitor;
a cholesterol absorption antagonist;
a MTP (microsomal transfer protein) inhibitor ;
a nicotinic acid derivative, including slow release and combination products;
a phytosterol compound;
probucol;
an anti-coagulant;
an omega-3 fatty acid ;
another anti-obesity compound for example sibutramine, phentermine, orlistat, bupropion, ephedrine, thyroxine;
an antihypertensive compound for example an angiotensin converting enzyme (ACE) inhibitor, an angiotensin II receptor antagonist, an adrenergic blocker, an alpha adrenergic blocker, a beta adrenergic blocker, a mixed alpha/beta adrenergic blocker, an adrenergic stimulant, calcium channel blocker, an AT-1 blocker, a saluretic, a diuretic or a vasodilator;
a CB1 receptor antagonist /inverse agonist;
a melanin concentrating hormone (MCH) modulator;
a melanocortin-4 receptor agonist;
an NPY receptor modulator;
an orexin receptor modulator;
a diacylglycerol acyltransferase-1 inhibitor;
a diacylglycerol acyltransferase-2 inhibitor;
a phosphoinositide-dependent protein kinase (PDK) modulator; or
modulators of nuclear receptors for example LXR, FXR, RXR, GR, ERRα, β, PPARα, β, γ and RORalpha;
a monoamine transmission-modulating agent, for example a selective serotonin reuptake inhibitor (SSRI), a noradrenaline reuptake inhibitor (NARI), a noradrenaline-serotonin reuptake inhibitor (SNRI), a monoamine oxidase inhibitor (MAOI), a tricyclic antidepressive agent (TCA), a noradrenergic and specific serotonergic antidepressant (NaSSA);
an antipsychotic agent for example olanzapine and clozapine;
a serotonin receptor modulator;
a leptin/leptin receptor modulator;
a ghrelin/ghrelin receptor modulator;
a DPP-IV inhibitor for example Saxagliptin, Sitagliptin, Vildagliptin or Alogliptin;
an SGLT-2 inhibitor for example Dapagliflozin;
a GLK activator;
or a pharmaceutically acceptable salt, solvate, solvate of such a salt or a prodrug thereof,
optionally together with a pharmaceutically acceptable diluent or carrier to a warm-blooded animal, such as man in need of such therapeutic treatment.

According to an additional further aspect of the present invention there is provided a combination treatment comprising the administration of an effective amount of a compound of the formula I, or a pharmaceutically acceptable salt thereof, optionally together with a pharmaceutically acceptable diluent or carrier, with the simultaneous, sequential or separate administration of very low calorie diets (VLCD) or low-calorie diets (LCD).

Therefore in an additional feature of the invention, there is provided a method for the treatment of obesity and its associated complications in a warm-blooded animal, such as man, in need of such treatment which comprises administering to said animal an effective amount of a compound of formula I, or a pharmaceutically acceptable salt thereof in simultaneous, sequential or separate administration with an effective amount of a compound from one of the other classes of compounds described in this combination section, or a pharmaceutically acceptable salt, solvate, solvate of such a salt or a prodrug thereof.

According to a further aspect of the invention there is provided a pharmaceutical composition which comprises a compound of formula I, or a pharmaceutically acceptable salt thereof, and a compound from one of the other classes of compounds described in this combination section or a pharmaceutically acceptable salt, solvate, solvate of such a salt or a prodrug thereof, in association with a pharmaceutically acceptable diluent or carrier.

According to a further aspect of the present invention there is provided a kit comprising a compound of formula I, or a pharmaceutically acceptable salt thereof, and a compound from one of the other classes of compounds described in this combination section or a pharmaceutically acceptable salt, solvate, solvate of such a salt or a prodrug thereof.

According to a further aspect of the present invention there is provided a kit comprising:
a) a compound of formula I, or a pharmaceutically acceptable salt thereof, in a first unit dosage form;
b) a compound from one of the other classes of compounds described in this combination section or a pharmaceutically acceptable salt, solvate, solvate of such a salt or a prodrug thereof; in a second unit dosage form; and
c) container means for containing said first and second dosage forms.

According to a further aspect of the present invention there is provided a kit comprising:
a) a compound of formula I, or a pharmaceutically acceptable salt thereof, together with a pharmaceutically acceptable diluent or carrier, in a first unit dosage form;
b) a compound from one of the other classes of compounds described in this combination section or a pharmaceutically acceptable salt, solvate, solvate of such a salt or a prodrug thereof, in a second unit dosage form; and
c) container means for containing said first and second dosage forms.

According to another feature of the invention there is provided the use of a compound of the formula I, or a pharmaceutically acceptable salt thereof, and one of the other compounds described in this combination section, or a pharmaceutically acceptable salt, solvate, solvate of such a salt or a prodrug thereof, in the manufacture of a medicament for use in the the treatment of obesity and its associated complications in a warm-blooded animal, such as man..

It will be understood that there are medically accepted definitions of obesity and being overweight. A patient may be identified by, for example, measuring body mass index (BMI), which is calculated by dividing weight in kilograms by height in metres squared, and comparing the result with the definitions.

### Pharmacological Activity

The compounds of the present invention are Growth Hormone Secretagogue-Receptor (GHS-R) modulators. The activity of the compounds of the invention was demonstrated using the following assay.

Ghrelin receptor agonist/antagonist Calcium mobilisation/Flux Assay (FLIPR) HEK 293s cells expressing human GHS receptor (In-house) were plated in black 384 poly-D-lysine plates with clear bottom (Greiner) and cultured to confluency overnight in plating media (UltraMDCK Cambrex) 37°C in a humidified cell incubator containing 5% CO₂. Growth media was aspirated and replaced with 28µl of loading buffer Dulbecco's phosphate-buffered saline (DPBS) containing 0.02M HEPES, 0.01% BSA (fatty acid free), 2.5mM Probenecid (Sigma) with calcium 3 (Molecular Devices) and Fluo-4/AM (Invitrogen) for 1 hour in cell incubator containing 5% CO₂.

Cell plates were then transferred to the FLIPR Tetra (Molecular Devices). Compound additions were 15µl in duplicate at 3 times final concentration in DPBS containing 0.02M HEPES, 0.01% BSA (fatty acid free), 2.5mM Probenecid and 1.5% DMSO. Fluorescence emissions from 384 wells were measured simultaneously at excitation and emission wavelength of 470 and 515 nm, respectively for 5 minutes in 1-second intervals. During this time agonist responses, if any, were recorded in the absence of ghrelin. Next 15µl at 4 times concentrated ghrelin IC80 (Tocris) solution in DPBS containing 0.02M HEPES, 0.01 % BSA (fatty acid free), 2.5mM Probenecid were added. Fluorescence emissions were measured for another 5 minutes as above. During this time the antagonist effects of compounds on ghrelin-stimulated calcium flux were recorded and expressed as % inhibition of ghrelin response (IC80). Sigmoidal curves were fitted by Origin 7.5 Client software and IC50 values determined. In addition, the agonist effects of the compounds could also be obtained and expressed as % maximal ghrelin response (100 nM). Sigmoidal curves were fitted by Origin 7.5 Client software and EC₅₀ values determined.

The compounds of the present invention were found to inhibit the activation of ghrelin receptor with IC₅₀s in a range of about 0.001µM to about 10µM in the FLIPR assays. In a preferred range, the compounds inhibit the activation of ghrelin receptor with IC₅₀s in a range of about 0.001µM to about 1.0µM. In a more preferred range, the compounds inhibit the activation of ghrelin receptor with IC₅₀ₛ in a range of about 0.001µM to about 0.1µM.

The results obtained by testing the compounds of the Examples in this assay are shown in Table1.

### Protocol for Ghrelin IP1 Agonist Assay

In HEK293s cells stably expressing human GHS receptor (In-house), agonist activity was assessed by measuring intracellular myo-Inositol 1 phosphate (IP1) using the IP1 HTRF assay kit (Cisbio International).

Test compound (14µl) plated into white 384-well plates (Matrix) in stimulation buffer (10mM HEPES, 1mM CaCl₂, 0.5mM MgCl₂, 4.2mM KCl, 146mM NaCl, 5.5mM glucose , 50mM LiCl pH to 7.4) containing 2% DMSO.

Cells grown in standard TC flasks to 80% confluency in DMEM (Sigma) containing 10% FCS, 1% penicillin/streptomycin/ glutamine and 50mg/ml gentacin at 37°C in 5% CO₂/95% air. Cells were harvested using accutase (Sigma), spun down at 1000rpm for 5 minutes and resuspended in stimulation buffer (as above) containing 0.02% fatty acid free BSA at a density of 1.43E6cells/ml.

Cell suspension (14µl) added to the compound plate and incubated for 2 hours at 37°C in 5% CO₂/95% air. Following lysis of cells using IP1-2 (6µl) and anti-IP1 (6µl) crypate provided with the kit for 1 hour at room temperature, fluorescence was measured on the Envision (Perkin Elmer) using HTRF setting (665nm/620nm). Agonist effect of the compound was expressed as % maximal effect of GHRP-6 (5µM: Bachem). Sigmoidal curves were fitted by Origin 7.5 Client and EC₅₀ values determined. The results obtained by testing the compounds of the Examples in this assay are shown in Table 1.

Compounds were considered to be antagonists if hBindingIC₅₀<5µM and
IP-One <20% effect and the ratio IP-One EC₅₀ hBindingIC₅₀ >50

**Table 1**

| Example No. | h bind IC50 (µM) | h IP1 ag EC50 (µM) | h IP1 ag % |
|---|---|---|---|
| 1 | 0.0644 | 0.02922 | 24.01 |
| 2 | 0.0133 | 0.01765 | 40.76 |
| 3 | 1.1232 | | · |
| 4 | 0.0773 | 0.009463 | 58.46 |
| 5 | 0.4025 | | · |
| 6 | 0.0444 | 0.04801 | 32.59 |
| 7 | 0.6704 | | · |
| 8 | 0.0696 | 0.03636 | 39.85 |
| 9 | 0.2474 | | · |
| 10 | 0.0273 | 0.01892 | 44.09 |
| 11 | 0.3472 | 0.005271 | 38.08 |
| 12 | 0.1786 | | · |
| 13 | 0.1623 | 0.05547 | 52.47 |
| 14 | 0.3213 | 0.1656 | 47.6 |
| 15 | 1.0754 | | · |
| 16 | 0.1 | 0.06444 | 34.14 |
| 17 | 0.0804 | 0.006032 | 42.07 |
| 18 | 0.2524 | 0.05362 | 47.05 |
| 19 | 1.054 | | · |
| 20 | 0.1839 | | · |
| 21 | 1.6775 | 0.2206 | 16.85 |
| 22 | 0.1842 | | · |
| 23 | 0.0827 | 0.01028 | 44.69 |
| 24 | 0.0253 | 0.006435 | 53.97 |
| 25 | 0.1892 | 0.01377 | 34.72 |
| 26 | 0.4281 | 0.6226 | 26.09 |
| 27 | 0.1146 | 0.02308 | 46.72 |
| 28 | 0.043 | 0.03366 | 44.58 |
| 29 | 0.0609 | | · |
| 30 | 0.2514 | 0.03924 | 23.83 |
| 31 | 0.0546 | | · |
| 32 | 0.4004 | 0.309 | 27.97 |
| 33 | 0.0885 | 0.02494 | 41.54 |
| 34 | 0.0993 | 0.01113 | 46.72 |
| 35 | 0.0715 | 0.01045 | 56.07 |
| 36 | 0.3047 | 0.009549 | 34.32 |
| 37 | 0.2343 | 0.0611 | 24.02 |
| 38 | 0.1792 | 0.01473 | 40.05 |
| 39 | 0.1792 | | · |
| 40 | 1.8222 | | · |
| 41 | 0.5349 | 0.09577 | 18.15 |
| 42 | 0.1768 | | · |
| 43 | 0.0517 | 0.04216 | 45.96 |
| 44 | 0.0182 | 0.005726 | 49.05 |
| 45 | 0.0214 | 25 | 0.9458 |
| 46 | 0.012 | 8.244 | 0.5 |
| 47 | 0.0816 | 25 | 0 |
| 48 | 0.0013 | 25 | 0.3196 |
| 49 | 0.0129 | 25 | 0 |
| 50 | 0.001 | 25 | 0 |
| 51 | 0.0142 | 0.01601 | 1 |
| 52 | 0.0037 | 25 | 0 |
| 53 | 0.01 | 25 | 0 |
| 54 | 0.0101 | 25 | 0.05273 |
| 55 | 0.0231 | 0.005697 | 48.89 |
| 56 | 0.0101 | 25 | 10.83 |
| 57 | 0.1616 | >25 | 0 |
| 58 | 1.3064 | 3.005 | 8.816 |
| 59 | 0.207 | >9.298 | 0.377 |
| 60 | 0.033 | >25 | 0.6468 |
| 61 | 0.1608 | >25 | 0.2758 |
| 62 | 1.7445 | 25 | 9.065 |
| 63 | 2.7675 | 0.06145 | 31.75 |
| 64 | 0.139 | 0.019 | 8.739 |
| 65 | 0.2721 | 0.08104 | 8.226 |
| 66 | 0.0231 | 25 | 0 |
| 67 | 0.5359 | >20 | 0 |
| 68 | 0.0441 | >25 | 0 |
| 69 | 0.0374 | 3.006 | 14.05 |
| 70 | 0.1406 | 0.3888 | 0.5 |
| 71 | 0.0004 | 25 | 0.04691 |
| 72 | 0.0003 | 6.068 | 6.601 |
| 73 | 0.0013 | 25 | 2.251 |
| 74 | 0.001 | 25 | 5.412 |
| 75 | 0.0074 | | · |
| 76 | 0.0114 | | · |
| 77 | 0.0017 | 25 | 0.9112 |
| 78 | 0.0008 | 4.221 | 0.8098 |
| 79 | 0.0006 | 25 | 1.95 |
| 80 | 0.0022 | 25 | 9.765 |
| 81 | 0.002 | 25 | 7.158 |
| 82 | 0.0016 | 0.62 | 0.8246 |
| 83 | 0.0013 | 25 | 0 |
| 84 | 0.0071 | | · |
| 85 | 0.0002 | 25 | 0.1803 |
| 86 | 0.0007 | 25 | 0.6398 |
| 87 | 0.0002 | 6.56 | 0.6309 |
| 88 | 0.0007 | 25 | 4.42 |
| 89 | 0.0011 | 13.47 | 3.034 |
| 90 | 0.0007 | >25 | 0 |
| 91 | 0.0005 | 4.821 | 0.5 |
| 92 | 0.0101 | >4.867 | 4.985 |
| 93 | 0.0017 | 0.0301 | 3.9 |
| 94 | 0.0003 | 0.007243 | 18.14 |
| 95 | 0.0107 | 1.159 | 9.482 |
| 96 | 0.0029 | 0.4325 | 13.08 |
| 97 | 0.0042 | 0.004282 | 8.134 |
| 98 | 0.0087 | 0.001501 | 9.188 |
| 99 | 0.0159 | 2.181 | 1.739 |
| 100 | 0.0273 | 20 | 0 |
| 101 | 0.2841 | 0.2061 | 7.887 |
| 102 | 0.1164 | 0.05776 | 24.19 |
| 103 | 0.2395 | 0.4541 | 20.46 |
| 104 | 0.7422 | 0.7724 | 15.06 |
| 105 | 0.2147 | 0.09858 | 39.35 |
| 106 | 0.2157 | 0.03281 | 12.9 |
| 107 | 0.3059 | 0.02757 | 19.3 |
| 108 | 1.6404 | 24.29 | 8.693 |
| 110 | 0.3915 | 0.09169 | 15.82 |
| 111 | 1.273 | 0.3354 | 14.13 |
| 112 | | 0.1424 | 38.44 |
| 113 | 1.6573 | 1.292 | 10.79 |
| 115 | 0.0634 | 2.031 | 9.953 |
| 116 | 0.036 | 0.4002 | 45.14 |
| 117 | 0.0378 | 0.01587 | 16.75 |
| 118 | 0.464 | 0.1137 | 9.594 |
| 119 | 0.0243 | 15.72 | 9.262 |
| 120 | | 0.2111 | 29.49 |
| 121 | 0.4003 | | |
| 123 | 0.1199 | 0.0545 | 31.18 |
| 124 | 0.1351 | 0.5238 | 54.5 |
| 125 | | 0.2522 | 31.82 |
| 126 | | 1.029 | 38.41 |
| 127 | 0.0561 | 0.3195 | 36.65 |
| 128 | 0.1 | 0.2187 | 17.33 |
| 129 | 0.2195 | | |
| 130 | 0.288 | 0.6676 | 31.15 |
| 131 | 0.2285 | 0.1905 | 17.51 |
| 132 | 0.6535 | 3.677 | 15.49 |
| 133 | 0.0215 | 11.04 | 41.67 |
| 134 | 0.2181 | 0.0133 | 63.89 |
| 135 | 2.2164 | 3.676 | 18.23 |
| 136 | 0.1971 | 0.2165 | 21.54 |
| 137 | 1.7509 | 2.322 | 24.05 |
| 138 | 0.448 | 25 | 0 |
| 139 | 0.1073 | 0.02732 | 46.71 |
| 140 | 0.2894 | 0.06203 | 42.53 |
| 141 | 0.1111 | 0.1836 | 22.67 |
| 142 | 0.8514 | 0.03508 | 24.51 |
| 143 | 0.4844 | | |
| 144 | 0.2008 | | |
| 145 | 0.0598 | | |
| 146 | 0.2686 | 0.03 | 40.7 |
| 147 | 0.0788 | | |
| 148 | 0.8303 | 0.05288 | 17.69 |
| 149 | 0.0522 | | |
| 150 | 0.0527 | 0.03089 | 33.37 |
| 151 | 0.0108 | 0.002226 | 74.3 |
| 152 | 0.0191 | 0.02394 | 46.57 |
| 153 | 0.0125 | 0.01086 | 47.03 |
| 154 | 0.1174 | | |
| 155 | 0.0012 | 0.002251 | 55.17 |
| 156 | 0.064 | | |
| 157 | 0.0785 | | |
| 158 | 0.1035 | | |
| 159 | 0.0085 | 0.01765 | 51.65 |
| 160 | 0.0296 | | |
| 161 | 0.1055 | 0.006183 | 56 |
| 162 | 0.2959 | 0.01135 | 48.28 |
| 163 | 0.6132 | | |
| 164 | 0.1738 | 0.07022 | 43.91 |
| 165 | 0.1273 | 0.06709 | 49.4 |
| 166 | 0.2796 | 0.04635 | 53.92 |
| 167 | 0.1094 | | |
| 168 | 0.0206 | 0.004129 | 36.99 |
| 169 | 0.1512 | | |
| 170 | 0.8311 | 0.07566 | 9.733 |
| 171 | 0.2958 | 0.01856 | 43.97 |
| 172 | 0.0283 | 0.007312 | 44.35 |
| 173 | 0.0762 | 0.008429 | 52.22 |
| 174 | 0.1394 | | |
| 175 | 0.0641 | 0.02239 | 30.15 |
| 176 | 0.0132 | | |
| 177 | 0.2479 | 0.06641 | 22.78 |
| 178 | 0.1075 | 0.07771 | 18.69 |
| 179 | 0.0183 | 0.00591 | 61.23 |
| 180 | 0.1268 | 0.06728 | 5.932 |
| 181 | 0.049 | 0.0006329 | 54.37 |
| 182 | 0.0089 | 0.02513 | 39.59 |
| 183 | 0.0067 | 0.01376 | 42.06 |
| 184 | 0.0337 | | |
| 185 | 0.0669 | 0.06923 | 39.7 |
| 186 | 0.0341 | | |
| 187 | 0.0581 | 0.005954 | 16.05 |
| 188 | 0.0538 | 0.05208 | 16.42 |
| 189 | 0.2741 | 0.2799 | 20.37 |
| 190 | 0.0519 | 0.0822 | 21.27 |
| 191 | 0.1282 | 0.07524 | 15.54 |
| 192 | 0.0524 | 0.05127 | 23.81 |
| 193 | 0.0259 | 0.0185 | 30.6 |
| 194 | 0.1722 | 0.009521 | 39.42 |
| 195 | 0.1144 | 0.03295 | 22.84 |
| 196 | 0.0015 | 0.0009345 | 79.44 |
| 197 | 0.0004 | 0.002016 | 55.82 |
| 198 | 0.0108 | 0.01096 | 32.1 |
| 199 | 0.0022 | 0.001823 | 42.21 |
| 200 | 0.0027 | 0.004529 | 41.36 |
| 201 | 0.0009 | 0.004539 | 29.74 |
| 202 | 0.0087 | 0.01338 | 51.28 |
| 203 | 0.0149 | 0.006222 | 29.41 |
| 204 | 0.0012 | 0.006923 | 36.25 |
| 205 | 0.0053 | 0.001998 | 39.55 |
| 206 | 0.0144 | 0.009578 | 69.05 |
| 208 | 0.0181 | 0.01352 | 30.66 |
| 209 | 0.0124 | 0.01537 | 42.25 |
| 210 | 0.0022 | 0.002262 | 51.47 |
| 211 | 0.0536 | 0.009579 | 27.69 |
| 212 | 0.0005 | 0.002033 | 27.61 |
| 213 | 0.2379 | 0.01248 | 12.93 |
| 214 | 0.0326 | 0.01241 | 52.14 |
| 215 | 0.0272 | 0.02299 | 50.2 |
| 216 | 0.0368 | 0.05399 | 64.64 |
| 217 | 0.0704 | 0.1179 | 36.86 |
| 218 | 0.0223 | 0.01849 | 39.17 |
| 219 | 0.0178 | 0.02931 | 64.53 |
| 220 | 0.0028 | 0.00401 | 21.39 |
| 221 | 0.0154 | 0.01873 | 65.02 |
| 222 | 0.0186 | 0.04305 | 58.25 |
| 223 | 0.0958 | 0.06214 | 24.76 |
| 224 | 0.08784 | 0.1394 | 24.33 |
| 225 | 0.06029 | 0.2239 | 27.83 |
| 226 | 0.00005 | 0.002192 | 22.28 |
| 227 | 3.78892 | 20 | 10.59 |
| 228 | 1.2385 | 1.372 | 9.894 |
| 229 | 1.9699 | 0.1167 | 18.18 |
| 230 | 0.0193 | 0.04652 | 12.57 |
| 231 | 0.06728 | 0.0721 | 15.69 |
| 232 | 0.03138 | 0.05605 | 12.61 |
| 233 | 0.03915 | 20 | 10.43 |
| 234 | 0.00382 | 20 | 6.733 |
| 235 | 0.02266 | 20 | 19.55 |
| 236 | 4.08984 | 20 | 3.722 |
| 237 | 0.0007 | 20 | 0 |
| 238 | 0.00047 | 0.04342 | 9.982 |
| 239 | 0.14967 | 20 | 0 |
| 240 | 0.00883 | 0.02809 | 80.91 |
| 241 | 0.00031 | 20 | 0 |
| 242 | 0.00062 | 20 | 9.319 |
| 243 | 0.01427 | 0.009344 | 7.671 |
| 244 | 2.06372 | 19.66 | 22.55 |
| 245 | 0.00652 | 20 | 0 |
| 246 | 0.00208 | 20 | 0 |
| 247 | 0.45246 | 20 | 0 |
| 248 | 0.059 | 0.3475 | 3.071 |
| 249 | 8.07676 | 4.899 | 3.129 |
| 250 | 3.78491 | 5.134 | 6.819 |
| 251 | 0.03435 | 20 | 0 |
| 252 | 0.01867 | 0.02774 | 1 |
| 253 | 7.59922 | 4.972 | 5.845 |
| 254 | 0.00569 | 20 | 0 |
| 255 | 0.00791 | 20 | 80.94 |
| 256 | 0.05889 | 20 | 0 |
| 257 | 0.84584 | 20 | 13.14 |
| 258 | 0.01054 | 3.339 | 2.451 |
| 259 | 0.00503 | 20 | 0.6532 |
| 260 | 0.00578 | 9.232 | 1.521 |
| 261 | 0.00093 | 20 | 0 |
| 262 | 0.03429 | 20 | 0 |
| 263 | 0.00112 | | |
| 264 | 0.00443 | 20 | 0 |
| 265 | 0.02957 | 0.0015 20 | 0 |
| 266 | 4.93501 | 20 | 6 |
| 267 | 0.0015 | | |
| 268 | 0.9647 | 7.32 | 11.27 |
| 269 | 8.7319 | | |
| 270 | 0.7615 | | |

The following compounds did not have IC₅₀s in the range of about 0.001µM to about 10µM in the binding assay: 2-chloro-5-fluoro-N-[(6-methylsulfonylbenzothiazol-2-yl)carbamoyl]benzamide and 2-chloro-4,5-dimethoxy-N-[(6-methylsulfonyl-1,3-benzothiazol-2-yl)carbamoyl]benzamide. In a preferred aspect these compounds are excluded from the claims of the present invention.

### Examples of the Invention

The invention will now be illustrated by the following Examples in which, unless stated otherwise:
(i) temperatures are given in degrees Celsius (°C); operations were carried out at room or ambient temperature, that is, at a temperature in the range of 18 - 25 °C and under an atmosphere of an inert gas such as nitrogen or argon;
(ii) organic solutions were dried over anhydrous magnesium sulfate or sodium sulfate; evaporation of solvent was carried out using a rotary evaporator under reduced pressure (600 - 4000 Pascals; 4.5 - 30 mmHg) with a bath temperature of up to 60 °C;
(iii) purification by chromatography generally refers to flash column chromatography, on silica unless otherwise stated. Column chromatography was generally carried out using prepacked silica cartridges (from 4 g up to 400 g) such as Redisep^{™} (available, for example, from Presearch Ltd, Hitchin, Herts, UK), Biotage (Biotage UK Ltd, Hertford, Herts, UK), or ISOLUTE (available, for example, from IST, Dyffryn Business Park, UK), eluted using a pump and fraction collector system;
(iv) purification by preparative HPLC generally refers to reverse phase preparative HPLC separations run on standard Gilson™ HPLC equipment using a 150 x 21.2 mm Phenomenex Luna 10 micron C18(2) 100 A column, and a standard gradient elution method (5 - 95% acetonitrile gradient with water as co-solvent and 0.2% trifluoroacetic acid as modifier, 12.5 minute gradient with a 2.5 minute hold at 95% acetonitrile) run on Unipoint software;
(v) in general, the course of reactions was followed by LCMS or TLC and reaction times are given for illustration only;
(vi) yields are given for illustration only and are not necessarily those which can be obtained by diligent process development; preparations were repeated if more material was required;
(vii) the structures of the end-products of the Formula (I) were confirmed by nuclear (generally proton) magnetic resonance (NMR) with a field strength (for proton) of 300 MHz (generally using a Varian Gemini 2000) or 400 MHz (generally using a Bruker Avance DPX400), unless otherwise stated, and mass spectral techniques; proton magnetic resonance chemical shift values were measured on the delta scale and peak multiplicities are shown as follows: s, singlet; d, doublet; t, triplet; q, quartet, quin, quintet; sept, septet; dd, double doublet; m, multiplet; br, broad;
   using perdeuterio dimethyl sulfoxide (DMSO-*d₆*) as solvent, unless otherwise stated
(viii) chemical symbols have their usual meanings; SI units and symbols are used;
(ix) solvent ratios are given in volume:volume (v/v) terms;
(x) mass spectra (MS) data were generated on an LCMS system where the HPLC component comprised generally either a Agilent 1100 or Waters Alliance HT (2790 & 2795) equipment and was run on a Phemonenex Gemini C18 5 µm, 50 x 2 mm column (or similar) eluting with either acidic eluent (for example, using a gradient between 0 - 95% water/acetonitrile with 5% of a 1% formic acid in 50:50 water:acetonitrile (v/v) mixture; or using an equivalent solvent system with methanol instead of acetonitrile), or basic eluent (for example, using a gradient between 0 - 95% water/acetonitrile with 5% of a 0.1% 880 ammonia in acetonitrile mixture); and the MS component comprised generally a Waters ZQ spectrometer. Chromatograms for Electrospray (ESI) positive and negative Base Peak Intensity, and UV Total Absorption Chromatogram from 220 - 300 nm, are generated and values for m/z are given; generally, only ions which indicate the parent mass are reported and unless otherwise stated the value quoted is (M+H)⁺;
(xi) suitable microwave reactors include "Smith Creator", "CEM Explorer", "Biotage Initiator sixty" and "Biotage Initiator eight";
(xii) the following abbreviations may be used below or in the process section hereinbefore:
   - BINAP: 2,2'-bis(diphenylphosphino)-1,1'-binaphthyl
   - DCM: dichloromethane
   - DCE: dichloroethane
   - DMA: dimethylacetamide
   - DMSO: dimethylsulfoxide
   - DMF: N,N-dimethylformamide
   - EtOAc: ethyl acetate
   - Et₂O: diethyl ether
   - H₂O: water
   - HCl: hydrochloric acid
   - mCPBA: 3-chloroperoxybenzoic acid
   - MeCN: acetonitrile
   - MeOH: methanol
   - EtOH: ethanol
   - THF: tetrahydrofuran
   - DIPEA: N-ethyldiisopropylamine
   - NBS: N-bromosuccinimide
   - AIBN: 2,2'-azobis(2-methylpropionitrile)
   - AcOH: acetic acid
   - RT: room temperature

Examples 15-18, 20-39 and 43-44 were isolated as trifluoroacetic acid salts.

Examples 59-60, 85, 235, 237, 253 and 263 were isolated as formic acid salts.

### Example 1: 2-Chloro-N-[[6-(2-morpholin-4-ylethylsulfonyl)benzothiazol-2-yl]carbamoyl]benzamide

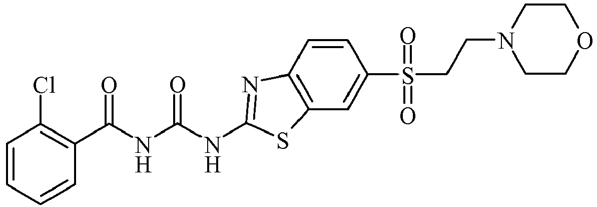

To a solution of 2-chloro-N-[(6-ethenylsulfonylbenzothiazol-2-yl)carbamoyl]benzamide (Intermediate 1, 61 mg, 0.15 mmol) in THF (2 mL) was added morpholine (15 µL, 0.17 mmol). The reaction was heated at 120 °C in a microwave for 5 minutes. Another portion of morpholine (30 µL, 0.34 mmol) was added and the reaction mixture was heated at 120 °C in a microwave for 5 minutes. The reaction mixture was concentrated *in vacuo* and the resulting solid was purified by chromatography on silica gel eluting with EtOAc (100%) to give the title compound as a white solid (25 mg, 33%):
¹H NMR δ2.21 (4H, s), 2.61 (2H, t), 3.24 (4H, s), 3.56 (2H, t), 7.50 - 7.52 (1H, m), 7.58 - 7.61 (2H, m), 7.67 (1H, d), 7.95 (2H, s), 8.65 (1H, s); MS 509.

### Example 2: 2-Chloro-N-[[6-[2-(2-methoxyethylamino)ethylsulfonyl]benzothiazol-2-yl]carbamoyl]benzamide

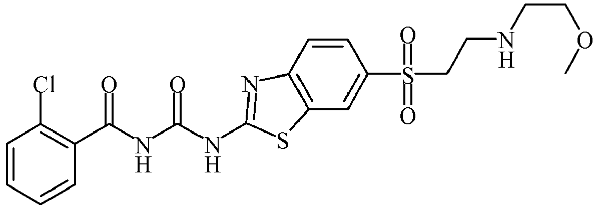

To a solution of 2-chloro-N-[(6-ethenylsulfonylbenzothiazol-2-yl)carbamoyl]benzamide (Intermediate 1, 50 mg, 0.12 mmol) in THF (2 mL) was added 2-methoxyethylamine (36 µL, 0.13 mmol). The reaction was heated at 120 °C in a microwave for 5 minutes. The reaction mixture was concentrated *in vacuo* and the resulting solid was purified by chromatography on silica gel eluting with MeOH/DCM (0 - 15%) to give the title compound as a white solid (22 mg, 37%):
¹H NMR δ2.67 (2H, t), 2.87 (2H, t), 3.19 (3H, s), 3.50 (2H, t), 7.45 - 7.63 (4H, m), 7.85 - 7.91 (2H, m), 8.56 (1H, s); MS 497.

### Example 3: 2-Chloro-N-[[6-(2-dimethylaminoethylsulfonyl)benzothiazol-2-yl]carbamoyl]benzamide

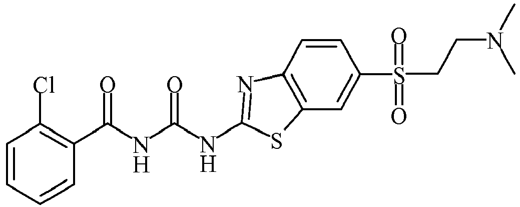

To a solution of 2-chloro-N-[(6-ethenylsulfonylbenzothiazol-2-yl)carbamoyl]benzamide (Intermediate 1, 49 mg, 0.12 mmol) in THF (2 mL) was added dimethylamine (2M in THF, 0.24 mL, 0.48 mmol). The reaction was heated at 120 °C in a microwave for 7 minutes. The reaction mixture was concentrated *in vacuo* and the resulting residue was purified by chromatography on silica gel eluting with MeOH/DCM (0 - 80%) to give the title compound as a white solid (40 mg, 72%):¹H NMR δ2.09 (6H, s), 3.51 (2H, t), 7.39 - 7.65 (4H, m), 7.92 (2H, s), 8.61 (1H, s), 11.75 (2H, s); MS 467.

### Example 4: 2-Chloro-N-[[6-(2-methylaminoethylsulfonyl)benzothiazol-2-yl]carbamoyl]benzamide

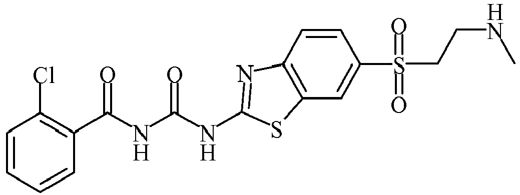

To a solution of 2-chloro-N-[(6-ethenylsulfonylbenzothiazol-2-yl)carbamoyl]benzamide (Intermediate 1, 49 mg, 0.12 mmol) in THF (2 mL) was added methylamine (8M in EtOH, 45 µL, 0.36 mmol). The reaction mixture was heated at 120 °C in a microwave for 7 minutes, and then concentrated *in vacuo* and the resulting residue was purified by chromatography on silica gel eluting with MeOH/DCM (0 - 10%) to give the title compound as a white solid (30 mg, 55%):¹H NMR δ2.37 (3H, s), 2.95 (2H, t), 3.54 (2H, t), 7.42 - 7.57 (4H, m), 7.74 - 7.80 (2H, m), 8.44 (1H, s);MS 453.

### Example 5: 2-Chloro-N-[[6-[2-(3-hydroxypyrrolidin-1-yl)ethylsulfonyl]benzothiazol-2-yl]carbamoyl]benzamide

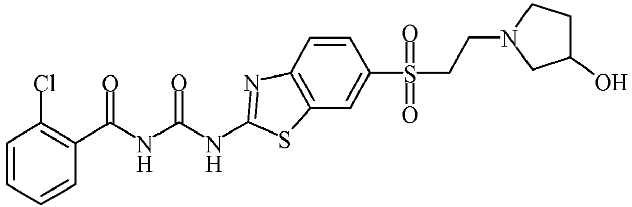

To a solution of 2-chloro-N-[(6-ethenylsulfonylbenzothiazol-2-yl)carbamoyl]benzamide (Intermediate 1, 49 mg, 0.12 mmol) in THF (2 mL) was added 3-hydroxypyrrolidine (33 µL, 0.4 mmol). The reaction mixture was heated at 120 °C in a microwave for 7 minutes and then concentrated *in vacuo* and the resulting residue was purified by chromatography on silica gel eluting with MeOH/DCM (0 - 10%) to give the title compound as a white solid (20 mg, 33%):¹H NMR 81.96 - 2.05 (1H, m), 2.13 - 2.19 (1H, m), 2.36 - 2.40 (1H, m), 2.55 (1H, d), 2.71 - 2.77 (1H, m), 2.85 (2H, t), 3.32 (2H, t), 4.17 (1H, m), 7.35 - 7.52 (3H, m), 7.78 (1H, d), 7.90 (2H, s), 8.36 (1H, s); MS 509.

### Examples 6 - 14

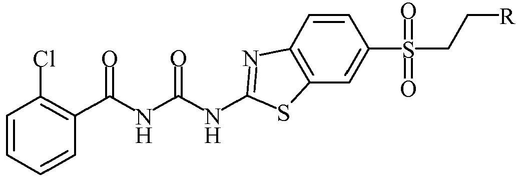

The following examples were prepared by the general procedure of Example 5, using commercially available amines and 2-chloro-N-[(6-ethenylsulfonylbenzothiazol-2-yl)carbamoyl]benzamide (Intermediate 1).

| Example | R | ¹H NMR | MS | Eluent* |
|---|---|---|---|---|
| 6 | 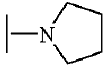 | 1.53 (4H, s), 2.37 (4H, s), 2.76 (2H, t), 3.55 (2H, t), 7.49 (1H, t), 7.58 (2H, q), 7.65 (1H, d), 7.92 (2H, s), 8.62 (1H, s), 11.75 (2H, br s) | 493 | A |
| 7 | 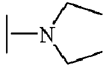 | 0.79 (6H, t), 2.35 (4H, q), 2.77 (2H, t), 3.46 - 3.50 (2H, m), 7.49 - 7.66 (4H, m), 7.94 (2H, s), 8.63 (1H, s) | 495 | A |
| 8 | 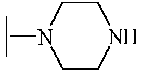 | 2.62 (2H, t), 3.45 - 3.50 (2H, m), 3.57 (2H, s), 7.36 - 7.49 (4H, m), 7.58 (1H, d), 7.67 - 7.70 (1H, m), 8.21 (1H, s) | 508 | C |
| 9 | 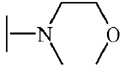 | 2.09 (3H, s), 2.42 (2H, t), 2.71 (2H, t), 3.12 (3H, s), 3.24 (2H, t), 3.51 (2H, t), 7.48 - 7.68 (4H, m), 7.95 (2H, s), 8.64 (1H, s), 11.81 (2H, s) | 511 | A |
| 10 | 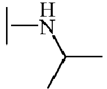 | 0.96 (6H, d), 2.83 (1H, sept), 2.91 (2H, t), 3.48 (2H, t), 7.37 - 7.63 (4H, m), 7.84 (2H, s), 8.51 (1H, s) | 481 | A |
| 11 | 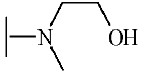 | 2.11 (3H, s), 2.37 (2H, t), 2.73 - 2.76 (2H, m), 3.52 (2H, t), 4.36 (1H, t), 7.47 - 7.67 (4H, m), 7.94 (2H, s), 8.64 (1H, s), 11.79 (2H, s) | 497 | C |
| 12 | 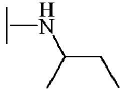 | 0.78 (3H, t), 0.91 (3H, d), 1.17 - 1.22 (1H, m), 1.34 - 1.38 (1H, m), 2.89 - 2.94 (2H, m), 3.48 (2H, t), 7.45 - 7.62 (4H, m), 7.87 (2H, s), 8.55 (1H, s) | 495 | C |
| 13 | 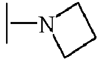 | 1.87 (2H, t), 2.66 (2H, t), 3.06 (4H, t), 3.33 (2H, t), 7.48 (1H, d), 7.56 - 7.58 (2H, m), 7.64 (1H, d), 7.91 (2H, s), 8.58 (1H, s) | 479 | A |
| 14 | 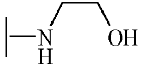 | 2.61 (2H, m), 2.89 (2H, m), 3.40 (2H, m), 3.50 (2H, m), 4.55 (1H, s), 7.45 - 7.49 (1H, m), 7.52 - 7.61 (3H, m), 7.86 (2H, s), 8.53 (1H, s) | 483 | B |

| | | | | |
|---|---|---|---|---|
| * A = MeOH/DCM (0 - 10%), B = MeOH/DCM (0 - 20%), C = MeOH/DCM (0 - 80%) | | | | |

### Examples 6 - 14

6: 2-Chloro-N-[[6-(2-pyrrolidin-1-ylethylsulfonyl)benzothiazol-2-yl]carbamoyl]benzamide
7: 2-Chloro-N-[[6-(2-diethylaminoethylsulfonyl)benzothiazol-2-yl]carbamoyl]benzamide
8: 2-Chloro-N-[[6-(2-piperazin-1-ylethylsulfonyl)benzothiazol-2-yl]carbamoyl]benzamide
9: 2-Chloro-N-[[6-[2-(2-methoxyethyl-methyl-amino)ethylsulfonyl]benzothiazol-2-yl]carbamoyl]benzamide
10: 2-Chloro-N-[[6-[2-(propan-2-ylamino)ethylsulfonyl]benzothiazol-2-yl]carbamoyl]benzamide
11: 2-Chloro-N-[[6-[2-(2-hydroxyethyl-methyl-amino)ethylsulfonyl]benzothiazol-2-yl]carbamoyl]benzamide
12: N-[[6-[2-(Butan-2-ylamino)ethylsulfonyl]benzothiazol-2-yl]carbamoyl]-2-chlorobenzamide
13: N-[[6-[2-(Azetidin-1-yl)ethylsulfonyl]benzothiazol-2-yl]carbamoyl]-2-chlorobenzamide
14: 2-Chloro-N-[[6-[2-(2-hydroxyethylamino)ethylsulfonyl]benzothiazol-2-yl]carbamoyl]benzamide

### Examples 15 - 39

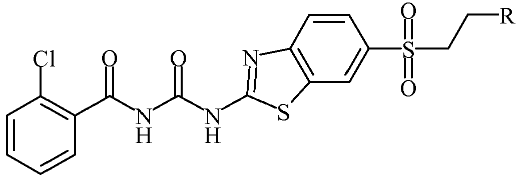

The following examples were prepared by the general procedure of Example 5, using commercially available amines and 2-chloro-N-[(6-ethenylsulfonylbenzothiazol-2-yl)carbamoyl]benzamide (Intermediate 1). The examples were purified by preparative HPLC.

| Example | R | ¹H NMR | MS |
|---|---|---|---|
| 15 | 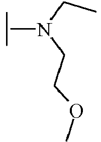 | 1.12 (3H, s), 3.20 (3H, s), 3.29 - 3.46 (6H, br m), 3.54 (2H, s), 3.90 (2H, s), 7.49 - 7.53 (1H, m), 7.57 - 7.64 (2H, m), 7.68 (1H, d), 7.98 (1H, dd), 8.05, (1H, d), 8.73 (1H, s), 11.85 (1H, s), 11.95 (1H, s) | 525 |
| 16 | 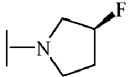 | 3.34 - 3.48 (6H, br m), 3.51 (2H, s), 3.86 (2H, s), 5.45 (1H, d), 7.49 - 7.54 (1H, m), 7.57 - 7.64 (2H, m), 7.68 (1H, d), 7.97 (1H, dd), 8.04 (1H, d), 8.71 (1H, s), 11.85 (1H, s), 11.94 (1H, s) | 511 |
| 17 | 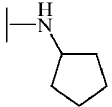 | 1.48 - 1.58 (4H, m), 1.63 - 1.69 (2H, m), 1.88 - 1.96 (2H, m), 3.21 (2H, s), 3.53 (1H, s), 3.69 - 3.74 (2H, m), 7.48 - 7.53 (1H, m), 7.57 - 7.64 (2H, m), 7.68 (1H, d), 7.99 (1H, dd), 8.04 (1H, d), 8.64 (1H, s), 8.74 (1H, s), 11.85 (1H, s), 11.95 (1H, s) | 507 |
| 18 | 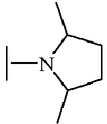 | 1.05 - 1.09 (2H, m), 1.24 - 1.31 (6H, m), 1.61 (2H, s), 2.13 (2H, s), 3.56 (2H, s), 3.96 (2H, s), 7.48 - 7.54 (1H, m), 7.56 - 7.64 (2H, m), 7.68 (1H, d), 7.99 - 8.06 (2H, m), 8.74 (1H, s), 11.85 (1H, s), 11.95 (1H, s) | 521 |
| 19 | 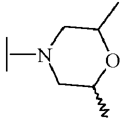 | 1.04 - 1.09 (6H, m), 3.50 - 3.80 (7H, br m), 3.82 - 3.89 (3H, br m), 7.48 - 7.54 (1H, m), 7.57 - 7.64 (2H, m), 7.68 (1H, d), 7.96 (1H, dd), 8.03 (1H, d), 8.70 (1H, s), 11.85 (1H, s), 11.95 (1H, s) | 537 |
| 20 | 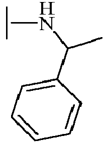 | 1.52 (3H, d), 3.62 - 3.69 (2H, m), 3.70 - 3.78 (2H, m), 4.41 - 4.47 (1H, m), 7.38 - 7.41 (3H, m), 7.42 - 7.45 (2H, m), 7.49 - 7.54 (1H, m) 7.57 - 7.64 (2H, m), 7.68 (1H, d), 7.89 (1H, dd), 8.00 (1H, d), 8.65 (1H, s), 11.86 (2H, s), 11.95 (1H, s) | 543 |
| 21 | 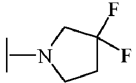 | 2.12 - 2.23 (2H, m), 2.88 (2H, s), 2.99 (2H, s), 3.08 (2H, s), 3.64 - 3.69 (2H, m), 7.48 - 7.53 (1H, m), 7.56 - 7.63 (2H, m), 7.68 (1H, d), 7.93 - 8.00 (2H, m), 8.67 (1H, s), 11.82 (1H, s), 11.92 (1H, s) | 529 |
| 22 | 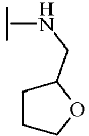 | 1.44 - 1.53 (1H, m), 1.79 - 1.83 (2H, m), 1.94 - 2.03 (1H, m), 2.93 - 2.99 (1H, m), 3.12 (1H, d), 3.21 - 3.27 (2H, m), 3.66 - 3.80 (4H, m), 3.99 - 4.06 (1H, m), 7.49 - 7.54 (1H, m), 7.57 - 7.64 (2H, m), 7.68 (1H, d), 7.96 (1H, dd), 8.05 (1H, d), 8.72 (2H, s), 11.86 (1H, s), 11.95 (1H, s) | 523 |
| 23 | 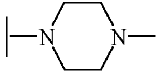 | 2.23 (2H, t), 2.32 - 2.35 (1H, m), 2.70 (3H, s), 2.74 (1H,d), 2.90 (2H, d), 3.29 (2H, d), 3.57 - 3.63 (4H, m), 7.48 - 7.53 (1H, m), 7.57 - 7.63 (2H, m), 7.68 (1H, d), 7.94 - 8.01 (2H, m), 8.68 (1H, s), 11.83 (1H, s), 11.92 (1H, s) | 522 |
| 24 | 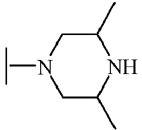 | 1.06 (6H, d), 1.85 (2H, t), 2.66 - 2.69 (2H, m), 2.69 - 2.74 (2H, m), 2.86 (2H, d), 3.59 (2H, t), 7.49 - 7.53 (1H, m), 7.57 - 7.64 (2H, m), 7.68 (1H, d), 7.93 - 8.00 (2H, m), 8.67 (2H, s), 11.85 (1H, s), 11.93 (1H, s) | 536 |
| 25 | 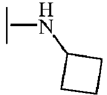 | 1.69 -1.81 (2H, m), 2.02 - 2.11 (2H, m), 2.11-2.20 (2H, m), 2.32 - 2.35 (1H, m), 3.10 (2H, t), 3.69 (2H, t), 7.49 - 7.54 (1H, m), 7.57 - 7.64 (2H, m), 7.68 (1H, d), 7.98 (1H, dd), 8.05 (1H, d), 8.73 (1H, s), 8.84 (1H, s), 11.86 (1H, s), 11.94 (1H, s) | 493 |
| 26 | 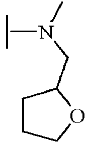 | 1.40 - 1.49 (1H, m), 1.84 (2H, s), 1.95 - 2.01 (1H, m), 2.80 (3H, s), 3.07 (1H, s), 3.62 - 3.68 (2H, m), 3.72 - 3.78 (2H, m), 3.89 - 3.94 (2H, m), 4.06 - 4.19 (2H, m), 7.46 - 7.56 (1H, m), 7.56 - 7.65 (2H, m), 7.68 (1H, d), 7.97 (1H, d), 8.05 (1H, d), 8.71 (1H, s), 11.85 (1H, s), 11.94 (1H, s) | 537 |
| 27 | 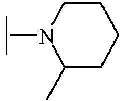 | 1.19 (3H, d), 1.35 - 1.48 (2H, m), 1.56 (1H, d), 1.67 (1H, d), 1.78 (1H, d), 1.86 (1H, d), 2.93 - 3.01 (1H, m), 3.23 - 3.28 (2H, m), 3.81 - 3.89 (2H, m), 3.99 - 4.07 (2H, m), 7.49 - 7.54 (1H, m), 7.57 - 7.64 (2H, m), 7.68 (1H, d), 7.99 - 8.07 (2H, m), 8.75 (1H, s), 11.85 (1H, s), 11.95 (1H, s) | 521 |
| 28 | 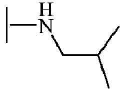 | 0.90 (6H, d), 1.82 - 1.89 (1H, m), 2.79 (2H, d), 3.18 - 3.24 (2H, m), 3.73 (2H, t), 7.49 - 7.54 (1H, m), 7.57 - 7.64 (2H, m), 7.68 (1H, d), 7.97 (1H, dd), 8.05 (1H, d), 8.39 (1H, s), 8.73 (1H, s), 11.86 (1H, s), 11.93 (1H, s) | 495 |
| 29 | 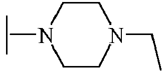 | 1.07 (3H, t), 2.32 - 2.34 (2H, m), 2.66 - 2.70 (4H, m), 3.31 - 3.36 (6H, br m), 3.58 (2H, t), 7.48 - 7.55 (1H, m), 7.56 - 7.63 (2H, m), 7.67 (1H, d), 7.86 - 7.91 (1H, m), 7.93 - 7.98 (1H, m), 8.66 (1H, s), 11.80 (2H, s) | 536 |
| 30 | 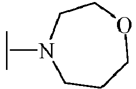 | 1.98 (2H, s), 3.23 - 3.58 (6H, br m), 3.63 - 3.81 (4H, m), 3.89 - 3.96 (2H, m), 7.49 - 7.54 (1H, m), 7.57 - 7.64 (2H, m), 7.68 (1H, d), 7.98 (1H, dd), 8.05 (1H, d), 8.71 (1H, s), 11.85 (1H, s), 11.94 (1H, s) | 523 |
| 31 | 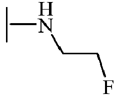 | 3.20 - 3.37 (2H, br m), 3.37 - 3.41 (2H, m), 3.73 (2H, t), 4.59 (1H, t), 4.75 (1H, t), 7.45 - 7.52 (1H, m), 7.54 - 7.62 (2H, m), 7.66 (1H, d), 7.93 - 7.98 (1H, m), 7.99 - 8.05 (1H, m), 8.70 (1H, s), 8.90 (1H, s), 11.78 (2H, s) | 485 |
| 32 | 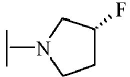 | 3.34 - 3.48 (6H, br m), 3.51 (2H, s), 3.84 - 3.92 (2H, m), 5.44 (1H, d), 7.49 - 7.53 (1H, m), 7.57 - 7.64 (2H, m), 7.68 (1H, d), 7.97 (1H, dd), 8.04 (1H, d), 8.71 (1H, s), 11.84 (1H, s), 11.94 (1H, s) | 511 |
| 33 | 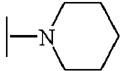 | 1.23 - 1.36 (2H, m), 1.47 - 1.62 (3H, m), 1.66 (1H, d), 1.80 (2H, d), 2.84 - 2.95 (2H, m), 3.46 (2H, d), 3.87 - 3.93 (2H, m), 7.49 - 7.54 (1H, m), 7.57 - 7.64 (2H, m), 7.68 (1H, d), 7.97 (1H, dd), 8.04 (1H, d), 8.71 (1H, s), 11.85 (1H, s), 11.94 (1H, s) | 507 |
| 34 | 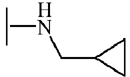 | 0.01 (2H, q), 0.23 - 0.31 (2H, m), 0.63 - 0.74 (1H, m), 2.57 (2H, d), 2.91 - 2.98 (2H, m), 3.43 (2H, t), 7.19 - 7.25 (1H, m), 7.26 - 7.35 (2H, m), 7.38 (1H, d), 7.65 - 7.71 (1H, m), 7.75 (1H, d), 8.32 (1H, s), 8.43 (1H, s), 11.52 (1H, s), 11.62 (1H, s) | 493 |
| 35 | 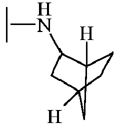 | 1.04 - 1.10 (2H, m), 1.11 - 1.18 (1H, m), 1.36 - 1.43 (1H, m), 1.45 - 1.54 (3H, m), 2.24 - 2.29 (1H, m), 2.36 (1H, d), 3.07 - 3.21 (3H, m), 3.65 - 3.72 (3H, m), 7.46 - 7.52 (1H, m), 7.56 - 7.62 (2H, m), 7.66 (1H, d), 7.96 (1H, dd), 8.02 (1H, d), 8.35 (1H, d), 8.71 (1H, s), 11.79 (1H, s), 11.91 (1H, s) | 533 |
| 36 | 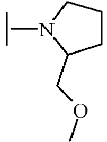 | 1.60 - 1.69 (1H, m), 1.76 - 1.85 (1H, m), 1.93 - 2.02 (1H, m), 2.04 - 2.14 (1H, m), 3.22 (3H, s), 3.45 - 3.52 (2H, m), 3.56 - 3.63 (4H, m), 3.95 - 4.04 (3H, m), 7.48 - 7.53 (1H, m), 7.57 - 7.64 (2H, m), 7.68 (1H, d), 7.97 (1H, dd), 8.05 (1H, d), 8.72 (1H, s), 11.85 (1H, s), 11.94 (1H, s) | 537 |
| 37 | 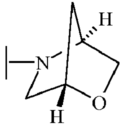 | 1.96 (1H, d), 2.26 - 2.32 (1H, m), 3.14 - 3.21 (2H, m), 3.49 (1H, br m), 3.58 (1H, br m), 3.72 (1H, br m), 3.79 - 3.92 (3H, m), 4.51 (1H, s), 4.65 (1H, s), 7.49 - 7.55 (1H, m), 7.57 - 7.64 (2H, m), 7.68 (1H, d), 7.97 (1H, d), 8.05 (1H, d), 8.71 (1H, s), 11.85 (1H, s), 11.95 (1H, s) | 521 |
| 38 | 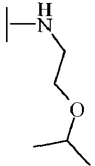 | 1.07 (6H, d), 3.14 (2H, s), 3.25 (1H, s), 3.52 - 3.59 (4H, m), 3.74 (2H, t), 7.49 - 7.54 (1H, m), 7.57 - 7.64 (2H, m), 7.68 (1H, d), 7.97 (1H, dd), 8.05 (1H, d), 8.57 (1H, s), 8.73 (1H, s), 11.86 (1H, s), 11.94 (1H, s) | 525 |
| 39 | 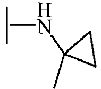 | 0.68 (2H, t), 0.97 (2H, m), 1.34 (3H, s), 3.27 - 3.32 (2H, m), 3.69 (2H, t), 7.48 - 7.54 (1H, m), 7.57 - 7.64 (2H, m), 7.68 (1H, d), 7.99 - 8.07 (2H, m), 8.75 (1H, s), 8.85 (1H, s), 11.85 (2H, s) | 493 |

### Examples 15 - 39

15: 2-Chloro-N-[[6-[2-(ethyl-(2-methoxyethyl)amino)ethylsulfonyl]benzothiazol-2-yl]carbamoyl]benzamide
16: 2-Chloro-N-[[6-[2-[(3S)-3-fluoropyrrolidin-1-yl]ethylsulfonyl]benzothiazol-2-yl]carbamoyl]benzamide
17: 2-Chloro-N-[[6-[2-(cyclopentylamino)ethylsulfonyl]benzothiazol-2-yl]carbamoyl]benzamide
18: 2-Chloro-N-[[6-[2-(2,5-dimethylpyrrolidin-1-yl)ethylsulfonyl]benzothiazol-2-yl]carbamoyl]benzamide
19: 2-Chloro-N-[[6-[2-(2,6-dimethylmorpholin-4-yl)ethylsulfonyl]benzothiazol-2-yl]carbamoyl]benzamide
20: 2-Chloro-N-[[6-[2-(1-phenylethylamino)ethylsulfonyl]benzothiazol-2-yl]carbamoyl]benzamide
21: 2-Chloro-N-[[6-[2-(3,3-difluoropyrrolidin-1-yl)ethylsulfonyl]benzothiazol-2-yl]carbamoyl]benzamide
22: 2-Chloro-N-[[6-[2-(oxolan-2-ylmethylamino)ethylsulfonyl]benzothiazol-2-yl]carbamoyl]benzamide
23: 2-Chloro-N-[[6-[2-(4-methylpiperazin-1-yl)ethylsulfonyl]benzothiazol-2-yl]carbamoyl]benzamide
24: 2-Chloro-N-[[6-[2-(3,5-dimethylpiperazin-1-yl)ethylsulfonyl]benzothiazol-2-yl]carbamoyl]benzamide
25: 2-Chloro-N-[[6-[2-(cyclobutylamino)ethylsulfonyl]benzothiazol-2-yl]carbamoyl]benzamide
26: 2-Chloro-N-[[6-[2-(methyl-(oxolan-2-ylmethyl)amino)ethylsulfonyl]benzothiazol-2-yl]carbamoyl]benzamide
27: 2-Chloro-N-[[6-[2-(2-methyl-1-piperidyl)ethylsulfonyl]benzothiazol-2-yl]carbamoyl]benzamide
28: 2-Chloro-N-[[6-[2-(2-methylpropylamino)ethylsulfonyl]benzothiazol-2-yl]carbamoyl]benzamide
29: 2-Chloro-N-[[6-[2-(4-ethylpiperazin-1-yl)ethylsulfonyl]benzothiazol-2-yl]carbamoyl]benzamide
30: 2-Chloro-N-[[6-[2-(1,4-oxazepan-4-yl)ethylsulfonyl]benzothiazol-2-yl]carbamoyl]benzamide
31: 2-Chloro-N-[[6-[2-(2-fluoroethylamino)ethylsulfonyl]benzothiazol-2-yl]carbamoyl]benzamide
32: 2-Chloro-N-[[6-[2-[(3R)-3-fluoropyrrolidin-1-yl]ethylsulfonyl]benzothiazol-2-yl]carbamoyl]benzamide
33: 2-Chloro-N-[[6-[2-(1-piperidyl)ethylsulfonyl]benzothiazol-2-yl]carbamoyl]benzamide 34: 2-Chloro-N-[[6-[2-(cyclopropylmethylamino)ethylsulfonyl]benzothiazol-2-yl]carbamoyl]benzamide
35: 2-Chloro-N-[[6-[2-(norbornan-2-ylamino)ethylsulfonyl]benzothiazol-2-yl]carbamoyl]benzamide
36: 2-Chloro-N-[[6-[2-[2-(methoxymethyl)pyrrolidin-1-yl]ethylsulfonyl]benzothiazol-2-yl]carbamoyl]benzamide
37: 2-Chloro-N-[[6-[2-[(1S,4S)-3-oxa-6-azabicyclo[2.2.1]hept-6-yl]ethylsulfonyl]benzothiazol-2-yl]carbamoyl]benzamide
38: 2-Chloro-N-[[6-[2-(2-propan-2-yloxyethylamino)ethylsulfonyl]benzothiazol-2-yl]carbamoyl]benzamide
39: 2-Chloro-N-[[6-[2-[(1-methylcyclopropyl)amino]ethylsulfonyl]benzothiazol-2-yl]carbamoyl]benzamide

### Example 40: 2-Chloro-N-[[6-(2-methoxyethylsulfonyl)benzothiazol-2-yl]carbamoyl]benzamide

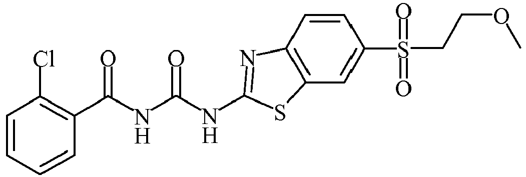

To a solution of 2-chloro-N-[(6-ethenylsulfonylbenzothiazol-2-yl)carbamoyl]benzamide (Intermediate 1, 96 mg, 0.23 mmol) in THF (4 mL) was added sodium methoxide (25% in MeOH, 1 mL). The reaction mixture stood for 30 minutes and was then concentrated *in vacuo.* The residue was dissolved in H₂O (10 mL) and acidified with HCl (2M in H₂O). The precipitate was filtered and dried to give the title compound as a white solid (46 mg, 44%):¹H NMR δ3.11 (3H, s), 3.61 - 3.66 (4H, m), 7.48 - 7.69 (4H, m), 7.94 (2H, q), 8.64 (1H, s), 11.76 (1H, s), 11.87 (1H, s);MS 454.

### Example 41: 2-Chloro-N-[[6-(2-hydroxyethylsulfonyl)benzothiazol-2-yl]carbamoyl]benzamide

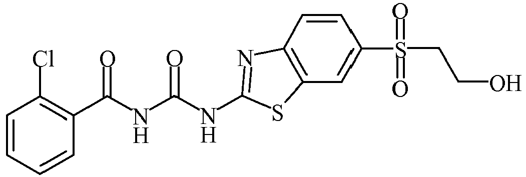

To a solution of 2-chloro-N-[(6-ethenylsulfonylbenzothiazol-2-yl)carbamoyl]benzamide (Intermediate 1, 0.12 g, 0.28 mmol) in THF (5 mL) was added Triton B (1 mL) and the solution was left to stand overnight. The solution was concentrated *in vacuo* and purified by preparative HPLC to give the title compound as a white solid (11 mg, 9%):¹H NMR δ3.49 (2H, t), 3.71 (2H, t), 7.50 - 7.69 (4H, m), 7.94 - 7.96 (2H, m), 8.64 (1H, s); MS 440. **Example 42:** 2-Chloro-N-[[6-[2-(2-methoxyethoxy)ethylsulfonyl]benzothiazol-2-yl]carbamoyl]benzamide

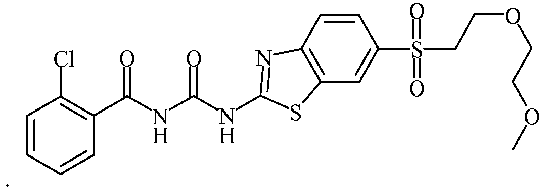

To a solution of 2-methoxyethanol (8 µL, 1.0 mmol) in THF (5 mL) was added sodium hydride (60%, 45 mg, 1.1 mmol). The reaction mixture was stirred until gas evolution ceased, then added to a solution of 2-chloro-N-[(6-ethenylsulfonylbenzothiazol-2-yl)carbamoyl]benzamide (Intermediate 1, 96 mg, 0.23 mmol) in THF (4 mL). The reaction mixture was concentrated *in vacuo* and then the residue was dissolved in H₂O (10 mL). The solution was acidified with HCl (2M in H₂O) and diluted with DCM (10 mL), then passed through a phase separation cartridge. The organic phase was concentrated *in vacuo* and the residue was purified by chromatography on silica gel eluting with EtOAc/isohexane (0 - 100%) to give the title compound as a white solid (45 mg, 39%): ¹H NMR δ3.1 (3H, s), 3.20 (2H, d), 3.36 (2H, d), 3.62 (2H, t), 3.71 - 3.74 (2H, m), 7.47 - 7.67 (4H, m), 7.92 (2H, d), 8.60 (1H, s); MS 498.

### Example 43: 2-Chloro-N-[[6-[2-(2-dimethylaminoethoxy)ethylsulfonyl]benzothiazol-2-yl]carbamoyl]benzamide

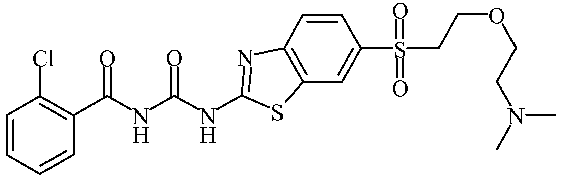

To a solution of N,N-dimethylethanolamine (10 µL, 1.0 mmol) in THF (5 mL) was added sodium hydride (60%, 45 mg, 1.1 mmol). The reaction mixture was stirred until gas evolution ceased, then added to a solution of 2-chloro-N-[(6-ethenylsulfonylbenzothiazol-2-yl)carbamoyl]benzamide (Intermediate 1, 96 mg, 0.23 mmol) in THF (4 mL). The reaction mixture was concentrated *in vacuo* and the residue dissolved in H₂O (10 mL). The solution was acidified with HCl (2M in H₂O) and diluted with DCM (10 mL), then passed through a phase separation cartridge. The organic phase was concentrated *in vacuo* and the residue was purified by preparative HPLC to give the title compound as a white solid (34 mg, 29%):
¹H NMR δ2.70 (6H, s), 3.13 (2H, s), 3.65 (2H, t), 3.70 (2H, t), 3.81 (2H, d), 7.50 - 7.53 (1H, m), 7.59 - 7.61 (2H, m), 7.67 (1H, d), 7.94 - 7.98 (2H, m), 8.66 (1H, s); MS 511.

### Example 44: 2-Chloro-N-[(6-pyrrolidin-3-ylsulfonylbenzothiazol-2-yl)carbamoyl]benzamide

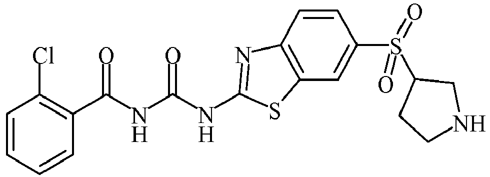

A solution of 2-chlorobenzamide (23 mg, 0.15 mmol) and oxalyl chloride (2 µL, 0.2 mmol) in THF (1.5 mL) was heated at 120 °C in a microwave for 5 minutes. To this was added *tert*-butyl 3-(2-aminobenzothiazol-6-yl)sulfonylpyrrolidine-1-carboxylate (Intermediate 49, 50 mg, 0.13 mmol) and the suspension was heated at 120 °C in a microwave for 5 minutes. One drop of HCl (conc.) and MeOH (1 mL) were added and the reaction mixture was heated at 120 °C in a microwave for 5 minutes. The reaction mixture was diluted with MeCN (5 mL) and purified by preparative HPLC to give the title compound as a white solid (26 mg, 43%): ¹H NMR δ2.22 (1H, m), 3.18 - 3.27 (2H, m), 3.47 - 3.49 (2H, m), 4.28 (1H, m), 7.48 - 7.52 (1H, m), 7.56 - 7.60 (2H, m), 7.66 (1H, d), 7.94 - 7.97 (1H, m), 8.01 (1H, d), 8.71 (1H, s);MS 435.

### Example 45: 2-Chloro-5-(pyridin-2-yl)-N-(6-(methylsulfonyl)benzothiazol-2-ylcarbamoyl)benzamide

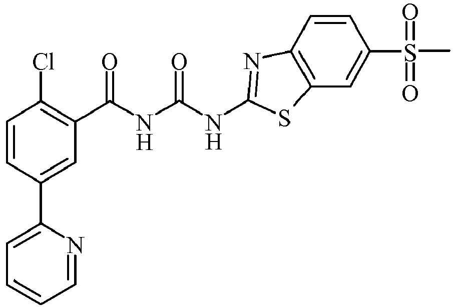

Oxalyl chloride (0.18 mL, 2.1 mmol) was added to a suspension of 2-chloro-5-pyridin-2-ylbenzamide (Intermediate 4, 0.47 g, 2.0 mmol) in THF (15 mL) and the mixture was heated at 120 °C in a microwave for 5 minutes. The reaction mixture was cooled and 2-amino-6-(methylsulfonyl)benzothiazole (0.41 g, 1.8 mmol) was added, then the mixture was heated at 100 °C in a microwave for 10 minutes. The reaction mixture was cooled, concentrated *in vacuo* and then suspended in MeOH. The suspension was filtered and washed with MeOH. A precipitate formed in the filtrate on standing and was filtered to give the title compound as a solid (90 mg, 10%): ¹H NMR δ3.24 (3H, s), 7.46 - 7.50 (1H, m), 7.69 - 7.78 (1H, m), 7.96 - 8.04 (3H, m), 8.07 - 8.20 (1H, m), 8.25 - 8.29 (1H, m), 8.39 (1H, d), 8.66 (1H, s), 8.72 (1H, d), 11.86 (1H, s);MS 487.

### Example 46: 2-Chloro-5-(pyrazol-1-yl)-N-(6-(methylsulfonyl)benzothiazol-2-ylcarbamoyl)benzamide

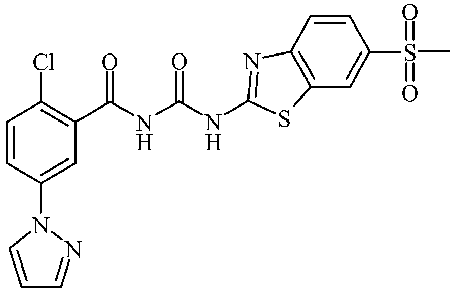

Oxalyl chloride (0.064 mL, 0.74 mmol) was added to a suspension of 2-chloro-5-pyrazol-1-ylbenzamide (Intermediate 13, 0.16 g, 0.70 mmol) in THF (3.5 mL) and the mixture was heated at 120 °C in a microwave for 5 minutes. The reaction mixture was cooled and 2-amino-6-(methylsulfonyl)benzothiazole (0.14 g, 0.63 mmol) was added, then the mixture was heated at 120 °C in a microwave for 5 minutes. The reaction mixture was cooled, concentrated *in vacuo* and then suspended in MeOH. The suspension was filtered and washed with MeOH to give the title compound as a solid (0.15 g, 46%):
¹H NMR δ3.25 (3H, s), 6.60 (1H, m), 7.72 (1H, d), 7.81 (1H, d), 7.96 (2H, s), 8.02 - 8.06 (1H, m), 8.18 (1H, d), 8.58 (1H, d), 8.66 (1H, s), 11.83 (2H, s);MS 476.

### Examples 47 - 57

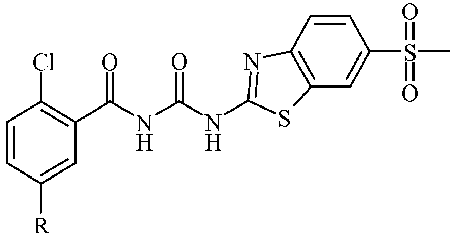

The following examples were prepared by the general procedure of Example 46, using commercially available 2-amino-6-(methylsulfonyl)benzothiazole and appropriate benzamides (Intermediates 6, 14, 16, 19-20, 24, 26-7, 29, 37 and 39).

| Example | R | ¹H NMR | MS |
|---|---|---|---|
| 47 | 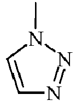 | 3.25 (3H, s), 7.79 (1H, d), 7.96 (2H, s), 8.16 - 8.20 (3H, m), 8.32 (1H, s), 8.66 (1H, s), 11.83 (2H, s) | 477 |
| 48 | 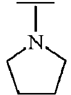 | 1.96 (4H, m), 3.25 (4H, m), 6.65 - 6.69 (1H, m), 6.76 (1H, d), 7.29 (1H, d), 7.96 (2H, s), 8.67 (1H, s), 11.68 (1H, s), 11.91 (1H, s) | 479 |
| 49 | 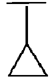 | 0.69 - 0.81 (2H, m), 0.98 - 1.04 (2H, m), 1.95 - 2.04 (1H, m), 3.25 (3H, s), 7.27 - 7.30 (1H, m), 7.36 (1H, s), 7.44 (1H, d), 7.96 (2H, s), 8.67 (1H, s), 11.73 (1H, s), 11.87 (1H, s) | 450 |
| 50 | 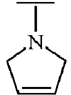 | 3.25 (3H, s), 4.08 (4H, s), 6.04 (2H, s), 6.67 (1H, d), 6.77 (1H, s), 7.32 (1H, t), 7.96 (2H, s), 8.67 (1H, s), 11.71 (1H, s), 11.91 (1H, s) | 476 |
| 51 | 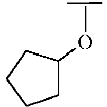 | 1.57 - 1.96 (8H, m), 3.32, (3H, s), 4.86 (1H, t), 7.07 - 7.10 (1H, m), 7.21 (1H, d), 7.45 (1H, d), 7.96 (2H, s), 8.67 (1H, s), 11.72 (1H, s), 11.84 (1H, s) | 494 |
| 52 | 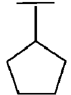 | 1.50 - 1.80 (6H, m), 2.03 (2H, m), 2.98 - 3.09 (1H, m), 3.25 (3H, s), 7.43 - 7.50 (2H, m), 7.55 (1H, s), 7.97 (2H, s), 8.67 (1H, s), 11.77 (1H, s), 11.88 (1H, s) | 478 |
| 53 | 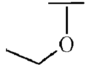 | 1.34 (3H, t), 3.25 (3H, s), 4.08 (2H, q), 7.09 - 7.13 (1H, m), 7.25 (1H, s), 7.45 (1H, d), 7.96 (2H, s), 8.67 (1H, s), 11.73 (1H, s), 11.84 (1H, s) | 454 |
| 54 | 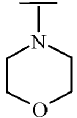 | 3.16 - 3.19 (4H, m), 3.24 (3H, s), 3.72 - 3.75 (4H, m), 7.06 - 7.13 (1H, m), 7.21 (1H, d), 7.38 (1H, d), 7.96 (2H, s), 8.66 (1H, s), 11.71 (1H, s) | 495 |
| 55 | 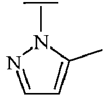 | 2.40 (3H, s), 3.24 (3H, s), 6.32 (1H, s), 7.62 (1H, d), 7.74 (2H, s), 7.87 (1H, s), 7.96 (2H, s), 8.67 (1H, s), 11.81 (2H, s) | 490 |
| 56 | 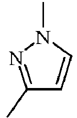 | 2.28 (3H, s), 3.25 (3H, s), 6.39 (1H, d), 7.67 (1H, d), 7.96 - 7.99 (3H, m), 8.12 (1H, s), 8.45 (1H, d), 8.67 (1H, s), 11.83 (2H, s) | 488 (M-H)⁻ |
| 57 | 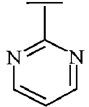 | 3.24 (3H, s), 7.52 (1H, t), 7.76 (1H, d), 7.96 (2H, s), 8.50 - 8.54 (1H, m), 8.63 - 8.66 (2H, m), 8.96 (2H, d), 11.85 (2H, s) | 488 |

### Examples 47 - 57

47: 2-Chloro-5-[1,2,3]triazol-1-yl-N-(6-(methylsulfonyl)benzothiazol-2-ylcarbamoyl)benzamide
48: 2-Chloro-5-(1-pyrrolidinyl)-N-(6-(methylsulfonyl)benzothiazol-2-ylcarbamoyl)benzamide
49: 2-Chloro-5-cyclopropyl-N-(6-(methylsulfonyl)benzothiazol-2-ylcarbamoyl)benzamide
50: 2-Chloro-5-(2,5-dihydro-pyrrol-1-yl)-N-(6-(methylsulfonyl)benzothiazol-2-ylcarbamoyl)benzamide
51: 2-Chloro-5-cyclopentyloxy-N-(6-(methylsulfonyl)benzothiazol-2-ylcarbamoyl)benzamide
52: 2-Chloro-5-cyclopentyl-N-(6-(methylsulfonyl)benzothiazol-2-ylcarbamoyl)benzamide
53 : 2-Chloro-5-ethoxy-N-(6-(methylsulfonyl)benzothiazol-2-ylcarbamoyl)benzamide
54: 2-Chloro-N-(6-(methylsulfonyl)benzothiazol-2-ylcarbamoyl)-5- morpholin-4-yl benzamide
55 : 2-Chloro-5-(5-methyl-1H-pyrazol-1-yl)-N-(6-(methylsulfonyl)benzothiazol-2-ylcarbamoyl)benzamide
56: 2-Chloro-5-(3-methyl-1H-pyrazol-1-yl)-N-(6-(methylsulfonyl)benzothiazol-2-ylcarbamoyl)benzamide
57: 2-Chloro-N-(6-(methylsulfonyl)benzothiazol-2-ylcarbamoyl)-5-(pyrimidin-2-yl)benzamide

### Example 58: 4-Chloro-3-(6-(methylsulfonyl)benzothiazol-2-ylcarbamoylcarbamoyl)phenyl methanesulfonate

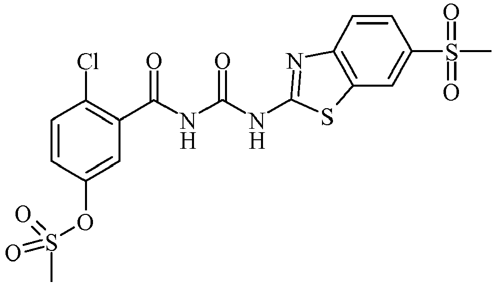

Example 58 was prepared by the general procedure of Example 46, using commercially available 2-amino-6-(methylsulfonyl)benzothiazole and 3-carbamoyl-4-chlorophenyl methanesulfonate (Intermediate 28). The crude solid was purified by chromatography on silica gel eluting with MeOH/DCM (1 - 15%) to give the title compound as a yellow solid (0.51 g, 50%):¹H NMR δ3.20 (3H, s), 3.42 (3H, s), 7.48 - 7.52 (1H, m), 7.66 - 7.69 (2H, m), 7.91 (2H, s), 8.62 (1H, s), 11.75 (1H, s), 11.82 (1H, s); MS (M-H)⁻ 502.

### Example 59: 2-Chloro-5-(4-methylpiperazin-1-yl)-N-(6-(methylsulfonyl)benzothiazol-2-ylcarbamoyl)benzamide

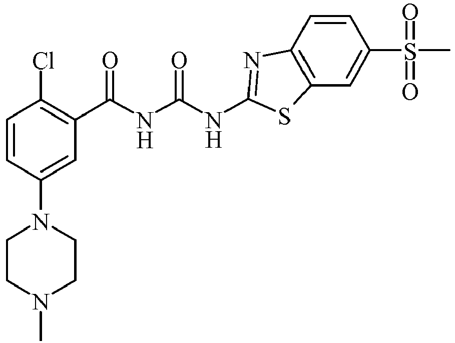

Example 59 was prepared by the general procedure of Example 46, using commercially available 2-amino-6-(methylsulfonyl)benzothiazole and 2-chloro-5-(4-methylpiperazin-1-yl)benzamide (Intermediate 30). The crude solid was purified by preparative HPLC to give the title compound as a solid (20 mg, 4%): ¹H NMR δ2.25 (3H, s), 7.07 - 7.11 (1H, m), 7.19 (1H, d), 7.35 (1H, d), 7.91 - 7.97 (2H, m), 8.13 (1H, s), 8.64 (1H, s). MS 508.

### Example 60: 2-Chloro-5-(3-(dimethylamino)pyrrolidin-1-yl)-N-(6-(methylsulfonyl)benzothiazol-2-ylcarbamoyl)benzamide

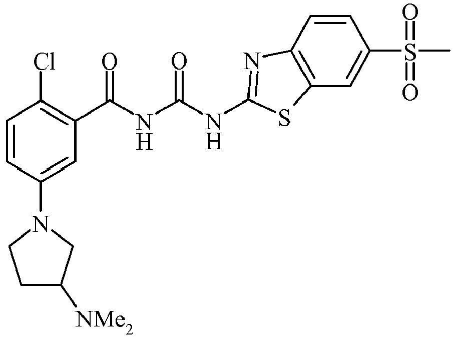

Example 60 was prepared by the general procedure of Example 59, using commercially available 2-amino-6-(methylsulfonyl)benzothiazole and 2-chloro-5-(3-(dimethylamino)pyrrolidin-1-yl)benzamide (Intermediate 31) to give the title compound as a solid (26 mg, 5%): ¹H NMR 81.77 - 1.90 (1H, m), 2.16 - 2.27 (7H, m), 2.87 (1H, m), 3.01 - 3.10 (1H, m), 3.24 (3H, s), 3.40 (1H, m), 3.47 (1H, m), 6.65 - 6.69 (1H, m), 6.77 (1H, d), 7.29 (1H, d), 7.92 (2H, d), 8.13 (1H, s), 8.63 (1H, s); MS 522.

### Example 61: 2-Chloro-5-ethynyl-N-(6-(methylsulfonyl)benzothiazol-2-ylcarbamoyl)benzamide

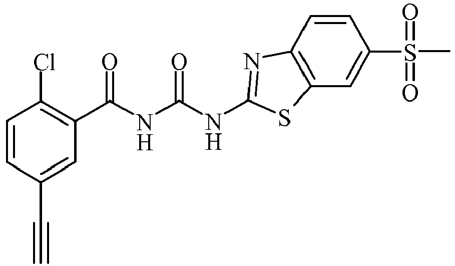

Oxalyl chloride (0.27 mL, 3.06 mmol) was added to 2-chloro-5-ethynylbenzamide (Intermediate 9, 0.50 g, 2.78 mmol) in THF (12 mL) and the solution was heated at 60 °C for 30 minutes under nitrogen. The solution was cooled and concentrated *in vacuo* to give the crude isocyanate. A solution of the isocyanate in THF (6 mL) was added dropwise over 5 minutes to a stirred solution of 6-(methylsulfonyl)benzothiazol-2-amine (0.64 g, 2.78 mmol) in THF (6 mL) at 60 °C and the solution was heated at 60 °C for 90 minutes under nitrogen. The reaction mixture was diluted with EtOAc and washed with H₂O (x 2) and saturated brine. The organic phase was left to stand for 30 minutes and the resulting precipitate was filtered and washed with EtOAc to give the title compound as an off-white solid (0.34 g, 28%): ¹H NMR δ3.25 (3H, s), 4.41 (1H, s), 7.54 - 7.71 (2H, m), 7.79 (1H, s), 7.96 (2H, s), 8.66 (1H, s), 11.59 - 12.05 (2H, m);MS 434.

### Example 62: 2-Chloro-5-pyrrolidin-1-ylmethyl-N-(6-(methylsulfonyl)benzothiazol-2-ylcarbamoyl)benzamide 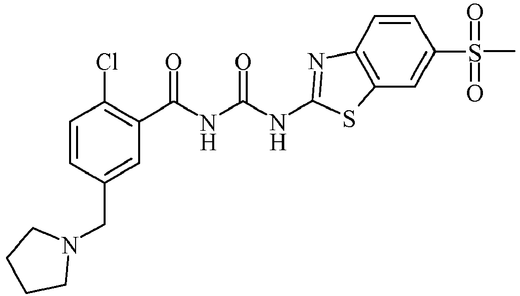

Pyrrolidine (0.1 mL, 1.2 mmol) was added to a suspension of 5-bromomethyl-2-chloro-N-(6-(methylsulfonyl)benzothiazol-2-ylcarbamoyl)benzamide (Intermediate 33, 0.20 g, 0.40 mmol) in MeCN (4 mL) and the solution was stirred for 15 minutes. The reaction mixture was concentrated *in vacuo* and the resulting residue was purified by chromatography on silica gel eluting with MeOH/DCM (0 - 10%) to give the title compound as a pale yellow solid (0.14 g, 71%):¹H NMR δ1.73 (4H, d), 2.56 (4H, s), 3.23 (3H, s), 3.71 (2H, s), 7.46 - 7.57 (3H, m), 7.87 - 7.94 (2H, m), 8.59 (1H, s), 11.5 (2H, br s); MS 493.

### Example 63: 2-Chloro-5-(2-(dimethylamino)ethoxy)-N-(6-(methylsulfonyl)benzothiazol-2-ylcarbamoyl)benzamide

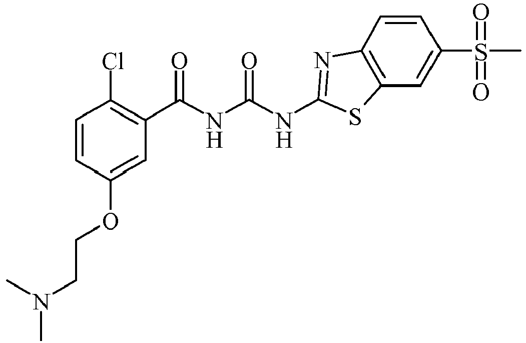

5-(2-Bromoethoxy)-2-chloro-N-(6-(methylsulfonyl)benzothiazol-2-ylcarbamoyl)benzamide (Intermediate 35, 0.18 g, 0.34 mmol) and dimethylamine (2M in THF, 0.34 mL, 0.68 mmol) were suspended in MeCN (3 mL) and the reaction mixture was heated at 140 °C in a microwave for 15 minutes, then cooled and concentrated *in vacuo.* The residue was triturated with MeOH, then filtered and the solid was purified by preparative HPLC to give the title compound as a colourless oil (10 mg, 6%): MS 497.

### Example 64: 2,5-Dichloro-N-[(6-methylsulfonylbenzothiazol-2-yl)carbamoyl]benzamide

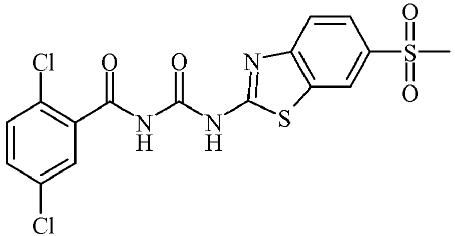

A solution of (4-fluorophenyl)-N-(6-methylsulfonylbenzothiazol-2-yl)carbamate (Intermediate 44, 0.30 g, 0.82 mmol) and 2,5-dichlorobenzamide (0.17 g, 0.90 mmol) in THF (10 mL) was treated with potassium *tert*-butoxide (1M in THF, 1.5 mL, 1.5 mmol) and the reaction mixture was heated at 65 °C for 4 hours under nitrogen. The mixture was treated with potassium *tert*-butoxide (1M in THF, 0.6 mL, 0.6 mmol) and heated at 65 °C for 2 hours and then more potassium *tert*-butoxide was added (1M in THF, 0.3 mL, 0.3 mmol) and the reaction mixture was heated at 65 °C overnight. More potassium *tert-*butoxide (1M in THF, 0.3 mL, 0.3 mmol) was added and the mixture was heated at 65 °C for 2 hours and then the mixture was diluted with H₂O (4 mL) and acidified with HCl (2M in H₂O, 2.5 mL, pH 4 - 5). The mixture was then diluted with H₂O (20 mL) and extracted with EtOAc (2 x 20 mL). The combined organic phases were concentrated *in vacuo* and the residue was triturated with MeOH (3 mL), then filtered and washed with MeOH (10 mL) to give the title compound as a white solid (0.12 g, 51%): ¹H NMR δ3.25 (3H, s), 7.64 (2H, s), 7.81 (1H, s), 7.96 (2H, s), 8.67 (1H, s), 11.77 (2H, s); MS 444.

### Example 65: 2-Chloro-5-methyl-N-(6-(methylsulfonyl)benzothiazol-2-ylcarbamoyl)benzamide

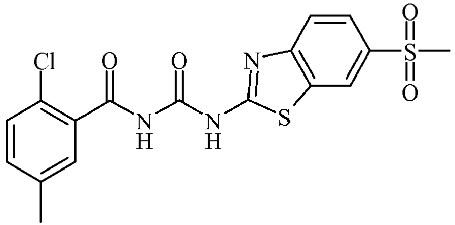

Example 65 was prepared by the general procedure of Example 64, using commercially available 2-chloro-5-methylbenzamide and (4-fluorophenyl)-N-(6-methylsulfonyl-benzothiazol-2-yl)carbamate (Intermediate 44) to give the title compound as a white solid (60 mg, 20%): ¹H NMR δ2.35 (3H, s), 3.25 (3H, s), 7.35 - 7.41 (1H, m), 7.44 - 7.50 (2H, m), 7.96 (2H, s), 8.67 (1H, s), 11.72 (1H, s), 11.85 (1H, s); MS 422.

### Example 66: 2-Chloro-N-[(6-methylsulfonylbenzothiazol-2-yl)carbamoyl]-5-pyrrol-1-yl-benzamide

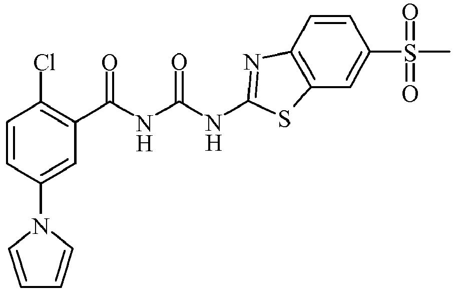

Example 66 was prepared by the general procedure of Example 64, using 2-chloro-5-pyrrol-1-yl-benzamide (Intermediate 40) and (4-fluorophenyl)-N-(6-methylsulfonylbenzothiazol-2-yl)carbamate (Intermediate 44). The crude solid was purified by preparative HPLC to give the title compound as a white solid (14 mg, 7%): ¹H NMR δ3.26 (3H, s), 6.33 (2H, t), 7.50 (2H, t), 7.67 (1H, d), 7.80 - 7.83 (1H, m), 7.98 - 8.00 (3H, m), 8.70 (1H, s), 11.82 (2H, s); MS 475.

### Example 67: 2-Chloro-N-(6-(methylsulfonyl)benzothiazol-2-ylcarbamoyl)-5-(1H-1,2,4-triazol-1-yl)benzamide

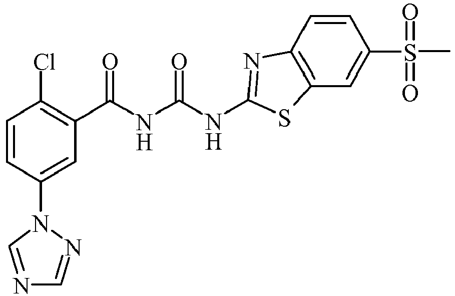

2-Chloro-5-(1H-1,2,4-triazol-1-yl)benzamide (Intermediate 15, 0.25 g, 1.12 mmol), 4-fluorophenyl 6-(methylsulfonyl)benzothiazol-2-ylcarbamate (Intermediate 44, 0.41 g, 1.12 mmol) and potassium *tert*-butoxide (1M in THF, 3.37 mL, 3.37 mmol) were suspended in THF (20 mL) and the reaction mixture was heated at 120 °C in a microwave for 15 minutes. The reaction mixture was cooled, diluted with THF and then neutralised with HCl (2M in H₂O) and concentrated *in vacuo.* The residue was purified by preparative HPLC to give the title compound as a colourless solid (58 mg, 11%): ¹H NMR δ3.20 (3H, s), 7.77 (1H, d), 7.92 (2H, s), 7.99 - 8.05 (1H, m), 8.19 (1H, s), 8.26 (1H, s), 8.62 (1H, s), 9.33 (1H, s), 11.68 - 11.97 (2H, m); MS 477.

### Example 68: 2-Chloro-N-(6-(methylsulfonyl)benzothiazol-2-ylcarbamoyl)-5-(thiazol-5-yl)benzamide

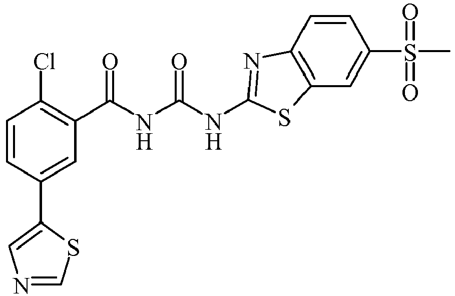

Example 68 was prepared by the general procedure of Example 67, using 2-chloro-5-(thiazol-5-yl)benzamide (Intermediate 12) and (4-fluorophenyl)-N-(6-methylsulfonylbenzothiazol-2-yl)carbamate (Intermediate 44) to give the title compound as a colourless solid (92 mg, 9%): ¹H NMR δ3.20 (3H, s), 7.63 (1H, d), 7.80 - 8.05 (4H, m), 8.39 (1H, s), 8.63 (1H, s), 9.11 (1H, s), 11.76 - 11.93 (2H, m); MS 493.

### Example 69: 2-Chloro-5-(1H-imidazol-1-yl)-N-(6-(methylsulfonyl)benzothiazol-2-ylcarbamoyl)benzamide

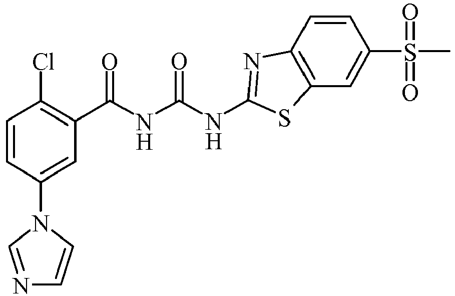

Example 69 was prepared by the general procedure of Example 67, using 2-chloro-5-(1H-imidazol-1-yl)benzamide (Intermediate 41) and (4-fluorophenyl)-N-(6-methylsulfonylbenzothiazol-2-yl)carbamate (Intermediate 44) to give the title compound as a brown solid (30mg, 17%):
¹H NMR δ3.26 (3H, s), 7.29 (1H, s), 7.78 (1H, d), 7.89 - 7.94 (2H, m), 7.97 (2H, s), 8.10 (1H, d), 8.58 (1H, s), 8.67 (1H, s), 11.66 - 12.23 (2H, m); MS 476.

### Example 70: 2-Chloro-N-(6-(methylsulfonyl)benzothiazol-2-ylcarbamoyl)-5-nitrobenzamide

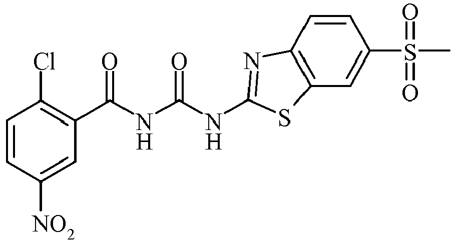

Oxalyl chloride (0.096 mL, 1.10 mmol) and 2-chloro-5-nitrobenzamide (0.20 g, 1.00 mmol) were dissolved in THF (3 mL) and heated at 120 °C in a microwave for 5 minutes. The reaction mixture was cooled, concentrated *in vacuo* and then the residue was diluted with DCE (3 mL) to give a solution of crude 2-chloro-5-nitrobenzoyl isocyanate. Trimethylaluminium (2M in hexanes, 0.75 mL, 1.50 mmol) was added to 6-(methylsulfonyl)benzothiazol-2-amine (0.23 g, 1.00 mmol) and DIPEA (0.35 mL, 1.99 mmol) in DCE (10 mL) and then the solution was stirred for 5 minutes under nitrogen and then cooled to 0 °C. The solution of crude 2-chloro-5-nitrobenzoyl isocyanate was added to the reaction mixture over 10 minutes at 0 °C, then the reaction mixture was warmed to room temperature and stirred for 30 minutes. The mixture was concentrated *in vacuo* and the residue was diluted with EtOAc (20 mL) and THF (20 mL), then washed with HCl (1M in H₂O, 20 mL) and then saturated brine (10 mL). The organic layer was dried, filtered and concentrated *in vacuo* and the residue was purified by chromatography on silica gel eluting with MeOH/DCM (0 - 10%) to give a solid that was then triturated with MeOH and filtered to give the title compound as an off-white solid (0.13 g, 27%): ¹H NMR δ3.24 (3H, s), 7.90 (1H, d), 7.96 (2H, s), 8.35 - 8.39 (1H, m), 8.58 (1H, s), 8.65 - 8.71 (1H, m), 11.84 (1H, s); MS 455.

### Example 71: 2-Chloro-N-[[6-(3-diethylaminopropylsulfonyl)benzothiazol-2-yl]carbamoyl]-5-pyrrol-1-yl-benzamide

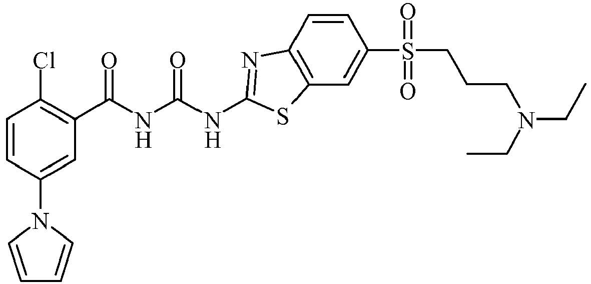

Oxalyl chloride (99 µL, 1.15 mmol) was added to a suspension of 2-chloro-5-pyrrol-1-yl-benzamide (Intermediate 40, 0.23 g, 1.05 mmol) in THF (10 mL) and the reaction mixture was heated at 60 °C for 90 minutes. The reaction mixture was cooled and 6-(3-iodopropylsulfonyl)benzothiazol-2-amine (Intermediate 47, 0.40 g, 1.05 mmol) was added, then the suspension was heated at 60 °C for 90 minutes. The reaction mixture was cooled and diethylamine (325 µL, 3.14 mmol) was added, then the reaction mixture was heated at 60 °C for 1 hour. The reaction mixture was cooled, concentrated *in vacuo* and the residue was purified by chromatography on silica gel eluting with MeOH/DCM (1 - 10%) to give the title compound as a white solid (0.14 g, 24%): ¹H NMR 81.03 - 1.10 (6H, m), 1.81 - 1.90 (2H, m), 2.54 - 2.57 (2H, m), 2.79 - 2.99 (4H, m), 3.40 - 3.46 (2H, m), 6.33 (2H, t), 7.48 (2H, t), 7.66 (1H, d), 7.81 (1H, dd), 7.89 - 7.99 (3H, m), 8.63 (1H, s); MS 574.

### Examples 72 - 74

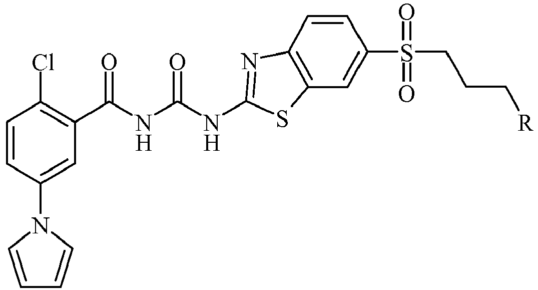

The following examples were prepared by the general procedure of Example 71, using 6-(3-iodopropylsulfonyl)benzothiazol-2-amine (Intermediate 47), 2-chloro-5-pyrrol-1-yl-benzamide (Intermediate 40) and commercially available amines.

| Example | R | ¹H NMR | MS |
|---|---|---|---|
| 72 | 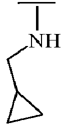 | 0.30 (2H, t), 0.53 - 0.56 (2H, m), 0.94 (1H, m), 1.89 (2H, t), 2.77 (2H, d), 2.98 (2H, t), 3.46 (2H, t), 6.31 (2H, t), 7.48 (2H, t), 7.61 (1H, d), 7.74 - 7.77 (1H, m), 7.84 (3H, d), 8.47 (1H, br s) | 572 |
| 73 | 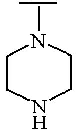 | 1.72 (2H, t), 2.36 (2H, q), 2.44 (4H, s), 3.03 (4H, t), 3.38 (2H, t), 6.33 (2H, t), 7.48 (2H, t), 7.67 (1H, d), 7.80 - 7.83 (1H, m), 7.90 -8.00 (3H, m), 8.65 (1H, s), 11.78 (2H, br s) | 587 |
| 74 | 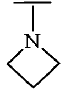 | 1.71 (2H, m), 2.23 (2H, t), 3.04 (2H, m), 3.38 - 3.42 (2H, m), 3.83 (4H, t), 6.33 (2H, t), 7.48 (2H, t), 7.66 (1H, d), 7.79 - 7.82 (1H, m), 7.89 - 7.91 (1H, m), 7.94 (2H, d), 8.61 (1H, s), 11.19 (2H, br s) | 558 |

### Examples 72 - 74

72: 2-Chloro-N-[[6-[3-(cyclopropylmethylamino)propylsulfonyl]benzothiazol-2-yl]carbamoyl]-5-pyrrol-1-yl-benzamide
73: 2-Chloro-N-[[6-(3-piperazin-1-ylpropylsulfonyl)benzothiazol-2-yl]carbamoyl]-5-pyrrol-1-yl-benzamide
74: N-[[6-[3-(Azetidin-1-yl)propylsulfonyl]benzothiazol-2-yl]carbamoyl]-2-chloro-5-pyrrol-1-yl-benzamide

### Example 75: 2-Chloro-N-[[6-[3-(propan-2-ylamino)propylsulfonyl]benzothiazol-2-yl]carbamoyl]-5-pyrrol-1-yl-benzamide

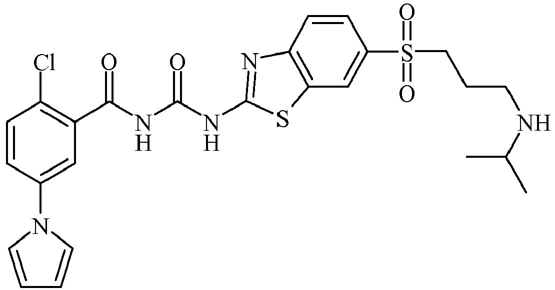

Isopropylamine (136 µL, 1.59 mmol) was added to a stirred solution of 2-chloro-N-[[6-(3-iodopropylsulfonyl)benzothiazol-2-yl]carbamoyl]-5-pyrrol-1-yl-benzamide (Intermediate 48, 0.25 g, 0.40 mmol) in THF (5 mL) and the reaction mixture was heated at 100 °C in a microwave for 15 minutes. The mixture was cooled, filtered and the solid was triturated with MeOH and Et₂O and then purified by reverse phase HPLC to give the title compound as a white solid (0.10 g, 45%): ¹H NMR δ1.18 (6H, d), 1.90 (2H, t), 2.97 (2H, m), 3.23 (1H, m), 3.52 (2H, t), 6.33 (2H, t), 7.49 (2H, t), 7.67 (1H, d), 7.81 - 7.84 (1H, m), 7.92 - 7.95 (1H, m), 7.98 (1H, s), 8.03 (1H, d), 8.49 (1H, br s), 8.69 (1H, s), 11.94 (1H, s), 11.99 (1H, s); MS 560.

### Example 76: 2-Chloro-N-[[6-[3-(2-methoxyethylamino)propylsulfonyl]benzothiazol-2-yl]carbamoyl]-5-pyrrol-1-yl-benzamide

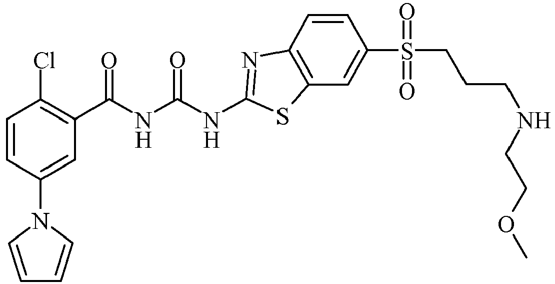

Example 76 was prepared by the general procedure of Example 75, using 2-chloro-N-[[6-(3-iodopropylsulfonyl)benzothiazol-2-yl]carbamoyl]-5-pyrrol-1-yl-benzamide (Intermediate 48) and commercially available 2-methoxyethylamine to give the title compound as a white solid (63 mg): ¹H NMR δ1.94 (2H, m), 3.00 (2H, m), 3.07 (2H, m), 3.28 (3H, s), 3.49 (2H, d), 3.54 (2H, m), 6.33 (2H, s), 7.49 (2H, s), 7.67 (1H, d), 7.82 (1H, d), 7.90 - 8.05 (4H, m), 8.68 (1H, br s), 11.95 (1H, s), 12.01 (1H, s); MS 576.

### Example 77: 2-Chloro-N-[[6-[(3S)-pyrrolidin-3-yl]sulfonylbenzothiazol-2-yl]carbamoyl]-5-pyrrol-1-yl-benzamide

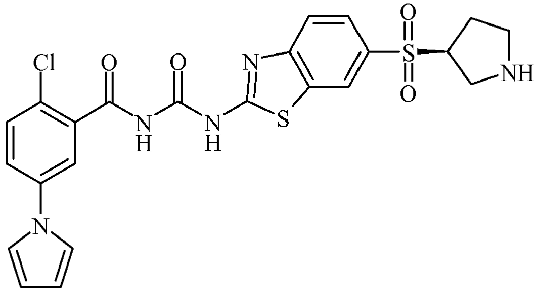

Oxalyl chloride (99 µL, 1.15 mmol) was added to a suspension of 2-chloro-5-pyrrol-1-yl-benzamide (Intermediate 40, 0.23 g, 1.04 mmol) in THF (10 mL) and the mixture was heated at 120 °C in a microwave for 5 minutes, then cooled and *tert*-butyl (3S)-3-(2-aminobenzothiazol-6-yl)sulfonylpyrrolidine-1-carboxylate (Intermediate 52, 0.40 g, 1.04 mmol) was added. The suspension was heated at 120 °C in a microwave for 5 minutes, then the reaction mixture was cooled, concentrated *in vacuo* and the residue was purified by chromatography on silica gel eluting with MeOH/DCM (2 - 15%) to give the title compound as a white solid (0.12 g, 22%): ¹H NMR 82.02 - 2.15 (2H, m), 3.04 - 3.07 (2H, m), 3.36 - 3.41 (2H, m), 4.06 - 4.10 (1H, m), 6.30 (2H, t), 7.48 (2H, t), 7.59 (1H, d), 7.72 - 7.75 (1H, m), 7.75 - 7.85 (3H, m), 8.45 (1H, s), 9.50 (1H, br s); MS 530.

### Example 78: 2-Chloro-N-[[6-[(3R)-pyrrolidin-3-yl]sulfonylbenzothiazol-2-yl]carbamoyl]-5-pyrrol-1-yl-benzamide

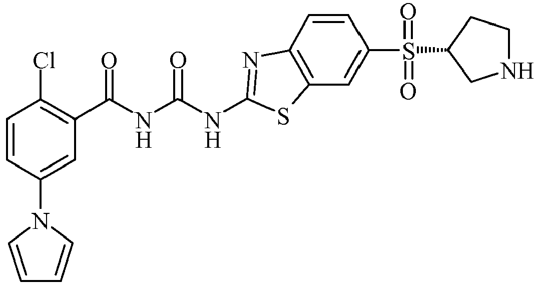

Example 78 was prepared by the general procedure of Example 77, using *tert*-butyl (3R)-3-(2-aminobenzothiazol-6-yl)sulfonylpyrrolidine-1-carboxylate (Intermediate 54) and 2-chloro-5-pyrrol-1-yl-benzamide (Intermediate 40) to give the title compound as a white solid (66 mg): ¹H NMR 82.18 - 2.28 (2H, m), 3.19 - 3.29 (2H, m), 3.44 - 3.50 (2H, m), 4.30 (1H, m), 6.33 (2H, t), 7.49 (2H, t), 7.67 (1H, d), 7.80 - 7.83 (1H, m), 7.94 - 8.02 (3H, m), 8.70 (1H, s); MS 530.

### Example 79: 2-Chloro-N-[[6-(2-piperazin-1-ylethylsulfonyl)benzothiazol-2-yl]carbamoyl]-5-pyrrol-1-yl-benzamide

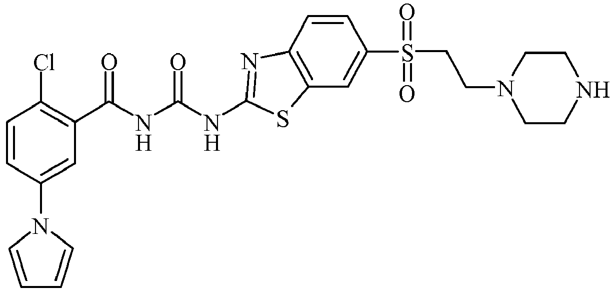

Oxalyl chloride (158 µL, 1.83 mmol) was added to a suspension of 2-chloro-5-pyrrol-1-yl-benzamide (Intermediate 40, 0.37 g, 1.66 mmol) in THF (4 mL) and the reaction mixture was heated at 120 °C in a microwave for 5 minutes. The mixture was cooled and 6-ethenylsulfonylbenzothiazol-2-amine (0.40 g, 1.66 mmol) was added and then the suspension was heated at 120 °C in a microwave for 5 minutes. The mixture was cooled, decanted, and the solution was concentrated *in vacuo* and then the residue was diluted with THF (4 mL). Piperazine (0.43 g, 4.99 mmol) was added to the solution and the reaction mixture was heated at 120 °C in a microwave for 5 minutes. The mixture was cooled, filtered and the solid was triturated with MeOH and Et₂O, and then filtered to give the title compound as a white solid (0.14 g, 15%): ¹H NMR 82.33 - 2.37 (4H, m), 2.63 (2H, t), 2.66 - 2.73 (4H, m), 3.48 (2H, t), 6.29 (2H, t), 7.47 (2H, t), 7.50 - 7.60 (2H, m), 7.65 - 7.72 (3H, m), 8.20 (1H, s), 9.20 (1H, br s); MS 573.

### Examples 80 - 82

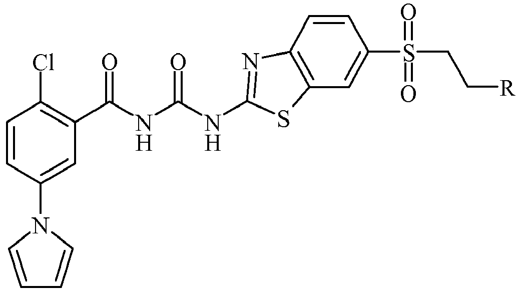

The following examples were prepared by the general procedure of Example 79, using 6-ethenylsulfonylbenzothiazol-2-amine, 2-chloro-5-pyrrol-1-yl-benzamide (Intermediate 40) and commercially available amines.

| Example | R | ¹H NMR | MS |
|---|---|---|---|
| 80 | 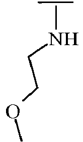 | 2.69 (2H, t), 2.90 (2H, t), 3.19 (3H, s), 3.35 - 3.37 (2H, m), 3.51 (2H, t), 6.32 (2H, t), 7.48 (2H, t), 7.63 (1H, d), 7.76 - 7.79 (1H, m), 7.86 - 7.91 (4H, m), 8.54 (1H, s) | 562 |
| 81 | 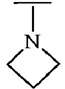 | 1.89 (2H, t), 2.65 - 2.72 (2H, m), 3.11 (4H, t), 3.30 - 3.40 (2H, m), 6.32 (2H, t), 7.49 (2H, t), 7.64 - 7.66 (1H, m), 7.78 - 7.82 (1H, m), 7.91 - 7.94 (3H, m), 8.59 (1H, s), 11.65 (2H, br s) | 544 |
| 82 | 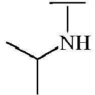 | 0.98 - 1.00 (6H, d), 2.95 (2H, t), 3.51 (2H, t), 6.31 (2H, t), 7.48 (2H, t), 7.61 (1H, d), 7.74 - 7.85 (4H, m), 8.48 (1H, s) | 546 |

### Examples 80 - 82

80: 2-Chloro-N-[[6-[2-(2-methoxyethylamino)ethylsulfonyl]benzothiazol-2-yl]carbamoyl]-5-pyrrol-1-yl-benzamide
81: N-[[6-[2-(Azetidin-1-yl)ethylsulfonyl]benzothiazol-2-yl]carbamoyl]-2-chloro-5-pyrrol-1-yl-benzamide
82: 2-Chloro-N-[[6-[2-(propan-2-ylamino)ethylsulfonyl]benzothiazol-2-yl]carbamoyl]-5-pyrrol-1-yl-benzamide

### Example 83: 2-Bromo-N-(6-(2-(isopropylamino)ethylsulfonyl)benzothiazol-2-ylcarbamoyl)-5-(1H-pyrrol-1-yl)benzamide

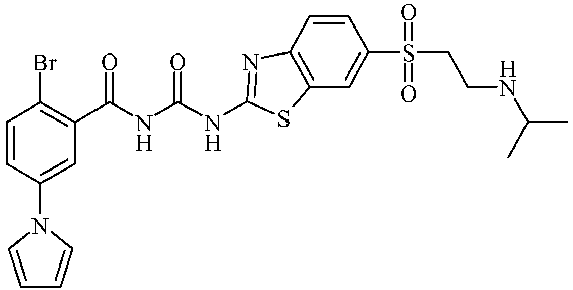

Example 83 was prepared by the general procedure of Example 79, using 6-ethenylsulfonylbenzothiazol-2-amine, 2-bromo-5-pyrrol-1-yl-benzamide (Intermediate 43) and commercially available isopropylamine. The crude solid was purified by preparative HPLC to give the title compound as an off-white solid (75 mg, 4%):
¹H NMR δ0.98 (6H, d), 2.88 (1H, m), 2.95 (2H, m), 3.50 (2H, m), 6.30 (2H, d), 7.45 (2H, d), 7.62 - 7.66 (1H, m), 7.73 (1H, d), 7.82 (3H, m), 8.14 (1H, s), 8.48 (1H, s); MS 592.

### Example 84: 2-Iodo-N-(6-(2-(isopropylamino)ethylsulfonyl)benzothiazol-2-ylcarbamoyl)-5-(1H-pyrrol-1-yl)benzamide

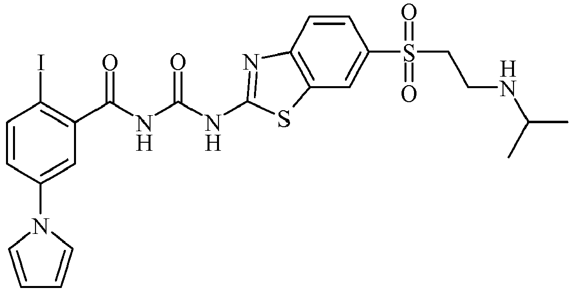

Copper(I) iodide (0.26 µL, 7.54 µmol) was added to sodium iodide (0.012 mL, 0.30 mmol), N,N'-dimethylethylenediamine (1.605 µL, 0.02 mmol) and 2-bromo-N-(6-(2-(isopropylamino)ethylsulfonyl)benzothiazol-2-ylcarbamoyl)-5-(1H-pyrrol-1-yl)benzamide (Example 83, 89 mg, 0.15 mmol) in dioxane (2 mL) under nitrogen and the reaction mixture was degassed and purged with nitrogen several times, then heated at 100 °C for 22 hours. The reaction mixture was purified by preparative HPLC to give the title compound as a yellow solid (2 mg, 2%): ¹H NMR (MeOD) δ1.17 (6H, d), 3.16 - 3.23 (2H, m), 3.52 (2H, t), 4.68 - 4.73 (1H, m), 6.23 (2H, s), 7.18 - 7.20 (2H, m), 7.32 - 7.36 (2H, m), 7.62 (1H, t), 7.91 (3H, m), 8.29 (1H, s), 8.51 (1H, s); MS 638.

### Example 85: 2-Chloro-N-(6-(3-(4-methylpiperazin-1-yl)propylsulfonyl)benzothiazol-2-ylcarbamoyl)-5-(1H-pyrrol-1-yl)benzamide

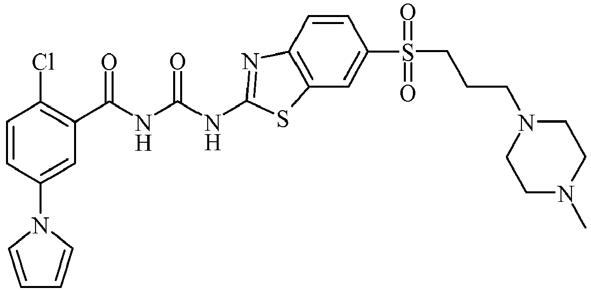

N-methylpiperazine (0.31 mL, 2.87 mmol) was added to 2-chloro-N-(6-(3-iodopropylsulfonyl)benzothiazol-2-ylcarbamoyl)-5-(1H-pyrrol-1-yl)benzamide (Intermediate 48, 0.60 g, 0.96 mmol) in THF (15 mL) and the reaction mixture was stirred for 16 hours. The mixture was diluted with THF (25 mL) and EtOAc (30 mL), then washed with H₂O (20 mL) and saturated brine (10 mL). The organic phase was dried, filtered, and concentrated *in vacuo.* The residue was purified by chromatography on silica gel eluting with MeOH/DCM (0 - 10%) and then by preparative HPLC to give the title compound as a white solid (0.11 g, 19%): ¹H NMR δ1.66 - 1.76 (2H, m), 2.36 - 2.43 (9H, m), 2.70 (4H, s), 3.35 (2H, t), 6.30 (2H, d), 7.43 (2H, d), 7.60 (1H, d), 7.72 - 7.75 (1H, m), 7.84 - 7.91 (3H, m), 8.16 (1H, s), 8.54 (1H, s); MS 601.

### Example 86: 2-Chloro-N-(6-(3-(4-methylpiperazin-1-yl)propylsulfonyl)benzothiazol-2-ylcarbamoyl)-5-(pyridin-2-yl)benzamide

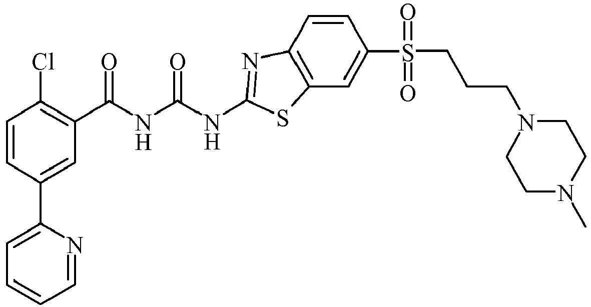

Oxalyl chloride (0.26 mL, 3.01 mmol) was added to a suspension of 2-chloro-5-(pyridin-2-yl)benzamide (Intermediate 4, 0.70 g, 3.01 mmol) in THF (15 mL) and the reaction mixture was heated at 120 °C in a microwave for 5 minutes. The reaction mixture was cooled and 6-(3-iodopropylsulfonyl)benzothiazol-2-amine (Intermediate 47, 1.04 g, 2.71 mmol) was added portionwise and the suspension was heated at 120 °C in a microwave for 5 minutes. The reaction mixture was cooled and 1-methylpiperazine (1.0 mL, 9.03 mmol) was added portionwise and the suspension was stirred for 16 hours. The reaction mixture was concentrated *in vacuo* and the residue was purified by chromatography on silica gel eluting with MeOH/DCM (0 - 10%) to give the title compound as an off-white solid (0.11 g, 6%): ¹HNMR δ1.66 - 1.76 (2H, m), 2.27 - 2.41 (9H, m), 2.58 (4H, s), 3.28 - 3.36 (2H, m), 7.39 - 7.44 (1H, m), 7.70 (1H, d), 7.86 - 7.97 (3H, m), 8.07 (1H, d), 8.24 - 8.28 (1H, m), 8.36 - 8.37 (1H, m), 8.59 (1H, s), 8.70 (1H, d), 11.30 (2H, br s); MS (M-H)⁺ 611.

### Examples 87 - 94

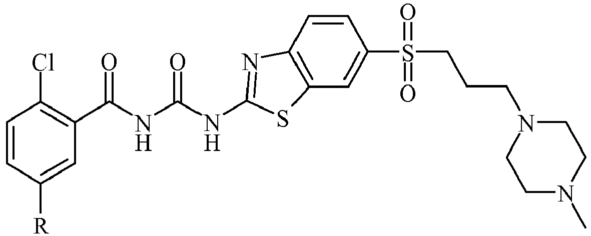

The following examples were prepared by the general procedure of Example 86, using 6-(3-iodopropylsulfonyl)benzothiazol-2-amine (Intermediate 47), benzamides (Intermediates 16, 13, 29, 37, 39, 28, 26 and 19 respectively) and commercially available 1-methylpiperazine.

| Example | R | ¹H NMR | MS |
|---|---|---|---|
| 87 | 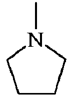 | 1.67 - 1.77 (2H, m), 1.96 (4H, s), 2.34 - 2.46 (9H, m), 2.72 (4H, s), 3.24 - 3.42 (6H, m), 6.63 - 6.69 (1H, m), 6.75 (1H, s), 7.29 (1H, d), 7.89 - 7.97 (2H, m), 8.64 (1H, s) | 607 |
| 88 | 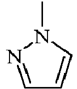 | 1.69 - 1.78 (2H, m), 2.37 - 2.47 (9H, m), 2.64 (4H, s), 3.33 - 3.38 (2H, m), 6.61 (1H, t), 7.72 (1H, d), 7.81 (1H, d), 7.89 - 7.93 (1H, m), 7.97 (1H, d), 8.02 - 8.06 (1H, m), 8.17 (1H, d), 8.58 (1H, d), 8.63 (1H, d) | 604 |
| 89 | 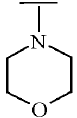 | 1.71 - 1.78 (2H, m), 2.38 - 2.47 (9H, m), 2.60 (4H, s), 3.18 - 3.20 (2H, m), 3.37 (4H, t), 3.75 (4H, t), 7.11 - 7.14 (1H, m), 7.22 (1H, d), 7.40 (1H, d), 7.91 - 7.99 (2H, m), 8.65 (1H, d), 11.14 (2H, br s) | 623 |
| 90 | 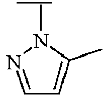 | 2.61 (4H, s), 2.94 (3H, s), 3.35 (2H, t), 6.32 (1H, s), 7.62 (1H, s), 7.73 (2H, s), 7.85 (1H, s), 7.89 - 7.99 (2H, m), 8.62 (1H, s), 11.10 (2H, br s) | 618 |
| 91 | 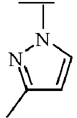 | 2.61 (4H, s), 2.95 (3H, s), 3.35 (2H, t), 6.39 (1H, d), 7.67 (1H, d), 7.89 - 7.99 (3H, m), 8.10 (1H, d), 8.45 (1H, d), 8.62 (1H, d), 11.13 (2H, br s) | 618 |
| 92 | 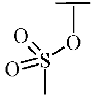 | 1.70 - 1.78 (2H, m), 2.39 - 2.45 (2H, m), 2.52 - 2.61 (4H, m), 2.67 (4H, s), 3.03 (3H, s), 3.35 - 3.39 (2H, m), 3.49 (3H, s), 7.56 - 7.59 (1H, m), 7.73 - 7.75 (2H, m), 7.91 - 7.98 (2H, m), 8.64 (1H, d), 11.16 (2H, br s) | 630 |
| 93 | 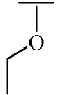 | 1.41 (3H, t), 1.76 - 1.84 (2H, m), 2.45 - 2.51 (9H, m), 2.71 (4H, s), 3.40 - 3.45 (2H, m), 4.13 - 4.18 (2H, m), 7.18 - 7.20 (1H, m), 7.31 (1H, d), 7.54 (1H, d), 7.97 - 8.06 (2H, m), 8.72 (1H, d), 11.21 (2H, br s) | 582 |
| 94 | 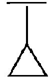 | 0.72 - 0.77 (2H, m), 0.98 - 1.04 (2H, m), 1.68 - 1.77 (2H, m), 1.95 - 2.04 (1H, m), 2.37 - 2.47 (9H, m), 2.86 (4H, s), 3.31 - 3.38 (2H, m), 7.26 - 7.30 (1H, m), 7.34 - 7.36 (1H, m), 7.43 (1H, d), 7.89 - 7.92 (1H, m), 7.95 - 7.98 (1H, m), 8.63 (1H, d), 11.15 (2H, br s) | 576 |

### Examples 87 - 94

87: 2-Chloro-N-(6-(3-(4-methylpiperazin-1-yl)propylsulfonyl)benzothiazol-2-ylcarbamoyl)-5-(pyrrolidin-1-yl)benzamide
88: 2-Chloro-N-(6-(3-(4-methylpiperazin-1-yl)propylsulfonyl)benzothiazol-2-ylcarbamoyl)-5-(1H-pyrazol-1-yl)benzamide
89: 2-Chloro-N-(6-(3-(4-methylpiperazin-1-yl)propylsulfonyl)benzothiazol-2-ylcarbamoyl)-5-morpholinobenzamide
90: 2-Chloro-5-(5-methyl-1H-pyrazol-1-yl)-N-(6-(3-(4-methylpiperazin-1-yl)propylsulfonyl)benzothiazol-2-ylcarbamoyl)benzamide
91: 2-Chloro-5-(3-methyl-1H-pyrazol-1-yl)-N-(6-(3-(4-methylpiperazin-1-yl)propylsulfonyl)benzothiazol-2-ylcarbamoyl)benzamide
92: 4-Chloro-3-(6-(3-(4-methylpiperazin-1-yl)propylsulfonyl)benzothiazol-2-ylcarbamoylcarbamoyl)phenyl methanesulfonate
93: 2-Chloro-5-ethoxy-N-(6-(3-(4-methylpiperazin-1-yl)propylsulfonyl)benzothiazol-2-ylcarbamoyl)benzamide
94: 2-Chloro-5-cyclopropyl-N-(6-(3-(4-methylpiperazin-1-yl)propylsulfonyl)benzothiazol-2-ylcarbamoyl)benzamide

### Example 95: 2-Chloro-N-(6-(3-(4-methylpiperazin-1-yl)propylsulfonyl)benzothiazol-2-ylcarbamoyl)-5-(1H-1,2,4-triazol-1-yl)benzamide

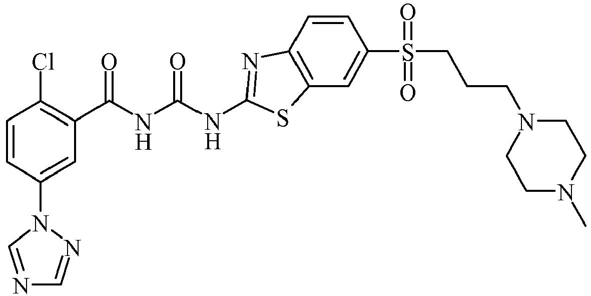

2-Chloro-5-(1H-1,2,4-triazol-1-yl)benzamide (Intermediate 15, 0.41 g, 1.86 mmol), 4-fluorophenyl 6-(3-(4-methylpiperazin-1-yl)propylsulfonyl)benzothiazol-2-ylcarbamate (Intermediate 58, 0.92 g, 1.86 mmol) and potassium *tert*-butoxide (1M in THF, 5.58 mL, 5.58 mmol) were suspended in THF (35 mL) and the reaction mixture was heated at 120 °C in a microwave for 15 minutes. The reaction mixture was cooled, diluted with THF and neutralised with HCl (2M in H₂O) and then concentrated *in vacuo.* The residue was purified by preparative HPLC to give the title compound as a white solid (0.11 g, 9%):
¹H NMR δ1.66 1.77 (2H, m), 2.30 - 2.42 (11H, m), 2.56 - 2.62 (2H, m), 3.33 (2H, t), 7.78 (1H, d), 7.82 - 7.90 (2H, m), 8.01 - 8.06 (1H, m), 8.16 (1H, d), 8.30 (1H, s), 8.52 (1H, s), 9.38 (1H, s), 10.46 - 12.85 (2H, m); MS 603.

### Example 96: 2-Chloro-N-(6-(3-(4-methylpiperazin-1-yl)propylsulfonyl)benzothiazol-2-ylcarbamoyl)-5-(thiazol-5-yl)benzamide

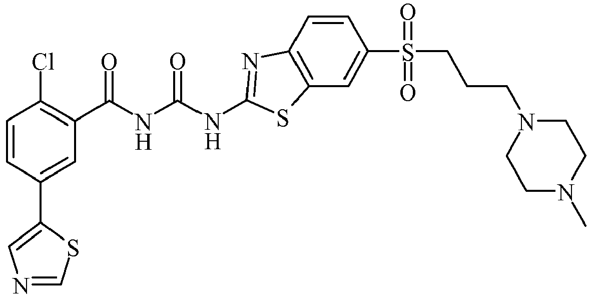

Example 96 was prepared by the general procedure of Example 95, using 2-chloro-5-(thiazol-5-yl)benzamide (Intermediate 12) and 4-fluorophenyl 6-(3-(4-methylpiperazin-1-yl)propylsulfonyl)benzothiazol-2-ylcarbamate (Intermediate 58) to give the title compound as a yellow solid (0.13 g, 10%):
¹H NMR δ1.59 - 1.72 (2H, m), 2.24 - 2.36 (11H, m), 2.49 - 2.58 (2H, m), 3.25 - 3.31 (2H, m), 7.59 (1H, d), 7.77 - 7.89 (3H, m), 7.93 (1H, d), 8.36 (1H, s), 8.51 (1H, s), 9.09 (1H, s), 10.72 - 12.16 (2H, m); MS 619.

### Example 97: 2-Chloro-N-(6-(3-(diethylamino)propylsulfonyl)benzothiazol-2-ylcarbamoyl)-5-morpholinobenzamide

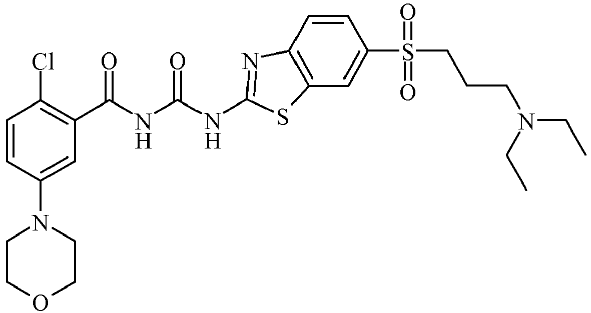

Oxalyl chloride (6.52 mL, 74.79 mmol) was added to 2-chloro-5-morpholin-4-yl-benzamide (Intermediate 29, 18 g, 74.79 mmol) in THF (400 mL) and the reaction mixture was heated at 60 °C for 2 hours under nitrogen. A solution of 6-(3-iodopropylsulfonyl)benzothiazol-2-amine (Intermediate 47, 26.0 g, 67.99 mmol) in THF (200 mL) was added to the reaction mixture and the suspension was heated at 60 °C for 90 minutes. The reaction mixture was cooled to 50 °C and diethylamine (21.10 mL, 203.96 mmol) was added. The suspension was stirred for 16 hours then heated at 50 °C for 90 minutes. DMF (100 mL) was added and the reaction mixture was heated at 50 °C for 2 hours then diethylamine (21.10 mL, 203.96 mmol) was added and the reaction mixture was heated at 50 °C overnight. Sodium iodide (5.56 mL, 135.97 mmol) and diethylamine (21.10 mL, 203.96 mmol) were added and the reaction mixture was heated at 50 °C for 3 days. The reaction mixture was cooled, diluted with THF (200 mL) and EtOAc (200 mL) and then washed with H₂O (2 x 300 mL) and saturated brine (200 mL). The organic phase was dried, filtered and concentrated *in vacuo* and the residue was purified by chromatography on silica gel eluting with MeOH/DCM (0 - 15%) and then triturated with MeOH to give the title compound as an off-white solid (13.40 g, 33%):
¹H NMR δ0.96 (6H, t), 1.71 - 1.81 (2H, m), 2.55 - 2.73 (6H, m), 3.26 - 3.56 (6H, m), 3.74 (4H, t), 7.07 - 7.14 (1H, m), 7.19 (1H, d), 7.38 (1H, d), 7.91 (2H, q), 8.61 (1H, s), 10.84 - 11.95 (2H, m); MS 594.

### Example 98: 2-Chloro-N-(6-(3-(diethylamino)propylsulfonyl)benzothiazol-2-ylcarbamoyl)-5-(1H-pyrazol-1-yl)benzamide

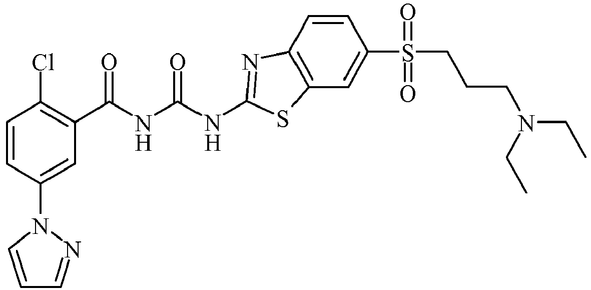

Diethylamine (28.3 mL, 273.39 mmol) was added to 2-chloro-N-(6-(3-iodopropylsulfonyl)benzothiazol-2-ylcarbamoyl)-5-(1H-pyrazol-1-yl)benzamide (Intermediate 60, 57.4 g, 91.13 mmol) in THF (300 mL) and the suspension was stirred for 16 hours under nitrogen. Diethylamine (9.4 mL, 91.13 mmol) was added and the mixture was stirred for 24 hours and then concentrated *in vacuo* and the residue was dissolved in EtOAc/THF (1:1, 200 mL) and washed with H₂O (100 mL) and saturated brine (100 mL). The organic phase was dried, filtered and concentrated *in vacuo* and the residue was purified by chromatography on silica gel eluting with MeOH/DCM (0 - 20%) and then chromatography on silica gel eluting with MeOH/EtOAc (0 - 20%) to give a solid that was triturated with H₂O and then slurried in MeOH, filtered and washed with Et₂O and then slurried in EtOAc and filtered to give the title compound as an off-white solid (7.26 g, 14%): ¹H NMR δ1.08 (6H, t), 1.91 - 1.99 (2H, m), 3.04 (6H, q), 3.45 (2H, t), 6.60 (1H, d), 7.72 (1H, d), 7.81 (1H, s), 7.90 - 8.05 (2H, m), 8.06 (1H, d), 8.17 (1H, d), 8.57 (1H, d), 8.65 (1H, d); MS 575.

### Example 99: 2-Chloro-N-(6-(1-methylpiperidin-4-ylsulfonyl)benzothiazol-2-ylcarbamoyl)-5-morpholinobenzamide

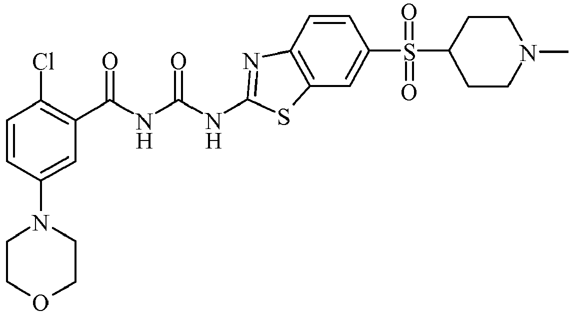

2-Chloro-5-morpholinobenzamide (Intermediate 29, 0.72 g, 3.0 mmol) and oxalyl chloride (0.28 mL, 3.15 mmol) were suspended in THF (15 mL) and heated at 120 °C in a microwave for 5 minutes. The reaction mixture was cooled and *tert*-butyl 4-(2-aminobenzothiazol-6-ylsulfonyl)piperidine-1-carboxylate (Intermediate 56, 1.07 g, 2.70 mmol) was added and then the reaction mixture was heated at 120 °C in a microwave for 5 minutes. The reaction mixture was cooled, concentrated *in vacuo* and the residue was dissolved in a mixture of acetyl chloride (10 mL) and MeOH (50 mL). The solution was stirred for 2 hours and then concentrated *in vacuo* and the residue was diluted with DCM (50 mL) and saturated aqueous sodium bicarbonate solution. The suspension was filtered to give crude 2-chloro-5-morpholino-N-(6-(piperidin-4-ylsulfonyl)benzothiazol-2-ylcarbamoyl)benzamide (1.02 g, 1.81 mmol) which was dissolved in formic acid (20 mL) and then formaldehyde (37% w/w in H₂O, 2.0 mL) was added and the solution was heated at 100 °C for 3 hours. The reaction mixture was concentrated *in vacuo* and the residue was dissolved in H₂O and then the solution was neutralised with saturated aqueous sodium bicarbonate solution and extracted with DCM (3 x 150 mL). The combined organic phases were concentrated *in vacuo* and the residue was purified by chromatography on silica gel eluting with MeOH/DCM (0 - 10%) to give the title compound as a white solid (0.23 g, 15%): ¹H NMR δ1.56 1.63 (2H, m), 1.83 - 1.97 (4H, m), 2.18 (3H, s), 2.88 (2H, d), 3.18 (4H, t), 3.75 (4H, t), 7.09 - 7.12 (1H, m), 7.20 (1H, d), 7.38 (1H, d), 7.83 - 7.86 (1H, m), 7.94 (1H, d), 8.57 (1H, d), 11.65 (2H, br s); MS 578.

### Example 100: 2-Chloro-N-(6-(1-methylpiperidin-4-ylsulfonyl)benzothiazol-2-ylcarbamoyl)-5-(1H-pyrazol-1-yl)benzamide

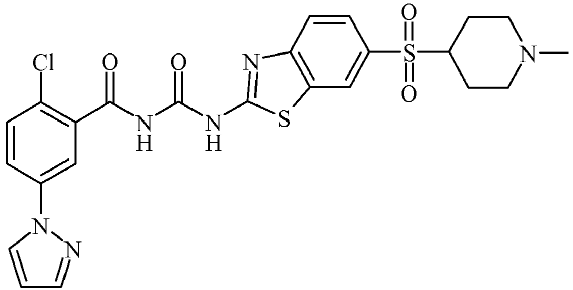

Acetyl chloride (30 mL, 421.9 mmol) and *tert*-butyl 4-(2-(3-(2-chloro-5-(1H-pyrazol-1-yl)benzoyl)ureido)benzothiazol-6-ylsulfonyl)piperidine-1-carboxylate (Intermediate 59, 15 g, 23.25 mmol) were added to ice-cold MeOH (500 mL) and the suspension was heated at 50 °C for 1 hour. The reaction mixture was concentrated *in vacuo* and suspended in MeOH (300 mL) and AcOH (60 mL) and then a solution of formaldehyde in water (37%, 14 mL) and sodium cyanoborohydride (4.40 g, 70 mmol) were added and the suspension was stirred for 3 hours. The reaction mixture was concentrated *in vacuo* and the residue was suspended in H₂O and then saturated aqueous sodium bicarbonate solution was added. The suspension was filtered and the solid was washed with H₂O, EtOH and Et₂O to give the title compound as a solid (9.95 g, 77%):
¹H NMR δ1.64 1.74 (2H, m), 1.95 (2H, d), 2.35 (2H, m), 2.40 (3H, s), 3.12 (2H, d), 6.61 - 6.62 (1H, m), 7.72 (1H, d), 7.82 - 7.86 (2H, m), 7.96 (1H, d), 8.02 - 8.05 (1H, m), 8.17 (1H, d), 8.57 - 8.58 (1H, m), 8.61 - 8.62 (1H, m), 11.60 (2H, br s); MS 559.

The following Examples were prepared in a similar manner:
**Example 101** 2,6-Dichloro-N-[(6-methylsulfonylbenzothiazol-2-yl)carbamoyl]benzamide
**Example 102** 2-Chloro-N-[(6-methylsulfonylbenzothiazol-2-yl)carbamoyl]benzamide
**Example 103** 2-Bromo-N-[(6-methylsulfonylbenzothiazol-2-yl)carbamoyl]benzamide
**Example 104** N-[(6-Methylsulfonylbenzothiazol-2-yl)carbamoyl]-2-nitro-benzamide
**Example 105** N-[(6-Methylsulfonylbenzothiazol-2-yl)carbamoyl]-2-phenyl-benzamide
**Example 106** 2-Chloro-6-fluoro-N-[(6-methylsulfonylbenzothiazol-2-yl)carbamoyl]benzamide
**Example 107** 2-Chloro-4-fluoro-N-[(6-methylsulfonylbenzothiazol-2-yl)carbamoyl]benzamide
**Example 108** 2-Fluoro-N-[(6-methylsulfonylbenzothiazol-2-yl)carbamoyl]benzamide
**Example 110** 2-Chloro-3-fluoro-N-[(6-methylsulfonylbenzothiazol-2-yl)carbamoyl]benzamide
**Example 111** 2-Chloro-3,4-dimethoxy-N-[(6-methylsulfonylbenzothiazol-2-yl)carbamoyl]benzamide
**Example 112** 2,6-Difluoro-N-[(6-methylsulfonylbenzothiazol-2-yl)carbamoyl]benzamide
**Example 113** 2-Chloro-4-methylsulfonyl-N-[(6-methylsulfonylbenzothiazol-2-yl)carbamoyl]benzamide
**Example 115** 2-Chloro-N-[(6-methylsulfonylbenzothiazol-2-yl)carbamoyl]-4-morpholin-4-yl-benzamide
**Example 116** 2-Chloro-N-[(6-methylsulfonylbenzothiazol-2-yl)carbamoyl]-4-pyrazol-1-yl-benzamide
**Example 117** 2-Chloro-N-[(6-methylsulfonylbenzothiazol-2-yl)carbamoyl]-4-pyrrolidin-1-yl-benzamide
**Example 118** 2-Iodo-N-[(6-methylsulfonylbenzothiazol-2-yl)carbamoyl]benzamide
**Example 119** 2-Chloro-4-(2,5-dimethylpyrrol-1-yl)-N-[(6-methylsulfonylbenzothiazol-2-yl)carbamoyl]benzamide
**Example 120** N-[[6-[2-(Carbamoylmethoxy)ethylsulfonyl]benzothiazol-2-yl]carbamoyl]-2-chloro-benzamide
**Example 121** 2-Chloro-N-[[6-[2-(2-hydroxyethoxy)ethylsulfonyl]benzothiazol-2-yl]carbamoyl]benzamide
**Example 122** 2-[2-[2-[(2-Chlorobenzoyl)carbamoylamino]benzothiazol-6-yl] sulfonylethoxy] acetic acid
**Example 123** 2-(4-Fluorophenyl)-4-methoxy-N-[(6-methylsulfonylbenzothiazol-2-yl)carbamoyl]benzamide
**Example 124** 2-(4-Methoxyphenyl)-N-[(6-methylsulfonylbenzothiazol-2-yl)carbamoyl]benzamide
**Example 125** 2-(2-Fluorophenyl)-N-[(6-methylsulfonylbenzothiazol-2-yl)carbamoyl]benzamide
**Example 126** 2-(4-Fluorophenyl)-N-[(6-methylsulfonylbenzothiazol-2-yl)carbamoyl]benzamide
**Example 127** N-[(6-Methylsulfonylbenzothiazol-2-yl)carbamoyl]-2-phenoxy-benzamide
**Example 128** 2-Methyl-N-[(6-methylsulfonylbenzothiazol-2-yl)carbamoyl]benzamide
**Example 129** 2-Chloro-N-[(6-ethenylsulfonylbenzothiazol-2-yl)carbamoyl]benzamide
**Example 130** 2-Ethylsulfanyl-N-[(6-methylsulfonylbenzothiazol-2-yl)carbamoyl]benzamide
**Example 131** N-[(6-Methylsulfonylbenzothiazol-2-yl)carbamoyl]-2-(phenoxymethyl)benzamide
**Example 132** 2-Methylsulfanyl-N-[(6-methylsulfonylbenzothiazol-2-yl)carbamoyl]benzamide
**Example 133** N-[(6-Methylsulfonylbenzothiazol-2-yl)carbamoyl]-2-phenylsulfanyl-benzamide
**Example 134** N-[(6-Methylsulfonylbenzothiazol-2-yl)carbamoyl]-2-pyrrol-1-yl-benzamide
**Example 135** 2-Ethylsulfonyl-N-[(6-methylsulfonylbenzothiazol-2-yl)carbamoyl]benzamide
**Example 136** N-[(6-Methylsulfonylbenzothiazol-2-yl)carbamoyl]-2-propan-2-yl-benzamide
**Example 137** N-[(6-Methylsulfonylbenzothiazol-2-yl)carbamoyl]-2-(trifluoromethylsulfonyloxy)benzamide
**Example 138** 2-Chloro-N-[(6-methylsulfonylbenzothiazol-2-yl)carbamoyl]-5-sulfamoyl-benzamide
**Example 139** 2-Chloro-N-[[6-(3-piperidylsulfonyl)benzothiazol-2-yl]carbamoyl]benzamide
**Example 140** 2-Chloro-N-[[6-[(1-propan-2-yl-3-piperidyl)sulfonyl]benzothiazol-2-yl]carbamoyl]benzamide
**Example 141** 2-Ethyl-N-[(6-methylsulfonylbenzothiazol-2-yl)carbamoyl]benzamide
**Example 142** 2-Chloro-N-[[6-[(1-ethyl-3-piperidyl)sulfonyl]benzothiazol-2-yl]carbamoyl]benzamide
**Example 143** 2-Chloro-N-[[6-[[1-(cyclopropylmethyl)-3-piperidyl]sulfonyl]benzothiazol-2-yl]carbamoyl]benzamide
**Example 144** 2-Chloro-N-[[6-[1-(cyclopropylmethyl)pyrrolidin-3-yl]sulfonylbenzothiazol-2-yl]carbamoyl]benzamide
**Example 145** 2-Chloro-N-[[6-[3-(propan-2-ylamino)propylsulfonyl]benzothiazol-2-yl]carbamoyl]benzamide
**Example 146** 2-Chloro-N-[[6-(3-morpholin-4-ylpropylsulfonyl)benzothiazol-2-yl]carbamoyl]benzamide
**Example 147** N-[[6-[3-(Azetidin-1-yl)propylsulfonyl]benzothiazol-2-yl]carbamoyl]-2-chloro-benzamide
**Example 148** 2-Chloro-N-[[6-(3-chloropropylsulfonyl)benzothiazol-2-yl]carbamoyl]benzamide
**Example 149** 2-Chloro-N-[[6-(3-pyrrolidin-1-ylpropylsulfonyl)benzothiazol-2-yl]carbamoyl]benzamide
**Example 150** 2-Chloro-N-[[6-[3-(1;1-dioxo-1,4-thiazinan-4-yl)propylsulfonyl]benzothiazol-2-yl]carbamoyl]benzamide
**Example 151** 2-Chloro-N-[[6-[3-(cyclopropylmethylamino)propylsulfonyl]benzothiazol-2-yl] carbamoyl]benzamide
**Example 152** 2-Chloro-N-[[6-[3-(1-piperidyl)propylsulfonyl]benzothiazol-2-yl]carbamoyl]benzamide
**Example 153** 2-Chloro-N-[[6-(3-diethylaminopropylsulfonyl)benzothiazol-2-yl]carbamoyl]benzamide
**Example 154** 2-Chloro-N-[[6-[3-(3,3-difluoropyrrolidin-1-yl)propylsulfonyl]benzothiazol-2-yl]carbamoyl]benzamide
**Example 155** 2-Chloro-N-[[6-[3-(4-methylpiperazin-1-yl)propylsulfonyl]benzothiazol-2-yl]carbamoyl]benzamide
**Example 156** 2-Chloro-N-[[6-[[(2R)-1-(cyclopropylmethyl)pyrrolidin-2-yl]methylsulfonyl]benzothiazol-2-yl]carbamoyl]benzamide
**Example 157** 2-Chloro-N-[[6-[3-(3-fluoropyrrolidin-1-yl)propylsulfonyl]benzothiazol-2-yl]carbamoyl]benzamide
**Example 158** 2-Chloro-N-[[6-[3-(4,4-difluoro-1-piperidyl)propylsulfonyl]benzothiazol-2-yl]carbamoyl]benzamide
**Example 159** 2-Chloro-N-[[6-[3-(2-hydroxyethylamino)propylsulfonyl]benzothiazol-2-yl]carbamoyl]benzamide
**Example 160** 2-Chloro-N-[[6-[3-(3-hydroxypyrrolidin-1-yl)propylsulfonyl]benzothiazol-2-yl]carbamoyl]benzamide
**Example 161** 5-Bromo-2-chloro-N-[(6-methylsulfonylbenzothiazol-2-yl)carbamoyl]benzamide
**Example 162** 2-Chloro-N-[[6-(1-propan-2-ylpyrrolidin-3-yl)sulfonylbenzothiazol-2-yl]carbamoyl]benzamide
**Example 163** 2-Chloro-N-[[6-(1-ethylpyrrolidin-3-yl)sulfonylbenzothiazol-2-yl]carbamoyl]benzamide
**Example 164** 2-Chloro-N-[[6-[[(2R)-1-ethylpyrrolidin-2-yl]methylsulfonyl]benzothiazol-2-yl] carbamoyl]benzamide
**Example 165** 2-Chloro-N-[[6-[[(2R)-pyrrolidin-2-yl]methylsulfonyl]benzothiazol-2-yl]carbamoyl]benzamide
**Example 166** 2-Chloro-N-[[6-[(1-methyl-3-piperidyl)sulfonyl]benzothiazol-2-yl]carbamoyl]benzamide
**Example 167** 2-Chloro-N-[[6-(1-methylpyrrolidin-3-yl)sulfonylbenzothiazol-2-yl]carbamoyl]benzamide
**Example 168** 2-Chloro-N-[[6-[[(2R)-1-propan-2-ylpyrrolidin-2-yl]methylsulfonyl]benzothiazol-2-yl]carbamoyl]benzamide
**Example 169** 2-Chloro-N-[[6-[[(2R)-1-methylpyrrolidin-2-yl]methylsulfonyl]benzothiazol-2-yl]carbamoyl]benzamide
**Example 170** N-[(6-Methylsulfonylbenzothiazol-2-yl)carbamoyl]-2-(trifluoromethyl)benzamide
**Example 171** 2-Chloro-N-[[6-[(3S)-pyrrolidin-3-yl]sulfonylbenzothiazol-2-yl]carbamoyl]benzamide
**Example 172** 2-Chloro-N-[[6-[(3R)-pyrrolidin-3-yl]sulfonylbenzothiazol-2-yl]carbamoyl]benzamide
**Example 173** 2-Chloro-N-[[6-[(1-ethyl-4-piperidyl)sulfonyl]benzothiazol-2-yl]carbamoyl]benzamide
**Example 174** 2-Chloro-N-[[6-[(1-propan-2-yl-4-piperidyl)sulfonyl]benzothiazol-2-yl]carbamoyl]benzamide
**Example 175** 2-Chloro-N-[[6-[1-(cyclopropylmethyl)azetidin-3-yl]sulfonylbenzothiazol-2-yl] carbamoyl]benzamide
**Example 176** 2-Chloro-N-[[6-(4-piperidylsulfonyl)benzothiazol-2-yl]carbamoyl]benzamide
**Example 177** 2-Chloro-4,5-difluoro-N-[(6-methylsulfonylbenzothiazol-2-yl)carbamoyl]benzamide
**Example 178** 2-Chloro-N-[[6-(pyridin-2-ylmethylsulfonyl)benzothiazol-2-yl]carbamoyl]benzamide
**Example 179** 2,4-Dichloro-N-[(6-methylsulfonylbenzothiazol-2-yl)carbamoyl]benzamide
**Example 180** 2-Chloro-N-[(6-methylsulfonylbenzothiazol-2-yl)carbamoyl]-4-nitrobenzamide
**Example 181** 2-Chloro-N-[(6-methylsulfonylbenzothiazol-2-yl)carbamoyl]-5-(trifluoromethyl)benzamide
**Example 182** 2-Chloro-N-[[6-[[1-(cyclopropylmethyl)-4-piperidyl]sulfonyl]benzothiazol-2-yl] carbamoyl]benzamide
**Example 183** N-[[6-(Azetidin-3-ylsulfonyl)benzothiazol-2-yl]carbamoyl]-2-chlorobenzamide
**Example 184** 2-Chloro-N-[[6-[(1-methyl-4-piperidyl)sulfonyl]benzothiazol-2-yl]carbamoyl]benzamide
**Example 185** 2-Chloro-N-[[6-(1-ethylazetidin-3-yl)sulfonylbenzothiazol-2-yl]carbamoyl]benzamide
**Example 186** 2-Chloro-N-[[6-(1-propan-2-ylazetidin-3-yl)sulfonylbenzothiazol-2-yl]carbamoyl]benzamide
**Example 187** 2-Chloro-N-[[6-(pyridin-3-ylmethylsulfonyl)benzothiazol-2-yl]carbamoyl]benzamide
**Example 188** 2-Chloro-N-[[6-[(5-methyl-1,2-oxazol-3-yl)methylsulfonyl]benzothiazol-2-yl]carbamoyl]benzamide
**Example 189** 2-Chloro-N-[[6-(1H-imidazol-2-ylmethylsulfonyl)benzothiazol-2-yl]carbamoyl]benzamide
**Example 190** 2-Chloro-N-[[6-(2-pyridin-2-ylethylsulfonyl)benzothiazol-2-yl]carbamoyl]benzamide
**Example 191** 2-Chloro-N-[[6-[(2-methyl-1,3-thiazol-4-yl)methylsulfonyl]benzothiazol-2-yl]carbamoyl]benzamide
**Example 192** 2-Chloro-N-[[6-(3-methoxypropylsulfonyl)benzothiazol-2-yl]carbamoyl]benzamide
**Example 193** 2-Chloro-N-[[6-(3-imidazol-1-ylpropylsulfonyl)benzothiazol-2-yl]carbamoyl]benzamide
**Example 194** 2-Chloro-6-fluoro-3-methyl-N-[(6-methylsulfonylbenzothiazol-2-yl)carbamoyl]benzamide
**Example 195** 6-Chloro-2-fluoro-3-methyl-N-[(6-methylsulfonylbenzothiazol-2-yl)carbamoyl]benzamide
**Example 196** 2-Chloro-N-[[6-[3-[(3S,SR)-3,5-dimethylpiperazin-1-yl]propylsulfonyl]benzothiazol-2-yl]carbamoyl]benzamide
**Example 197** 2-Chloro-N-[[6-[3-(4-ethylpiperazin-1-yl)propylsulfonyl]benzothiazol-2-yl]carbamoyl]benzamide
**Example 198** N-[[6-[3-(4-Acetylpiperazin-1-yl)propylsulfonyl]benzothiazol-2-yl]carbamoyl]-2-chloro-benzamide
**Example 199** 2-Chloro-N-[[6-[3-(4-propan-2-ylpiperazin-1-yl)propylsulfonyl]benzothiazol-2-yl]carbamoyl]benzamide
**Example 200** 2-Chloro-N-[[6-[3-[4-(2-methoxyethyl)piperazin-1-yl]propylsulfonyl]benzothiazol-2-yl]carbamoyl]benzamide
**Example 201** 2-Chloro-N-[[6-[3-(4-dimethylamino-1-piperidyl)propylsulfonyl]benzothiazol-2-yl]carbamoyl]benzamide
**Example 202** 2-Chloro-N-[[6-(3-dimethylaminopropylsulfonyl)benzothiazol-2-yl]carbamoyl]benzamide
**Example 203** 2-Chloro-N-[[6-[3-(2-methoxyethylamino)propylsulfonyl]benzothiazol-2-yl]carbamoyl]benzamide
**Example 204** 2-Chloro-N-[[6-[3-(4-methyl-1,4-diazepan-1-yl)propylsulfonyl]benzothiazol-2-yl]carbamoyl]benzamide
**Example 205** *tert*-Butyl 4-[3-[2-[(2-chlorobenzoyl)carbamoylamino]benzothiazol-6-yl]sulfonylpropyl]piperazine-1 -carboxylate
**Example 206** 2-Chloro-N-[[6-(3-piperazin-1-ylpropylsulfonyl)benzothiazol-2-yl]carbamoyl]benzamide
**Example 207** 2-Chloro-N-[[6-[3-[4-(2-cyanoethyl)piperazin-1-yl]propylsulfonyl]benzothiazol-2-yl]carbamoyl]benzamide
**Example 208** 2-Chloro-N-[[6-[3-(4-methylsulfonylpiperazin-1-yl)propylsulfonyl]benzothiazol-2-yl]carbamoyl]benzamide
**Example 209** 2-Chloro-N-[[6-[3-(3-methylpiperazin-1-yl)propylsulfonyl]benzothiazol-2-yl]carbamoyl]benzamide
**Example 210** 2-Chloro-N-[[6-[4-(4-methylpiperazin-1-yl)butylsulfonyl]benzothiazol-2-yl]carbamoyl]benzamide
**Example 211** 2-Chloro-N-[[6-(4-diethylaminobutylsulfonyl)benzothiazol-2-yl]carbamoyl]benzamide
**Example 212** 2-Chloro-N-[[6-[3-(4-methylpiperazin-1-yl)propylsulfonyl]benzothiazol-2-yl]carbamoyl]-4-(3-methylpyrazol-1-yl)benzamide
**Example 213** *tert*-Butyl N-[3-[2-[(2-chlorobenzoyl)carbamoylamino]benzothiazol-6-yl] sulfonylcyclobutyl] carbamate
**Example 214** N-[[6-(3-Aminocyclobutyl)sulfonylbenzothiazol-2-yl]carbamoyl]-2-chlorobenzamide
**Example 215** 2-Chloro-N-[[6-(3-methylaminocyclobutyl)sulfonylbenzothiazol-2-yl]carbamoyl]benzamide
**Example 216** 2-Chloro-N-[[6-(3-dimethylaminocyclobutyl)sulfonylbenzothiazol-2-yl]carbamoyl]benzamide
**Example 217** *tert*-Butyl 4-[[2-[(2-chlorobenzoyl)carbamoylamino]benzothiazol-6-yl]sulfonylmethyl]piperidine-1-carboxylate
**Example 218** *tert*-Butyl 4-[2-[2-[(2-chlorobenzoyl)carbamoylamino]benzothiazol-6-yl]sulfonylethyl]piperidine-1-carboxylate
**Example 219** 2-Chloro-N-[[6-(4-piperidylmethylsulfonyl)benzothiazol-2-yl]carbamoyl]benzamide
**Example 220** 2-Chloro-5-ethynyl-N-[[6-[3-(4-methylpiperazin-1-yl)propylsulfonyl]benzothiazol-2-yl]carbamoyl]benzamide 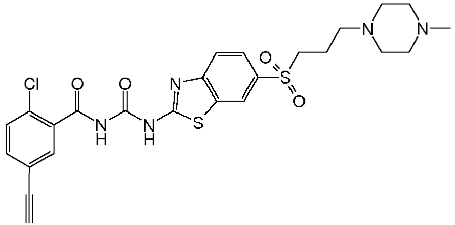 Oxalyl chloride (0.979 mL, 11.2 mmol) was added to 2-chloro-5-ethynylbenzamide (Intermediate 9, 1.69 g, 9.41 mmol) in THF (50 mL) under nitrogen. The resulting solution was stirred at 60 °C for 90 minutes. The solution was then concentrated and dried on the high vac line for 5mins to afford the crude isocyanate. A solution of the acyl isocyanate in THF (5 mL) was added to a stirred solution of 6-(3-iodopropylsulfonyl)benzo[d]thiazol-2-amine (3.60 g, 9.41 mmol) in THF (50 mL) at 60 °C, over a period of 5 minutes under nitrogen. The resulting solution was stirred at 60 °C for 2 hours. To the reaction mixture was added the 1-methylpiperazine (3.13 mL, 28.2 mmol), and the reaction was left to stir at 60 °C for 2 hours. The reaction mixture was diluted with EtOAc (100 mL) and THF (100mL), and washed sequentially with water (50 mL), and saturated brine (50 mL). The organic layer was dried over MgSO₄, filtered and evaporated to afford crude product. The crude product was purified by flash silica chromatography, elution gradient 0 to 40% MeOH in DCM. Pure fractions were evaporated to dryness to afford 2-chloro-5-ethynyl-N-(6-(3-(4-methylpiperazin-1-yl)propylsulfonyl)benzo[d]thiazol-2-ylcarbamoyl)benzamide (1.70 g, 32 %) as a beige solid.
   m/z (ESI+) (M+H)+ = 560.15; HPLC tR = 1.49 min
   ¹H NMR (400.13 MHz, DMSO-d6) δ 1.66 - 1.73 (2H, m), 2.28 (3H, s), 2.31- 2.40 (m, 6H), 2.50 -2.52 (4H, m), 3.38 (2H, q), 4.37 (1H, s), 7.54 - 7.60 (2H, m), 7.69 (1H, s), 7.80 - 7.86 (2H, m), 8.48 (1H, s).
**Example 221** 2-Chloro-N-[[6-[2-(4-piperidyl)ethylsulfonyl]benzothiazol-2-yl]carbamoyl]benzamide
**Example 222** 2-Chloro-N-[[6-[3-(2-methoxyethylamino)cyclobutyl]sulfonylbenzothiazol-2-yl]carbamoyl]benzamide and
**Example 223** N-[[6-(3-Acetamidocyclobutyl)sulfonylbenzothiazol-2-yl]carbamoyl]-2-chlorobenzamide
**Example 224** 2-chloro-N-[[6-(isopropylsulfamoyl)-1,3-benzothiazol-2-yl]carbamoyl]benzamide 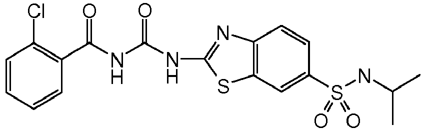 To a suspension of 2-chlorobenzamide (126 mg, 0.80 mmol) in THF (10 mL) was added oxalyl chloride (76 µL, 0.88 mmol). The resultant solution was heated to 60 °C in a glass solutions tube for around 90 minutes then cooled to RT. To this was added 2-amino-6-sulfonylbenzothiazole chloride (200 mg, 0.80 mmol) (caution, gas evolved!) in one portion and the suspension heated to 60 °C for a further 90 minutes then the reaction was cooled back to room temperature. Isopropylamine (206 µL, 2.41 mmol) was then added in one portion and the reaction further stirred for 1 hour at room temperature. The product precipitated from solution and was collected by filtration, washed with MeOH followed by Et₂O and isohexane to give 2-chloro-N-[[6-(isopropylsulfamoyl)-1,3-benzothiazol-2-yl]carbamoyl]benzamide as a fine white solid (100 mg, 38%).
   m/z (ESI+) (M+H)+ = 433; HPLC tR = 2.50 min.
**Example 225** 2-chloro-N-(6-(1-(isopropylamino)-2-methylpropan-2-ylsulfonyl)benzo[d]thiazol-2-ylcarbamoyl)benzamide 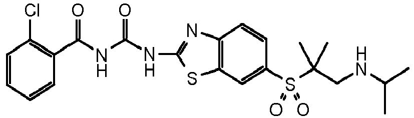 Diethylamine (0.67 mL, 6.50 mmol) was added to (9H-fluoren-9-yl)methyl 2-(2-(3-(2-chlorobenzoyl)ureido)benzo[d]thiazol-6-ylsulfonyl)-2-methylpropyl(isopropyl)carbamate (Intermediate 68, 0.095 g, 0.13 mmol) in THF (7 mL) at 25 °C. The resulting solution was stirred at 25 °C for 16 hours. The reaction mixture was concentrated in vacuo and the crude product was purified by preparative HPLC (Phenomenex Gemini C18 110A (axia) column, 5µ silica, 30 mm diameter, 100 mm length), using decreasingly polar mixtures of water (containing 0.1 % formic acid) and MeCN as eluents. The crude product was purified by flash silica chromatography, elution gradient 0 to 10% MeOH in DCM. Pure fractions were evaporated to dryness to afford 2-chloro-N-(6-(1-(isopropylamino)-2-methylpropan-2-ylsulfonyl)benzo[d]thiazol-2-ylcarbamoyl)benzamide (3.00 mg, 4.53 %) as a white solid. m/z (ESI+) (M+H)+ = 509; HPLC tR = 2.38 min.
   ¹H NMR (400.132 MHz, CDCl₃) d 1.03 (d, 6H), 1.37 (s, 6H), 2.74 (m, 1H), 2.86 (s, 2H), 7.47 (m, 1H), 7.54 (m, 2H), 7.85 (d, 1H), 7.93 (s, 2H), 8.36 (s, 1H)
**Example 226** 2-chloro-5-ethyl-N-(6-(3-(4-methylpiperazin-1-yl)propylsulfonyl)-benzo[d]thiazol-2-ylcarbamoyl)benzamide 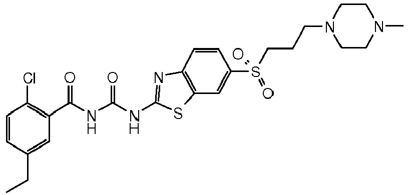 2-Chloro-5-ethynyl-N-(6-(3-(4-methylpiperazin-1-yl)propylsulfonyl)benzo[d]thiazol-2-ylcarbamoyl)benzamide (Example 220, 400mg, 0.71 mmol) and 80 mg (20%) Pt/C in MeOH (20ml) and THF (20 mL) were stirred under an atmosphere of hydrogen at 1 atm and 30 °C for 1 hour. The reaction was progressing very slowly and so another 320mg (80% by wt) Pt/C was added and the reaction was allowed to stir for 2 hours. The reaction was filtered through celite and washed through with MeOH/THF, and evaporated to dryness to give a gum which was triturated with ether to give a cream solid (260 mg, 64%). m/z (ESI+) (M+H)+ = 564.17; HPLC tR = 1.55 min
   ¹H NMR (400.13 MHz, DMSO-d6) δ 1.16 (3H, m), 1.66 - 1.73 (2H, m), 2.27 (3H, s), 2.31- 2.40 (6H, m),2.48 - 2.50 (4H, m), 2.64 (2H, q), 3.32 (2H, t), 7.37 (1H, d), 7.43 - 7.44 (2H, m), 7.83 - 7.88 (2H, m), 8.53 (1H, s).
**Example 227** 5-(1-acetylpyrrolidin-3-yloxy)-2-chloro-N-(6-(methylsulfonyl)benzo[d]thiazol-2-ylcarbamoyl)benzamide 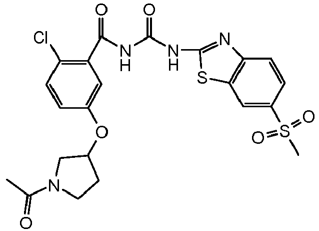 Acetyl chloride (0.032 ml, 0.440 mmol) was added to 2-chloro-N-(6-(methylsulfonyl)benzo[d]thiazol-2-ylcarbamoyl)-5-(pyrrolidin-3-yloxy)benzamide (Example 249 , 200 mg, 0.400 mmol), and triethylamine (0.113 ml, 0.810 mmol) in DMF (5 mL) under air. The resulting solution was stirred at ambient temperature for 2 hours. The reaction was evaporated to dryness, and 10ml of water added yielding the crude product as a solid. The crude product was purified by flash silica chromatography, elution gradient 0 to 10% MeOH in DCM. Pure fractions were evaporated to dryness to afford 5-(1-acetylpyrrolidin-3-yloxy)-2-chloro-N-(6-(methylsulfonyl)benzo[d]thiazol-2-ylcarbamoyl)benzamide (95 mg, 44 %) as a white solid. ¹H NMR (400.13 MHz, DMSO-d6) 1.90 (3H, d), 2.05 - 2.30 (2H, m), 3.20 (3H, s), 3.30 - 3.45 (2H, m), 3.50 - 3.60 (2H, m), 5.09 - 5.15 (1H, m), 7.10 - 7.15 (1H, m), 7.28 (1H, d), 7.49 - 7.52 (1H, m), 7.95 (2H, s), 8.65 (1H, s).
**Example 228** (S)-2-chloro-5-((1-isopropylpiperidin-3-yl)methoxy)-N-(6-(methylsulfonyl)benzo[d]thiazol-2-ylcarbamoyl)benzamide 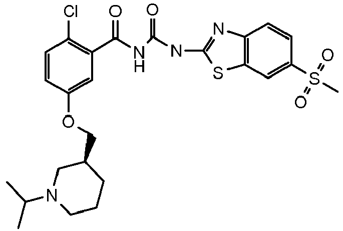 2-Iodopropane was added to (S)-2-chloro-N-(6-(methylsulfonyl)benzo[d]thiazol-2-ylcarbamoyl)-5-(piperidin-3-ylmethoxy)benzamide (Intermediate 174c), and potassium carbonate in DMF (10 mL) . The resulting slurry was stirred at 70 °C for 2 hours. Reaction was evaporated to dryness. The reaction mixture was diluted with EtOAc (100 mL), and washed with water (50 mL). The organic layer was dried over MgSO₄, filtered and evaporated to afford crude product. The crude product was purified by flash silica chromatography, elution gradient 0 to 10% MeOH in DCM. Pure fractions were evaporated to dryness to afford (S)-2-chloro-5-((1-isopropylpiperidin-3-yl)methoxy)-N-(6-(methylsulfonyl)benzo[d]thiazol-2-ylcarbamoyl)benzamide (130 mg,) as a cream solid. m/z (ESI+) (M+H)+ = 565.26; HPLC tR = 1.39 min
   ¹H NMR (400.13 MHz, DMSO-d₆) δ 1.04 - 1.11 (6H, m), 1.57 - 1.65 (1H, t), 1.76 (2H, s), 2.11 (1H, s), 2.30 - 2.45 (2H, m), 2.87 - 2.91 (3H, m), 3.20 (4H, t), 3.97 (2H, t), 7.11 (1H, t), 7.20 - 7.22 (1H, m), 7.40 - 7.45 (1H, m), 7.90 (2H, q), 8.55 (1H, d).
   The following compounds were made in a similar manner to those listed above using a different building block as stated. The building block numbers correspond to the numbers of the intermediates unless EX (EX = Example no.) is used.
   Following the method described for the formation of example 99 using oxalyl chloride coupling then optional reaction with an amine is called method A.
   Following the method described for the formation of example 224 using oxalyl chloride coupling then sulphonamide formation is called method B.
   Following the method described for the formation of example 95 using the active carbamate method to join the building blocks is called method C.
   Following the method described for the formation of intermediate 122 using a reductive amination method is called method D.
   Following the method described for the formation of example 100 using acid to remove a tert-butylcarbonyl amine-protecting group is called method E. 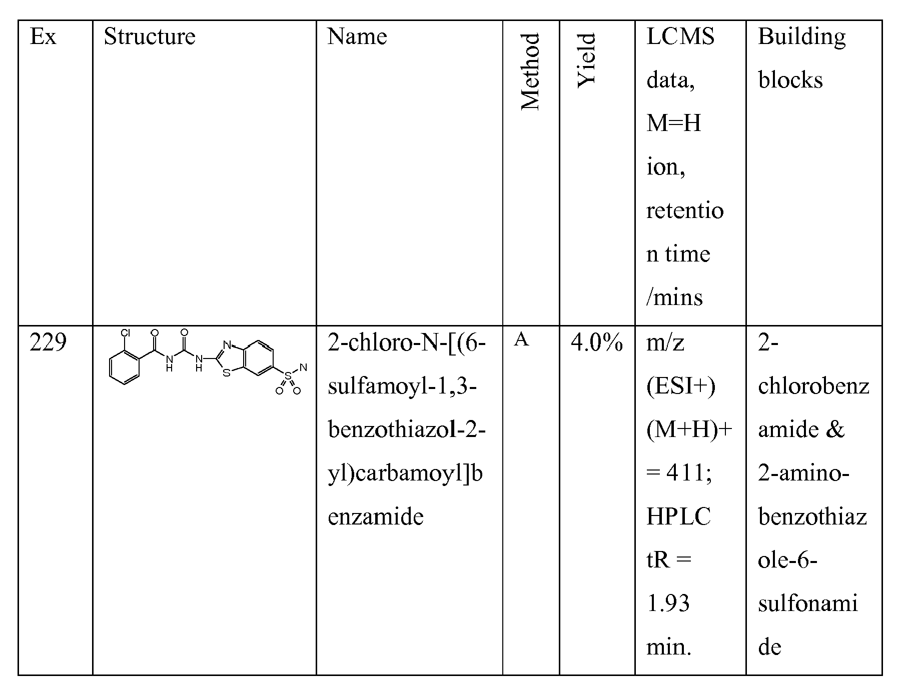 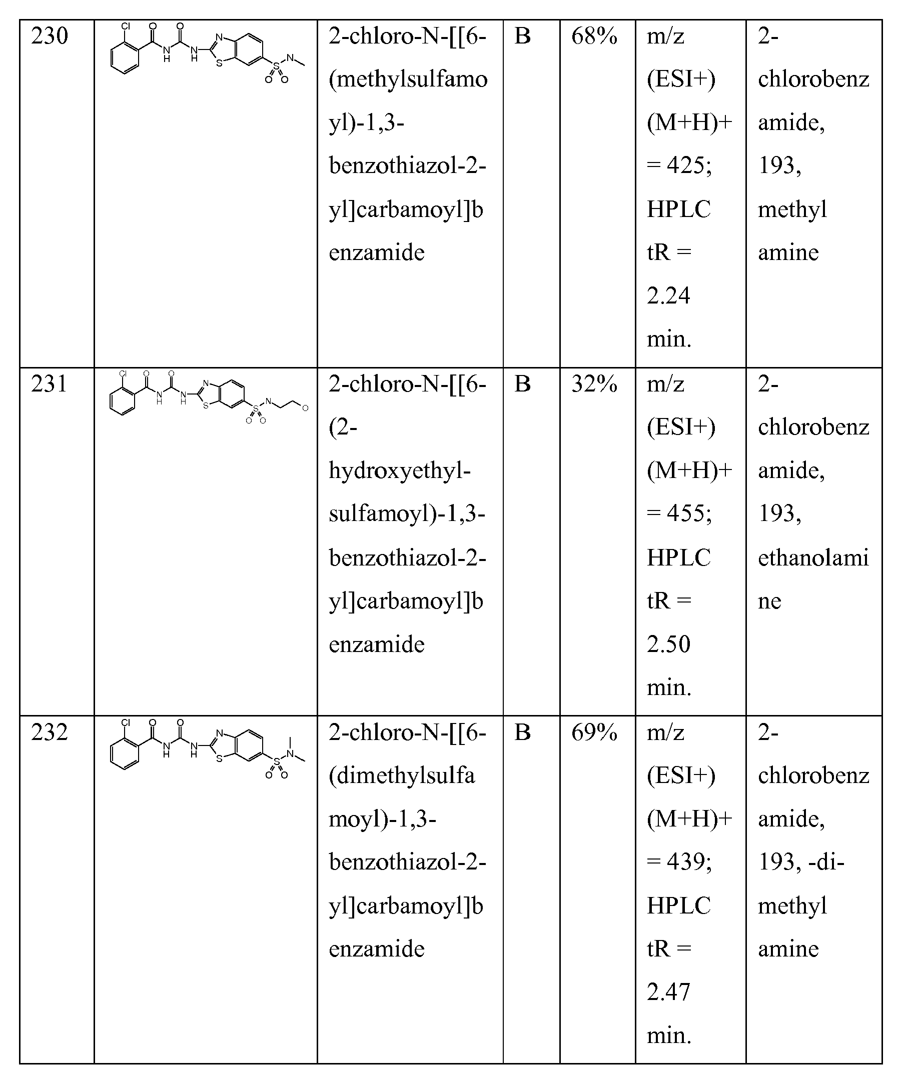 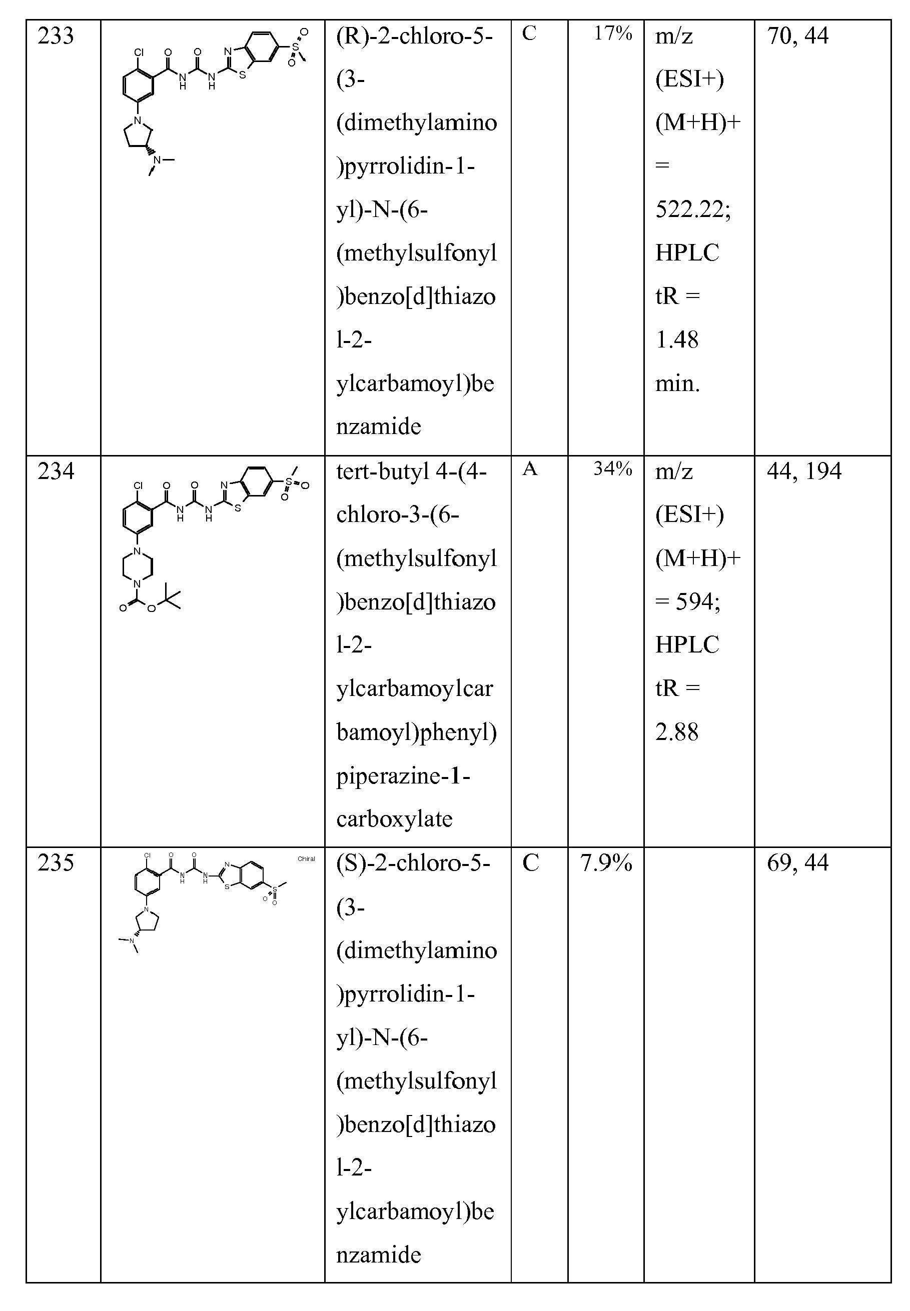 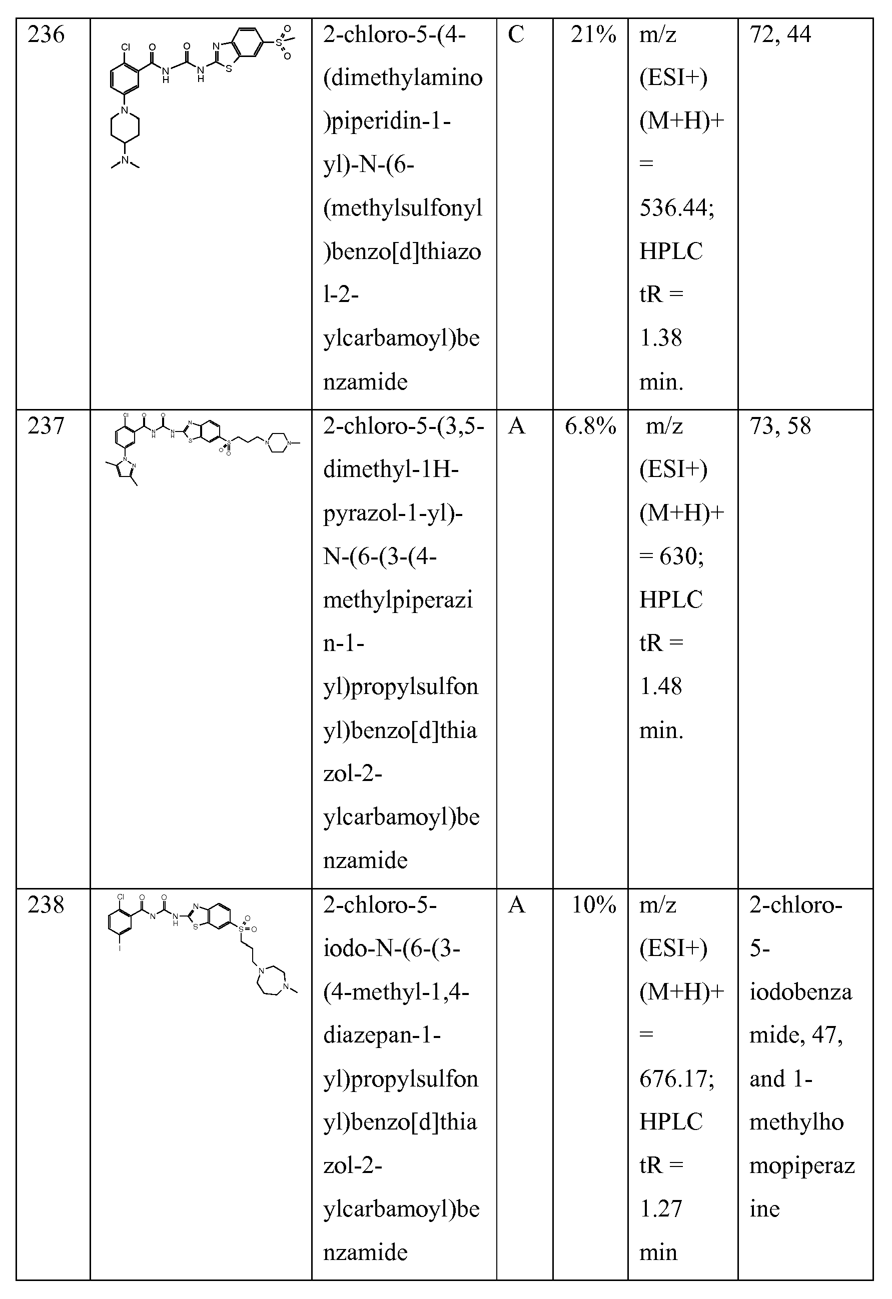 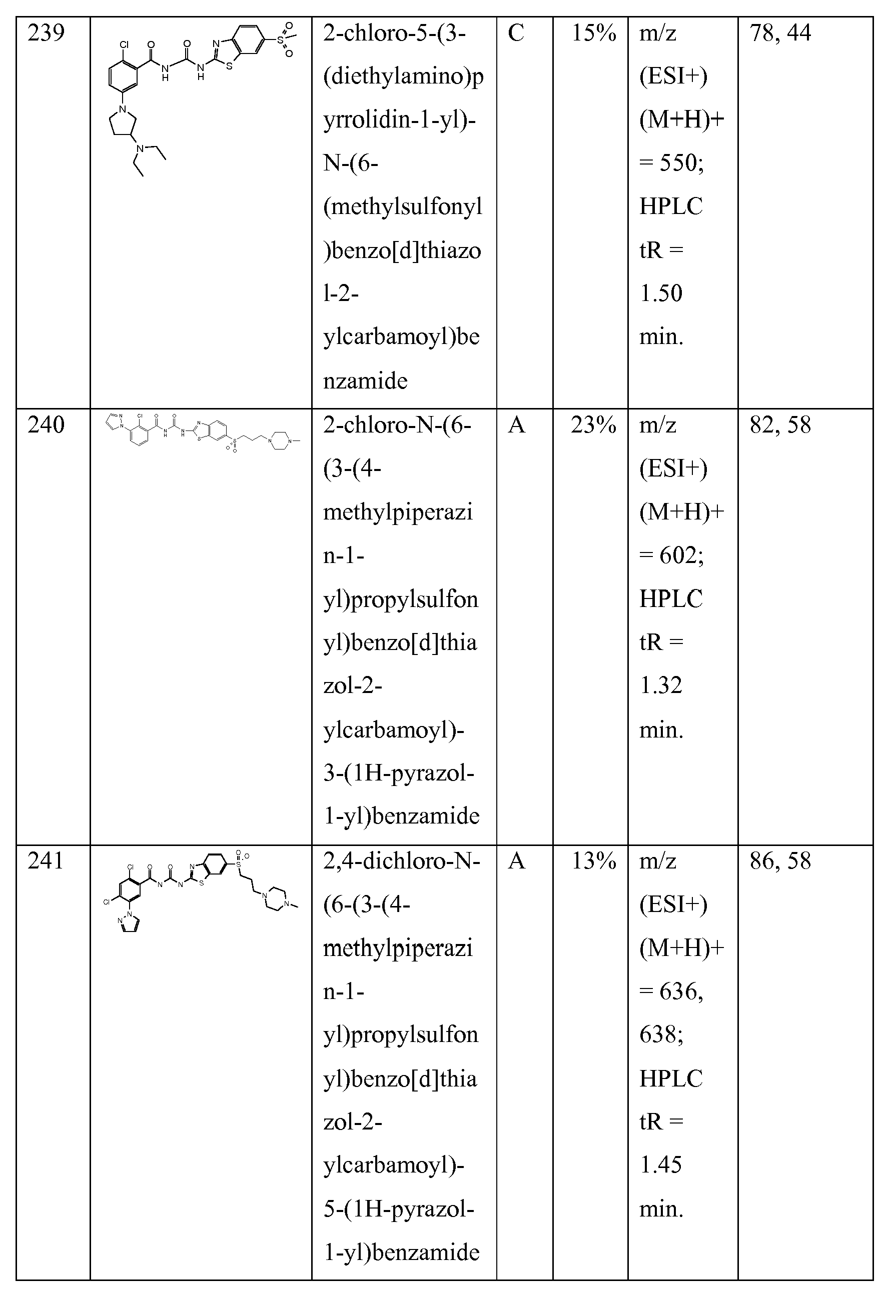 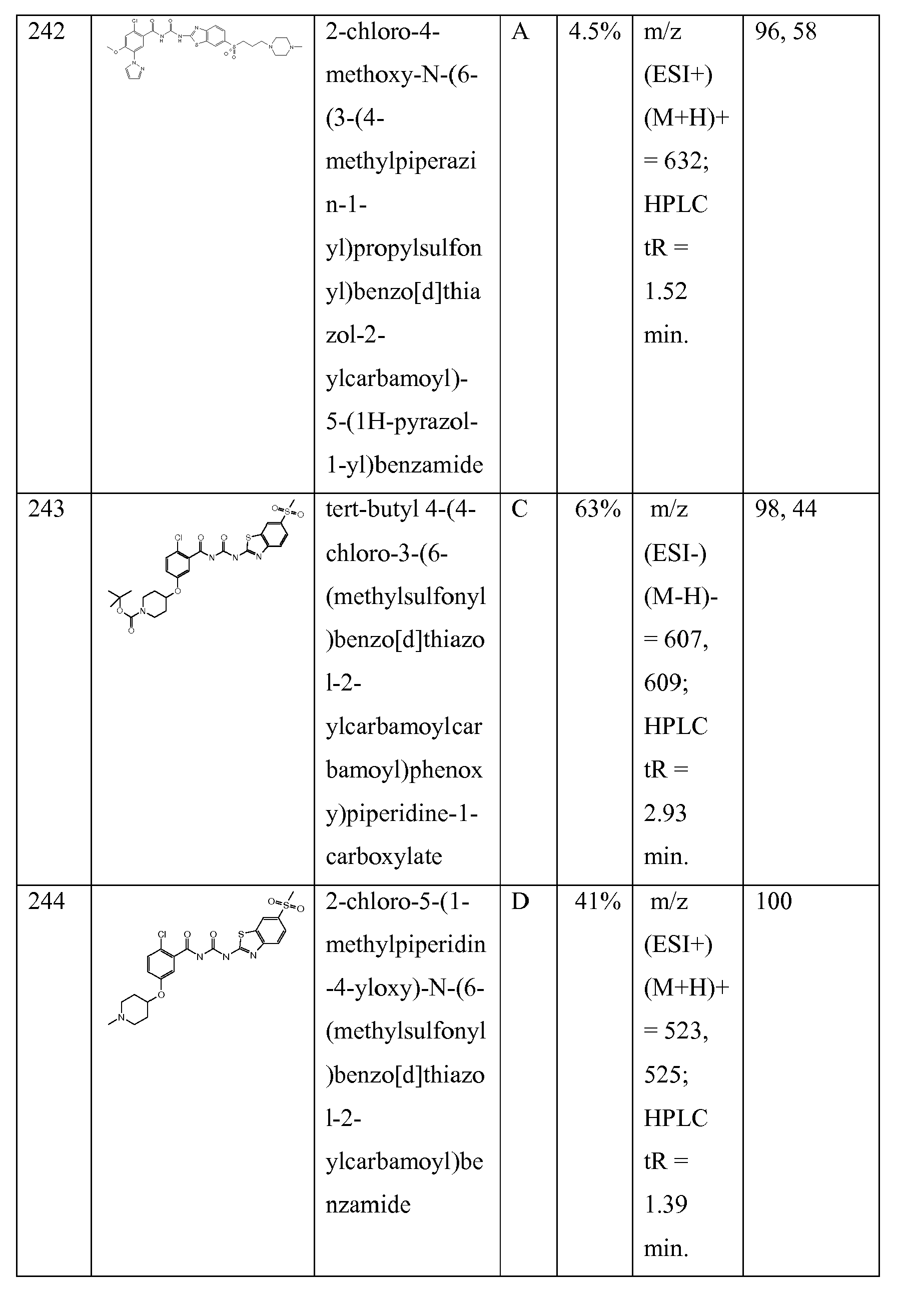 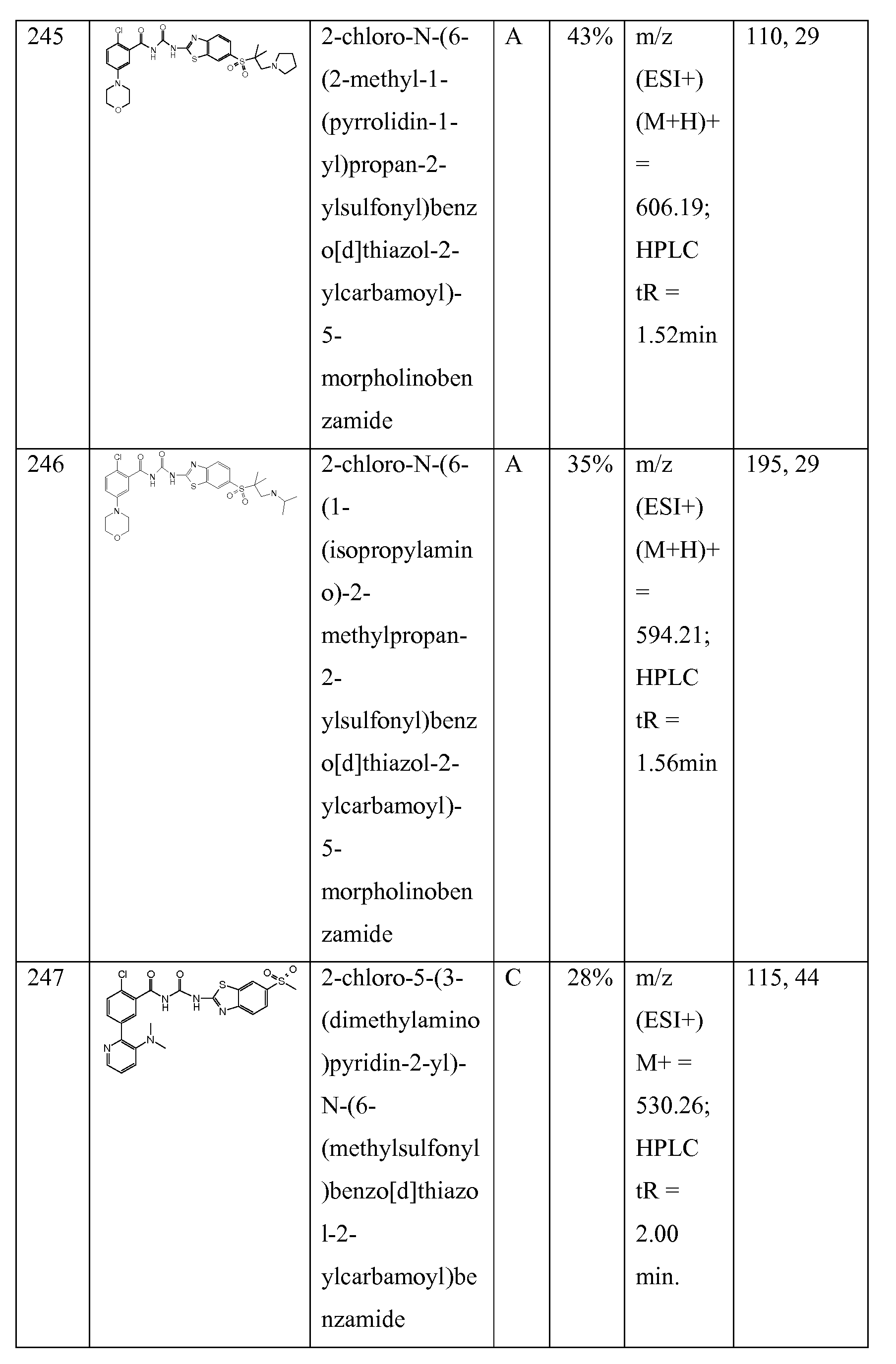 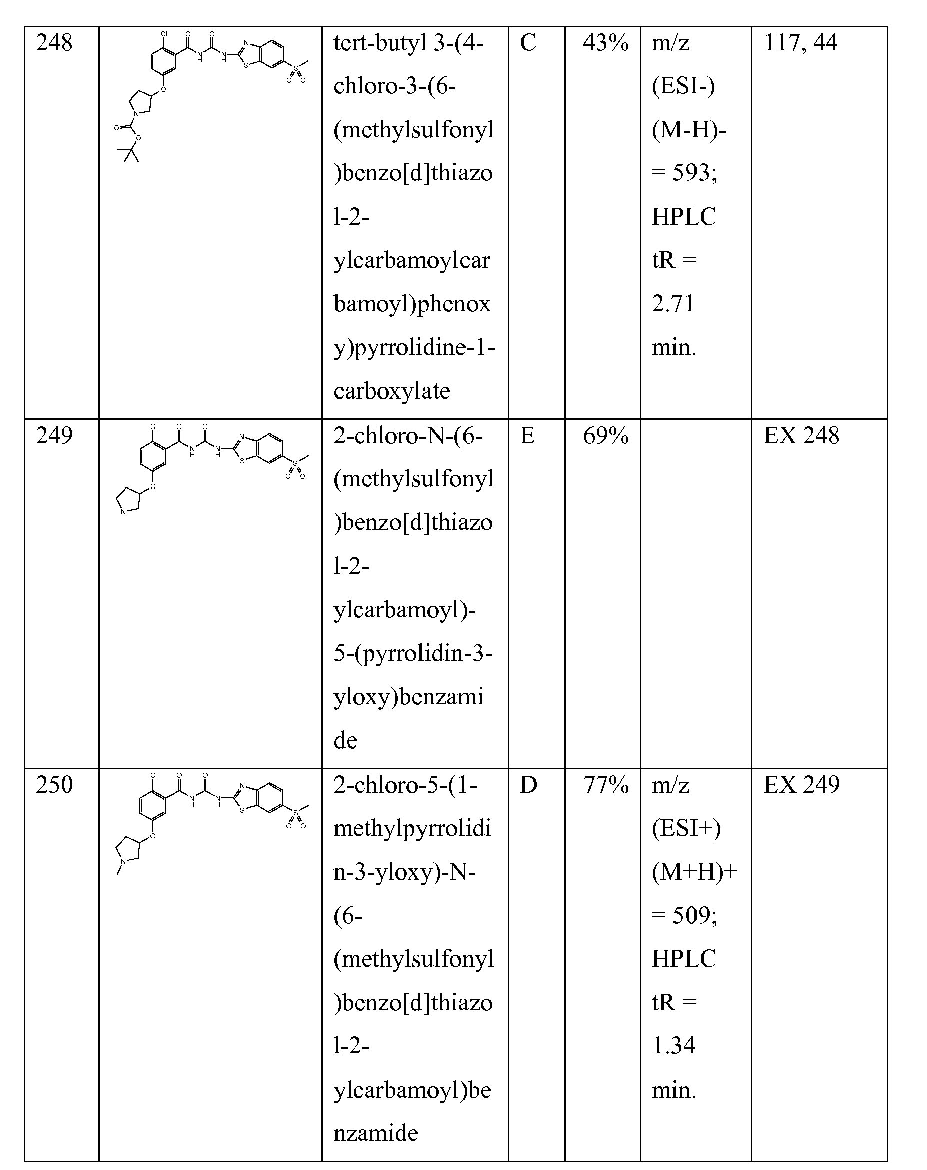 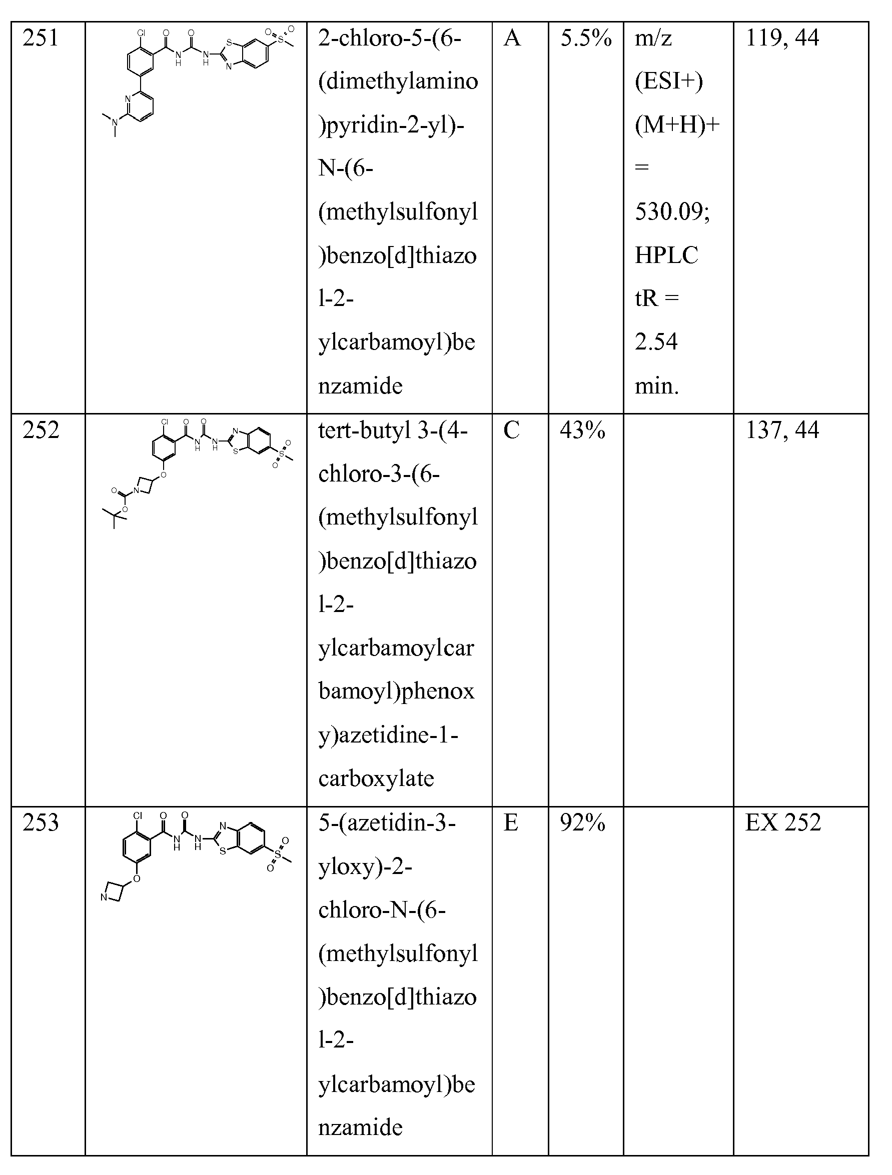 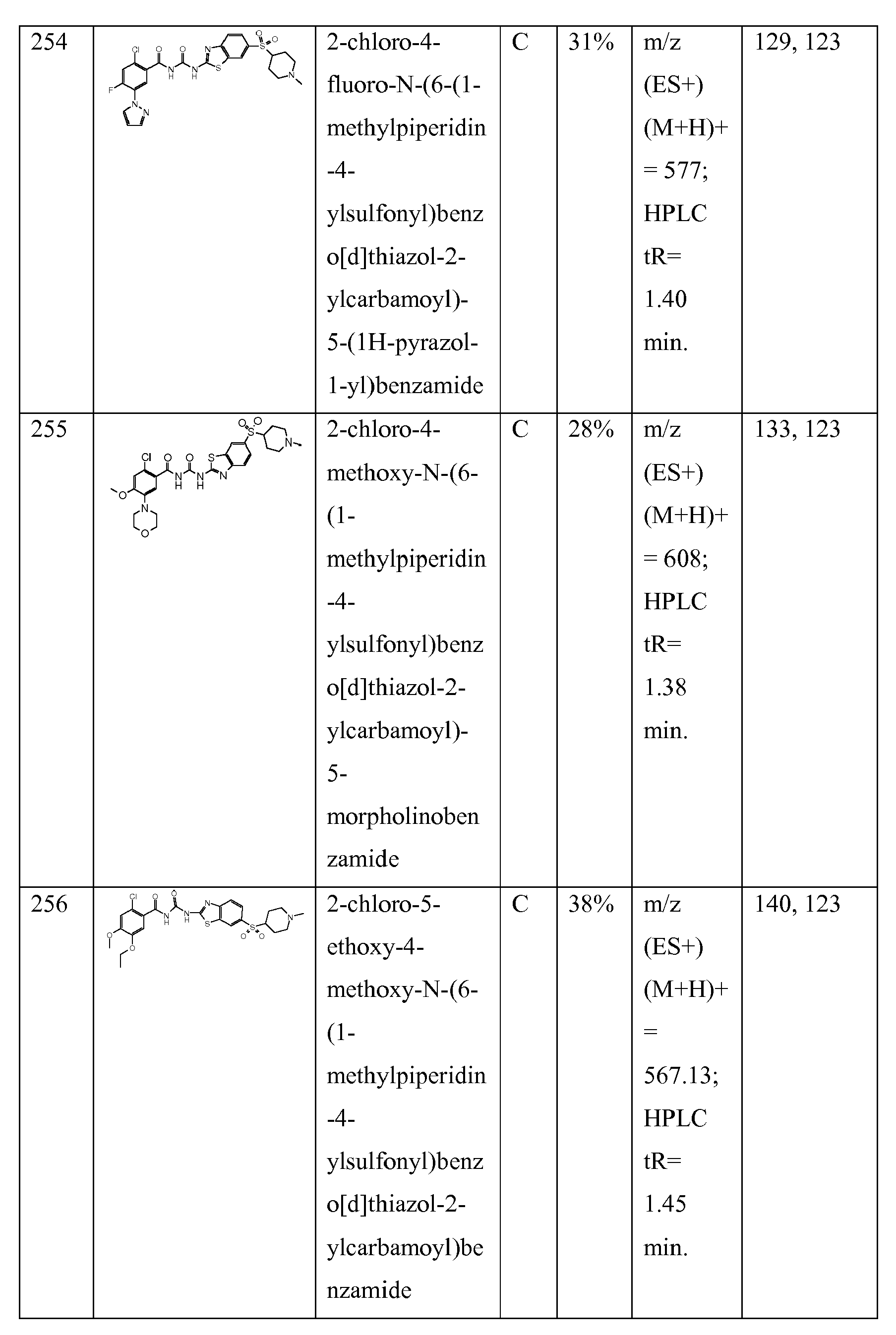 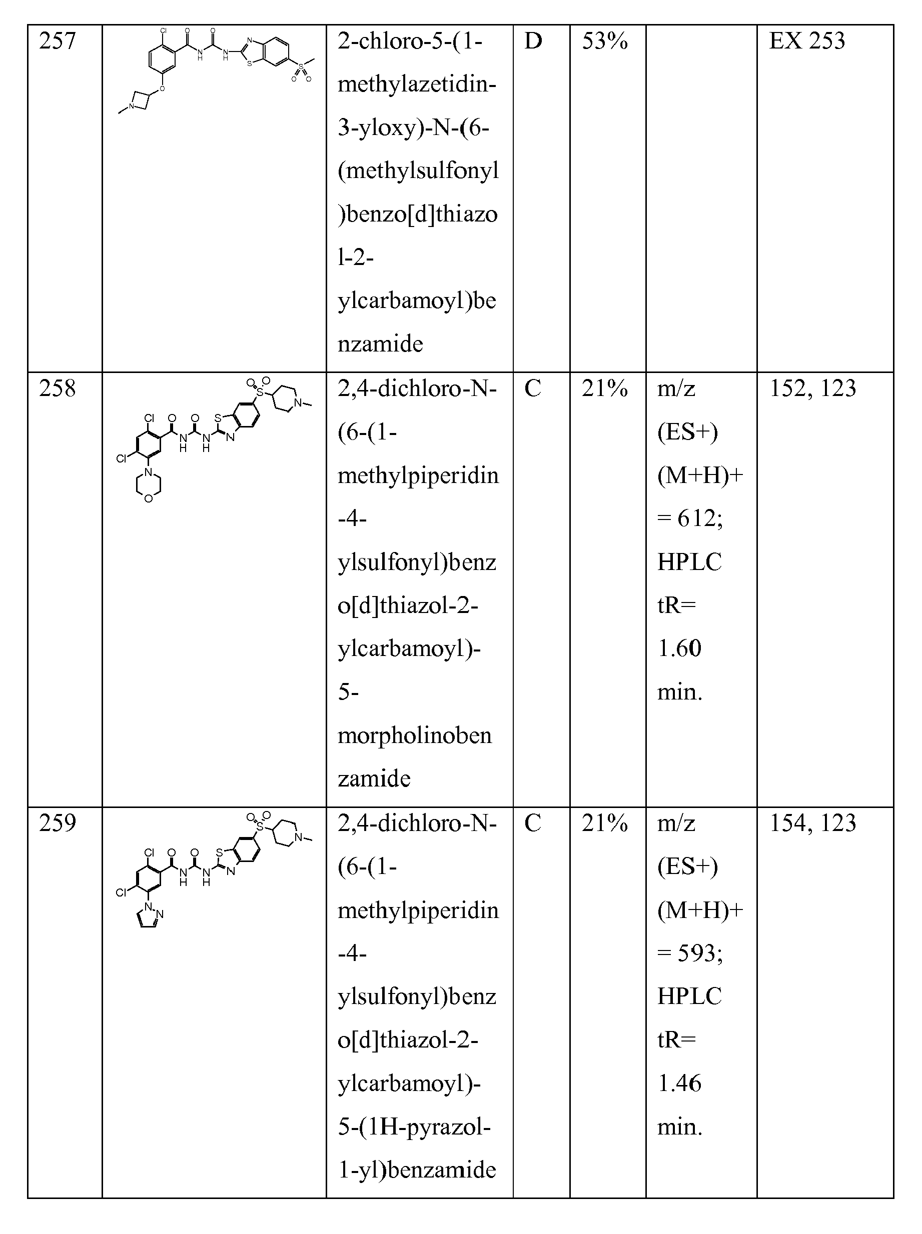 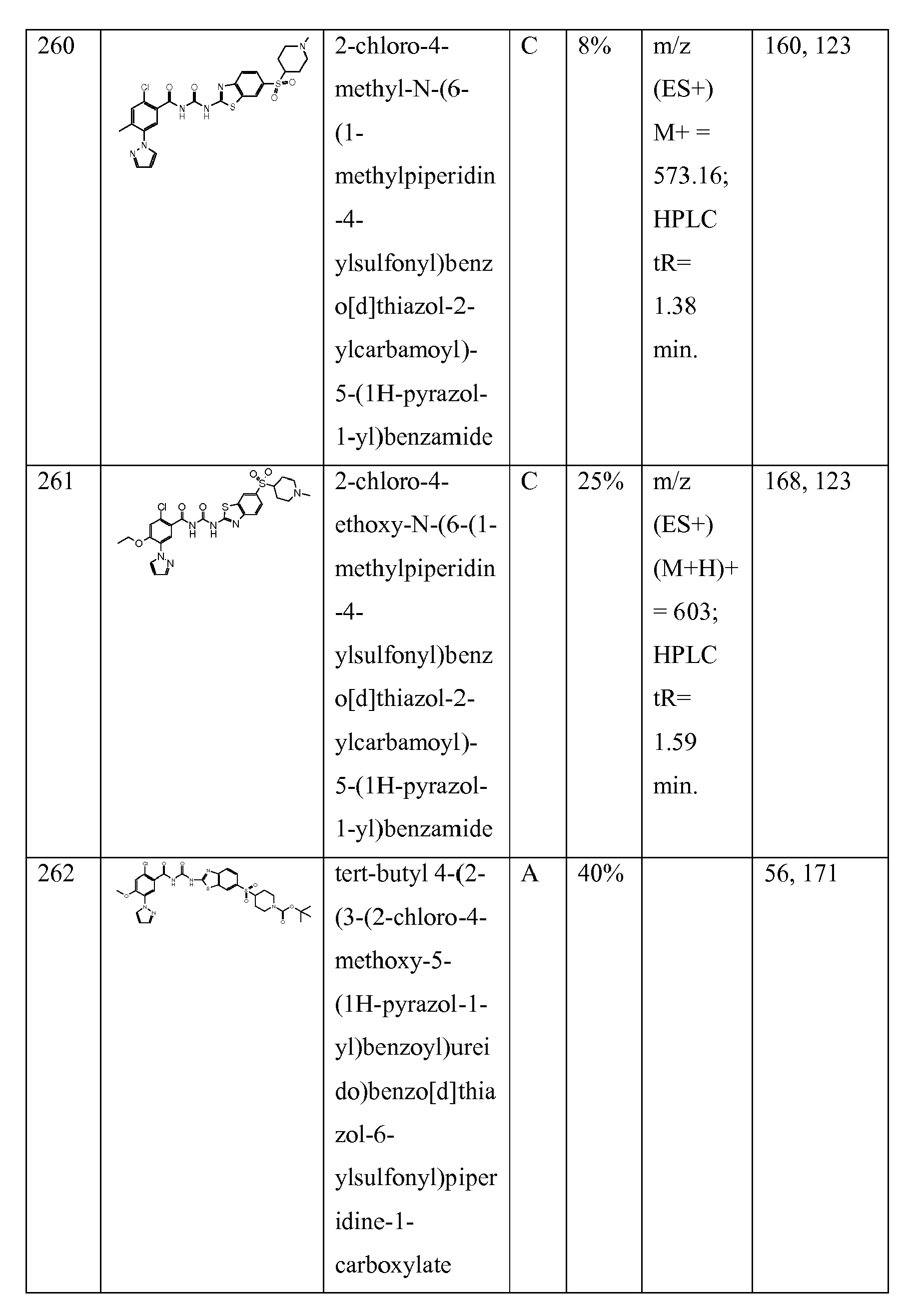 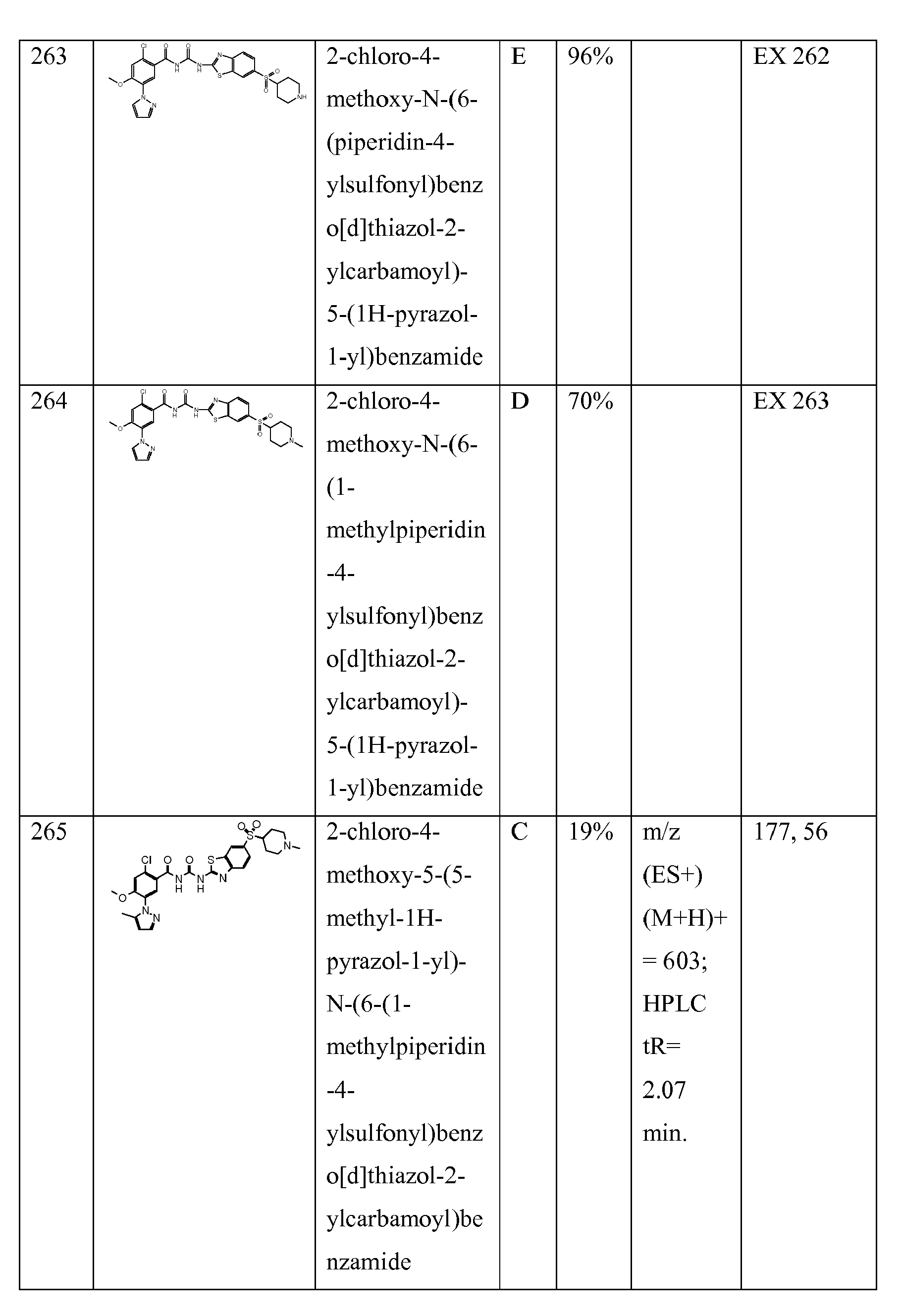 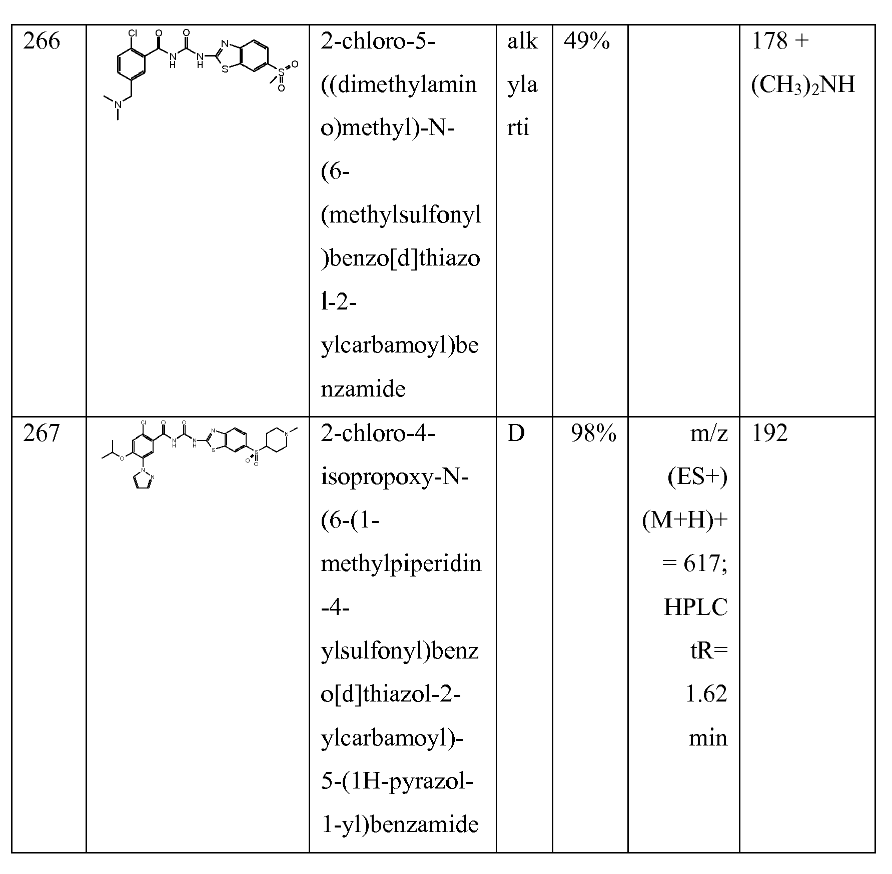
**Example 233** ¹H NMR (400.132 MHz, DMSO) δ 1.79 - 1.92 (1H, m), 2.15 - 2.23 (1H, m), 2.27 (6H, s), 2.91 (1H, q), 3.09 (1H, t), 3.19 - 3.30 (1H, m), 3.41 (4H, t), 3.48 (1H, t), 6.65 - 6.71 (1H, m), 6.78 (1H, d), 7.30 (1H, d), 7.89 - 8.00 (2H, m), 8.65 (1H, s).
**Example 235** ¹H NMR (400.13 MHz, DMSO-d6) 8 2.26 (1H, s), 2.83 (6H, s), 3.27 (3H, s), 3.52 - 3.55 (2H, m), 3.64 - 3.69 (1H, m), 4.00 (1H, br s), 6.76 - 6.79 (1H, m), 6.88 (1H, s), 7.39 (1H, d), 7.98 (2H, s), 8.69 (1H, s)..
**Example 236** ¹H NMR (400.13 MHz, DMSO-d6) δ 1.58 (2H, s), 1.96 (2H, s), 2.51 (1H, m), 2.60 (3H, s), 2.78 (2H, t), 3.36 (6H, s), 3.88 (2H, d), 7.11 (1H, d), 7.20 (1H, s), 7.35 (1H, d), 7.92 (2H, d), 8.63 (1H, s).
**Example 237** ¹H NMR (400.13 MHz, DMSO-d6) δ 1.67 - 1.74 (2H, m), 2.18 (3H, s), 6.11 (1H, s), 7.67 (2H, d), 7.78 (1H, s), 7.85 - 7.91 (2H, m), 8.56 (1H, d).
**Example 239** ¹H NMR (400.13 MHz, DMSO-d6) δ 1.03 (6H, t), 1.90 - 2.23 (2H, m), 2.53 (3H, s), 2.75 (4H, m), 3.08 - 3.18 (2H, m), 3.24 (3H, s), 3.44 - 3.56 (3H, m), 6.67 - 6.70 (1H, m), 6.79 (1H, d), 7.30 (1H, d), 7.94 - 7.96 (2H, m), 8.13 (1H, s), 8.64 (1H, t), 11.7 (1H, s).
**Example 240** ¹H NMR (400.13 MHz, DMSO-d6) δ1.69 (2H, q), 2.25 - 2.32 (9H, m), 3.31 (2H, m), 6.55 (1H, t), 7.57 - 7.61 (1H, m), 7.66 - 7.69 (2H, m), 7.78 - 7.79 (1H, m), 7.81 - 7.86 (2H, m), 8.12 - 8.13 (1H, m), 8.50 (1H, s).
**Example 241** ¹H NMR (400.132 MHz, DMSO) δ 1.68 - 1.76 (2H, m), 2.31 - 2.33 (2H, m), 2.36 - 2.44 (2H, m), 2.65 - 2.68 (4H, m), 3.32 (3H, s), 3.36 (4H, t), 6.59 (1H, t), 7.82 (1H, d), 7.89 - 7.97 (3H, m), 8.09 (1H, s), 8.23 (1H, d), 8.63 (1H, s), 11.6 (1H, s).
**Example 242** ¹H NMR (400.13 MHz, DMSO-d6) δ 1.68 - 1.75 (2H, m), 2.35 - 2.38 (2H, m), 2.42 (1H, s), 2.47 (4H, s), 2.51 (1H, d), 2.65 - 2.67 (1H, m), 2.75 (3H, br s), 3.16 (1H, d), 3.34 (2H, m), 3.99 (3H, s), 6.53 (1H, t), 7.48 (1H, s), 7.75 (1H, d), 7.88 - 7.91 (1H, m), 7.94 (1H, d), 7.99 (1H, s), 8.25 (1H, d), 8.62 (1H, d).
**Example 243** ¹H NMR (400.132 MHz, DMSO) δ 1.46 (9H, s), 1.56 - 1.65 (2H, m), 1.96 - 2.02 (2H, m), 3.22 - 3.28 (2H, m), 3.32 (3H, s), 3.69 - 3.76 (2H, m), 4.65 - 4.71 (1H, m), 7.22 - 7.25 (1H, m), 7.38 (1H, s), 7.54 (1H, d), 8.03 (2H, s), 8.73 (1H, s), 11.80 (1H, s), 11.92 (1H, s).
**Example 244** ¹H NMR (400.132 MHz, DMSO) δ 1.57 - 1.65 (2H, m), 1.85 - 1.91 (2H, m), 2.22 (3H, s), 2.28 - 2.34 (2H, m), 2.64 - 2.70 (2H, m), 3.13 (3H, s), 4.35 - 4.41 (1H, m), 7.00 - 7.03 (1H, m), 7.14 (1H, d), 7.33 (1H, d), 7.76 - 7.82 (2H, m), 8.47 (1H, d), 11.40 (2H, s).
**Example 245** ¹H NMR (400.13 MHz, DMSO-d6) δ 1.34 (6H, s), 1.75 (4H, s), 2.93 (4H, s), 3.15 - 3.19 (4H, m), 3.73 - 3.75 (4H, m), 7.09 - 7.12 (1H, m), 7.21 (1H, d), 7.38 (1H, d), 7.84 (1H, d), 7.96 (1H, d), 8.61 (1H, s).
**Example 246** ¹H NMR (400.13 MHz, DMSO-d6) δ 1.22 (6H, d), 1.37 (6H, s), 3.15 (5H, s), 3.14 - 3.17 (4H, m), 3.73 (4H, t), 7.09 - 7.12 (1H, m), 7.20 (1H, d), 7.38 (1H, d), 7.88 - 7.91 (1H, m), 7.99 (1H, d), 8.68 (1H, s).
**Example 247** ¹H NMR (400.13 MHz, DMSO-d6) δ 2.59 (6H, s), 3.24 (3H, s), 7.29 - 7.33 (1H, m), 7.51 - 7.53 (1H, m), 7.64 (1H d, J= 8.1 Hz), 7.96 (2H, s), 8.09 (2H d, J= 8.7 Hz), 8.24 - 8.26 (1H, m), 8.66 (1H, s), 11.78 (1H, s), 11.86 (1H, s).
**Example 248** ¹H NMR (400.13 MHz, DMSO-d6) δ 1.40 (9H, s), 2.07 (1H, s), 2.16 (1H, s), 3.24 (3H, s), 3.34 - 3.46 (3H, m), 3.56 (1H, d), 5.05 (1H, s), 7.13 - 7.16 (1H, m), 7.29 (1H, s), 7.48 (1H, d), 7.96 (2H, s), 8.66 (1H, s), 11.72 - 11.89 (2H, m).
**Example 249** ¹H NMR (400.132 MHz, DMSO) d 2.00 - 2.08 (m, 1H), 2.10 - 2.22 (m, 1H), 3.16 (s, 3H), 3.27 (m, 4H), 5.09 (m, 1H), 7.01 (m, 2H), 7.37 (m, 1H), 7.59 (m, 1H), 7.71 (m, 1H), 8.23 (s, 1H).
**Example 250** ¹H NMR (400.13 MHz, DMSO-d6) δ 1.77 - 1.84 (1H, m), 2.24 - 2.31 (1H, m), 2.33 (3H, s), 2.44 (1H, d), 2.66 - 2.79 (2H, m), 2.83 - 2.87 (1H, m), 3.22 (3H, s), 4.91 - 4.94 (1H, m), 7.03 - 7.06 (1H, m), 7.14 (1H, d), 7.43 (1H, d), 7.86 - 7.92 (2H, m), 8.57 (1H, s).
**Example 251** ¹H NMR (400.132 MHz, DMSO) δ 3.15 - 3.19 (6H, m), 3.32 (3H, s), 6.75 (1H, d), 7.31 (1H, d), 7.64 - 7.75 (2H, m), 8.04 (2H, s), 8.28 - 8.33 (1H, m), 8.38 - 8.41 (1H, m), 8.74 (1H, s), 11.83 - 12.09 (2H, m).
**Example 252** ¹H NMR (400.13 MHz, DMSO-d6) 1.39 - 1.40 (9H, m), 3.17 - 3.18 (3H, m), 3.70 - 3.80 (2H, m), 4.35 -4.45 (2H, m), 4.95 - 5.05 (1H, m), 7.06 (1H, s), 7.14 (1H, s), 7.49 (1H, s), 7.97 (2H, s), 8.67 (1H, s), 11.76 (1H, s), 11.84 (1H, s).
**Example 253:** ¹H NMR (400.13 MHz, DMSO-d6) 3.25 3H, s), 3.98 - 4.02 (2H, m), 4.43 - 4.48 (2H, m), 5.09 - 5.15 (1H, m), 7.07 - 7.10 (1H, m), 7.18 (1H, d), 7.49 - 7.52 (1H, m), 7.95 (2H, s), 8.65 (1H, t).
**Example 254** ¹H NMR (400.13 MHz, DMSO-d6) 1.54 - 1.63 (2H, m), 1.87 (2H, d), 2.05 (2H, t), 2.24 (3H, s), 2.94 (2H, d), 3.22 - 3.29 (1H, m), 6.64 (1H, t), 7.79 - 7.82 (1H, m), 7.87 - 7.93 (3H, m), 8.12 (1H, d), 8.30 (1H, t), 8.50 (1H, s), 11.35 - 11.70 (2H, s).
**Example 255** ¹H NMR (400.13 MHz, DMSO-d6) 1.50 - 1.60 (2H, m), 1.81 - 1.92 (4H, m), 2.14 (3H, s), 2.84 (2H, d), 3.00 (4H, t), 3.16 - 3.24 (1H, m), 3.72 (4H, t), 3.88 (3H, s), 7.11 (1H, s), 7.16 (1H, s), 7.82 - 7.84 (1H, m), 7.92 (1H, d), 8.56 (1H, d), 11.66 (2H, s).
**Example 256** ¹H NMR (400.13 MHz, DMSO-d6) 1.34 (3H, t), 1.77 - 1.82 (2H, m), 2.01 (2H, d), 2.52 (1H, s), 2.61 (2H, t), 3.28 (3H, d), 3.85 (3H, s), 4.08 (2H, q), 7.13 (1H, s), 7.27 (1H, s), 7.85 - 7.87 (1H, m), 7.98 (1H, d), 8.61 (1H, d).
**Example 257** ¹H NMR (400.13 MHz, DMSO-d6) 2.53 (3H, s), 3.22 (3H, s), 3.47 (2H, q), 4.05 - 4.09 (2H, m), 4.89 - 4.95 (1H, m), 7.01 - 7.04 (1H, m), 7.11 (1H, d), 7.46 (1H, d), 7.90 - 7.95 (2H, m), 8.60 - 8.62 (1H, m).
**Example 258** ¹H NMR (400.13 MHz, DMSO-d6) ä 1.52 - 1.62 (2H, m), 1.85 (2H, d), 1.98 (2H, t), 2.19 (3H, s), 2.89 (2H, d), 3.01 (4H, t), 3.20 (1H, m), 3.75 (4H, t), 7.41 (1H, s), 7.69 (1H, s), 7.79 - 7.82 (1H, m), 7.89 (1H, d), 8.51 (1H, d), 11.60 (2H, s).
**Example 259** ¹H NMR (400.13 MHz, DMSO-d6) ä 1.53 - 1.63 (2H, m), 1.86 (2H, d), 2.05 (2H, t), 2.23 (3H, s), 2.95 (2H, d), 3.21 (1H, m), 6.57 - 6.58 (1H, m), 7.79 - 7.82 (2H, m), 7.85 - 7.91 (2H, m), 8.02 (1H, s), 8.20 - 8.21 (1H, m), 8.46 (1H, s), 11.45 (2H, s).
**Example 260** ¹H NMR (400.13 MHz, DMSO-d6) 1.77 (2H, d), 2.07 (2H, d), 2.29 (3H, s), 2.63 (3H, s), 2.77 - 2.83 (2H, m), 3.38 (2H, d), 3.48 (1H, d), 6.51 (1H, t), 7.62 (1H, s), 7.69 (1H, s), 7.74 (1H, d), 7.84 - 7.87 (1H, m), 7.96 (1H, d), 8.06 (1H, s), 8.58 (1H, d).
**Example 261** ¹H NMR (400.13 MHz, DMSO-d6) 1.38 (3H, t), 1.51 - 1.61 (2H, m), 1.84 (2H, d), 1.94 (2H, t), 2.17 (3H, s), 2.87 (2H, d), 3.18 - 3.24 (1H, m), 4.27 (2H, q), 6.53 - 6.54 (1H, m), 7.44 (1H, s), 7.75 - 7.76 (1H, m), 7.80 - 7.82 (1H, m), 7.90 (1H, d), 7.98 (1H, s), 8.29 - 8.30 (1H, m), 8.53 (1H, d), 11.63 (2H, s)
**Example 262** ¹H NMR (400.13 MHz, DMSO-d6) 1.35 (11H, s), 1.84 (2H, d), 2.60 - 2.70 (2H, s), 3.40 -3.45 (1H, m) 3.93 - 3.99 (5H, m), 6.52 - 6.53 (1H, m), 7.49 (1H, s), 7.76 (1H, d), 7.84 - 7.87 (1H, m), 8.01 (2H, s), 8.25 - 8.26 (1H, m), 8.60 (1H, s).
**Example 263** ¹H NMR (400.13 MHz, DMSO-d6) 1.70 - 1.79 (2H, m), 2.04 (2H, d), 2.85 (2H, q), 3.34 (2H, d), 3.99 (3H, s), 6.52 - 6.53 (1H, m), 7.49 (1H, s), 7.75 (1H, s), 7.84 - 7.87 (1H, m), 7.99 (2H, d), 8.25 - 8.26 (1H, m), 8.52 (1H, d), 8.63 (1H, d), 9.07 (1H, d), 11.77 (1H, s).
**Example 264** ¹H NMR (400.13 MHz, DMSO-d6) 1.50 - 1.58 (2H, m), 1.80 (2H, d), 1.83 (1H, s), 1.86 (1H, s), 2.12 (3H, s), 2.82 (2H, d), 3.12 (1H, t), 3.96 (3H, s), 6.50 (1H, t), 7.39 (1H, s), 7.70 - 7.73 (3H, m), 7.83 (1H, s), 8.23 (1H, d), 8.33 (1H, s).
**Example 265** ¹H NMR (400.13 MHz, DMSO-d6) 1.50 - 1.61 (2H, m), 1.83 (2H, d), 1.93 (2H, t), 2.11 (3H, s), 2.15 (3H, s), 2.86 (2H, d), 3.16 - 3.24 (1H, m), 3.88 (3H, s), 6.21 - 6.23 (1H, m), 7.46 (1H, s), 7.54 (1H, d), 7.63 (1H, s), 7.80 - 7.83 (1H, m), 7.91 (1H, d), 8.53 (1H, d), 11.60 (2H, s).
**Example 266** ¹H NMR (400.13 MHz, DMSO-d6) 2.22 (6H, s), 3.16 (3H, s), 3.50 (2H, s), 7.45 - 7.48 (1H, m), 7.50 -7.58 (2H, m), 7.90 - 7.95 (2H, m), 8.62 (1H, s), 11.70 (2H, s).
**Example 267** ¹H NMR (400.13 MHz, DMSO-d₆) δ 1.37 (6H, d), 1.58 - 1.62 (2H, m), 1.85 - 2.00 (4H, m), 2.20 (3H, s), 2.90 (2H, d), 3.23 (1H, m), 4.96 (1H, m), 6.57 - 6.58 (1H, m), 7.52 (1H, s), 7.79 (1H, d), 7.84 - 7.86 (1H, m), 7.93 (1H, d), 8.02 (1H, s), 8.30 - 8.31 (1H, m), 8.56 (1H, d), 11.70 (2H, br s).
**Example 268** 2-Chloro-5-(3-dimethylamino-1-piperidyl)-N-[(6-methylsulfonyl-1,3-benzothiazol-2-yl)carbamoyl]benzamide was prepared in a similar manner to Examples 233 and 235.
**Example 269** 2-Chloro-5-(3-dimethylaminocyclobutoxy)-N-[(6-methylsulfonyl-1,3-benzothiazol-2-yl)carbamoyl]benzamide was prepared in a similar manner to Example 243.
**Example 270** 2-Chloro-5-[3-(dimethylaminomethyl)pyrrolidin-1-yl]-N-[(6-methylsulfonyl-1,3-benzothiazol-2-yl)carbamoyl]benzamide was prepared in a similar manner to Examples 233 and 235.

### Preparation of Starting Materials

### Intermediate 1: 2-Chloro-N-[(6-ethenylsulfonylbenzothiazol-2-yl)carbamoyl]benzamide.

To a solution of 2-chlorobenzamide (0.16 g, 1.0 mmol) in THF (10 mL) was added oxalyl chloride (95 µL, 1.1 mmol) and the reaction mixture was heated at 120 °C in a microwave for 5 minutes. To this was added 6-ethenylsulfonylbenzothiazol-2-amine (0.24 g, 1.0 mmol) and the suspension was heated at 120 °C in a microwave for 5 minutes. The organic phase was decanted and evaporated to give the title compound (0.30 g, 71 %) that was used crude in the next reaction: ¹H NMR δ6.21 (1H, d), 6.36 (1H, d), 7.16 (1H, dd), 7.50 - 7.52 (1H, m), 7.58 - 7.61 (2H, m), 7.66 - 7.68 (1H, m), 7.87 - 7.90 (1H, m), 7.97 (1H, d), 8.66 (1H, d), 11.81 (2H, s); MS 422.

### Intermediate 2: Methyl 2-chloro-5-pyridin-2-ylbenzoate

A suspension of methyl 2-chloro-5-iodobenzoate (8.0 g, 27 mmol), 2-pyridineboronic acid N-phenyldiethanolamine ester (14.5 g, 54 mmol), palladium acetate (0.30 g, 1.35 mmol), potassium carbonate (7.46 g, 54.0 mmol), triphenylphosphine (1.42 g, 5.4 mmol) and copper iodide (2.06 g, 10.8 mmol) in THF (100 mL) was heated at 65 °C overnight under nitrogen. The reaction mixture was cooled, filtered through Celite^{®} and concentrated *in vacuo,* then the residue was suspended in DCM (250 mL) and filtered through Celite^{®}. The filtrate was concentrated *in vacuo* and the residue was purified by chromatography on silica gel eluting with EtOAc/isohexane (0 - 50%) to give the title compound as a solid (3.0 g, 49%):¹H NMR δ3.90 (3H, s), 7.39 - 7.43 (1H, m), 7.69 (1H, d), 7.89 - 7.95 (1H, m), 8.05 (1H, d), 8.24 - 8.28 (1H, m), 8.54 (1H, d), 8.68 - 8.70 (1H, m);MS 248.

### Intermediate 3: 2-Chloro-5-pyridin-2-ylbenzoic acid

To a solution of methyl 2-chloro-5-pyridin-2-ylbenzoate (Intermediate 2, 3.0 g, 12.1 mmol) in MeOH (120 mL) was added potassium hydroxide (2.0 g, 36.3 mmol) and the solution was heated at 60 °C overnight under nitrogen. The reaction mixture was acidified with glacial acetic acid, concentrated *in vacuo* and then the solid was suspended in H₂O (100 mL), filtered and dried to give the title compound as a solid (3.0 g) that was used crude in the next reaction: MS 234.

### Intermediate 4: 2-Chloro-5-pyridin-2-ylbenzamide

To a solution of 2-chloro-5-pyridin-2-ylbenzoic acid (Intermediate 3, 3.0 g, 12.8 mmol) in THF (100 mL) was added isopropyl chloroformate (1.0M in toluene, 15.5 mL, 15.5 mmol) and DIPEA (5.0 mL, 28.3 mmol) and the solution was stirred overnight under nitrogen. Ammonia (0.5M in dioxane, 300 mL, 150 mmol) was added and the suspension was concentrated *in vacuo,* then the solid was suspended in H₂O (100 mL), filtered, dried and purified by chromatography on silica gel eluting with EtOAc/isohexane (10 - 100%) to give the title compound as a white solid (1.9 g, 64%):
¹H NMR 87.36 - 7.41 (1H, m), 7.58 (1H, d), 7.63 (1H, s), 7.87 - 7.95 (2H, m), 8.01 - 8.04 (1H, m), 8.10 - 8.14 (2H, m), 8.66 - 8.69 (1H, m); MS 233.

### Intermediate 5: Methyl 2-chloro-5-(pyrimidin-2-yl)benzoate

A mixture of 2-bromopyrimidine (2.8 g, 17.63 mmol), 4-chloro-3-(methoxycarbonyl)phenylboronic acid (4.2 g, 19.59 mmol), palladium acetate (0.22 g, 0.98 mmol) and triphenylphosphine (1.028 g, 3.92 mmol) in THF (80 mL) was heated at 65 °C overnight, then the reaction mixture was cooled, filtered through Celite^{®} and concentrated *in vacuo.* The residue was partitioned between EtOAc (200 mL) and saturated brine (200 mL) and the organic phase was dried, concentrated *in vacuo,* and then the residue was purified by chromatography on silica gel eluting with EtOAc/isohexane (0 - 50%) to give the title compound as a white solid (2.0 g, 42%): MS 249.

### Intermediate 6: 2-Chloro-5-(pyrimidin-2-yl)benzamide

880 Ammonia (200 mL) was added to a solution of methyl 2-chloro-5-(pyrimidin-2-yl)benzoate (Intermediate 5, 2.0 g, 8.2 mmol) in MeOH (130 mL) and the reation mixture was stirred overnight, then filtered to give the title compound as a white solid (1.0 g, 54%) that was used crude in the next reaction: ¹H NMR 87.47 - 7.51 (1H, m), 7.64 (2H, d), 7.99 (1H, s), 8.37 - 8.41 (2H, m), 8.92 - 8.94 (2H, m); MS 234.

### Intermediate 7: Methyl 2-chloro-5-((trimethylsilyl)ethynyl)benzoate

Copper(I) iodide (4.91 µL, 0.15 mmol) and dichlorobis(triphenylphosphine)-palladium(II) (0.20 g, 0.29 mmol) were added to methyl 2-chloro-5-iodobenzoate (4.3 g, 14.50 mmol) and ethynyltrimethylsilane (3.07 mL, 21.8 mmol) in triethylamine (80 mL) and the suspension was stirred for 45 minutes. The reaction mixture was diluted with EtOAc and washed with H₂O and saturated brine. The organic phase was dried, filtered and concentrated *in vacuo,* and then the residue was purified by chromatography on silica gel eluting with EtOAc/isohexane (0 - 10%) to give the title compound as a yellow liquid (3.49 g, 90%): ¹H NMR δ0.00 (9H, s), 3.62 (3H, s), 7.31 - 7.42 (2H, m), 7.61 (1H, d).

### Intermediate 8: 2-Chloro-5-ethynylbenzoic acid

Sodium hydroxide (2M in H₂O, 16.35 mL, 32.70 mmol) was added to methyl 2-chloro-5-((trimethylsilyl)ethynyl)benzoate (Intermediate 7, 3.49 g, 13.08 mmol) in MeOH (70 mL) and the solution was stirred for 1 hour. The reaction mixture was concentrated *in vacuo,* acidified with HCl (2M in H₂O), diluted with EtOAc and then washed with water and saturated brine. The organic phase was dried, filtered and concentrated *in vacuo* to give the title compound as a white solid (2.25 g, 95%) that was used crude in the next reaction:
¹H NMR δ4.36 (1H, s), 7.51 - 7.65 (2H, m), 7.82 (1H, d), 13.58 (1H, s); MS (M-H)⁻ 179.

### Intermediate 9: 2-Chloro-5-ethynylbenzamide

Isopropyl chloroformate (1M in toluene, 14.95 mL, 14.95 mmol) was added to 2-chloro-5-ethynylbenzoic acid (Intermediate 8, 2.25 g, 12.46 mmol) and DIPEA (4.77 mL, 27.41 mmol) in THF (40 mL) and the solution was stirred for 6 hours. Ammonia (0.5M in dioxane, 249 mL, 124.59 mmol) was added and the suspension was stirred for 15 minutes and then concentrated *in vacuo.* The residue was triturated with H₂O, filtered and then washed with H₂O to give the title compound as an orange solid (1.74 g, 78%) that was used crude in the next reaction.

### Intermediate 10: Methyl 2-chloro-5-(thiazol-5-yl)benzoate

Tetrakis(triphenylphosphine)palladium(0) (33.5 mg, 0.03 mmol) was added to 5-(tributylstannyl)thiazole (0.33 g, 0.87 mmol) and methyl 2-chloro-5-iodobenzoate (0.17 g, 0.58 mmol) in toluene (2 mL) and the solution was degassed and heated at 110 °C for 4 hours. The reaction mixture was cooled, diluted with acetone and then a solution of cesium fluoride (0.3 g) in H₂O (3 mL) was added. The suspension was stirred at room temperature for 3 hours and then filtered through Celite^{®} and washed with acetone. The solution was diluted with EtOAc and the organic phase was washed with H₂O (x 2) and saturated brine. The organic phase was dried, filtered and concentrated *in vacuo,* and then the residue was purified by chromatography on silica gel eluting with EtOAc/isohexane (0 - 50%) to give the title compound as a pale yellow solid (98 mg, 67%): ¹H NMR δ3.89 (3H, s), 7.65 (1H, d), 7.83 - 7.94 (1H, m), 8.05 (1H, d), 8.42 (1H, s), 9.14 (1H, s); MS 254.

### Intermediate 11: 2-Chloro-5-(thiazol-5-yl)benzoic acid

Sodium hydroxide (2M in H₂O, 42.4 mL, 84.74 mmol) was added to methyl 2-chloro-5-(thiazol-5-yl)benzoate (Intermediate 10, 4.3 g, 16.95 mmol) in MeOH (80 mL) and the solution was stirred for 2 hours. The reaction mixture was acidified with HCl (2M in H₂O) and the precipitate was filtered and washed with H₂O to give the title compound as a pale yellow solid (3.35 g, 82%) that was used crude in the next reaction: ¹H NMR δ7.61 (1H, d), 7.84 (1H, d), 8.02 (1H, s), 8.42 (1H, s), 9.13 (1H, s), 12.78 - 14.47 (1H, m); MS 240.

### Intermediate 12: 2-Chloro-5-(thiazol-5-yl)benzamide

DIPEA (5.36 mL, 30.75 mmol) was added to 2-chloro-5-(thiazol-5-yl)benzoic acid (Intermediate 11, 3.35 g, 13.98 mmol) and isopropyl chloroformate (1M in toluene, 16.77 mL, 16.77 mmol) in THF (50 mL) and the solution was stirred for 2 hours. Ammonia (0.5M in dioxane, 280 mL, 139.8 mmol) was added and the suspension was stirred for 10 minutes, then the reaction mixture was concentrated *in vacuo* and the residue was dissolved in DCM and H₂O. The resulting precipitate was filtered, washed with DCM and dried to give the title compound as an off-white solid (2.04 g, 61 %) that was used crude in the next reaction:¹H NMR δ7.55 (1H, d), 7.62 - 7.78 (3H, m), 7.96 (1H, s), 8.40 (1H, s), 9.12 (1H, s); MS 239.

### Intermediate 13: 2-Chloro-5-pyrazol-1-ylbenzamide

To a solution of pyrazole (0.89 g, 13.04 mmol) in DMF (50 mL) was added sodium hydride (60%, 0.52 g, 13.04 mmol) and the suspension was stirred for 15 minutes then 2-chloro-5-fluorobenzamide (2.27 g, 13.04 mmol) was added and the solution was stirred for 15 minutes then heated at 120 °C overnight. The reaction mixture was concentrated *in vacuo* and the residue was suspended in H₂O (50 mL), then the solid was filtered and purified by chromatography on silica gel eluting with EtOAc/isohexane (5 - 100%) to give the title compound as a white solid (0.48 g, 17%): ¹H NMR δ6.56 (1H, m), 7.57 - 7.61 (1H, m), 7.68 (1H, s), 7.77 (1H, d), 7.87 - 7.96 (3H, m), 8.58 (1H, d); MS 222.

### Intermediate 14: 2-Chloro-5-[1,2,3]triazol-1-yl-benzamide

Intermediate 14 was prepared by the general procedure of Intermediate 13, using commercially available 1,2,3-triazole and 2-chloro-5-fluorobenzamide to give the title compound as a white solid (80 mg, 3%): ¹H NMR 87.67 - 7.73 (2H, m), 8.02 - 8.07 (3H, m), 8.17 (2H, s); MS 223.

### Intermediate 15: 2-Chloro-5-(1H-1,2,4-triazol-1-yl)benzamide

To a solution of 2-chloro-5-fluorobenzamide (6.66 g, 38.3 mmol) in DMSO (40 mL) was added potassium carbonate (10.61 g, 76.7 mmol) and 1,2,4-triazole (7.95 g, 115.1 mmol) and the suspension was heated at 150 °C for 18 hours under nitrogen. The reaction mixture was cooled, diluted with H₂O and extracted with EtOAc. The organic phase was washed with water (x 3) and saturated brine, dried, filtered and then concentrated *in vacuo* and the residue was purified by chromatography on silica gel eluting with MeOH/DCM (0 - 5%) to give the title compound as a white solid (0.95 g, 11%): ¹H NMR δ7.64 (1H, d), 7.73 (1H, s), 7.85 - 7.90 (1H, m), 7.93 (1H, d), 7.99 (1H, s), 8.23 (1H, s), 9.34 (1H, s); MS 223.

### Intermediate 16: 2-Chloro-5-(1-pyrrolidinyl)benzamide

To a solution of 2-chloro-5-fluorobenzamide (1.0 g, 5.8 mmol) in DMF (15 mL) was added pyrrolidine (2.4 mL, 29.0 mmol) and the solution was heated at 200 °C in a microwave for 1 hour. The reaction mixture was concentrated *in vacuo* and the solid was suspended in H₂O (30 mL), filtered, dried, then triturated with MeOH and filtered to give the title compound as a solid (0.20 g, 15%) that was used crude in the next reaction:
¹H NMR δ1.90 1.98 (4H, m), 3.20 (4H, t), 6.52 - 6.55 (2H, m), 7.15 - 7.19 (1H, m), 7.41 (1H, s), 7.67 (1H, s); MS 225.

### Intermediate 17: Ethyl 2-chloro-5-cyclopropylbenzoate

Ethyl 5-bromo-2-chlorobenzoate (5.0 g, 19.0 mmol), cyclopropyl boronic acid (2.12 g, 24.7 mmol), dichlorobis(tricyclohexylphosphine) palladium (II) (0.70 g, 0.95 mmol) and tripotassium phosphate (14.1 g, 66.4 mmol) was added to a degassed mixture of toluene (80 mL) and H₂O (5 mL) and the suspension was heated at 100 °C for 4 hours. The reaction mixture was concentrated *in vacuo* and the residue was purified by chromatography on silica gel eluting with EtOAc/isohexane (0 - 5%) to give the title compound as an oil (2.11 g): ¹H NMR 80.61 - 0.72 (2H, m), 0.91 - 1.01 (2H, m), 1.31 (3H, t), 1.94 - 2.03 (1H, m), 4.31 (2H, q), 7.21 - 7.25 (1H, m), 7.41 (1H, d), 7.47 (1H, d).

### Intermediate 18: 2-Chloro-5-cyclopropylbenzoic acid

To a solution of ethyl 2-chloro-5-cyclopropylbenzoate (Intermediate 17, 2.11 g, 9.39 mmol) was added lithium hydroxide monohydrate (1.97 g, 47.0 mmol) and the solution was left to stand overnight then heated at 60 °C for 3 hours. The reaction mixture was concentrated *in vacuo* and the residue was dissolved in H₂O (150 mL) and washed with EtOAc (50 mL). The aqueous phase was acidified with HCl (1M in H₂O) and extracted with DCM (2 x 150 mL). The combined organic phases were dried and concentrated *in vacuo* to give the title compound as a white solid (1.75 g, 96%) that was used crude in the next reaction: ¹H NMR 80.66 - 0.71 (2H, m), 0.94 - 1.01 (2H, m), 1.93 - 2.02 (1H, m), 7.18 - 7.21 (1H, m), 7.37 (1H, d), 7.46 (1H, d), 13.24 (1H, s); MS (M-H)⁻ 195.

### Intermediate 19: 2-Chloro-5-cyclopropylbenzamide

To a solution of 2-chloro-5-cyclopropylbenzoic acid (Intermediate 18, 1.75 g, 8.9 mmol) in THF (100 mL) was added isopropyl chloroformate (1.0M in toluene, 11 mL, 11 mmol) and DIPEA (3.5 mL, 20 mmol) and the solution was stirred overnight under nitrogen. Ammonia (0.5M in dioxane, 400 mL, 200 mmol) was added and the suspension was concentrated *in vacuo* then the residue was partitioned between DCM (250 ml) and H₂O (250 mL) and the aqueous phase was extracted with DCM (100 mL). The combined organic phases were dried, concentrated *in vacuo* and the residue was purified by chromatography on silica gel eluting with EtOAc/isohexane (10 - 100%) to give the title compound as a white solid (0.75 g, 43%): ¹H NMR 80.61 - 0.76 (2H, m), 0.88 - 1.01 (2H, m), 1.90 - 1.99 (1H, m), 7.11 (2H, m), 7.30 (1H, d), 7.50 (1H, s), 7.78 (1H, s); MS 196.

### Intermediate 20: 2-Chloro-5-(2,5-dihydro-pyrrol-1-yl)-benzamide

To a solution of 2-chloro-5-fluorobenzamide (0.50 g, 2.88 mmol) in DMF (10 mL) was added 3-pyrroline (1.09 mL, 14.4 mmol) and the solution was heated at 200 °C in a microwave for 1 hour. The reaction mixture was concentrated *in vacuo* and the solid was triturated with MeOH and filtered to give the title compound as a white solid (0.14 g, 22%) that was used crude in the next reaction: ¹H NMR δ4.04 (4H, s), 6.02 (2H, s), 6.52 - 6.55 (2H, m), 7.20 - 7.23 (1H, m), 7.44 (1H, s), 7.70 (1H, s); MS 223.

### Intermediate 21: Methyl 2-chloro-5-cyclopent-2-enyl-benzoate

To a solution of methyl 2-chloro-5-iodobenzoate (1.80 g, 6.0 mmol) in DMF (6 mL) and DIPEA (6 mL) was added palladium acetate (0.14 g, 0.60 mmol), tri-*o*-toluyl phosphine (0.37 g, 1.2 mmol) and cyclopentene (3 mL) and the reaction mixture was heated at 150 °C in a microwave for 50 minutes. The reaction mixture was concentrated *in vacuo* and the residue was partitioned between Et₂O (50 mL) and H₂O (50 mL) and the organic phase was washed with saturated brine (2 x 50 mL), dried and concentrated *in vacuo,* then the crude product was purified by chromatography on silica gel eluting with DCM/isohexane (0 - 100%) to give the title compound as an oil (0.45 g) that was used crude in the next reaction.

### Intermediate 22: 2-Chloro-5-cyclopent-2-enyl-benzoic acid

To a solution of methyl 2-chloro-5-cyclopent-2-enyl-benzoate (Intermediate 21, 0.45 g, 1.9 mmol) in MeOH (20 mL) was added potassium hydroxide (0.21 g, 3.8 mmol) and the solution was heated at 50 °C overnight. The reaction mixture was concentrated *in vacuo* and dissolved in H₂O (20 mL) then acidified with HCl (2M in H₂O) and extracted with DCM (2 x 20 mL). The combined organic phases were concentrated *in vacuo* to give the title compound as an oil (0.44 g) that was used crude in the next reaction.

### Intermediate 23: 2-Chloro-5-cyclopent-2-enylbenzamide

To a solution of 2-chloro-5-cyclopent-2-enyl-benzoic acid (Intermediate 22, 0.44 g, 1.98 mmol) in THF (20 mL) was added isopropyl chloroformate (1.0M in toluene, 2.4 mL, 2.4 mmol) and DIPEA (0.76 mL, 4.35 mmol) and the solution was stirred overnight under nitrogen. Ammonia (0.5M in dioxane, 40 mL, 20 mmol) was added and the reaction mixture was stirred for 30 minutes then concentrated *in vacuo.* The residue was partitioned between DCM (20 ml) and H₂O (20 mL) and the aqueous phase was extracted with DCM (20 mL). The combined organic phases were concentrated *in vacuo* and the residue was purified by chromatography on silica gel eluting with EtOAc/isohexane (10 - 100%) to give the title compound as a white solid (0.30 g, 68%): ¹H NMR 81.56 - 1.66 (1H, m), 2.31 - 2.44 (3H, m), 3.89 - 3.93 (1H, m), 5.72 - 5.76 (1H, m), 5.95 - 5.99 (1H, m), 7.17 - 7.20 (2H, m), 7.35 - 7.38 (1H, m), 7.51 (1H, s), 7.80 (1H, s); MS 222.

### Intermediate 24: 2-Chloro-5-cyclopentylbenzamide

To a solution of 2-chloro-5-cyclopent-2-enylbenzamide (Intermediate 23, 0.30 g, 1.35 mmol) in EtOAc (15 mL) was added 10% platinum on carbon (60 mg) under nitrogen. The suspension was stirred for 4 hours under hydrogen and then the reaction mixture was filtered and concentrated *in vacuo* to give the title compound as a white solid (0.27 g, 89%) that was used crude in the next reaction: ¹H NMR δ1.44 - 1.81 (6H, m), 1.96 - 2.05 (2H, m), 2.95 - 3.04 (1H, m), 7.27 - 7.30 (2H, m), 7.35 (1H, m), 7.51 (1H, s), 7.80 (1H, s); MS 224.

### Intermediate 25: 2-Chloro-5-hydroxybenzamide

A solution of methyl 2-chloro-5-hydroxybenzoate (5.0 g, 26.8 mmol) in 880 ammonia (100 mL) was stirred for 17 hours then the reaction mixture was concentrated *in vacuo* and suspended in EtOAc (100 mL). The precipitate was filtered and washed with EtOAc (75 mL) and isohexane (75 mL) to give the title compound as a white solid (3.02 g, 66%) that was used crude in the next reaction: ¹H NMR 86.76 - 6.80 (2H, m), 7.20 - 7.24 (1H, m), 7.44 (1H, s), 7.76 (1H, s), 9.65 (1H, s).

### Intermediate 26: 2-Chloro-5-ethoxybenzamide

To a solution of 2-chloro-5-hydroxybenzamide (Intermediate 25, 0.53 g, 3.06 mmol) in DMF (10 mL) was added potassium carbonate (1.06 g, 7.65 mmol) and ethyl bromide (0.57 mL, 7.65 mmol) and the suspension was heated at 50 °C for 24 hours. The reaction mixture was filtered, concentrated *in vacuo* and then the residue was purified by chromatography on silica gel eluting with EtOAc/isohexane (0 - 100%) to give the title compound as a white solid (0.47 g, 77%): ¹H NMR δ1.3 (3H, t), 4.04 (2H, q), 6.95 - 6.98 (2H, m), 7.32 - 7.35 (1H, m), 7.52 (1H, s), 7.79 (1H, s); MS 200.

### Intermediate 27: 2-Chloro-5-cyclopentyloxybenzamide

To a solution of 2-chloro-5-hydroxybenzamide (Intermediate 25, 0.53 g, 3.06 mmol) in DMF (10 mL) was added potassium carbonate (0.85 g, 6.12 mmol) and cyclopentyl bromide (0.40 mL, 4.0 mmol) and the suspension was heated at 50 °C for 24 hours. The reaction mixture was cooled, filtered and concentrated *in vacuo,* then the residue was purified by chromatography on silica gel eluting with EtOAc/isohexane (0 - 100%) to give the title compound as a white solid (0.45 g, 61%): ¹H NMR δ1.63 (6H, d), 1.84 - 1.98 (2H, m), 4.79 - 4.83 (1H, m), 6.90 - 6.96 (2H, m), 7.29 - 7.35 (1H, m), 7.51 (1H, s), 7.79 (1H, s); MS 240.

### Intermediate 28: 3-Carbamoyl-4-chlorophenyl methanesulfonate

Methanesulfonyl chloride (0.74 mL, 9.62 mmol) was added dropwise to triethylamine (2.70 mL, 19.23 mmol) and 2-chloro-5-hydroxybenzamide (Intermediate 25, 1.50 g, 8.74 mmol) in DCM (40 mL) and the suspension was stirred for 45 minutes under nitrogen. The reaction mixture was concentrated *in vacuo* and the residue was purified by chromatography on silica gel eluting with MeOH/DCM (0 - 10%) to give the title compound as a white solid (1.30 g, 60%): ¹H NMR δ3.42 (3H, s), 7.38 - 7.43 (2H, m), 7.60 (1H, d), 7.70 (1H, s), 7.95 (1H, s).

### Intermediate 29: 2-Chloro-5-morpholin-4-yl-benzamide

2-Chloro-5-fluorobenzamide (1.0 g, 5.76 mmol) and morpholine (2.51 mL, 28.81 mmol) were dissolved in NMP (10 mL) and the reaction mixture was heated at 180 °C for 16 hours then cooled, diluted with H₂O (100 mL) and extracted with EtOAc (3 x 75 mL). The combined organic phases were dried, filtered and concentrated *in vacuo* and then the residue was purified by chromatography on silica gel eluting with EtOAc/isohexane (0 - 100%). The crude solid was triturated with Et₂O to give the title compound as a white solid (0.22 g, 15%): ¹H NMR δ3.11 (4H, t), 3.72 (4H, t), 6.93 - 6.99 (2H, m), 7.26 (1H, d), 7.47 (1H, s), 7.74 (1H, s); MS 241.

### Intermediate 30: 2-Chloro-5-(4-methylpiperazin-1-yl)benzamide

To a solution of 2-chloro-5-fluorobenzamide (5.21 g, 30.0 mmol) in DMSO (60 mL) was added potassium carbonate (8.29 g, 60.0 mmol) and 1-methylpiperazine (16.6 mL, 150 mmol) and the suspension was heated at 130 °C for 90 minutes, then at 150 °C for 48 hours under nitrogen. The reaction mixture was cooled and poured into water (500 mL), then extracted with EtOAc (4 x 400 mL). The combined organic phases were dried, filtered and concentrated *in vacuo,* and then the residue was triturated with EtOAc and filtered to give the title compound as a white solid (3.05 g, 40%) that was used crude in the next reaction:
¹H NMR δ2.20 (3H, s), 2.42 (4H, t), 3.14 (4H, t), 6.91 - 6.97 (2H, m), 7.23 (1H, d), 7.45 (1H, s), 7.73 (1H, s); MS 254.

### Intermediate 31: 2-Chloro-5-(3-(dimethylamino)pyrrolidin-1-yl)benzamide

Intermediate 31 was prepared by the general procedure of Intermediate 30, using 2-chloro-5-fluorobenzamide and N,N-dimethylpyrrolidin-3-amine to give the title compound as a solid (1.71 g, 55%): ¹H NMR 81.75 - 1.82 (1H, m), 2.10 - 2.19 (7H, m), 2.76 (1H, m), 3.01 (1H, t), 3.17 - 3.45 (3H, m), 6.53 - 6.56 (2H, m), 7.16 - 7.19 (1H, m), 7.42 (1H, s), 7.67 (1H, s); MS 268.

### Intermediate 32: 5-Bromomethyl-2-chlorobenzamide

To a suspension of 2-chloro-5-methylbenzamide (3.7 g, 21.8 mmol) in MeCN (125 mL) was added NBS (3.9 g, 21.8 mmol) and AIBN (0.18 g, 1.09 mmol) and the reaction mixture was heated at 80 °C for 5 hours. The reaction mixture was cooled and concentrated *in vacuo,* then the residue was purified by chromatography on silica gel eluting with EtOAc/isohexane (0 - 100%) to give the title compound as a white solid (2.3 g, 42%):
¹H NMR δ4.71 (2H, s), 7.44 - 7.50 (3H, m), 7.60 (1H, s), 7.89 (1H, s); MS 250.

### Intermediate 33: 5-Bromomethyl-2-chloro-N-(6-(methylsulfonyl)benzothiazol-2-ylcarbamoyl)benzamide

ntermediate 33 was prepared by the general procedure of Example 46, using commercially available 2-amino-6-(methylsulfonyl)benzothiazole and 5-bromomethyl-2-chlorobenzamide (Intermediate 32) to give the title compound as a pale yellow solid (0.66 g); MS 504.

### Intermediate 34: 5-(2-Bromoethoxy)-2-chlorobenzamide

To a stirred solution of 2-chloro-5-hydroxybenzamide (Intermediate 25, 0.55 g, 3.19 mmol) in THF (30 mL) was added triphenylphosphine (1.0 g, 3.83 mmol), 2-bromoethanol (0.27 mL, 3.83 mmol) and di-*tert*-butyl azodicarboxylate (0.81 g, 3.51 mmol) and the solution was stirred overnight. The reaction mixture was concentrated *in vacuo* and then the residue was purified by chromatography on silica gel eluting with EtOAc/isohexane (5 - 100%) to give the title compound as a white solid (0.36 g, 40%): ¹H NMR δ3.80 (2H, t), 4.34 (2H, t), 7.01 (1H, d), 7.03 (1H, d), 7.35 - 7.38 (1H, m), 7.54 (1H, s), 7.82 (1H, s); MS 280.

### Intermediate 35: 5-(2-Bromoethoxy)-2-chloro-N-(6-(methylsulfonyl)benzothiazol-2-ylcarbamoyl)benzamide

Intermediate 35 was prepared by the general procedure of Example 46, using commercially available 2-amino-6-(methylsulfonyl)benzothiazole and 5-(2-bromoethoxy)-2-chlorobenzamide (Intermediate 34) to give the title compound as a pale yellow solid (0.18g, 44%).

### Intermediate 36: Methyl 2-chloro-5-(5-methyl-1H-pyrazol-1-yl)benzoate

To a solution of methyl 2-chloro-5-hydrazinylbenzoate (2.26 g, 11.25 mmol) in MeOH (120 mL) was added acetylacetaldehyde dimethyl acetal (1.75 mL, 11.81 mmol) and the solution was heated at 65 °C for 2 hours. The reaction mixture was concentrated *in vacuo* and the residue was purified by chromatography on silica gel eluting with EtOAc/isohexane (0 - 25%) to give the title compound as a colourless oil (0.88 g, 31%): ¹H NMR δ2.37 (3H, s), 3.88 (3H, s), 6.31 (1H, t), 7.60 - 7.61 (1H, m), 7.70 - 7.78 (2H, m), 7.94 - 7.95 (1H, m); MS 251.

### Intermediate 37: 2-Chloro-5-(5-methyl-1H-pyrazol-1-yl)benzamide

To methyl 2-chloro-5-(5-methyl-1H-pyrazol-1-yl)benzoate (Intermediate 36, 0.87 g, 3.47 mmol) was added 880 ammonia (80 mL) and MeOH (60 mL) and the solution was stirred for 4 hours. The reaction mixture was concentrated *in vacuo* and the residue was purified by chromatography on silica gel eluting with EtOAc/isohexane (0 - 100%) to give the title compound as a white solid (0.62 g, 76%): ¹H NMR δ2.37 (3H, s), 6.29 (1H, t), 7.56 - 7.63 (4H, m), 7.66 (1H, s), 7.97 (1H, s); MS 236.

### Intermediate 38: Methyl 2-chloro-5-(3-methyl-1H-pyrazol-1-yl)benzoate

Intermediate 38 was prepared by the general procedure of Intermediate 36, using commercially available methyl 2-chloro-5-hydrazinylbenzoate and acetylacetaldehyde dimethyl acetal to give the title compound as a white solid (0.94 g, 33%):
¹H NMR δ2.27 (3H, s), 3.89 (3H, s), 6.37 (1H, d), 7.64 - 7.67 (1H, m), 7.95 - 8.00 (1H, m), 8.20 (1H, d), 8.45 (1H, d); MS 251.

### Intermediate 39: 2-Chloro-5-(3-methyl-1H-pyrazol-1-yl)benzamide

To methyl 2-chloro-5-(3-methyl-1H-pyrazol-1-yl)benzoate (Intermediate 38, 0.80 g, 3.19 mmol) was added 880 ammonia (80 mL) and MeOH (60 mL) and the suspension was stirred for 4 hours. The reaction mixture was concentrated *in vacuo* to give the title compound as a white solid (0.75 g, 100%) that was used crude in the next reaction:
¹H NMR δ2.26 (3H, s), 6.35 (1H, d), 7.53 - 7.57 (1H, m), 7.66 (1H, s), 7.81 - 7.86 (2H, m), 7.94 (1H, s), 8.44 (1H, d); MS 236.

### Intermediate 40: 2-Chloro-5-pyrrol-1-yl-benzamide

To a suspension of 2-chloro-5-pyrrol-1-ylbenzoic acid (6.5 g, 29.3 mmol) in THF (100 mL) was added isopropyl chloroformate (1.0M in toluene, 29.3 mL, 29.3 mmol) and DIPEA (10.7 mL, 61.6 mmol) and the reaction mixture was stirred for 3 hours. Ammonia (0.5M in dioxane, 250 mL, 125 mmol) was added and the reaction mixture was stirred for 16 hours then concentrated *in vacuo.* The resulting solid was suspended in H₂O (200 mL) and DCM (200 mL), filtered and washed with H₂O (x 2), Et₂O and isohexane to give the title compound as a white solid (5.1 g, 78%) that was used crude in the next reaction:
¹H NMR δ6.28 (2H, d), 7.43 (2H, d), 7.51 - 7.54 (1H, m), 7.62 (2H, t), 7.65 (1H, s), 7.93 (1H, s);MS 222.

### Intermediate 41: 2-Chloro-5-(1H-imidazol-1-yl)benzamide

Copper(I) iodide (9.55 mg, 0.05 mmol) was added to 2-chloro-5-boronobenzamide (0.20 g, 1.00 mmol) and imidazole (82 mg, 1.20 mmol) in MeOH (4 mL) and the solution was heated at 65 °C for 18 hours. The reaction mixture was concentrated *in vacuo* and the residue was purified by chromatography on silica gel eluting with MeOH/DCM (0 - 10%) to give the title compound as a colourless solid (86 mg, 39%): ¹H NMR δ7.18 (1H, t), 7.68 - 7.71 (1H, m), 7.75 (1H, s), 7.77 - 7.81 (1H, m), 7.84 (1H, d), 7.88 (1H, t), 8.00 (1H, s), 8.39 (1H, s); MS 222.

### Intermediate 42: 2-Bromo-5-(IH-pyrrol-1-yl)benzoic acid

Dimethoxytetrahydrofuran (3.0 mL, 23.1 mmol) was added to a stirred solution of 5-amino-2-bromobenzoic acid (5.0 g, 23.1 mmol) in AcOH (20 mL) and the reaction mixture was heated at 120 °C for 30 minutes. The reaction mixture was cooled, concentrated *in vacuo* and the residue was diluted with MeOH/DCM (20:80) and filtered through silica gel, then the filtrate was concentrated *in vacuo* to give the title compound as an off-white solid (4.20 g, 68%) that was used crude in the next reaction: ¹H NMR δ6.28 (2H, t), 7.43 (2H, t), 7.63 - 7.67 (1H, m), 7.75 (1H, d), 7.87 (1H, d), 13.57 (1H, s); MS 267.

### Intermediate 43: 2-Bromo-5-(1H-pyrrol-1-yl)benzamide

To a solution of 2-bromo-5-(1H-pyrrol-1-yl)benzoic acid (Intermediate 42, 4.20 g, 15.8 mmol) in THF (200 mL) was added DIPEA (6.07 mL, 34.7 mmol) and isopropyl chloroformate (1M in toluene, 18.9 mL, 18.9 mmol) and the solution was stirred for 5 hours under nitrogen. Ammonia (0.5M in dioxane, 300 mL) was added and the suspension was concentrated *in vacuo,* then suspended in H₂O and filtered to give the title compound as an off-white solid (4.70 g, 97%) that was used crude in the next reaction: ¹H NMR δ6.28 (2H, t), 7.43 (2H, t), 7.53 - 7.57 (1H, m), 7.65 (3H, m), 7.91 (1H, s); MS 306.

### Intermediate 44: (4-Fluorophenyl) N-(6-methylsulfonylbenzothiazol-2-yl)carbamate

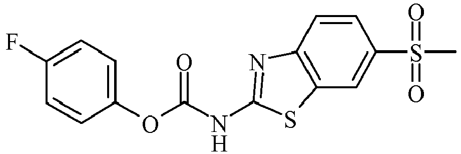

To 2-amino-6-(methylsulfonyl)benzothiazole (3.0 g, 13.14 mmol) and pyridine (1.30 mL, 15.77 mmol) in DCM (50 mL) at 0 °C was added 4-fluorophenyl chloroformate (2.10 mL, 15.77 mmol) dropwise over 2 minutes under nitrogen. The reaction mixture was warmed to ambient temperature and stirred for 2 hours, then filtered and washed with H₂O (100 mL), Et₂O (200 mL) and isohexane (100 mL) to give the title compound as a white solid (4.13 g, 86%) that was used crude in the next reaction: ¹H NMR δ3.25 (3H, s), 7.28 - 7.34 (2H, m), 7.38 - 7.41 (2H, m), 7.93 - 7.97 (2H, m), 8.65 (1H, s), 12.95 (1H, s); MS 367.

### Intermediate 45: 6-(3-Chloropropylsulfanyl)benzothiazol-2-amine

To a stirred solution of 2-aminobenzothiazole-6-thiol (10.0 g, 54.86 mmol) in MeCN (250 mL) was added potassium carbonate (11.38 g, 82.30 mmol) and 1-chloro-3-iodopropane (6.19 mL, 57.61 mmol) and the reaction mixture was heated at 85 °C for 1 hour under nitrogen. The reaction mixture was cooled, filtered and the solid was washed with MeCN (2 x 100 mL), then the filtrate was concentrated *in vacuo* and the resulting solid was triturated with isohexane to give the title compound as a white solid (16 g) that was used crude in the next reaction: ¹H NMR 81.89 - 1.97 (2H, m), 2.98 (2H, t), 3.72 (2H, t), 7.23 - 7.30 (2H, m), 7.55 (2H, s), 7.75 (1H, s); MS 259.

### Intermediate 46: 6-(3-Chloropropylsulfonyl)benzothiazol-2-amine

To a stirred solution of 6-(3-chloropropylsulfanyl)benzothiazol-2-amine (Intermediate 45, 16.3 g, 89.92 mmol) in DCM (250 mL) was added mCPBA (32.6 g, 188.8 mmol) and the reaction mixture was stirred for 1 hour, then washed with aqueous sodium metabisulfite (10% w/v, 100 mL) and saturated aqueous sodium bicarbonate solution (200 mL). The organic phase was dried, filtered, and concentrated *in vacuo* and then the resulting solid was triturated with isohexane to give the title compound as an orange solid (9.9 g, 38%) that was used crude in the next reaction:
¹H NMR 81.95 - 2.02 (2H, m), 3.37 - 3.39 (2H, m), 3.68 (2H, t), 7.49 (1H, d), 7.67 - 7.70 (1H, m), 8.05 (2H, s), 8.25 (1H, d); MS 291.

### Intermediate 47: 6-(3-Iodopropylsulfonyl)benzothiazol-2-amine

To a stirred solution of 6-(3-chloropropylsulfonyl)benzothiazol-2-amine (Intermediate 46, 6.0 g, 20.63 mmol) in acetone (100 mL) was added sodium iodide (30.9 mL, 206.3 mmol) and the reaction mixture was heated at 55 °C for 16 hours. The reaction mixture was cooled, filtered and concentrated *in vacuo* and the residue was diluted with DCM (200 mL) and then washed with water (200 mL) and saturated brine (100 mL). The organic phase was dried, filtered and concentrated *in vacuo* and then the resulting solid was triturated with isohexane to give the title compound as an orange solid (7.1 g, 90%) that was used crude in the next reaction:
¹H NMR δ2.04 (2H, q), 3.27 (2H, t), 3.33 (2H, t), 7.48 - 7.51 (1H, m), 7.66 - 7.70 (1H, m), 8.06 - 8.07 (2H, s), 8.24 (1H, d); MS 381.

### Intermediate 48: 2-Chloro-N-[[6-(3-iodopropylsulfonyl)benzothiazol-2-yl]carbamoyl]-5-pyrrol-1-yl-benzamide

To a suspension of 2-chloro-5-pyrrol-1-yl-benzamide (Intermediate 40, 0.58 g, 2.62 mmol) in THF (15 mL) was added oxalyl chloride (248 µL, 2.88 mmol) and the solution was heated at 120 °C in a microwave for 5 minutes. The reaction mixture was cooled and 6-(3-iodopropylsulfonyl)benzothiazol-2-amine (Intermediate 47, 1.0 g, 2.62 mmol) was added and the suspension was heated at 120 °C in a microwave for 5 minutes. The reaction mixture was cooled, filtered and the solid was triturated with MeOH and then Et₂O to give the title compound as an orange solid (0.50 g, 30%) that was used crude in the next reaction: ¹H NMR δ2.07 (2H, m), 3.28 (2H, t), 3.40 - 3.44 (2H, m), 6.33 (2H, t), 7.49 (2H, t), 7.68 (1H, d), 7.81 - 7.84 (1H, m), 7.92 - 7.94 (1H, m), 8.01 (2H, d), 8.69 (1H, s), 11.89 (2H, s); MS 629.

### Intermediate 49: tert-Butyl 3-(2-aminobenzothiazol-6-yl)sulfanylpyrrolidine-1-carboxylate

To a solution of *tert*-butyl 3-methylsulfonyloxypyrrolidine-1-carboxylate (1.7 g, 6.4 mmol) in MeCN (100 mL) was added 2-aminobenzothiazole-6-thiol (1.2 g, 6.4 mmol) and potassium carbonate (1.2 g, 8.3 mmol). The reaction mixture was stirred overnight, then heated at reflux for 24 hours. The reaction mixture was cooled to 60 °C, then MeOH (10 mL) and sodium borohydride (0.3 g) were added and the reaction mixture was heated for 3 hours. The reaction mixture was cooled and then concentrated *in vacuo* and the residue partitioned between DCM (100 mL) and H₂O (100 mL). The aqueous phase was extracted with DCM (50 mL) and the combined organic phases were dried, then concentrated *in vacuo* and the residue was purified by chromatography on silica gel eluting with EtOAc/isohexane (0 - 80%) to give the title compound as an off-white solid (1.1 g, 49%):
¹H NMR δ1.38 (9H, s), 1.77 (1H, s), 2.14 (1H, s), 3.12 - 3.17 (1H, m), 3.35 - 3.41 (1H, m), 3.48 - 3.54 (1H, m), 3.76 (1H, s), 7.27 (2H, s), 7.56 (2H, s), 7.77 (1H, s);MS (M-*^{t}*Bu)⁺ 296.

### Intermediate 50: tert-Butyl 3-(2-aminobenzothiazol-6-yl)sulfonylpyrrolidine-1-carboxylate

To a stirred solution of *tert*-butyl 3-(2-aminobenzothiazol-6-yl)sulfanylpyrrolidine-1-carboxylate (Intermediate 49, 1.1 g, 3.13 mmol) in DCM (100 mL) was added mCPBA (1.63 g, 9.39 mmol). The reaction mixture was stirred for 2 hours, then saturated aqueous sodium bicarbonate solution (100 mL) was added. The organic phase was separated and dried, then concentrated *in vacuo* and the residue was purified by chromatography on silica gel eluting with EtOAc/isohexane (20 - 100%) to give the title compound as a red/brown solid (0.68 g, 57%):
¹H NMR δ1.36 (9H, s), 2.14 (2H, s), 3.22 (2H, m), 3.44 (1H, m), 3.56 (1H, m), 4.02 (1H, s), 7.47 (1H, d), 7.64 - 7.68 (1H, m), 8.03 (2H, s), 8.23 (1H, d); MS (M-*^{t}*Bu)⁺ 328.

### Intermediate 51: tert-Butyl (3S)-3-(2-aminobenzothiazol-6-yl)sulfanylpyrrolidine-1-carboxylate

To a solution of *tert*-butyl (3R)-3-methylsulfonyloxypyrrolidine-1-carboxylate (6.4 g, 24.0 mmol) in MeCN (300 mL) and EtOH (30 mL) was added 2-aminobenzothiazole-6-thiol (4.0 g, 22.1 mmol), potassium carbonate (4.3 g, 31.1 mmol) and sodium borohydride (2.7 g, 71.4 mmol) and the reaction mixture was heated at 80 °C for 16 hours. The reaction mixture was cooled, concentrated *in vacuo* and the residue was diluted with DCM (500 mL) and H₂O (500 mL). The aqueous phase was separated and extracted with DCM (100 mL) and then the combined organic layers were concentrated *in vacuo* to give the title compound as a yellow solid (8.8 g) that was used crude in the next reaction: MS 350.

### Intermediate 52: tert-Butyl (3S)-3-(2-aminobenzothiazol-6-yl)sulfonylpyrrolidine-1-carboxylate

To a solution of *tert*-butyl (3S)-3-(2-aminobenzothiazol-6-yl)sulfanylpyrrolidine-1-carboxylate (Intermediate 51, 8.84 g, 25.2 mmol) in DCM (250 mL) was added mCPBA (13.2 g, 52.1 mmol) and the solution was stirred for 30 minutes. The reaction mixture was quenched by addition of aqueous sodium metabisulphite (10% w/v, 300 mL), then the organic phase was washed with saturated aqueous sodium bicarbonate solution (300 mL) and concentrated *in vacuo.* The residue was suspended in DCM and mCPBA was added (6.1 g, 26 mmol) and the reaction mixture was stirred overnight. The reaction mixture was quenched by addition of aqueous sodium metabisulphite (10% w/v, 300 mL) and the organic phase was separated and washed with saturated aqueous sodium bicarbonate solution (300 mL) and then concentrated *in vacuo.* The residue was purified by chromatography on silica gel eluting with EtOAc/isohexane (10 - 100%) to give the title compound as a white solid (0.65 g, 7%):
¹H NMR δ1.36 (9H, d), 2.14 (2H, s), 3.22 (2H, m), 3.38 - 3.67 (2H, m), 4.02 (1H, m), 7.47 (1H, d), 7.64 - 7.68 (1H, m), 8.04 (2H, s), 8.23 (1H, d); MS 382.

### Intermediate 53: tert-Butyl (3R)-3-(2-aminobenzothiazol-6-yl)sulfanylpyrrolidine-1-carboxylate

Intermediate 53 was prepared by the general procedure of Intermediate 51, using commercially available 2-aminobenzothiazole-6-thiol and *tert*-butyl (3S)-3-methylsulfonyloxypyrrolidine-1-carboxylate to give the title compound as a yellow solid (8.8 g): ¹H NMR δ1.39 (9H, s), 1.75 - 1.79 (1H, m), 2.08 - 2.13 (1H, m), 3.17 (1H, m), 3.35 - 3.41 (1H, m), 3.48 - 3.54 (1H, m), 3.76 (1H, s), 7.28 (2H, s), 7.56 (2H, s), 7.77 (1H, s); MS (M-^{t}But 296.

### Intermediate 54: tert-Butyl (3R)-3-(2-aminobenzothiazol-6-yl)sulfonylpyrrolidine-1-carboxylate

To a solution of *tert*-butyl (3R)-3-(2-aminobenzothiazol-6-yl)sulfanylpyrrolidine-1-carboxylate (Intermediate 53, 8.84 g, 25.2 mmol) in DCM (250 mL) was added mCPBA (13.2 g, 52.1 mmol) and the solution was stirred for 90 minutes and then quenched with aqueous sodium metabisulphite (10% w/v, 150 mL). The organic phase was separated and washed with saturated aqueous sodium bicarbonate solution (150 mL) and then concentrated *in vacuo* to give the title compound as an off-white solid (6.4 g) that was used crude in the next reaction.

### Intermediate 55: tert-Butyl 4-(2-aminobenzothiazol-6-ylthio)piperidine-1-carboxylate

To a solution of *tert*-butyl 4-(methylsulfonyloxy)piperidine-1-carboxylate (20.9 g, 74.82 mmol) in MeCN (900 mL) was added EtOH (100 mL), 2-aminobenzothiazole-6-thiol (13.64 g, 74.82 mmol), potassium carbonate (13.44 g, 97.26 mmol) and sodium borohydride (7.91 mL, 224.5 mmol) and the suspension was heated at 80 °C for 16 hours under nitrogen. The reaction mixture was cooled, concentrated *in vacuo* and the residue was partitioned between H₂O (900 mL) and DCM (900 mL). The aqueous phase was extracted with DCM (500 mL) and the combined organic phases were dried and concentrated *in vacuo* to give the title compound as a yellow solid (25.1 g, 92%) that was used crude in the next reaction: ¹H NMR δ1.29 - 1.34 (2H, m), 1.38 (9H, s), 1.81 - 1.85 (2H, m), 2.85 - 2.87 (2H, m), 3.20 (1H, m), 3.81 (2H, m), 7.28 (2H, d), 7.54 (2H, s), 7.77 (1H, t); MS (M-^{t}Bu+H)⁺ 310.

### Intermediate 56: tert-Butyl 4-(2-aminobenzothiazol-6-ylsulfonyl)piperidine-1-carboxylate

To a solution of *tert*-butyl 4-(2-aminobenzothiazol-6-ylthio)piperidine-1-carboxylate (Intermediate 55, 31.8 g, 87.00 mmol) in DCM (900 mL) was added mCPBA (43.1 g, 182.70 mmol) portionwise and the solution was stirred for 45 minutes, and then aqueous sodium metabisulphite (20% w/v, 500 mL) was added. The organic phase was separated, washed with saturated aqueous sodium bicarbonate solution and dried. A precipitate formed on standing which was filtered to give the title compound (23.40 g, 39%). The filtrate was concentrated *in vacuo* (100 mL) and a precipitate formed on standing which was filtered to give the title compound (9.90 g, 29%). The combined products were used crude in the next reaction: ¹H NMR 81.31 - 1.39 (11H, m), 1.83 - 1.86 (2H, m), 2.68 - 2.71 (2H, m), 3.38 (1H, t), 3.98 - 4.01 (2H, m), 7.49 (1H, d), 7.61 - 7.64 (1H, m), 8.01 (2H, s), 8.17 (1H, d); MS (M-H)⁻ 396.

### Intermediate 57: 6-(3-(4-Methylpiperazin-1-yl)propylsulfonyl)benzothiazol-2-amine

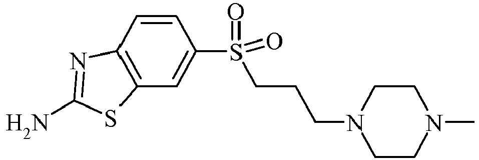

1-Methylpiperazine (12.19 mL, 109.88 mmol) was added to 6-(3-iodopropylsulfonyl)benzothiazol-2-amine (Intermediate 47, 28 g, 73.25 mmol) and potassium carbonate (11.05 mL, 183.13 mmol) in THF (250 mL) and the suspension was stirred for 20 hours. The reaction mixture was filtered and concentrated *in vacuo* and then the residue was purified by chromatography on silica gel eluting with MeOH/DCM (0 - 20%) to give the title compound as a brown oil (0.65 g, 7%):MS 355.

### Intermediate 58: 4-fluorophenyl 6-(3-(4-methylpiperazin-1-yl)propylsulfonyl)benzo-thiazol-2-ylcarbamate

4-Fluorophenyl chloroformate (4.0 mL, 30.42 mmol) was added to 6-(3-(4-methylpiperazin-1-yl)propylsulfonyl)benzothiazol-2-amine (Intermediate 57, 11.3 g, 28.97 mmol) and DIPEA (7.0 mL, 86.92 mmol) in DCM (400 mL) and the resulting mixture was stirred for 2 hours. The reaction mixture was washed with H₂O (100 mL) and the organic layer was dried, filtered and concentrated *in vacuo.* The residue was purified by chromatography on silica gel eluting with MeOH/DCM (0 - 10%) to give the title compound as a brown oil (2.8 g, 20%): MS 493.

### Intermediate 59: tert-Butyl 4-(2-(3-(2-chloro-5-(1H-pyrazol-1-yl)benzoyl)ureido)benzothiazol-6-ylsulfonyl)piperidine-1-carboxylate

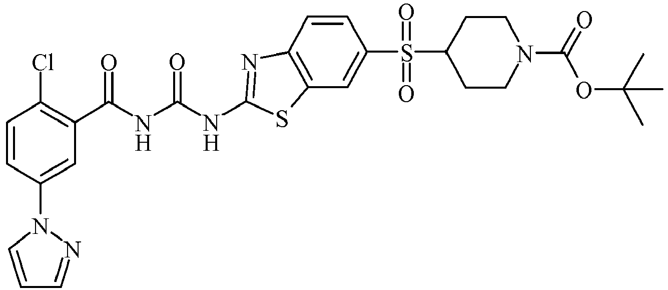

To a suspension of 2-chloro-5-(1H-pyrazol-1-yl)benzamide (Intermediate 13, 9.12 g, 41.15 mmol) in DCE (300 mL) was added oxalyl chloride (5.38 mL, 61.72 mmol) and the suspension was heated at 65 °C for 90 minutes. Dioxane (300 mL) was added and the reaction mixture was concentrated *in vacuo.* A solution of *tert*-butyl 4-(2-aminobenzothiazol-6-ylsulfonyl)piperidine-1-carboxylate (Intermediate 56, 15.54 g, 39.09 mmol) in DMF (50 mL) was added to the concentrated mixture (320 mL) at 80 °C and the solution was heated at 80 °C for 15 minutes. The reaction mixture was concentrated *in vacuo* and the residue was diluted with Et₂O and stirred overnight. The precipitate was filtered to give the title compound as a solid (14.95 g, 59%) that was used crude in the next reaction: ¹H NMR 81.33 - 1.41 (10H, m), 1.87 (2H, d), 2.54 (1H, t), 2.68 - 2.68 (1H, m), 2.73 (1H, d), 3.51 (1H, t), 3.98 - 4.02 (2H, m), 6.62 - 6.63 (1H, m), 7.74 (1H, d), 7.83 (1H, d), 7.86 - 7.89 (1H, m), 8.00 (1H, d), 8.05 - 8.07 (1H, m), 8.21 (1H, s), 8.62 (1H, d), 8.63 (1H, s), 11.91 (2H, s); MS 645.

### Intermediate 60: 2-Chloro-N-(6-(3-iodopropylsulfonyl)benzothiazol-2-ylcarbamoyl)-5-(1H-pyrazol-1-yl)benzamide

Oxalyl chloride (0.275 mL, 3.15 mmol) was added to 2-chloro-5-(1H-pyrazol-1-yl)benzamide (Intermediate 13, 0.67 g, 3.00 mmol) in THF (15 mL) and the reaction mixture was heated at 120 °C in a microwave for 5 minutes. The reaction mixture was cooled and 6-(3-iodopropylsulfonyl)benzothiazol-2-amine (Intermediate 47, 1.03 g, 2.70 mmol) was added and the suspension was heated at 120 °C in a microwave for 5 minutes. This procedure was repeated 26 times and then the reaction mixtures were combined, concentrated *in vacuo* and added to sodium iodide (18.64 mL, 455.96 mmol) in acetone (391 mL) and the suspension was heated at 65 °C for 16 hours. The reaction mixture was cooled, concentrated *in vacuo* and the residue was dissolved in THF/EtOAc (1:1, 300 mL) and washed with H₂O (100 mL) and saturated brine (100 mL). The organic phase was dried, filtered and concentrated *in vacuo* to give the title compound as a solid that was used crude in the next reaction: ¹H NMR 82.06 - 2.09 (2H, m), 3.25 - 3.29 (4H, m), 6.61 (1H, s), 7.71 (1H, d), 7.81 (1H, s), 7.87 - 8.09 (3H, m), 8.19 (1H, s), 8.57 (2H, d); MS 630.

### Intermediate 61 : Ethyl 2-(2-aminobenzo[d]thiazol-6-ylthio)-2-methylpropanoate

Sodium hydride (0.966 g, 24.1 mmol) was added portionwise to 2-aminobenzo-[d]thiazole-6-thiol (4.00 g, 22.0 mmol) in DMF (30 mL) cooled to 0 °C over a period of 10 minutes under nitrogen. The resulting solution was stirred at 0 °C for 20 minutes before adding ethyl 2-bromoisobutyrate (3.54 mL, 24.1 mmol) and warming to 25 °C and stirring for 1.5 hours. The reaction mixture was diluted with water (500 mL), and the yellow precipitate filtered off and washed with water. Material used crude. m/z (ESI+) (M+H)+ = 297; HPLC tR = 2.00 min.

### Intermediate 62 :2-(2-aminobenzo[d]thiazol-6-ylthio)-2-methylpropanoic acid

Sodium hydroxide (60.4 mL, 120.7 mmol) was added to ethyl 2-(2-aminobenzo[d]thiazol-6-ylthio)-2-methylpropanoate (Intermediate 61, 7.16 g, 24.1 mmol) in ethanol (50 mL) and THF (50 mL) at 25 °C. The resulting solution was stirred at 50 °C for 2 hours. The reaction mixture was concentrated *in-vacuo* and then acidified with 2M HCl. The solid was filtered, washed with water and dried to yield 2-(2-aminobenzo[d]-thiazol-6-ylthio)-2-methylpropanoic acid (2.61 g, 40 %) as a yellow solid. m/z (ESI+) (M+H)+ = 269; HPLC tR = 1.40 min. ¹H NMR (400.13 MHz, DMSO-d6) δ 1.37 (6H, s), 7.30 - 7.30 (1H, m), 7.72 (1H, s), 7.75 (1H, t), 12.50 (1H, s).

### Intermediate 63: 2-(2-aminobenzo[d]thiazol-6-ylthio)-N-isopropyl-2-methyl-propanamide

N-Ethyldiisopropylamine (2.52 mL, 14.6 mmol) was added to isopropylamine (1.25 mL, 14.6 mmol), 2-(2-aminobenzo[d]thiazol-6-ylthio)-2-methylpropanoic acid (2.61 g, 9.73 mmol) and 1-(3-dimethylaminopropyl)-3-ethylcarbodiimide hydrochloride (2.24 g, 11.7 mmol) in DMF (20 mL) at 25 °C under nitrogen. The resulting solution was stirred at 0.486 molar for 2.5 hours. The reaction was incomplete and further Isopropylamine (1.25 mL, 14.6 mmol) and 1-(3-dimethylaminopropyl)-3-ethylcarbodiimide hydrochloride (2.24 g, 11.7 mmol) were added and the solution was stirred at 25 °C for a further 1 hour. The reaction was incomplete so the temperature was increased to 50 °C and the reaction mixture was stirred for a further 16 hours. The reaction mixture was concentrated and diluted with water (100 mL), extracted with EtOAc (2 x 200ml) and washed with more water (100 mL). The organic layer was dried over MgSO₄, filtered and evaporated to afford crude product. The crude product was purified by flash silica chromatography, elution gradient 0 to 70% EtOAc in isohexane. Pure fractions were evaporated to dryness to afford 2-(2-aminobenzo[d]thiazol-6-ylthio)-N-isopropyl-2-methylpropanamide (0.655 g, 21.76 %) as a yellow solid. m/z (ESI+) (M+H)+ = 310; HPLC tR = 1.44 min.¹H NMR (400.13 MHz, CDCl₃) δ 1.14 - 1.17 (6H, d), 1.48 (6H, s), 4.02 - 4.07 (1H, m), 5.45 (2H, s), 6.57 (1H, d), 7.36 - 7.38 (1H, m), 7.44 (1H, d), 7.67 (1H, d)

### Intermediate 64: 2-(2-aminobenzo[d]thiazol-6-ylthio)-N-isopropyl-2-methylpropanamine

Borane-methyl sulfide complex (2.12 mL, 4.23 mmol) was added to 2-(2-aminobenzo[d]thiazol-6-ylthio)-N-isopropyl-2-methylpropanamide (Intermediate 63, 655 mg, 2.12 mmol) in THF (20 mL) at room temperature under nitrogen. The resulting solution was stirred at 60 °C for 1 hour. The reaction was incomplete and further borane-methyl sulfide complex (2.12 mL, 4.23 mmol) was added and the solution was stirred at 60 °C for a further 1 hours. MeOH (20.0 mL) was added slowly (exotherm) to the reaction mixture and this was stirred at reflux for a further 30 minutes. The reaction mixture was absorbed onto silica and The crude product was purified by flash silica chromatography, elution gradient 0 to 5% 7N NH₃ in MeOH in DCM. Pure fractions were evaporated to dryness to afford 6-(1-(isopropylamino)-2-methylpropan-2-ylthio)benzo[d]thiazol-2-amine (213 mg, 34.1 %) as a yellow gum and 2-(2-aminobenzo[d]thiazol-6-ylthio)-N-isopropyl-2-methylpropanamide (189 mg, 28.9 %) as a yellow gum.

### Intermediate 66 :(9H-fluoren-9-yl)methyl 2-(2-aminobenzo[d]thiazol-6-ylthio)-2-methylpropyl(isopropyl)carbamate

9-Fluorenylmethyl chloroformate (202 mg, 0.78 mmol) in THF (14 mL) was added dropwise to 6-(1-(isopropylamino)-2-methylpropan-2-ylthio)benzo[d]thiazol-2-amine (Intermediate 64, 210 mg, 0.71 mmol) and sodium carbonate (6.70 mL, 3.35 mmol) in THF (21 mL) and water (7 mL) at 25 °C. The resulting mixture was stirred at 25 °C for 20 hours. The reaction mixture was diluted with EtOAc (2 x 100 mL), and washed with water (2 x 100 mL). The organic layer was dried over MgSO₄, filtered and evaporated to afford crude product. The crude product was purified by flash silica chromatography, elution gradient 0 to 100% EtOAc in isohexane. Pure fractions were evaporated to dryness to afford (9H-fluoren-9-yl)methyl 2-(2-aminobenzo[d]thiazol-6-ylthio)-2-methylpropyl(isopropyl)carbamate (69.0 mg, 18 %) as a colourless gum.
m/z (ESI+) (M+H)+ = 518; HPLC tR = 3.10 min.¹H NMR (400.13 MHz, CDCl₃) δ 0.90 - 1.2 (12H, broad hump), 3.28 (3H, s), 4.17 (1H, s), 4.50 - 4.62 (2H, m), 5.59 (2H, s), 7.26 - 7.30 (2H, m), 7.36 (3H, t), 7.42 - 7.44 (1H, m), 7.55 (2H, d), 7.68 (1H, s), 7.73 (2H, d).

### Intermediate 67 :(9H-fluoren-9-yl)methyl 2-(2-(3-(2-chlorobenzoyl)ureido)benzo[d]thiazol-6-ylthio)-2-methylpropyl(isopropyl)carbamate

Oxalyl chloride (0.019 mL, 0.22 mmol) was added to 2-chlorobenzamide (31.1 mg, 0.20 mmol) in DCE (5 mL) at 25 °C. The resulting solution was stirred at 85 °C for 90 minutes. Dioxane (5 mL) was added to the reaction mixture and the DCE (5 mL) was removed by distillation at atmospheric pressure, collecting fractions that distilled at 90 °C. The reaction mixture was allowed to cool to 85 °C before adding :(9H-fluoren-9-yl)methyl 2-(2-aminobenzo[d]thiazol-6-ylthio)-2-methylpropyl(isopropyl)carbamate (intermediate 66, 69.0 mmol, 0.133 mmol) as a suspension in dioxane (3 mL). The reaction mixture was stirred for a further 90 minutes. The reaction mixture was absorbed onto silica and the crude product was purified by flash silica chromatography, elution gradient 0 to 50% EtOAc in isohexane. Pure fractions were evaporated to dryness to afford (9H-fluoren-9-yl)methyl 2-(2-(3-(2-chlorobenzoyl)ureido)benzo[d]thiazol-6-ylthio)-2-methylpropyl(isopropyl)carbamate (122 mg, 131 %) as a yellow gum.
m/z (ESI+) (M+H)+ = 699; HPLC tR = 3.94 min.

### Intermediate 68 :(9H-fluoren-9-yl)methyl 2-(2-(3-(2-chlorobenzoyl)ureido)benzo-[d]thiazol-6-ylsulfonyl)-2-methylpropyl(isopropyl)carbamate

Potassium monopersulphate triple salt (0.088 g, 0.140 mmol) was added in one portion to (9H-fluoren-9-yl)methyl 2-(2-(3-(2-chlorobenzoyl)ureido)benzo[d]thiazol-6-ylthio)-2-methylpropyl(isopropyl)carbamate (Intermediate 67, 0.091 g, 0.13 mmol) in THF (6 mL) and water (3 mL) at 25 °C. The resulting suspension was stirred at 25 °C for 5 days. The reaction mixture was diluted with EtOAc (20 mL), and washed with water (20 mL). The organic layer was dried over MgSO₄, filtered and evaporated to afford crude product.

### Intermediate 69 : (S)-2-chloro-5-(3-(dimethylamino)pyrrolidin-1-yl)benzamide

To a solution of (S)-*N,N*-dimethylpyrrolidin-3-amine (5.92 g, 51.9 mmol) in DMSO (35 mL) was added 2-chloro-5-fluorobenzamide (3.00 g, 17.3 mmol) and potassium carbonate (1.97 ml, 34.6 mmol). The suspension was heated to 150 °C for 6 days. The reaction mixture was allowed to cool and poured into water (1000 mL). The aqueous phase was extracted with EtOAc (4 x 250ml). The combined organics were dried (Na₂SO₄) and evaporated to a brown solid. The crude solid was triturated with EtOAc to give a solid which was collected by filtration and dried under vacuum to give (S)-2-chloro-5-(3-(dimethylamino)pyrrolidin-1-yl)benzamide (2.75 g, 59.4 %) as an off-white solid. m/z (ESI+) (M+H)+ = 268; HPLC tR = 0.59 min. ¹H NMR (400.13 MHz, DMSO-d6) δ 1.80 (1H, m), 2.16 - 2.21 (7H, m), 2.77 (1H, m), 2.99 - 3.06 (1H, m), 3.21 - 3.27 (1H, m), 3.44 (1H, m), 6.56 (2H, m), 7.20 (1H, m), 7.49 (1H, s), 7.74 (1H, s) one CH not seen.

### Intermediate 70 : (R)-2-chloro-5-(3-(dimethylamino)pyrrolidin-1-yl)benzamide

Potassium carbonate (3.98 g, 28.8 mmol) was added to 2-chloro-5-fluorobenzamide (2.5 g, 14.40 mmol) and (3R)-(+)-3-(dimethylamino)pyrrolidine (4.93 g, 43.2 mmol) in DMSO (50 mL). The resulting suspension was stirred at 150 °C for 3 days. The reaction mixture was diluted with water and extracted into EtOAc. The organic layer was washed with water (3 x 30 mL) and brine (30 mL), dried over MgSO₄, filtered and evaporated to afford crude product. The crude product was purified by flash silica chromatography, elution 100% MeOH. The column was opened and the contents were slurried in 50/50 methanol/DCM (1000ml) for 1 hour. The silica was filtered off and the liquors concentrated to a brown solid. (R)-2-chloro-5-(3-(dimethylamino)pyrrolidin-1-yl)benzamide (1.20 g, 31.1 %). m/z (ESI+) (M+H)+ = 268.33; HPLC tR = 1.48 min.
¹H NMR (400.132 MHz, DMSO) δ 1.78 - 1.91 (1H, m), 2.16 - 2.31 (8H, m), 2.79 - 2.86 (1H, m), 3.07 (1H, t), 3.22 - 3.34 (1H, m), 3.45 - 3.53 (1H, m), 6.57 - 6.64 (2H, m), 7.25 (1H, d), 7.54 (1H, s), 7.79 (1H, s).

### Intermediate 71 :methyl 2-chloro-5-(4-(dimethylamino)piperidin-1-yl)benzoate

Methyl 2-chloro-5-iodobenzoate (1.00g, 3.37 mmol), cesium carbonate (0.360 mL, 4.50 mmol), BINAP (0.070 g, 0.11 mmol), palladium(II) acetate (0.025 g, 0.11 mmol) and 4-(dimethylamino)piperidine (0.519 g, 4.05 mmol) were suspended in dioxane (18 mL) and sealed into a microwave tube. The reaction was heated to 120 °C for 3 hours in the microwave reactor and cooled to RT. The reaction mixture was filtered through celite. The crude product was purified by flash silica chromatography, elution gradient 0 to 10% NH₃/MeOH in DCM. Pure fractions were evaporated to dryness to afford methyl 2-chloro-5-(4-(dimethylamino)piperidin-1-yl)benzoate (0.456 g, 68.3 %) as a yellow oil.
m/z (ESI+) (M+H)+ =297.45; HPLC tR = 1.01 min. ¹H NMR (400.13 MHz, CDCl₃) δ 1.56 - 1.66 (2H, m), 1.93 (2H, d), 2.24 - 2.29 (1H, m), 2.31 (6H, s), 2.71 - 2.78 (2H, m), 3.71 (2H, d), 3.92 (3H, s), 6.94 - 6.97 (1H, m), 7.27 - 7.28 (1H, m), 7.33 (1H, d).

### Intermediate 72:2-chloro-5-(4-(dimethylamino)piperidin-1-yl)benzamide

Methyl 2-chloro-5-(4-(dimethylamino)piperidin-1-yl)benzoate (Intermediate 72, 458 mg, 1.54 mmol) was treated with ammonia (60 mL, 1080 mmol) in MeOH (40 mL). The resulting solution was stirred at room temperature for 16 hours. The crude product was purified by ion exchange chromatography, using an SCX column. The desired product was eluted from the column using MeOH and pure fractions were evaporated to dryness to afford 2-chloro-5-(4-(dimethylamino)piperidin-1-yl)benzamide (365 mg, 84 %) as a cream solid. m/z (ESI+) (M+H)+ = 282.33; HPLC tR = 0.64 min. ¹H NMR (400.13 MHz, CDCl₃) δ 1.60 (2H, m), 1.91 - 1.94 (2H, m), 2.24 - 2.29 (1H, m), 2.31 (6H, s), 2.72 - 2.79 (2H, m), 3.74 (2H, d), 5.85 - 5.91 (1H, s), 6.49 - 6.51 (1H, s), 6.91 - 6.94 (1H, m), 7.24 (1H, d), 7.37 (1H, d)

### Intermediate 73 :2-chloro-5-(3,5-dimethyl-1H-pyrazol-1-yl)benzamide

To a solution of 2-chloro-5-(3,5-dimethyl-1H-pyrazol-1-yl)benzoic acid (0.500 g, 1.99 mmol) in dichloromethane (20 mL) at ambient temperature under nitrogen was added *N*-ethyldiisopropylamine (0.659 mL, 3.99 mmol) and isopropyl chloroformate (2.79 mL, 2.79 mmol). The resultant solution was stirred at room temperature for 16 h. The reaction mixture was poured into a solution of ammonia in dioxane (0.5M, 40mL, 20mmol). The reaction mixture was evaporated, and the residue suspended in water. The solid was collected by filtration and dried to give 2-chloro-5-(3,5-dimethyl-1H-pyrazol-1-yl)benzamide (0.358 g, 71.9 %) as a solid. m/z (ESI+) (M+H)+ = 250; HPLC tR = 1.53 min. ¹H NMR (400.13 MHz, DMSO-d6) δ 2.18 (3H, s), 2.34 (3H, d), 6.11 (1H, s), 7.54 - 7.55 (1H, m), 7.57 - 7.61 (2H, m), 7.71 (1H, s), 8.01 (1H, s)

### Intermediate 77 :methyl 2-chloro-5-(3-(diethylamino)pyrrolidin-1-yl)benzoate

Methyl 2-chloro-5-iodobenzoate (1.00 g, 3.37 mmol), cesium carbonate (1.648 g, 5.06 mmol), BINAP (0.105 g, 0.17 mmol), palladium(II) acetate (0.038 g, 0.17 mmol) and *N,N*-diethylpyrrolidin-3-amine (0.576 g, 4.05 mmol) were suspended in dioxane (15 mL) and sealed into a microwave tube. The reaction was heated to 120 °C for 2 hours in the microwave reactor and cooled to RT. The reaction mixture was filtered through celite before concentrating in vacuo. The crude product was purified by flash silica chromatography, elution gradient 0 to 5% NH₃/MeOH in DCM. The product was isolated but still contained less polar impurities. The crude product was purified by ion exchange chromatography, using an SCX column. The desired product was eluted from the column using 7M NH₃/MeOH and pure fractions were evaporated to dryness to afford methyl 2-chloro-5-(3-(diethylamino)pyrrolidin-1-yl)benzoate (0.257 g, 24.52 %) as a brown gum. m/z (ESI+) (M+H)+ =311; HPLC tR = 1.16 min.
¹H NMR (400.13 MHz, DMSO-d6) δ 0.96 (6H, t), 1.80 - 1.85 (1H, m), 2.11 - 2.17 (1H, m), 2.54 - 2.63 (4H, m), 2.99 (1H, t), 3.15 - 3.22 (2H, m), 3.40 - 3.44 (2H, m), 6.53 - 6.56 (2H, m), 7.16 - 7.18 (1H, m), 7.42 (1H, s), 7.67 (1H, s).

### Intermediate 78 :2-chloro-5-(3-(diethylamino)pyrrolidin-1-yl)benzamide

Methyl 2-chloro-5-(3-(diethylamino)pyrrolidin-1-yl)benzoate (Intermediate 77, 257 mg, 0.830 mmol) was treated with ammonia (30 mL, 540 mmol) in MeOH (20 mL). The resulting solution was stirred at room temperature for 16 hours. The crude product was purified by ion exchange chromatography, using an SAX column. The desired product was eluted from the column using MeOH and pure fractions were evaporated to dryness, triturated with ether and filtered to afford 2-chloro-5-(3-(diethylamino)pyrrolidin-1-yl)benzamide (180 mg, 73.6 %) as a beige solid. m/z (ESI+) (M+H)+ = 296; HPLC tR = 0.73 min. ¹H NMR (400.13 MHz, DMSO-d6) δ 0.96 (6H, t), 1.80 - 1.85 (1H, m), 2.11 - 2.17 (1H, m), 2.54 - 2.63 (4H, m), 2.99 (1H, t), 3.15 - 3.22 (2H, m), 3.40 - 3.44 (2H, m), 6.53 - 6.56 (2H, m), 7.16 - 7.18 (1H, m), 7.42 (1H, s), 7.67 (1H, s).

### Intermediate 79 :2-chloro-3-hydrazinylbenzoic acid hydrochloride

To a suspension of 3-amino-2-chlorobenzoic acid (2.00 g, 11.7 mmol) in hydrochloric acid, 37% (25 mL) at 0 °C was added a cold solution of sodium nitrite (0.965 g, 13.99 mmol) in water (10.0 mL) dropwise maintaining the temperature below 5 °C. The resultant solution was stirred in an ice-bath for 30 min before dropwise addition of a solution of Tin(II) chloride dihydrate (7.89 g, 35.0 mmol) in hydrochloric acid, 37% (10 mL) maintaining the temperature below 5 °C. The resultant suspension was stirred at 0 °C for 3 h and the resultant suspension filtered and the collected solid dried to give crude 2-chloro-3-hydrazinylbenzoic acid hydrochloride (2.60 g, 100 %) as a solid. This was used without purification in the next step. ¹H NMR (400.13 MHz, DMSO-d6) δ 7.11 - 7.14 (1H, m), 7.24 - 7.26 (1H, m), 7.33 (1H, t), 7.58 (1H, s).

### Intermediate 80 :methyl 2-chloro-3-hydrazinylbenzoate hydrochloride

To ice-cooled methanol (200 mL) was added (cautiously) acetyl chloride (40 mL, 562 mmol) . To the resultant solution was added crude 2-chloro-3-hydrazinylbenzoic acid hydrochloride (intermediate 79, 2.60 g, 11.7 mmol). The reaction mixture was heated to 50 °C for 2 h . The reaction mixture was evaporated to give methyl 2-chloro-3-hydrazinylbenzoate hydrochloride (2.76 g, 100 %) as a solid. ¹H NMR (400.13 MHz, DMSO-d6) δ 3.85 (3H, s), 7.24 - 7.26 (1H, m), 7.30 - 7.32 (1H, m), 7.43 (1H, t), 8.24 (1H, s), 10.20 (2H, br. s).

### Intermediate 81 :methyl 2-chloro-3-(1H-pyrazol-1-yl)benzoate

To a suspension of the crude methyl 2-chloro-3-hydrazinylbenzoate hydrochloride (Intermediate 80, 2.76 g, 11.6 mmol) in ethanol (25 mL) was added malonaldehyde bis(dimethyl acetal) (1.92 mL, 11.6 mmol). The reaction mixture was heated to 80 °C for 2 h. The reaction mixture was allowed to cool and evaporated. The resultant solid was suspended in DCM (50 mL), filtered and dried to give methyl 2-chloro-3-(1H-pyrazol-1-yl)benzoate (2.76 g, 100 %) as a yellow solid. m/z (ESI+) (M+H)+ = 237; HPLC tR = 1.84 min. ¹H NMR (400.13 MHz, DMSO-d6) δ 3.89 (3H, s), 6.53 - 6.54 (1H, m), 7.60 (1H, t), 7.72 - 7.74 (1H, m), 7.76 (1H, d), 7.83 - 7.85 (1H, m), 8.14 - 8.15 (1H, m).

### Intermediate 82 :2-chloro-3-(1H-pyrazol-1-yl)benzamide

Methyl 2-chloro-3-(1H-pyrazol-1-yl)benzoate (2.75 g, 11.6 mmol) was suspended in 37% aqueous ammonia (250 mL, 11.6 mmol) and methanol (200 mL) and stirred at room temperature overnight. The reaction mixture was concentrated to approx. 20mL. The aqueous phase was extracted with DCM (2 x 50 mL). The combined organics were evaporated to give 2-chloro-3-(1H-pyrazol-1-yl)benzamide (1.31 g, 51 %) as a solid. m/z (ESI+) (M+H)+ = 222; HPLC tR = 0.99 min.
¹H NMR (400.13 MHz, DMSO-d6) δ 6.53 (1H, t), 7.50 - 7.54 (2H, m), 7.56 - 7.60 (1H, m), 7.66 (1H, s), 7.75 (1H, d), 7.98 (1H, s), 8.08 (1H, d).

### Intermediate 83 : 2,4-dichloro-5-hydrazinylbenzoic acid

To a suspension of 5-amino-2,4-dichlorobenzoic acid (5.00 g, 24.3 mmol) in hydrochloric acid, 37% (50 mL) cooled to 0 °C was added a cold solution of sodium nitrite (2.01 g, 29.1 mmol) in water (25.0 mL) dropwise maintaining an internal temperature below 5 °C. The resultant solution was stirred in an ice-bath for 30 min before dropwise addition of a solution of tin(II) chloride (13.8 g, 72.8 mmol) in hydrochloric acid, 37% (20 mL) maintaining the temperature below 5°C. The resultant suspension was stirred at 0 °C for 3 hours. The solid was filtered off and washed with cold water (50 mL) and dried. The crude 2,4-dichloro-5-hydrazinylbenzoic acid (6.28 g, 100 %) was used without further purification. ¹H NMR (400.132 MHz, DMSO) δ 7.53 (1H, s), 7.67 (1H, s), 8.30 (1H, s), 10.64 (2H, brs).

### Intermediate 84 :methyl 2,4-dichloro-5-hydrazinylbenzoate

Acetyl chloride (12.0 mL, 168 mmol) was added dropwise to MeOH (150 mL) cooled to 0 °C over a period of 10 minutes. To the resulting solution was added 2,4-dichloro-5-hydrazinylbenzoic acid, HCl (Intermediate 83, 6.72 g, 22.4 mmol). The mixture was allowed to warm to 20 °C and stirred for 3 days. The reaction mixture was evaporated to dryness to give crude methyl 2,4-dichloro-5-hydrazinylbenzoate (6.25 g, 103 %) as an orange solid which was used without further purification. m/z (ESI+) mass ion not seen; HPLC tR = 1.25 min. ¹H NMR (400.132 MHz, DMSO) δ 3.88 (3H, s), 7.56 (1H, s), 7.75 (1H, s), 8.46 (1H, brs), 10.44 (2H, brs).

### Intermediate 85 :methyl 2,4-dichloro-5-(1H-pyrazol-1-yl)benzoate

Malonaldehyde bis(dimethyl acetal) (4.08 mL, 24.8 mmol) was added in one portion to methyl 2,4-dichloro-5-hydrazinylbenzoate (Intermediate 84, 6.69 g, 24.8 mmol) in EtOH (80 mL) at 20 °C. The resulting suspension was heated to and stirred at 80 °C for 2 hours. The reaction mixture was evaporated to dryness to afford crude methyl 2,4-dichloro-5-(1H-pyrazol-1-yl)benzoate (3.84 g, 57 %). The crude product was purified by flash silica chromatography, elution gradient 0 to 40% EtOAc in isohexane. Pure fractions were evaporated to dryness to afford methyl 2,4-dichloro-5-(1H-pyrazol-1-yl)benzoate (3.84 g, 57%) as a yellow oil which crystallised on standing. m/z (ESI+) (M+H)+ = 271, 273; HPLC tR = 2.26 min.
¹H NMR (400.132 MHz, DMSO) δ 3.94 (3H, s), 6.63 - 6.64 (1H, m), 7.87 (1H, d), 8.08 (1H, s), 8.12 (1H, s), 8.29 (1H, d).

### Intermediate 86 :2,4-dichloro-5-(1H-pyrazol-1-yl)benzamide

Methyl 2,4-dichloro-5-(1H-pyrazol-1-yl)benzoate (Intermediate 85, 3.84 g, 14.2 mmol) was added in one portion to 880 ammonia (150mL) which formed a suspension. MeOH (75 mL) was added to give a cloudy solution which was stirred at 20 °C for 16 hours. The mixture was evaporated to dryness, re-suspended in water (100 mL) and treated with NaHCO₃ (sat, aq, 100mL). The resulting solid was filtered off, washed with water (50 mL) and dried to give an orange solid. This was triturated with ether (100 mL), filtered off and dried to afford 2,4-dichloro-5-(1H-pyrazol-1-yl)benzamide (2.09 g, 58 %) which was used withour further purification. m/z (ESI+) (M+H)+ = 256, 258; HPLC tR = 1.40 min. ¹H NMR (400.132 MHz, DMSO) δ 6.55 - 6.56 (1H, m), 7.63 (1H, s), 7.73 (1H, s), 7.79 (1H, d), 7.93 (1H, s), 8.01 (1H, s), 8.17 (1H, d).

### Intermediate 87 :2-chloro-4,5-dinitrobenzoic acid

To 95% sulfuric acid (40 mL) was added red fuming nitric acid (6 mL, 144 mmol) followed by 2-chloro-4-nitrobenzoic acid (10.0 g, 49.6 mmol). The suspension was heated to 90 °C for 40 min. The reaction mixture was allowed to cool and then quenched with ice-water (400 mL), The suspension was filtered and washed with water to give 2-chloro-4,5-dinitrobenzoic acid (11.6 g, 95 %) as a solid. Used crude.

### Intermediate 88 :2-chloro-4-methoxy-5-nitrobenzoic acid

To a solution of 2-chloro-4,5-dinitrobenzoic acid (11.6 g, 47.0 mmol) in methanol (55 mL) was added a solution of potassium hydroxide (5.79 g, 103 mmol) in methanol (55 mL). The reaction mixture was stirred whilst heating to 50 °C. After 40 min the thick suspension was diluted with water (220 mL) and the resultant solution acidified with hydrochloric acid. The resultant precipitate was collected by filtration to give 2-chloro-4-methoxy-5-nitrobenzoic acid (7.16 g, 66 %) as a white solid.m/z (ESI-) (M-H)- = 230; HPLC tR = 1.60 min. ¹H NMR (400.13 MHz, DMSO-d6) δ 4.01 (3H, s), 7.55 (1H, s), 8.39 (1H, s), 13.58 (1H, br s).

### Intermediate 89 :methyl 2-chloro-4-methoxy-5-nitrobenzoate

To ice-cooled methanol (600 mL) was added acetyl chloride (120 mL, 1.60 mol) (CAUTION: exotherm). To this was added 2-chloro-4-methoxy-5-nitrobenzoic acid (7.16 g, 30.9 mmol). The reaction mixture was warmed to 50 °C for 3 h. The solution was evaporated to give methyl 2-chloro-4-methoxy-5-nitrobenzoate (7.50 g, 99 %) as white solid. No mass ion detected; RT = 2.06 min. ¹H NMR (400.13 MHz, DMSO-d6) δ 3.86 (3H, s), 4.03 (3H, s), 7.60 (1H, s), 8.41 (1H, s).

### Intermediate 90 :methyl 5-amino-2-chloro-4-methoxybenzoate

To a suspension of methyl 2-chloro-4-methoxy-5-nitrobenzoate (7.50 g, 30.5 mmol) in methanol (300 mL) was added tin(II) chloride (29.0 g, 152 mmol) in a single portion (reaction mixture turns yellow and gradually becomes homogeneous). The reaction mixture was heated to 65 °C for 2 h. The reaction mixture was evaporated and the residue diluted with water (300 mL). This was then neutralised with saturated aquoeus sodium bicarbonate (CAUTION: Froths - the froth could be dispersed by addition of a little EtOAc). To the resultant suspension was added EtOAc (300 mL). The mixture looked very difficult to separate and so was filtered to remove precipitated tin residues. The organic layer was separated, washed with brine, dried (Na₂SO₄) and evaporated to give methyl 5-amino-2-chloro-4-methoxybenzoate (5.30 g, 80 %) as a brown oil. This was used in the next step without purification. m/z (ESI+) (M+H)+ = 216; HPLC tR = 1.58 min.

### Intermediate 91 :potassium 5-amino-2-chloro-4-methoxybenzoate

To a solution of methyl 5-amino-2-chloro-4-methoxybenzoate (5.30 g, 24.6 mmol) in ethanol (125 mL) was added potassium hydroxide (1.52 g, 27.0 mmol). The resultant brown solution was stirred at 70 °C 2 hours. Water (2 mL) was added and the reaction mixture heated for a further 1h. The reaction mixture was evaporated to give potassium 5-amino-2-chloro-4-methoxybenzoate (5.89 g, 100 %) as a brown solid. This was used in the next stage without purification. m/z (ESI-) (M-H)- = 200; HPLC tR = 0.90 min. ¹H NMR (400.13 MHz, DMSO-d6) δ 3.71 (3H, s), 6.59 (1H, s), 6.80 (1H, s).

### Intermediate 92 :2-chloro-5-hydrazinyl-4-methoxybenzoic acid hydrochloride

To a suspension of potassium 5-amino-2-chloro-4-methoxybenzoate (5.89 g, 24.6 mmol) in hydrochloric acid, 37% (60 mL) at -10°C was added a cold solution of sodium nitrite (2.04 g, 29.5 mmol) in water (24.0 mL) dropwise maintaining the temperature below 0°C. The resultant solution was stirred in an ice-bath for 30 min before dropwise addition of a solution of tin(II) chloride dihydrate (16.6 g, 73.7 mmol) in hydrochloric acid, 37% (25 mL) maintaining the temperature below 5 °C. The resultant suspension was stirred at - 10 °C to 0 °C for 2.5 h and the resultant suspension filtered and the collected solid dried to give crude 2-chloro-5-hydrazinyl-4-methoxybenzoic acid hydrochloride (4.00 g, 64.3 %) as a solid. This was used without purification in the next step. The filtrate was concentrated to approx. 100mL and then filtered to give a second crop (approximately 7.50 g). Material used crude in the next step.

### Intermediate 94 :methyl 2-chloro-5-hydrazinyl-4-methoxybenzoate hydrochloride

To ice-cooled methanol (500 mL) was added (cautiously) acetyl chloride (100 mL, 1.40 mol) . To the resultant solution was added crude 2-chloro-5-hydrazinyl-4-methoxybenzoic acid hydrochloride (4.00 g, 15.8 mmol). The reaction mixture was heated to 50 °C for 2 h . The reaction mixture was evaporated to give methyl 2-chloro-5-hydrazinyl-4-methoxybenzoate hydrochloride (4.22 g, 100 %) as a solid.

### Intermediate 95 :methyl 2-chloro-4-methoxy-5-(1H-pyrazol-1-yl)benzoate

To a suspension of the crude methyl 2-chloro-5-hydrazinyl-4-methoxybenzoate hydrochloride (4.22 g, 15.8 mmol) in ethanol (100 mL) was added malonaldehyde bis(dimethyl acetal) (4.05 mL, 24.6 mmol). The reaction mixture was heated to 80 °C for 2 h. The reaction mixture was allowed to cool and evaporated. The crude product was purified by flash silica chromatography, elution gradient 0 to 40% isohexane in EtOAc. Pure fractions were evaporated to dryness to afford methyl 2-chloro-4-methoxy-5-(1H-pyrazol-1-yl)benzoate (1.500 g, 35.6 %) as a yellow oil. m/z (ESI+) (M+H)+ = 267; HPLC tR = 2.13 min. ¹H NMR (400.13 MHz, DMSO-d6) δ 3.84 (3H, s), 3.99 (3H, s), 6.51 - 6.52 (1H, m), 7.45 (1H, s), 7.74 (1H, d), 8.19 (1H, s), 8.24 - 8.25 (1H, m).

### Intermediate 96 :2-chloro-4-methoxy-5-(1H-pyrazol-1-yl)benzamide

Methyl 2-chloro-4-methoxy-5-(1H-pyrazol-1-yl)benzoate (1.50 g, 5.62 mmol) was suspended in 37% aqueous ammonia (100 mL, 5.62 mmol) and methanol (80 mL) and stirred at room temperature overnight. The reaction hadn't gone to completion so the reaction mixture was evaporated and fresh ammonia (100 mL, 5.62 mmol) and methanol (80 mL) were added. The reaction mixture was stirred at room temperature overnight and then evaporated. The crude product was stirred with water (50 mL) and NaHCO₃ (sat aq) (50 mL). The resulting solid was filtered off, washed with water (50 mL) and then ether (50 mL) and then left to dry overnight to give 2-chloro-4-methoxy-5-(1H-pyrazol-1-yl)benzamide (0.672 g, 47.5 %) as a white solid. m/z (ESI+) (M+H)+ = 252; HPLC tR = 1.30 min. ¹H NMR (400.13 MHz, DMSO-d6) δ 3.93 (3H, s), 6.50 (1H, t), 7.36 (1H, s), 7.57 (1H, s), 7.73 - 7.74 (2H, m), 7.88 (1H, s), 8.21 (1H, d).

### Intermediate 98 :tert-butyl 4-(3-carbamoyl-4-chlorophenoxy)piperidine-1-carboxylate

Potassium carbonate (4.83 g, 35.0 mmol) was added to 2-chloro-5-hydroxybenzamide (1.50 g, 8.74 mmol) and tert-butyl 4-(methylsulfonyloxy)piperidine-1-carboxylate (4.88 g, 17.5 mmol) in DMF (40 mL) at 20°C. The resulting suspension was stirred at 110 °C for 16 hours. The reaction mixture was evaporated to dryness and redissolved in EtOAc (500 mL), and washed sequentially with water (200 mL) and saturated brine (200 mL). The organic layer was dried over MgSO₄, filtered and evaporated to afford crude product. The crude product was purified by flash silica chromatography, elution gradient 50 to 100% EtOAc in isohexane. Pure fractions were evaporated to dryness to afford tert-butyl 4-(3-carbamoyl-4-chlorophenoxy)piperidine-1-carboxylate (2.59 g, 83 %) as a white solid.
m/z (ESI+) (M-tBu)+ = 209, 301; HPLC tR = 2.15 min. ¹H NMR (400.132 MHz, DMSO) δ 1.39 (9H, s), 1.45 - 1.54 (2H, m), 1.85 - 1.91 (2H, m).

### Intermediate 100 : 2-chloro-N-(6-(methylsulfonyl)benzo[d]thiazol-2-ylcarbamoyl)-5-(piperidin-4-yloxy)benzamide

Acetyl chloride (0.195 mL, 2.74 mmol) was cautiously added dropwise to MeOH (2.00 mL) cooled to 0°C. The resulting solution was treated with tert-butyl 4-(4-chloro-3-(6-(methylsulfonyl)benzo[d]thiazol-2-ylcarbamoylcarbamoyl)phenoxy)piperidine-1-carboxylate (83.6 mg, 0.14 mmol) and stirred at 20 °C for 3 hours. Material used crude.

### Intermediate 106 :2-(2-aminobenzo[d]thiazol-6-ylthio)-2-methyl-1-(pyrrolidin-1-yl)propan-1-one

To a stirred solution of 2-(2-aminobenzo[d]thiazol-6-ylthio)-2-methylpropanoic acid (Intermediate 62, 1.34 g, 4.99 mmol), 1-(3-dimethylaminopropyl)-3-ethylcarbodiimide hydrochloride (1.20 g, 6.24 mmol) and 4-dimethylaminopyridine (0.610 g, 4.99 mmol) in *N,N*-dimethylformamide (9.99 mL) under an atmosphere of nitrogen at ambient temperature was added pyrrolidine (0.459 mL, 5.49 mmol). When the addition was completed, the mixture was stirred at ambient temperature for 6 hours, poured onto water (150mL) and extracted with ethyl acetate (3 x 50 mL). The combined ethyl acetate extracts were washed with brine, dried (MgSO₄) and evaporated in vacuo to a residue which was chromatographed on silica with ethyl acetate as eluant to give a solid which was triturated with diethyl ether to give 2-(2-aminobenzo[d]thiazol-6-ylthio)-2-methyl-1-(pyrrolidin-1-yl)propan-1-one (0.700 g, 43%).
¹H NMR (CDCl₃): δ 1.5 (s, 6H), 1.75 - 1.95 (m, 4H), 3.4 - 3.5 (m, 2H), 3.9 - 4.0 (m, 2H), 5.4 (s, 2H), 7.2 (d, 1H), 7.35 (d, 1H) and 7.5 (s, 1H).

### Intermediate 109 :6-(2-methyl-1-(pyrrolidin-1-yl)propan-2-ylthio)benzo[d]thiazol-2-amine

To a stirred solution of 2-(2-aminobenzo[d]thiazol-6-ylthio)-2-methyl-1-(pyrrolidin-1-yl)propan-1-one (643 mg, 2.00 mmol) in tetrahydrofuran (20mL) under an atmosphere of nitrogen at ambient temperature was added borane-tetrahydrofuran complex (10.0 mL, 10.0 mmol). When the addition was completed, the mixture was stirred at ambient temperature for 16 hours, treated dropwise with methanol (20 mL), evaporated in vacuo to a residue which was taken up in methanol (10 mL) and treated with 4M hydrogen chloride in dioxane (10 mL). The mixture was stirred at ambient temperature for 2 hours, evaporated in vacuo to a residue which was taken up in saturated sodium hydrogen carbonate solution (25 mL), extracted with ethyl acetate (3 x 25 mL), the combined ethyl acetate extracts washed with brine, dried (MgSO₄) and evaporated *in vacuo* to a residue which was chromatographed on silica with 10% methanol in dichloromethane as eluant to give 6-(2-methyl-1-(pyrrolidin-1-yl)propan-2-ylthio)benzo[d]thiazol-2-amine (340 mg, 55.3 %). ¹H NMR (CDCl₃): δ 1.2 (s, 6H), 1.7 (dt, 4H), 2.5 (s, 2H), 2.6 (dt, 4H), 5.3 (s, 2H), 7.2 (d, 1H), 7.3 (d, 1H) and 7.5 (s, 1H).

### Intermediate 110 : 6-(2-methyl-1-(pyrrolidin-1-yl)propan-2-ylsulfonyl)benzo[d]thiazol-2-amine

To a stirred solution of 6-(2-methyl-1-(pyrrolidin-1-yl)propan-2-ylthio)benzo[d]thiazol-2-amine (154 mg, 0.50 mmol) in methanol (5.00 mL) was added hydrochloric acid (1.50 mL, 1.50 mmol) followed by a solution of sodium tungstate dihydrate (3.30 mg, 10.02 µmol) in water (0.11 ml). The mixture was heated to 55 °C and treated with hydrogen peroxide (0.146 ml, 1.65 mmol). When the addition was completed, the mixture was heated at 55 °C for 30 minutes, cooled to ambient temperature, treated with saturated sodium hydrogen carbonate solution (5 mL), the methanol evaporated *in vacuo* and the aqueous residue extracted with ethyl acetate (3 x 10mL). The combined ethyl acetate extracts were dried (MgSO₄) and evaporated *in vacuo* to a residue which was triturated with ethyl acetate to give 6-(2-methyl-1-(pyrrolidin-1-yl)propan-2-ylsulfonyl)benzo[d]thiazol-2-amine (106 mg, 62.3 %). ¹H NMR (DMSO d6): δ 1.2 (s, 6H), 1.6 (dt, 4H), 2.5 (dt, 4H), 2.7 (s, 2H), 7.45 (d, 1H), 7.6 (d, 1H), 7.95 (s, 2H) and 8.15 (s, 1H).

### Intermediate 112 :methyl 2-chloro-5-(3-nitropyridin-2-yl)benzoate

2-Bromo-3-nitropyridine (1.25 g, 6.16 mmol), 4-chloro-3-(methoxycarbonyl)phenylboronic acid (1.32 g, 6.16 mmol), tetrakis(triphenylphosphine)palladium(0) (0.36 g, 0.310 mmol) and sodium carbonate (0.653 g, 6.16 mmol) were suspended in toluene (15 mL) and ethanol (15.0 mL) and sealed into a microwave tube. The reaction was heated to 100 °C for 5 hours in the microwave reactor and cooled to RT. The reaction was incomplete and further 2-bromo-3-nitropyridine (0.625 g, 3.08 mmol) was added and the mixture was heated to 100 °C for a further 1 hour. The reaction mixture was filtered through celite, rinsed through with ethyl acetate (25 mL) and washed with water (1 x 50 mL) then brine (1 x 50 mL). The organic layer was dried over MgSO₄, filtered and evaporated to afford crude product. The crude product was purified by flash silica chromatography, elution gradient 10 to 50% EtOAc in isohexane. Pure fractions were evaporated to dryness to afford methyl 2-chloro-5-(3-nitropyridin-2-yl)benzoate (1.100 g, 61.0 %) as a yellow solid.
m/z (ESI+) (M+H)+ = 293.25; HPLC tR = 2.31 min. ¹H NMR (400 MHz, CDCl₃) δ 3.94 (3H, s), 7.48 - 7.52 (1H, m), 7.54 - 7.56 (1H, m), 7.57 - 7.60 (1H, m), 8.10 - 8.11 (1H, m), 8.21 - 8.23 (1H, m), 8.87 - 8.89 (1H, m).

### Intermediate 113 :methyl 5-(3-aminopyridin-2-yl)-2-chlorobenzoate

Methyl 2-chloro-5-(3-nitropyridin-2-yl)benzoate (0.900 g, 3.08 mmol) and palladium, 10% on charcoal (0.1 g, 0.94 mmol) in ethyl acetate (40 mL) were stirred under an atmosphere of hydrogen at atmospheric pressure and room temperature for 5 hours. The reaction was incomplete and further palladium, 10% on charcoal (0.100 g, 0.940 mmol) was added and the mixture was stirred at room temperature for a further 1 hour. The reaction mixture was filtered through celite and washed with ethyl acetate. The solvent was removed in vacuo to yield methyl 5-(3-aminopyridin-2-yl)-2-chlorobenzoate (0.60 g, 74.3 %) as a light brown oil that solidified on standing, which was used without purification. m/z (ESI+) (M+H)+ = 263.28; HPLC tR = 0.83 min. ¹H NMR (400.13 MHz, CDCl₃) δ 3.80 (2H, s), 3.93 (3H, s), 7.06 - 7.12 (2H, m), 7.56 (1H, d), 7.78 - 7.80 (1H, m), 8.14 - 8.15(1H,m),8.20(1H,d).

### Intermediate 114 :methyl 2-chloro-5-(3-(dimethylamino)pyridin-2-yl)benzoate

Formaldehyde (0.685 mL, 9.14 mmol) was added to methyl 5-(3-aminopyridin-2-yl)-2-chlorobenzoate (0.800 g, 3.05 mmol) in formic acid (20 mL). The resulting solution was stirred at 100 °C for 16 hours. The reaction mixture was allowed to cool to room temperature then basified with saturated NaHCO₃. This was partitioned with ethyl acetate (2 x 50 mL) and the organic layer dried with MgSO₄. The solvent was removed in vacuo to yield crude product. The crude product was purified by flash silica chromatography, elution gradient 0 to 30% EtOAc in isohexane. Pure fractions were evaporated to dryness to afford methyl 2-chloro-5-(3-(dimethylamino)pyridin-2-yl)benzoate (0.428 g, 48%) as a pale yellow oil. m/z (ESI+) (M+3H)+ = 293.24; HPLC tR = 1.46 min. ¹H NMR (400 MHz, CDCl₃) δ 2.61 (6H, s), 3.94 (3H, s), 7.16 - 7.20 (1H, m), 7.34 - 7.36 (1H, m), 7.49 (1H, d), 8.03 - 8.06 (1H, m), 8.28 - 8.30 (1H, m), 8.41 (1H, d).

### Intermediate 115 :2-chloro-5-(3-(dimethylamino)pyridin-2-yl)benzamide

35% Ammonia in methanol (60 mL, 1.08 mol) was added to methyl 2-chloro-5-(3-(dimethylamino)pyridin-2-yl)benzoate (428 mg, 1.47 mmol) in methanol (30 mL). The resulting solution was stirred at room temperature over the weekend. The methanol was removed *in vacuo,* resulting in a white precipitate. The flask was cooled in ice water and the precipitate collected by vacuum filtration, washed with water and dried under vacuum to afford 2-chloro-5-(3-(dimethylamino)pyridin-2-yl)benzamide (300 mg, 73.9 %) as a white crystalline solid, which was used without further purification. m/z (ESI+) (M+H)+ = 276.24; HPLC tR = 0.85 min. ¹H(400 MHz, CDCl₃) δ 1.67 (2H, s), 2.61 (6H, s), 7.16 - 7.20 (1H, m), 7.34 - 7.36 (1H, m), 7.46 (1H d, J= 8.4 Hz), 7.98 - 8.00 (1H, m), 8.26 - 8.27 (1H, m), 8.31 (1H d, J= 2.2 Hz)

### Intermediate 116 :(S)-tert-butyl 3-(methylsulfonyloxy)pyrrolidine-1-carboxylate

To a stirred solution of (S)-tert-butyl 3-hydroxypyrrolidine-1-carboxylate (4.38 g, 23.4 mmol) and N-ethyldiisopropylamine (6.11 mL, 35.1 mmol) in dichloromethane (94 mL) at 0 °C was added methanesulfonyl chloride (2.173 mL, 28.1 mmol). The mixture stirred at ambient temperature for 16 hours, evaporated *in vacuo* to a residue which was taken up in ethyl acetate (125 mL), washed with water, citric acid solution, brine, dried (MgSO₄) and evaporated in vacuo to a residue which was chromatographed on silica with 50% ethyl acetate in isohexane as eluant to give (S)-tert-butyl 3-(methylsulfonyloxy)pyrrolidine-1-carboxylate (4.92 g, 79 %). ¹H NMR (CDCl₃) δ: 1.4 (s, 9H), 2.0 - 2.2 (m, 2H), 3.0 (s, 3H), 3.4 - 3.6 (m, 4H) and 5.2 (dt, 1H).

### Intermediate 117 :(S)-tert-butyl 3-(3-carbamoyl-4-chlorophenoxy)pyrrolidine-1-carboxylate

To a stirred solution of 2-chloro-5-hydroxybenzamide (343 mg, 2.00 mmol) and (S)-tert-butyl 3-(methylsulfonyloxy)pyrrolidine-1-carboxylate (557 mg, 2.10 mmol) in acetonitrile (10.0 mL) in a 20 mL microwave vial was added cesium carbonate (1.95 g, 6.00 mmol). The mixture was heated at 120 °C in microwave for 1 hour, cooled to ambient temperature and pressure, evaporated *in vacuo* to a residue which was taken up in ethyl acetate (25 mL), the ethyl acetate layer was washed with brine, dried (MgSO₄) and evaporated in vacuo to a residue which was chromatographed on silica with ethyl acetate as eluant to give (S)-tert-butyl 3-(3-carbamoyl-4-chlorophenoxy)pyrrolidine-1-carboxylate (510 mg, 74.9 %). ¹H NMR (CDCl₃): 1.4 (s, 9H), 2.0 - 2.1 (m, 2H), 3.4 - 3.6 (m, 4H), 4.8 (br s, 1H), 5.9 (s, 1H), 6.4 (s, 1H), 6.8 (d, 1H) and 7.2 - 7.3 (m, 2H).

### Intermediate 118 :6-bromo-N,N-dimethylpyridin-2-amine

Split into 2 microwave vials and done in parallel dimethylamine (33% soln in ethanol) (2.73 mL, 15.2 mmol), 2,6-dibromopyridine (1.80 g, 7.60 mmol) were dissolved in acetonitrile (30 mL) and sealed into a microwave tube. The reaction was heated to 130 °C for 1 hour in the microwave reactor and cooled to RT. The reaction was not complete hence a further quantity of dimethylamine (33% soln in ethanol) (0.91 mL, 5.06 mmol) was added and the mixture heated for a further 30 mins at 130 °C. The reaction mixture was evaporated to dryness and redissolved in DCM (50 mL), and washed sequentially with saturated NaHCO₃ (50 mL) and water (50 mL). The organic layer was dried by passing through a phase separating cartridge and evaporated to afford desired product. 6-bromo-N,N-dimethylpyridin-2-amine (1.54 g, 100 %). m/z (ESI+) (M+H)+ =201.22 + 203.18 (equal heights) ; HPLC tR = 2.40 min.¹H NMR (400.13 MHz, CDCl₃) δ 1.54 (1H, s), 3.06 (6H, s), 6.36 - 6.38 (1H, m), 6.66 (1H, d), 7.22 - 7.26 (1H, m).

### Intermediate 118b: methyl 2-chloro-5-(6-(dimethylamino)pyridin-2-yl)benzoate

6-Bromo-*N,N-*dimethylpyridin-2-amine (0.938 g, 4.66 mmol), 4-chloro-3-(methoxycarbonyl)phenylboronic acid (1.00 g, 4.66 mmol), tetrakis(triphenylphosphine)-palladium(0) (0.269 g, 0.230 mmol) and sodium carbonate (0.989 g, 9.33 mmol) were suspended in toluene (1.5 mL) and ethanol (1.5 mL) and sealed into a microwave tube. The reaction was heated to 100 °C for 5 hours in the microwave reactor and cooled to RT. The reaction mixture was evaporated to dryness and redissolved in DCM (25 mL), and washed with water (25 mL). The organic layer was dried by passing through a phase seperating cartridge and evaporated to afford crude product. The crude product was purified by flash silica chromatography (gradient 0 to 12.5% EtOAc in isohexane). Pure fractions were evaporated to dryness to afford methyl 2-chloro-5-(6-(dimethylamino)pyridin-2-yl)benzoate (0.900 g, 66.4 %) as a colourless gum. m/z no obvious mass ion = ; HPLC tR = 1.12 min. ¹H NMR (400.13 MHz, DMSO-d6) δ 3.09 (6H, s), 3.89 (3H, s), 6.67 (1H, d), 7.19 - 7.21 (1H, m), 7.58 - 7.65 (1H, m), 8.21 - 8.24 (1H, m), 8.44 (1H, d)

### Intermediate 119 :2-chloro-5-(6-(dimethylamino)pyridin-2-yl)benzamide

Ammonia (39.9 mL, 644 mmol) was added in one portion to methyl 2-chloro-5-(6-(dimethylamino)pyridin-2-yl)benzoate (0.88 g, 3.03 mmol) in MeOH (40 mL) at ambient temperature under air. The resulting mixture was stirred vigorously at ambient temperature for 3 days. (warmed to 50 °C for 8hrs in the last 24 hours) Removal of the solvent under reduced pressure gave crude material. Stirrred in water (25 mL) and filtered and dried (high vac). Gave 2-chloro-5-(6-(dimethylamino)pyridin-2-yl)benzamide (0.620 g, 74.3 %) as a colourless solid. m/z (ESI+) (M+H)+ = 276.24; HPLC tR = 1.20 min. ¹H NMR (400.13 MHz, DMSO-d6) δ 3.09 (6H, s), 6.65 (1H, d), 7.19 (1H, d), 7.53 (1H, t), 7.57 - 7.61 (2H, m), 7.93 (1H, s), 8.06 - 8.09 (2H, m).

### Intermediate 120 :2-chloro-5-hydroxybenzamide

To a stirred solution of methyl 2-chloro-5-hydroxybenzoate (18.7 g, 100 mmol) in methanol (200 mL) was added ammonia (339 mL, 5.01 mol) and the mixture heated at 50 °C for 3 days. The mixture was evaporated in vacuo to a residue which was taken up in ethyl acetate (500 mL), washed with 2M citric acid solution, saturated sodium hydrogen carbonate solution, brine, dried (MgSO₄) and evaporated in vacuo to a residue which was crystallised from ethyl acetate in isohexane to give 2-chloro-5-hydroxybenzamide (13.8 g, 80 %). Material used crude.

### Intermediate 121 : 6-(piperidin-4-ylsulfonyl)benzo[d]thiazol-2-amine

Acetyl chloride (74 mL, 1.04 mol) was added to tert-butyl 4-(2-aminobenzo[d]thiazol-6-ylsulfonyl)piperidine-1-carboxylate (15.5 g, 39.0 mmol) in methanol (1L). The resulting solution was stirred at 50 °C for 1 hour. The reaction mixture was evaporated to dryness, and triturated with ether to give a cream solid of the HCl salt which was filtered off and dried. m/z (ESI+) (M+H)+ = 298.12; HPLC tR = 0.56 min

### Intermediate 122 : 6-(1-methylpiperidin-4-ylsulfonyl)benzo[d]thiazol-2-amine

A solution of formaldehyde (10.8 mL, 145 mmol) in methanol (30 mL) was added dropwise to a stirred solution of 6-(piperidin-4-ylsulfonyl)benzo[d]thiazol-2-amine (HCl salt) (20.2 g, 60.5 mmol), sodium cyanoborohydride (7.60 g, 121 mmol) in methanol (950 mL) and acetic acid (30 mL) at 20 °C, over a period of 10 minutes under nitrogen. The reaction mixture was stirred at room temperature for 60 mins and was then neutralised with saturated NaHCO₃ (approx 150 mL). The mixture was extracted with ethyl acetate (2 x 500 mL), the organics were combined, dried over magnesium sulphate, filtered and evaporated to dryness yielding 6-(1-methylpiperidin-4-ylsulfonyl)benzo[d]thiazol-2-amine (12.1 g, 64 %) as a pale yellow solid. m/z (ESI+) (M+H)+ = 312.14; HPLC tR = 0.64min.

### Intermediate 123 : 4-fluorophenyl 6-(1-methylpiperidin-4-ylsulfonyl)benzo[d]thiazol-2-ylcarbamate

4-Fluorophenyl chloroformate (4.94 mL, 37.6 mmol) was added dropwise to 6-(1-methylpiperidin-4-ylsulfonyl)benzo[d]thiazol-2-amine (10.6 g, 34.2 mmol), and pyridine (5.53 ml, 68.40 mmol) in DMF (100 mL) under air. The resulting solution was stirred at ambient temperature overnight. The reaction was evaporated in vacuo and the reaction mixture was diluted with EtOAc (300 mL) and THF (300 mL), and washed sequentially with water (200 mL). The organic layer was dried over MgSO₄, filtered and evaporated to afford crude product as a white solid (1.2 g). Further material could be extracted by concentration the aqueous layer until a white solid precipitated, this was filtered, overnight and dried to 4-fluorophenyl 6-(1-methylpiperidin-4-ylsulfonyl)benzo[d]thiazol-2-ylcarbmate (4.00g, total yield 5.20 g, 34%). Material used without further purification.
m/z (ESI+) (M+H)+ = 450.14 ; HPLC tR = 1.52 min

### Intermediate 124 :methyl 2-chloro-4-fluoro-5-nitrobenzoate

A solution of trimethylsilyldiazomethane 2M solution in ether (14.2 mL, 28.4 mmol) was added dropwise to a stirred solution of 2-chloro-4-fluoro-5-nitrobenzoic acid (4.80 g, 21.9 mmol) in toluene (70 mL) and methanol (35.0 mL) over a period of 15 minutes. The resulting solution was stirred at ambient temperature for 30 minutes. The reaction mixture was quenched with acetic acid (3.75 mL, 65.6 mmol) and stirred at ambient temperature for 10 minutes. The reaction mixture was evaporated to afford methyl 2-chloro-4-fluoro-5-nitrobenzoate (5.11 g, 100 %) which was used without further purification.

### Intermediate 125 :2-chloro-4-fluoro-5-hydrazinylbenzoic acid hydrochloride

To a suspension of 5-amino-2-chloro-4-fluorobenzoic acid (5.12 g, 27.0 mmol) in hydrochloric acid, 37% (50 mL) cooled to 0 °C was added a cold solution of sodium nitrite (2.00 g, 29.1 mmol) in water (25.0 mL) dropwise maintaining an internal temperature below 5 °C. The resultant solution was stirred in an ice-bath for 30 min before dropwise addition of a solution of tin(II) chloride (13.8 g, 72.8 mmol) in hydrochloric acid, 37% (20 mL) maintaining the temperature below 5°C. The resultant suspension was stirred at 0°C for 3 hours. The solid was filtered off, washed with cold water (50 mL) and dried under high vac. The crude 2-chloro-4-fluoro-5-hydrazinylbenzoic acid hydrochloride (3.94 g, 60 %) was used without further purification. m/z (ESI-) (M-H)- = 203; HPLC tR = 0.40 min.

### Intermediate 126 :methyl 2-chloro-4-fluoro-5-hydrazinylbenzoate hydrochloride

Acetyl chloride (8.72 mL, 122 mmol) was added portionwise to methanol (150 mL) at 0 °C. The resulting solution was stirred at 0 °C for 30 minutes before the addition of 2-chloro-4-fluoro-5-hydrazinylbenzoic acid hydrochloride (3.94 g, 16.4 mmol). The resulting reaction was warmed to 50 °C and stirred at this temperature for 4 hours. The solvent was evaporated in vacuo to yield crude methyl 2-chloro-4-fluoro-5-hydrazinylbenzoate hydrochloride (4.17 g, 100 %) which was used without further purification. m/z (ESI+) (M+H)+ = 219; HPLC tR = 1.06 min.

### Intermediate 128 :methyl 2-chloro-4-fluoro-5-(1H-pyrazol-1-yl)benzoate

Malonaldehyde bis(dimethyl acetal) (2.69 mL, 16.4 mmol) was added to methyl 2-chloro-4-fluoro-5-hydrazinylbenzoate hydrochloride (4.17 g, 16.4 mmol) in ethanol (80 mL) at ambient temperature. The resulting solution was stirred at 80 °C for 2 hours. The solvent was removed in vacuo to yield crude product. The crude product was purified by flash silica chromatography, elution gradient 10 to 100% EtOAc in isohexane. Pure fractions were evaporated to dryness to afford ethyl 2-chloro-4-fluoro-5-(1H-pyrazol-1-yl)benzoate (0.057 g, 1.3 %) as a white solid, methyl 2-chloro-4-fluoro-5-(1H-pyrazol-1-yl)benzoate (2.28 g, 55 %) as a white solid and 2-chloro-4-fluoro-5-(1H-pyrazol-1-yl)benzoic acid (0.025 g, 0.6 %) as a yellow solid. m/z (ESI+) (M+H)+ = 269; HPLC tR = 2.47 min.
¹H NMR (400.13 MHz, DMSO-d6) δ 1.33 (3H, t), 4.35 (2H, q), 6.61 - 6.62 (1H, m), 7.85 - 7.85 (1H, m), 7.90 (1H, d), 8.26 - 8.29 (2H, m).

### Intermediate 129 :2-chloro-4-fluoro-5-(1H-pyrazol-1-yl)benzamide

A solution of methyl 2-chloro-4-fluoro-5-(1H-pyrazol-1-yl)benzoate (2.19 g, 8.60 mmol) in ammonia (28% w/w solution) (100 mL, 8.60 mmol) / MeOH (20 mL) was stirred at 60 °C for 4 hours. The reaction was cooled to ambient temperature and the resulting solid was filtered off and dried to yield 2-chloro-4-fluoro-5-(1H-pyrazol-1-yl)benzamide (1.52 g, 74 %) as a white solid. m/z (ESI+) (M+H+CH3CN)+ = 281; HPLC tR = 1.37 min. ¹H NMR (400.13 MHz, DMSO-d6) δ 6.59 - 6.60 (1H, m), 7.69 (1H, s), 7.79 (1H, d), 7.84 (1H, d), 7.88 (1H, d), 7.99 (1H, s), 8.25 (1H, t).

### Intermediate 130 :methyl 2-chloro-4-methoxy-5-nitrobenzoate

Sodium methoxide (0.5M in MeOH) (48.1 mL, 24.0 mmol) was added to a solution of methyl 2-chloro-4-fluoro-5-nitrobenzoate (5.11 g, 21.9 mmol) in methanol (150 mL) at ambient temperature. The resulting solution was stirred at ambient temperature for 2 minutes during which time a precipitate formed. The reaction mixture was diluted with Et₂O (400 mL), and washed sequentially with water (100 mL) and saturated brine (100 mL). The organic layer contained a precipitate which was filtered off and dried to yield product. The filtrate was evaporated to yield further product. These two batches were identical by TLC so were combined to yield methyl 2-chloro-4-methoxy-5-nitrobenzoate (5.37 g, 100 %) as a pale yellow solid. Material used crude in the next step.

### Intermediate 131 :methyl 5-amino-2-chloro-4-methoxybenzoate

Tin(II) chloride (5.23 mL, 108.91 mmol) was added to a suspension of methyl 2-chloro-4-methoxy-5-nitrobenzoate (5.35 g, 21.78 mmol) in methanol (200 mL) at ambient temperature under nitrogen. The resulting suspension was stirred at 65 °C for 90 minutes. The suspension dissolves to a yellow solution during this time. The reaction was evaporated *in vacuo* and the residue was dissolved in water (300mL) then cautiously treated with sat NaHCO₃ solution until ∼pH9. The resulting suspension was extracted with DCM (3 x 250 mL). The DCM layers were combined, washed with brine (100 mL) then dried (MgSO₄), filtered and evaporated to crude product. The crude product was purified by flash silica chromatography, elution gradient 20 to 60% EtOAc in isohexane. Pure fractions were evaporated to dryness to afford methyl 5-amino-2-chloro-4-methoxybenzoate (2.68 g, 57.1 %) as a yellow solid. m/z (ESI+) (M+H)+ = 216; HPLC tR = 1.64 min. ¹H NMR (400.13 MHz, DMSO-d6) δ 3.77 (3H, s), 3.83 (3H, s), 5.08 (2H, s), 6.89 (1H, s), 7.15 (1H, s).

### Intermediate 132 :methyl 2-chloro-4-methoxy-5-morpholinobenzoate

Bis(2-bromoethyl) ether (0.26 mL, 2.09 mmol), methyl 5-amino-2-chloro-4-methoxybenzoate (300 mg, 1.39 mmol) and potassium carbonate (577 mg, 4.17 mmol) were suspended in DMA (4 mL) and sealed into a microwave tube. The reaction was heated to 140 °C for 2 hours in the microwave reactor and cooled to RT. The reaction was scaled up and repeated in 2 twice further using methyl 5-amino-2-chloro-4-methoxybenzoate (1.14 g, 1.39 mmol). All three reactions were combined for workup. The reaction mixture was evaporated to dryness and redissolved in EtOAc (150 mL), and washed sequentially with water (50 mL) and saturated brine (50 mL). The organic layer was dried over MgSO₄, filtered and evaporated to afford crude product. The crude product was purified by flash silica chromatography, elution gradient 0.5 to 1% 7M ammonia in MeOH in DCM. Pure fractions were evaporated to dryness to afford methyl 2-chloro-4-methoxy-5-morpholinobenzoate (2.06 g, 60%) as a yellow solid. m/z (ESI+) (M+H)+ = 286; HPLC tR = 1.96 min. ¹H NMR (400.13 MHz, CDCl₃) δ 3.04 - 3.07 (4H, m), 3.86 - 3.91 (10H, m), 6.89 (1H, s), 7.44 (1H, s).

### Intermediate 133 : 2-chloro-4-methoxy-5-morpholinobenzamide

A solution of methyl 2-chloro-4-methoxy-5-morpholinobenzoate (1.97 g, 6.89 mmol) in ammonia (7N in MeOH) (50 mL, 2287.07 mmol) and ammonia (28% w/w solution) (150 mL, 6.89 mmol) was stirred at 75 °C for 48 hours. The solvent was removed in vacuo and solid was dissolved in ammonia (7N in MeOH) (50 mL, 2.28 mol) and ammonia (28% w/w solution) (150 mL, 6.89 mmol) and heated at 75 °C for a further 4 days. The reaction was cooled to ambient temperature and the resulting solid was filtered off and washed with ice cold MeOH to yield 2-chloro-4-methoxy-5-morpholinobenzamide (0.880 g, 47 %) as a white solid. The filtrate was acidified to ∼pH3 with 2M HCl and the resulting solid was filtered off and recrystallised from MeOH to yield 2-chloro-4-methoxy-5-morpholinobenzoic acid (0.480 g, 25.6 %) as a white solid.
m/z (ESI+) (M+H)+ = 272; HPLC tR = 1.44 min. ¹H NMR (400.13 MHz, DMSO-d6) δ 2.95 (4H, t), 3.71 (4H, t), 3.86 (3H, s), 7. 05 (1H, s), 7.32 (1H, s), 12.95 (1H, s)

### Intermediate 136 :tert-butyl 3-(methylsulfonyloxy)azetidine-1-carboxylate

To a stirred solution of *tert*-butyl 3-hydroxyazetidine-1-carboxylate (5.00 g, 28.9 mmol) and *N*-ethyldiisopropylamine (7.54 mL, 43.3 mmol) in dichloromethane (115 mL) at 0 °C was added methanesulfonyl chloride (2.68 mL, 34.6 mmol). The mixture stirred at ambient temperature for 16 hours, evaporated *in vacuo* to a residue which was taken up in ethyl acetate (125 mL), washed with water (50 mL), aqueous1M citric acid (50 mL) solution, brine (50 mL), dried (MgSO₄) and evaporated *in vacuo* to a residue which was chromatographed on silica with 50% ethyl acetate in *iso*hexane as eluant to give tert-butyl 3-(methylsulfonyloxy)azetidine-1-carboxylate (6.23 g, 86 %).
¹H NMR (CDCl₃): δ 1.4 (s, 9H), 3.05 (s, 3H), 4.1 (dd, 2H), 4.3 (dd, 2H) and 5.2 (dt, 1H).

### Intermediate 137 :tert-butyl 3-(3-carbamoyl-4-chlorophenoxy)azetidine-1-carboxylate

To a stirred solution of 2-chloro-5-hydroxybenzamide (2.05 g, 12.0 mmol) and tert-butyl 3-(methylsulfonyloxy)azetidine-1-carboxylate (3.30 g, 13.1 mmol) in acetonitrile (30.0 mL) and DMA (6.0 mL) in 2 x 20 mL microwave vials was added cesium carbonate (11.7 g, 35.8 mmol). The mixture was heated at 120° in the microwave for 1 hour, cooled to ambient temperature and pressure, evaporated in vacuo to a residue which was taken up in diethyl ether (125mL), washed with brine, dried (MgSO₄) and evaporated in vacuo to a residue which was chromatographed on silica with ethyl acetate as eluant, then on basic alumina with ethyl acetate as eluant to give tert-butyl 3-(3-carbamoyl-4-chlorophenoxy)-azetidine-1-carboxylate (0.380 g, 9.7 %).

### Intermediate 138 :2-chloro-5-ethoxy-4-methoxybenzoic acid

To a stirred solution of sodium hydroxide (1.20 g, 30.0 mmol) in water (20 mL) was added silver(I) oxide (1.16 g, 5.00 mmol), the mixture heated to 50 °C and treated with 2-chloro-5-ethoxy-4-methoxybenzaldehyde (1.07 g, 5.00 mmol). When the addition was completed, the mixture was heated at 60 °C for 1 hour, then cooled to ambient temperature and stirred for 16 hours. The mixture was filtered through celite, acidified to pH 1 with 2M hydrochloric acid, extracted with ethyl acetate (2 x 100 mL), the combined ethyl acetate extracts washed with brine, dried (MgSO₄) and evaporated in vacuo to a residue which was crystallised from ethyl acetate to give 2-chloro-5-ethoxy-4-methoxybenzoic acid (0.893 g, 77 %). ¹H NMR (DMSOd6): δ 1.3 (t,3H), 3.8 (s, 3H), 4.0 (q, 2H), 7.05 (s, 1H), 7.35 (s, 1H) and 13.0 (s, 1H).

### Intermediate 140 :2-chloro-5-ethoxy-4-methoxybenzamide

To a stirred solution of 2-chloro-5-ethoxy-4-methoxybenzoic acid (3.62 g, 15.7 mmol) in dichloromethane (62.8 mL) was added oxalyl chloride (1.64 mL, 18.8 mmol) and dry dimethylformamide (1 drop) and the mixture stirred at ambient temperature for 4 hours. The mixture was evaporated *in vacuo* to a residue which was taken up in dichloromethane (62.8 mL) and added to ammonia (17.1 mL, 784 mmol) at 0°C. When the addition was completed, the mixture was allowed to come to ambient temperature and stirred for 10 minutes. The precipitated solid was filtered off, washed with water and dried to give 2-chloro-5-ethoxy-4-methoxybenzamide (3.38 g, 94 %). ¹H NMR (DMSOd6): δ 1.3 (t,3H), 3.8 (s, 3H), 4.0 (q, 2H), 7.05 (d, 2H), 7.4 (s, 1H) and 7.65 (s, 1H).

### Intermediate 143 : 2,4-dichloro-5-nitrobenzoic acid

Nitric acid (2.66 mL, 62.8 mmol) was added dropwise to 2,4-dichlorobenzoic acid (10.0 g, 52.4 mmol) in sulfuric acid (50 mL) at 0 °C. The resulting mixture was stirred at room temperature for 30 minutes. The reaction mixture was poured into ice water and left to cool for 10 minutes, after which the precipitate was collected by filtration. The material was dried in the vacuum oven overnight, yielding crude 2,4-dichloro-5-nitrobenzoic acid (16.7 g, 135 %) as a cream solid. This material was used in the next reaction without any further purification.m/z (ES-) (M-H)- = 234; HPLC tR= 1.98 min.

### Intermediate 144 :methyl 2,4-dichloro-5-nitrobenzoate

Diazomethyltrimethylsilane (2M in ether) (31.8 mL, 63.6 mmol) was added portionwise to 2,4-dichloro-5-nitrobenzoic acid (10.0 g, 42.4 mmol) in methanol (30 mL) /toluene (90 mL) at ambient temperature over a period of 10 minutes under nitrogen. The resulting solution was stirred at ambient temperature for 20 minutes. Acetic acid was added to the reaction until effervescence stopped (∼5mL). The reaction was stirred at ambient temperature for a further 30 minutes then evaporated *in vacuo.* The resulting solid was recrystallised from EtOAc/isohexane to yield methyl 2,4-dichloro-5-nitrobenzoate (4.00 g, 38 %) as a yellow solid. m/z (EI+) M+ = 249; tR= 11.72 min. ¹H NMR (400.13 MHz, DMSO-d6) 3.90 (3H, s), 8.18 (1H, s), 8.54 (1H, s).

### Intermediate 146 :methyl 5-amino-2,4-dichlorobenzoate

Methyl 2,4-dichloro-5-nitrobenzoate (1.00 g, 4.00 mmol) and platinum (10% on carbon) (50.0 mg, 0.26 mmol) in methanol (30 mL) were stirred under an atmosphere of hydrogen at 1 atm and ambient temperature for 1 hour. The reaction mixture was filtered through celite and the solvent was evaporated to yield crude product. The crude product was purified by flash silica chromatography, elution gradient 10 to 40% EtOAc in isohexane. Pure fractions were evaporated to dryness to afford methyl 5-amino-2,4-dichlorobenzoate (0.800 g, 91 %) as a white solid. m/z (ES+) (M+MeCN+H)+ = 261; HPLC tR= 2.05 min. ¹H NMR (400.13 MHz, DMSO-d6) δ 3.81 (3H, s), 5.78 (2H, s), 7.24 (1H, s), 7.42 (1H, s).

### Intermediate 151 :methyl 2,4-dichloro-5-hydrazinylbenzoate

To a suspension of methyl 5-amino-2,4-dichlorobenzoate (5.00 g, 22.7 mmol) in hydrochloric acid, 37% (50 mL) cooled to 0 °C was added a solution of sodium nitrite (1.88 g, 27.3 mmol) in water (25.0 mL) dropwise maintaining an internal temperature below 5 °C. The resultant solution was stirred in an ice-bath for 30 min before dropwise addition of a solution of tin(II) chloride (12.9 g, 68.2 mmol) in hydrochloric acid, 37% (20 mL) maintaining the temperature below 5 °C. The resultant suspension was stirred at 0 °C for 3 hours. The solid was filtered off, washed with cold water (50 mL) and dried under high vac. The crude methyl 2,4-dichloro-5-hydrazinylbenzoate (5.40 g, 101 %) was used without further purification. m/z (ES+) (M+MeCN+H)+ = 276; HPLC tR= 1.40 min.
¹H NMR (400.13 MHz, DMSO-d6) δ 3.88 (3H, s), 7.53 (1H, s), 7.75 (1H, s), 8.43 (1H, s), 10.00 (2H, s).

### Intermediate 152 :2,4-dichloro-5-morpholinobenzamide

A suspension of methyl 2,4-dichloro-5-morpholinobenzoate (Intermediate 146, 691 mg, 2.38 mmol) in ammonia (28% w/w in water) (20 mL, 2.38 mmol) was treated with ammonia (7N in MeOH) (5.0 mL, 2.38 mmol). The resulting solution was stirred overnight at ambient temperature. The solvent was removed in vacuo to yield crude material. The crude product was purified by flash silica chromatography, elution gradient 10 to 100% EtOAc in isohexane. Pure fractions were evaporated to dryness to afford 2,4-dichloro-5-morpholinobenzamide (282 mg, 43.0 %) as a white solid. m/z (ES+) (M+H)+ = 275; HPLC tR= 1.55 min. ¹H NMR (400.13 MHz, CDCl₃) δ 3.06 - 3.08 (4H, m), 3.85 - 3.90 (4H, m), 5.90 (1H, s), 6.49 (1H, s), 7.45 (1H, s), 7.55 (1H, s).

### Intermediate 153 :methyl 2,4-dichloro-5-(1H-pyrazol-1-yl)benzoate

1,1,3,3-tetramethoxypropane (3.78 mL, 23.0 mmol) was added to methyl 2,4-dichloro-5-hydrazinylbenzoate (5.40 g, 23.0 mmol) in MeOH (80 mL) at ambient temperature under nitrogen. The resulting solution was stirred at 65 °C for 2 hours. The solvent was evaporated to yield crude product. The crude product was purified by flash silica chromatography, elution gradient 5 to 20% EtOAc in isohexane. Pure fractions were evaporated to dryness to afford methyl 2,4-dichloro-5-(1H-pyrazol-1-yl)benzoate (2.67 g, 43 %) as a pale yellow oil which crystallised on standing. m/z (ES+) (M+H)+ = 271; HPLC tR= 2,.27 min. ¹H NMR (400.13 MHz, CDCl₃) δ 3.86 (3H, s), 6.42 - 6.44 (1H, m), 7.59 (1H, s), 7.70 (1H, d), 7.84 - 7.84 (1H, m), 8.08 (1H, s).

### Intermediate 154 :2,4-dichloro-5-(1H-pyrazol-1-yl)benzamide

A solution of methyl 2,4-dichloro-5-(1H-pyrazol-1-yl)benzoate (2.58 g, 9.52 mmol) in ammonia (28% w/w in water) (80 mL, 9.52 mmol) / ammonia (7N in MeOH) (20 mL, 140.0 mmol) was stirred at ambient temperature for 18 hours. The bulk of the MeOH was removed by evaporation in vacuo and the resulting solution was extracted with DCM (3 x 50 mL). The combined organic layers were washed with brine (50 mL), dried (MgSO₄), filtered and evaporated to yield 2,4-dichloro-5-(1H-pyrazol-1-yl)benzamide (2.130 g, 87 %) as a white solid. m/z (ES+) (M+H)+ = 257; HPLC tR= 1.75 min. ¹H NMR (400.13 MHz, DMSO-d6) δ 6.56 - 6.57 (1H, m), 7.80 (1H, d), 7.96 (1H, s), 8.00 (1H, s), 8.2.

### Intermediate 155 :2-chloro-4-methyl-5-nitrobenzoic acid

Nitric acid (1.48 mL, 35.2 mmol) was added dropwise to 2-chloro-4-methylbenzoic acid (5.00 g, 29.31 mmol) in sulfuric acid (25 mL) at 0 °C. The resulting mixture was stirred at room temperature for 30 minutes. The reaction mixture was poured into ice water and left to cool for 10 minutes, after which the precipitate was collected by filtration. The material was dried in the vacuum oven overnight, yielding crude product as a beige solid, which was a mixture of isomers and starting material. This was recrystallised from ethanol/water, yielding 2-chloro-4-methyl-5-nitrobenzoic acid (3.25 g, 51 %) as a beige crystalline solid. m/z (ES-) (M+H)+ = 214.24; HPLC tR= 1.75 min. ¹H NMR(400 MHz, CDCl₃) δ 2.67 (3H, s), 7.52 (1H, s), 8.70 (1H, s).

### Intermediate 156 :methyl 2-chloro-4-methyl-5-nitrobenzoate

Acetyl chloride (52.8 mL, 742.15 mmol) was cautiously added portionwise to MeOH (500 mL) at 0°C and allowed to stir for 5 minutes. 2-chloro-4-methyl-5-nitrobenzoic acid (3.20 g, 14.8 mmol) was added and the solution stirred at 50 °C for 4.5 hours. The solvent was removed in vacuo to leave methyl 2-chloro-4-methyl-5-nitrobenzoate (3.41 g, 100 %) as a yellow solid. m/z (ES+) (M-H-NO2) = 182.40; HPLC tR= 2.41 min.
¹H NMR (400 MHz, CDCl₃) δ 2.65 (3H, s), 3.97 (3H, s), 7.47 (1H, s), 8.54 (1H, s).

### Intermediate 157 :methyl 5-amino-2-chloro-4-methylbenzoate

Palladium, 10% on charcoal (0.310 g, 2.91 mmol) was added to methyl 2-chloro-4-methyl-5-nitrobenzoate (3.10g, 13.5 mmol). The resulting mixture was stirred at ambient temperature for 2.5 hours under an atmosphere of hydrogen. The reaction mixture was filtered through celite and the solvent removed *in vacuo* to yield crude methyl 5-amino-2-chloro-4-methylbenzoate (2.55 g, 95 %) as a yellow oil. m/z (ES+) (M+H)+ = 200.30; HPLC tR= 1.81 min. ¹H NMR (400.13 MHz, CDCl₃) δ 2.15 (3H, s), 3.89 (3H, s), 7.11 (1H, s), 7.15 (1H, s).

### Intermediate 158 :methyl 2-chloro-5-hydrazinyl-4-methylbenzoate

A cold solution of sodium nitrite (1.06 g, 15.3 mmol) in water was added dropwise to methyl 5-amino-2-chloro-4-methylbenzoate (2.55 g, 12.8 mmol) in hydrochloric acid, 37% (60 mL) at 0°C at a rate that maintained an internal temperature below 5°C. The resulting mixture was stirred at 0°C for 40 minutes. A solution of tin(II) chloride (7.27 g, 38.3 mmol) in hydrochloric acid (20 mL) was added portionwise, keeping the temperature below 5 °C. The reaction mixture was stirred at 3 °C for 3 hours. The solid was filtered and washed with cold water (10 mL) and dried on the filter, yielding crude methyl 2-chloro-5-hydrazinyl-4-methylbenzoate (2.00 g, 62 %) as a pale brown solid. m/z (ES-) (M-H-Cl)- = 249.07; HPLC tR= 0.97 min. ¹H NMR (400.13 MHz, DMSO-d6) δ 2.22 (3H, s), 3.85 (3H, s), 7.355 (1H, s), 7.374 (1H, s), 10.26 (3H, s).

### Intermediate 159 :methyl 2-chloro-4-methyl-5-(1H-pyrazol-1-yl)benzoate

1,1,3,3-tetramethoxypropane (1.31 mL, 7.96 mmol) was added to methyl 2-chloro-5-hydrazinyl-4-methylbenzoate (2.00 g, 7.96 mmol) in ethanol (50 mL). The resulting mixture was stirred at 80 °C for 2 hours. The mixture was allowed to cool and solvent removed *in vacuo.* The crude product was purified by flash silica chromatography, using an isocratic gradient of 1 :1 EtOAc : hexanes. Pure fractions were evaporated to dryness to afford methyl 2-chloro-4-methyl-5-(1H-pyrazol-1-yl)benzoate (1.04 g, 52 %) as a yellow oil that solidified on standing over the weekend. m/z (ES+) (M+H)+ = 251.28; HPLC tR= 2.26 min. ¹H NMR (400.13 MHz, CDCl₃) δ 2.29 (3H, s), 3.91 (3H, s), 6.46 (1H, t), 7.43 (1H, s), 7.62 (1H, d, J=2.56Hz), 7.73 (1H, d, J=1.80Hz), 7.87 (1H, s).

### Intermediate 160:2-chloro-4-methyl-5-(1H-pyrazol-1-yl)benzamide

Methyl 2-chloro-4-methyl-5-(1H-pyrazol-1-yl)benzoate (1.04 g, 4.15 mmol) was dissolved in 7N ammonia in methanol (50 mL, 4.15 mmol). The resulting solution was stirred at ambient temperature for 3 hours. The reaction was incomplete so the temperature was increased to 50 °C and the reaction mixture was stirred for a further 2 hours. The reaction was allowed to cool and 35% ammonia solution (50 mL) added and the mixture stirred at ambient temperature over night. The methanol was removed in vacuo and the residue diluted with ethyl acetate, which was washed with water and dried over MgSO₄. The solvent evaporated to leave a yellow gum, which was triturated with ether to give 2-chloro-4-methyl-5-(1H-pyrazol-1-yl)benzamide (0.800 g, 82 %) as a pale yellow solid.
¹H NMR (400.13 MHz, CDCl₃) δ 2.29 (3H, s), 6.17 (1H, s), 6.45 (1H, t, J=2.28Hz), 6.54 (1H, s), 7.39 (1H, s), 7.62 (1H, d, J=2.28Hz), 7.72 (1H, d, J=2.04Hz), 7.81 (1H, s).

### Intermediate 161 :methyl 2-chloro-4-ethoxy-5-nitrobenzoate

To a stirred solution of methyl 2-chloro-4-hydroxy-5-nitrobenzoate (5.00 g, 21.6 mmol) and Iodoethane (2.07 mL, 25.9 mmol) in butan-2-one (121 mL) was added potassium carbonate (8.95 g, 64.8 mmol) and the stirred mixture heated at 60° for 16 hours. The mixture was cooled to ambient temperature, filtered, the filtrates evaporated in vacuo to a residue which was taken up in water (50 mL), extracted with ethyl acetate (2 x 150mL), the combined ethyl acetate extracts washed with 1M citric acid solution, brine, dried (MgSO₄) and evaporated *in vacuo* to a residue which was chromatographed on alumina with ethyl acetate as eluant to give methyl 2-chloro-4-ethoxy-5-nitrobenzoate (2.72 g, 48 %).
¹H NMR (CDCl₃): δ 1.45 (t, 3H), 3.85 (s, 3H), 4.2 (q, 2H), 7.05 (s, 1H) and 8.4 (s, 1H).

### Intermediate 163 :methyl 5-amino-2-chloro-4-ethoxybenzoate

To a stirred solution of methyl 2-chloro-4-ethoxy-5-nitrobenzoate (5.35 g, 20.6 mmol) in methanol (275 mL) and tetrahydrofuran (137 mL) was added platinum (0.804 g, 0.21 mmol) and the mixture stirred at ambient temperature under an atmosphere of hydrogen gas for 5 hours. The mixture was filtered through Celite, washed with methanol (25 mL), the filtrates evaportaed *in vacuo* to a residue which was chromatographed on silica with 20% ethyl acetate in isohexane as eluant to give methyl 5-amino-2-chloro-4-ethoxybenzoate (3.88 g, 82 %). ¹H NMR (CDCl₃): δ 1.45 (t,3H), 3.85 (s, 2H), 3.9 (s, 3H), 4.1 (q, 2H), 6.8 (s, 1H) and 7.25 (s, 1H).

### Intermediate 165 :2-chloro-4-ethoxy-5-hydrazinylbenzoic acid

To a suspension of potassium 5-amino-2-chloro-4-ethoxybenzoate (5.09 g, 20.1 mmol) in hydrochloric acid, 37% (30 mL) cooled to 0 °C was added a solution of sodium nitrite (1.66 g, 24.0 mmol) in water (20.0 mL) dropwise maintaining an internal temperature below 5 °C. The resultant solution was stirred in an ice-bath for 30 min before dropwise addition of a solution of tin(II) chloride (11.1 g, 60.2 mmol) in hydrochloric acid, 37% (20 mL) maintaining the temperature below 5 °C. The resultant suspension was stirred at 0 °C for 3 hours. The solid was filtered off, washed with cold water (100 mL) and dried under high vac. The crude 2-chloro-4-ethoxy-5-hydrazinylbenzoic acid (4.06 g, 88 %) was used without further purification. m/z (ES-) (M-H)- = 229; HPLC tR= 0.73 min. ¹H NMR (400.13 MHz, DMSO-d6) δ 1.37 (3H, t), 4.18 (2H, q), 7.10 (1H, s), 7.53 (1H, s), 10.10 (3H, s), 12.70 (1H, br s).

### Intermediate 166 :methyl 2-chloro-4-ethoxy-5-hydrazinylbenzoate hydrochloride

Acetyl chloride (8.11 mL, 114 mmol) was added portionwise to methanol (150 mL) at 0 °C. The resulting solution was stirred at 0 °C for 30 minutes before the addition of 2-chloro-4-ethoxy-5-hydrazinylbenzoic acid hydrochloride (4.06 g, 15.2 mmol). The resulting reaction was warmed to 50 °C and stirred at this temperature for 4 hours. The solvent was evaporated *in vacuo* to yield crude methyl 2-chloro-4-ethoxy-5-hydrazinylbenzoate hydrochloride (3.80 g, 89 %) which was used without further purification. ¹H NMR (400.13 MHz, DMSO-d6) δ 1.37 (3H, t), 3.83 (3H, s), 4.19 (2H, q), 7.14 (1H, s), 7.53 (1H, s), 7.82 (1H, s), 10.10 (3H, s).

### Intermediate 167 :methyl 2-chloro-4-ethoxy-5-(1H-pyrazol-1-yl)benzoate

Malonaldehyde bis(dimethyl acetal) (2.23 mL, 13.5 mmol) was added to methyl 2-chloro-4-ethoxy-5-hydrazinylbenzoate hydrochloride (3.80 g, 13.5 mmol) in MeOH (60 mL) at ambient temperature under nitrogen. The resulting solution was stirred at 70 °C for 3 hours. The solvent was evaporated to yield crude product. The crude product was purified by flash silica chromatography, elution with DCM. Pure fractions were evaporated to dryness to afford methyl 2-chloro-4-ethoxy-5-(1H-pyrazol-1-yl)benzoate (2.60 g, 68 %) as a yellow solid. m/z (ES+) (M+H)+ = 281; HPLC tR= 2.40 min. ¹H NMR (400.13 MHz, DMSO-d6) δ 1.37 (3H, t), 3.84 (3H, s), 4.28 (2H, q), 6.52 - 6.53 (1H, m), 7.42 (1H, s), 7.74 - 7.74 (1H, m), 8.22 (1H, s), 8.29 (1H, d).

### Intermediate 168 :2-chloro-4-ethoxy-5-(1H-pyrazol-1-yl)benzamide

A solution of methyl 2-chloro-4-ethoxy-5-(1H-pyrazol-1-yl)benzoate (2.56 g, 9.12 mmol) in ammonia (28% w/w in water) (300 mL, 9.12 mmol) / ammonia (7N in MeOH) (50 mL, 350 mmol) was stirred at ambient temperature for 48 hours. The bulk of the MeOH was removed under reduced pressure and the resulting solution was partitioned between DCM (250 mL) and water (100 mL). The layers were separated and the aqueous layer was re-extracted with DCM (2 x 50 mL). The combined organic layers were washed with brine (50mL), then dried (MgSO₄), filtered and evaporated to yield 2-chloro-4-ethoxy-5-(1H-pyrazol-1-yl)benzamide (1.82 g, 75 %) as a pale yellow solid. m/z (ES+) (M+H)+ = 266; HPLC tR= 1.54 min. ¹H NMR (400.13 MHz, DMSO-d6) δ 1.35 (3H, t), 4.22 (2H, q), 6.50 - 6.51 (1H, m), 7.34 (1H, s), 7.52 (1H, s), 7.73 (1H, d), 7.77 (1H, s), 7.83 (1H, s), 8.25 (1H, d).

### Intermediate 169: methyl 2-chloro-5-hydrazinyl-4-methoxybenzoate

This compound was prepared from methyl 5-amino-2-chloro-4-methoxybenzoate by adapting the method described in Intermediate 158.

### Intermediate 170 :methyl 2-chloro-4-methoxy-5-(1H-pyrazol-1-yl)benzoate

To a suspension of the crude methyl 2-chloro-5-hydrazinyl-4-methoxybenzoate hydrochloride (3.40 g, 12.7 mmol) in ethanol (100 mL) was added malonaldehyde bis(dimethyl acetal) (4.05 mL, 24.6 mmol). The reaction mixture was heated to 80 °C for 2 h. The reaction mixture was allowed to cool, stood overnight and evaporated. The crude product was purified by flash silica chromatography, elution gradient 0 to 40% isohexane in EtOAc. Pure fractions were evaporated to dryness to afford methyl 2-chloro-4-methoxy-5-(1H-pyrazol-1-yl)benzoate (1.95 g, 57 %) as a yellow oil which solidified on standing to a pale yellow solid. ¹H NMR (400.13 MHz, CDCl₃) δ 3.90 (3H, s), 3.96 (3H, s), 6.44 - 6.45 (1H, m), 7.11 (1H, s), 7.72 (1H, d), 8.03 (1H, d), 8.38 (1H, s).

### Intermediate 171 :2-chloro-4-methoxy-5-(1H-pyrazol-1-yl)benzamide

Methyl 2-chloro-4-methoxy-5-(1H-pyrazol-1-yl)benzoate (2.50 g, 9.37 mmol) was stirred in methanol (150 mL) and added to this was ammonia 33% in water (300 mL). The resulting mixture was stirred at ambient temperature for 50 hours. The reaction was evaporated *in vacuo* to remove the methanol, whereupon a white solid dropped out of solution. The solid was filtered off and air dried yielding 2-chloro-4-methoxy-5-(1H-pyrazol-1-yl)benzamide (0.990 g, 42.0 %) as a white solid. Used crude.

### Intermediate 172 :methyl 2-chloro-5-hydroxybenzoate

To a stirred mixture of 5-bromo-2-chlorobenzoic acid (481 mg, 2.04 mmol), potassium hydroxide (458 mg, 8.17 mmol), 2-Di-tert-butylphosphino-2',4',6'-triisopropylbiphenyl (69.4 mg, 0.16 mmol) and tris(dibenzylideneacetone)dipalladium(0) (37.4 mg, 0.04 mmol) under an atmosphere of nitrogen in a sealed microwave vial was added de-gassed water (1 mL) and 1,4-dioxane (1 mL). The stirred mixture was heated at 80 °C for 4 hours and cooled to ambient temperature. The mixture was treated with hydrochloric acid (8.17 mL, 8.17 mmol), extracted with ethyl acetate (3 x 20mL), the combined ethyl acetate extracts washed with brine, dried (MgSO₄) and evaporated in vacuo to a residue which was taken up in a mixture of diethyl ether (6 mL) and methanol (6 mL) and treated dropwise at 0 °C with (trimethylsilyl)diazomethane solution (1.28 mL, 2.55 mmol). When the addition was completed the mixture was allowed to come to ambient temperature and stirred for a further 10 minutes (N.B. reaction not gone to completion; a further 0.72 mL (trimethylsilyl)diazomethane added) then treated with acetic acid (0.351 ml, 6.13 mmol). The mixture was stirred at ambient temperature for 20 minutes, evaporated *in vacuo* to a residue which was taken up in ethyl acetate (25 ml), washed with saturated sodium hydrogen carbonate, brine, dried (MgSO₄) and evaporated in vacuo to a residue which was chromatographed on silica with 20% ethyl acetate in isohexane as eluant to give methyl 2-chloro-5-hydroxybenzoate (259 mg, 67.9 %).

### Intermediate 173 :(S)-tert-butyl 3-((methylsulfonyloxy)methyl)piperidine-1-carboxylate

To a stirred solution of (S)-tert-butyl 3-(hydroxymethyl)piperidine-1-carboxylate (3.90 g, 18.1 mmol) and N-ethyldiisopropylamine (4.73 mL, 27.1 mmol) in dichloromethane (72.5 mL) at 0 °C was added methanesulfonyl chloride (1.68 mL, 21.7 mmol). The mixture stirred at ambient temperature for 2 hours, evaporated in vacuo to a residue which was taken up in ethyl acetate (125 mL), washed with water, citric acid solution, brine, dried (MgSO₄) and evaporated *in vacuo* to a residue which was chromatographed on silica with 50% ethyl acetate in isohexane as eluant to give (S)-tert-butyl 3-((methylsulfonyloxy)methyl)piperidine-1-carboxylate (4.98 g, 94 %). ¹H NMR (CDCl₃): δ 1.2 - 1.3 (m, 3H), 1.4 (s, 3H), 1.6 (m, 1H), 1.75 (m, 1H), 1.9 (m, 1H), 2.7 (m, 1H), 2.85 (m, 1H), 2.95 (s, 3H), 3.7 (m, 1H), 3.9 (m,1H) and 4.05 (m, 2H).

### Intermediate 174a :(S)-tert-butyl 3-((3-carbamoyl-4-chlorophenoxy)methyl)piperidine-1-carboxylate

To a stirred solution of 2-chloro-5-hydroxybenzamide (2.46 g, 14.3 mmol) and (S)-tert-butyl 3-((methylsulfonyloxy)methyl)piperidine-1-carboxylate (4.21 g, 14.3 mmol) in DMA (39.8 mL) in 3 x 20 mL Microwave vial was added cesium carbonate (14.0 g, 43.0 mmol). The mixture was heated at 120° in the microwave for 2 hours, cooled to ambient temperature and pressure, poured onto water (600mL), and extracted with diethyl ether (3 x 200 mL). The combined diethyl ether extracts were washed with brine, dried (MgSO₄) and evaporated *in vacuo* to a residue which was chromatographed on silica with ethyl acetate as eluant to give a solid which was crystallised from ethyl acetate / isohexane to give (S)-tert-butyl 3-((3-carbamoyl-4-chlorophenoxy)methyl)piperidine-1-carboxylate (4.16 g, 79 %). ¹H NMR (CDCl₃): δ 1.2 - 1.3 (m, 2H), 1.35 (s, 9H), 1.4 (m, 2H), 1.6 (m, 1H), 1.8 (m, 1H), 1.9 (m, 1H), 2.8 (m, 2H), 3.8 (dd, 2H), 5.9 (s, 1H), 6.4 (s, 1H), 6.85 (dd, 1H), 7.2 (d, 1H) and 7.3 (dd, 1H).

### Intermediate 174b : (S)-tert-butyl 3-((4-chloro-3-(6-(methylsulfonyl)benzo[d]thiazol-2-ylcarbamoylcarbamoyl)phenoxy)methyl)piperidine-1-carboxylate

Split equally between 3 microwave vials were (S)-tert-butyl 3-((3-carbamoyl-4-chlorophenoxy)methyl)piperidine-1-carboxylate, potassium tert-butoxide (1M in THF) and 4-fluorophenyl 6-(methylsulfonyl)benzo[d]-thiazol-2-ylcarbamate each in THF (12 mL) and sealed into the microwave tubes. The reactions were heated to 120 °C for 10 minutes in the microwave reactor and cooled to RT. The reactions were combined and was made neutral by the addition of 2N HCl. The reaction mixture was diluted with EtOAc (100 mL), and washed with water (25 mL). The organic layer was dried over MgSO₄, filtered and evaporated to afford crude product. The crude product was purified by flash silica chromatography, elution gradient 0 to 10% MeOH in DCM. Pure fractions were evaporated to dryness to afford (S)-tert-butyl 3-((4-chloro-3-(6-(methylsulfonyl)benzo[d]thiazol-2-ylcarbamoylcarbamoyl)phenoxy)methyl)-piperidine-1-carboxylate (as a cream solid. m/z (ES+) (M+H)+ = 623 ; HPLC tR= 2.93 min.

### Intermediate 174c : (S)-2-chloro-N-(6-(methylsulfonyl)benzo[d]thiazol-2-ylcarbamoyl)-5-(piperidin-3-ylmethoxy)benzamide

Acetyl chloride (1.54 ml, 21.7 mmol) was added dropwise to MeOH (25 mL) at 22 °C over a period of 15 minutes under air, added to this was then (S)-tert-butyl 3-((4-chloro-3-(6-(methylsulfonyl)benzo[d]thiazol-2-ylcarbamoylcarbamoyl)phenoxy)methyl)-piperidine-1-carboxylate (500 mg, 0.80 mmol). The resulting solution was stirred at 55 °C for 2 hours. The reaction was evaporated to dryness, triturated with a little diethyl ether and filtered off yielding (S)-2-chloro-N-(6-(methylsulfonyl)benzo[d]thiazol-2-ylcarbamoyl)-5-(piperidin-3-ylmethoxy)benzamide (435 mg, 97 %) as a white solid. m/z (ES+) (M+H)+ = 523 ; HPLC tR= 1.35 min.

### Intermediate 175 :methyl 2-chloro-4-methoxy-5-(5-methyl-1H-pyrazol-1-yl)benzoate and methyl 2-chloro-4-methoxy-5-(5-methyl-1H-pyrazol-1-yl)benzoate.

4,4-Dimethoxybutan-2-one (2.08 g, 15.7 mmol) was added to methyl 2-chloro-5-hydrazinyl-4-methoxybenzoate hydrochloride (Intermediate 169, 4.20 g, 15.7 mmol) in methanol (100 mL) at ambient temperature under nitrogen. The resulting solution was stirred at 65 °C for 3 hours. The solvent was evaporated under reduced pressure to yield crude product. The crude product was purified by flash silica chromatography, elution gradient 40 to 100% EtOAc in isohexane. Pure fractions were evaporated to dryness to afford the two products. Different batches of product were combined to yield methyl 2-chloro-4-methoxy-5-(3-methyl-1H-pyrazol-1-yl)benzoate (0.964 g, 21 %) as a yellow solid and methyl 2-chloro-4-methoxy-5-(5-methyl-1H-pyrazol-1-yl)benzoate (0.758 g, 17%) as a yellow solid. m/z (ES+) (M+H)+ = 281; HPLC tR= 2.03 min.
¹H NMR (400.13 MHz, DMSO-d6) δ 2.08 (3H, s), 3.82 (3H, s), 3.89 (3H, s), 6.21 (1H, q), 7.45 (1H, s), 7.53 (1H, d), 7.78 (1H, s).

### Intermediate 177 :2-chloro-4-methoxy-5-(5-methyl-1H-pyrazol-1-yl)benzamide

A solution of methyl 2-chloro-4-methoxy-5-(5-methyl-1H-pyrazol-1-yl)benzoate (729 mg, 2.60 mmol) in ammonia (28% w/w in water) (50 mL, 2.60 mmol) / ammonia (7N in MeOH) (10 mL, 70.0 mmol) was stirred at ambient temperature for 16 hours. The bulk of the MeOH was removed under reduced pressure and the resulting solution was partitioned between DCM (50 mL) and water (20 mL). The layers were separated and the aqueous layer was re extracted with DCM (2 x 20 mL). The combined organic layers were washed with brine (15 mL), dried (MgSO₄), filtered and evaporated to yield 2-chloro-4-methoxy-5-(5-methyl-1H-pyrazol-1-yl)benzamide (572 mg, 83 %) as a pale yellow solid. m/z (ES+) (M+H)+ = 266; HPLC tR= 1.36 min. ¹H NMR (400.13 MHz, DMSO-d6) δ 2.08 (3H, s), 3.83 (3H, s), 6.20 (1H, q), 7.35 (1H, s), 7.37 (1H, s), 7.50 - 7.53 (2H, m), 7.84 (1H, s).

### Intermediate 178 :5-(bromomethyl)-2-chloro-N-(6-(methylsulfonyl)benzo[d]thiazol-2-ylcarbamoyl)benzamide

5-(Bromomethyl)-2-chlorobenzamide (Intermediate 33, 670mg, 2.70 mmol), and oxalyl chloride (0.259 mL, 2.97 mmol) was suspended in THF (12 mL) and sealed into a microwave tube. The reaction was heated to 120 °C for 5 minutes in the microwave reactor and cooled to RT. In another microwave tube 6-(methylsulfonyl)benzo[d]thiazol-2-amine (Intermediate 44, 616 mg, 2.70 mmol) with THF (2 mL) was stirred with *N,N-*diisopropylethylamine (0.470 mL, 2.70 mmol), and added to this with stirring, was the crude solution of the acyl isocyanate. The reaction mixture was again heated in the microwave at 120 °C for another 5mins. The reaction mixture was evaporated to dryness and then triturated with water (20 mL) and this gave a sticky pale yellow solid. This solid was then triturated and stirred in MeOH (20 mL) and filtered off, washed through with a little methanol and diethyl ether yielding 5-(bromomethyl)-2-chloro-N-(6-(methylsulfonyl)benzo[d]thiazol-2-ylcarbamoyl)benzamide (574 mg, 42.3 %) as a cream solid. Material carried through crude to the next stage and displacement of the Br with an amine.

### Intermediate 179 :2-chloro-4-hydroxy-5-nitrobenzoic acid

2-chloro-4-fluoro-5-nitrobenzoic acid (55.0 g, 0.250 mol) was added to sodium hydroxide (1.25 L, 2.50 mol) and the mixture stirred at ambient temperature for 2 hours. The mixture was acidified with conc. hydrochloric acid, the precipitated solid filtered off, washed with water and dried to give 2-chloro-4-hydroxy-5-nitrobenzoic acid (53.2 g, 98 %).

### Intermediate 180 :methyl 2-chloro-4-hydroxy-5-nitrobenzoate

To stirred methanol (1.45 L) at 0 °C was added dropwise acetyl chloride (0.290 L, 4.07 mmol). When the addition was completed, the mixture was allowed to come to ambient temperature and stirred for a further 10 minutes, then treated with 2-chloro-4-hydroxy-5-nitrobenzoic acid (17.7 g, 81.5 mmol). The mixture was stirred at ambient temperature for 16 hours then heated at 50 °C for 2 hours, evaporated in vacuo to a residue which was dried under high vacuum to give methyl 2-chloro-4-hydroxy-5-nitrobenzoate (17.2 g, 91 %).

### Intermediate 185 :methyl 2-chloro-4-isopropoxy-5-nitrobenzoate

To a stirred solution of methyl 2-chloro-4-hydroxy-5-nitrobenzoate (3.51 g, 15.16 mmol) and 2-Iodopropane (2.27 mL, 22.7 mmol) in butan-2-one (84 mL) was added potassium carbonate (6.28 g, 45.5 mmol) and the stirred mixture heated under reflux at 85 °C for 3 days. The mixture was cooled to ambient temperature, evaporated in vacuo to a residue which was taken up in water (75 mL), acidified to pH 2 with concentrated hydrochloric acid, extracted with ethyl acetate (3 x 50mL), the combined ethyl acetate extracts washed with brine, dried (MgSO₄) and evaporated in vacuo to a residue which was chromatographed on alumina with ethyl acetate as eluant to give methyl 2-chloro-4-isopropoxy-5-nitrobenzoate (3.28 g, 79 %).

### Intermediate 186 :methyl 5-amino-2-chloro-4-isopropoxybenzoate

To a stirred solution of methyl 2-chloro-4-isopropoxy-5-nitrobenzoate (5.33 g, 19.5 mmol) in methanol (130 mL) and tetrahydrofuran (64.9 mL) was added platinum (0.760 g, 0.19 mmol) and the mixture stirred at ambient temperature under an atmosphere of hydrogen gas for 16 hours. The mixture was filtered through celite, washed with methanol (25 mL), the filtrates evaporated in vacuo to a residue which was chromatographed on silica with 20% ethyl acetate in isohexane as eluant to give methyl 5-amino-2-chloro-4-isopropoxybenzoate (3.70 g, 78 %) which was shown by LCMS & NMR to contain ∼15% of the des-chloro compound. Separation by reverse phase chromatography gave methyl 5-amino-2-chloro-4-isopropoxybenzoate (3.04 g, 64%) and methyl 3-amino-4-isopropoxybenzoate (0.315 g, 7.9%). ¹H NMR (CDCl₃); δ 1.3 (d, 6H), 3.75 (s, 2H), 3.8 (s, 3H), 4.5 (dq, 1H), 6.7 (s, 1H) and 7.2 (s, 1H).

### Intermediate 189 :methyl 2-chloro-5-hydrazinyl-4-isopropoxybenzoate

To a suspension of methyl 5-amino-2-chloro-4-isopropoxybenzoate (2.95 g, 12.1 mmol) in hydrochloric acid, 37% (40 mL) at -10 °C was added a cold solution of sodium nitrite (1.00 g, 14.5 mmol) in water (12 mL) dropwise maintaining the temperature below 0 °C. The resultant solution was stirred in an ice-bath for 30 min before dropwise addition of a solution of tin(II) chloride (6.89 g, 36.3 mmol) in hydrochloric acid, 37% (12 mL) maintaining the temperature below 5 °C. The resultant suspension was attempted to be stirred at -10 °C to 0 °C for 1 h. The reaction mixture was filtered and the solid washed with water (200 mL) {this seemed to take the product into the aqueous phase, leaving behind impurities}. The aqueous phase was concentrated to 50 mL which caused the product to precipitate. This was filtered and dried to give methyl 2-chloro-5-hydrazinyl-4-isopropoxybenzoate (2.90 g, 81 %) as a white solid as the HCl salt. m/z (Ionization Method) Ion Type = No Ion; HPLC tR= 1.09 min.
¹H NMR (400.13 MHz, DMSO-d6) δ 1.30 (6H, d), 3.82 (3H, s), 4.78 - 4.84 (1H, m), 7.15 (1H, s), 7.52 (1H, s), 7.61 (1H, s), 9.74 (3H, s).

### Intermediate 190 :methyl 2-chloro-4-isopropoxy-5-(1H-pyrazol-1-yl)benzoate

To a solution of the hydrochloride salt of methyl 2-chloro-5-hydrazinyl-4-isopropoxybenzoate (2.90 g, 9.83 mmol) in ethanol (100 mL) was added malonaldehyde bis(dimethyl acetal) (3.24 mL, 19.6 mmol). The solution was heated to 80 °C for 2 h. The reaction mixture was allowed to cool and evaporated. The crude product was purified by flash silica chromatography, elution gradient 0 to 50% isohexane in EtOAc. Pure fractions were evaporated to dryness to afford methyl 2-chloro-4-isopropoxy-5-(1H-pyrazol-1-yl)benzoate (2.190 g, 76 %) as a cream solid.

### Intermediate 191 :2-chloro-4-isopropoxy-5-(1H-pyrazol-1-yl)benzamide

To a solution of methyl 2-chloro-4-isopropoxy-5-(1H-pyrazol-1-yl)benzoate (2.19 g, 7.43 mmol) in methanol (150 mL) was added 30 % (w/w) aqueous ammonia (200 mL). The reaction mixture was stirred at room temperature for 90 min. then reaction mixture was warmed to 55 °C for 3 h. The reaction mixture was concentrated until a white precipitate formed. This was filtered and dried to give 2-chloro-4-isopropoxy-5-(1H-pyrazol-1-yl)benzamide (0.647 g, 31.1 %) as a white solid.

### Intermediate 192 :2-chloro-4-isopropoxy-N-(6-(piperidin-4-ylsulfonyl)benzo[d]thiazol-2-ylcarbamoyl)-5-(1H-pyrazol-1-yl)benzamide

To a solution of 2-chloro-4-isopropoxy-5-(1H-pyrazol-1-yl)benzamide (0.546 g, 1.95 mmol) in THF (10 mL) was added oxalyl chloride (0.187 mL, 2.15 mmol). The solution was heated to 120 °C in a microwave for 5 min and then cooled to room temperature. The vial was vented and tert-butyl 4-(2-aminobenzo[d]thiazol-6-ylsulfonyl)piperidine-1-carboxylate (0.698 g, 1.76 mmol) added. The reaction mixture was reheated to 120 °C for 5 min. Seven drops of conc HCl were added and the suspension heated to 120 °C for 10 min. The reaction mixture was evaporated to a solid. This was suspended in methanol, filtered and dried to give 2-chloro-4-isopropoxy-N-(6-(piperidin-4-ylsulfonyl)benzo[d]thiazol-2-ylcarbamoyl)-5-(1H-pyrazol-1-yl)benzamide (0.418 g, 34 %) as a white solid.
m/z (ES+) (M+H)+ = 603; HPLC tR= 1.69 min.
¹H NMR (400.13 MHz, DMSO-d₆) δ 1.33 (6H, d), 1.68 - 1.78 (2H, m), 2.04 (2H, d), 2.85 (2H, m), 3.34 (2H, m), 3.57 - 3.64 (1H, m), 4.91 - 4.97 (1H, m), 6.53 - 6.54 (1H, m), 7.51 (1H, s), 7.75 (1H, d), 7.85 - 7.87 (1H, m), 7.99 - 8.03 (2H, m), 8.27 - 8.28 (1H, m), 8.35 (1H, br s), 8.63 (1H, s), 8.85 (1H, br s), 11.75 (1H, s), 11.94 (1H, s).

### Intermediate 193 : 2-Aminobenzenthiazole-6-sulfonyl chloride

Potassium nitrate (694 mg, 6.86 mmol) and sulfuryl chloride (551 µL, 6.86 mmol) were added slowly to a solution of 2-aminobenzothiazole-6-thiol (0.50 g, 2.74 mmol) in dry acetonitrile (10 mL) at 0 °C under N₂. Reaction was stirred for 2 hours at 0 °C and allowed to warm to room temperature overnight. The reaction was neutralised with saturated sodium carbonate, extracted with ethyl acetate (3 x 50 mL), washed with brine (50 mL). The organic layer was dried over MgSO₄ and concentrated under reduced pressure to give a pale yellow solid which was triturated with isohexane to give the crude product as an orange solid. Yield = 350 mg. (∼51% yield).

### Intermediate 194: tert-butyl 4-(3-carbamoyl-4-chlorophenyl)piperazine-1-carboxylate

To a solution of 2-chloro-5-fluorobenzamide (5.21 g, 30.0 mmol) in DMSO (60 mL) was added potassium carbonate (8.29 g, 60.0 mmol) and *tert*-butyl 1-piperazinecarboxylate (16.8 g, 90.0 mmol). The resultant suspension was stirred, under nitrogen, at 150 °C for 90 min and then 150 °C for 72 h. The reaction mixture was poured into water (500 mL), extracted with EtOAc (400 mL), the organic layer was dried over Na₂SO₄, filtered and evaporated to afford an oil. The crude product was purified by flash silica chromatography (20 to 100% EtOAc in isohexane) to afford tert-butyl 4-(3-carbamoyl-4-chlorophenyl)piperazine-1-carboxylate (0.960 g, 9.4 %) as an off-white solid.m/z (ESI-) (M-H)- = 338; HPLC tR = 2.12 min.

### Intermediate 195: 6-(1-(isopropylamino)-2-methylpropan-2-ylsulfonyl)benzo[d]-thiazol-2-amine

To a stirred solution of 6-(1-(isopropylamino)-2-methylpropan-2-ylthio)benzo[d]thiazol-2-amine (670 mg, 2.27 mmol) in methanol (22.7 mL) was added hydrochloric acid (6.80 mL, 6.80 mmol) followed by a solution of sodium tungstate dihydrate (15.0 mg, 0.05 mmol) in water (0.5 mL). The mixture was heated to 55° and treated with hydrogen peroxide (0.66 mL, 7.48 mmol). When the addition was completed, the mixture was heated at 55 °C for 1 hour, cooled to ambient temperature, treated with saturated sodium hydrogen carbonate solution (23 mL), the methanol evaporated *in vacuo* and the aqueous residue extracted with ethyl acetate (3 x 25 mL). The combined ethyl acetate extracts were dried (MgSO₄) and evaporated *in vacuo* to a residue which was triturated with ethyl acetate to give 6-(1-(isopropylamino)-2-methylpropan-2-ylsulfonyl)benzo[d]thiazol-2-amine (299 mg, 40%). ¹H NMR δ (DMSO d6): 0.9 (d, 6H), 1.2 (s, 6H), 2.55-2.65 (m, 1H), 2.65 (s, 2H), 7.45 (d, 1H), 7.6 (d, 1H), 8.0 (s, 2H) and 8.15 (s, 1H).

## Claims

1. A compound of formula I 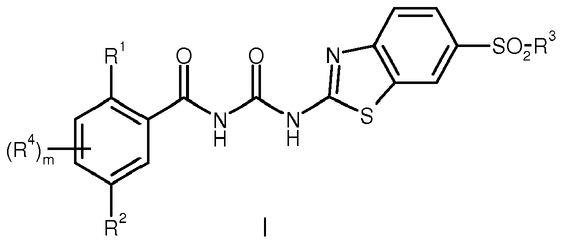 or a pharmaceutically acceptable salt thereof in which
**R¹** represents halo, nitro, a C₁₋₆alkyl group optionally substituted by one, two or three fluoro, a C₂₋₆alkenyl group, a C₃₋₆cycloalkyl group, phenyl, phenoxy, a phenylC₁₋₄alkyl group, a phenoxyC₁₋₄alkyl group, pyrrolyl, a group R^{a}S(O)ₙ(O)ₒ in which R^{a} represents phenyl or a C₁₋₄alkyl optionally substituted by one or more fluoro, n is 0, 1 or 2 and o is 0 except that when n is 2 then o is 0 or 1; wherein any aromatic ring in a substituent R¹ is optionally substituted by one or more of the following: halo, a C₁₋₃alkyl group and a C₁₋₃alkoxy group;
**R²** represents H, halo, a C₁₋₆alkyl group optionally substituted by one, two or three fluoro, a C₂₋₆alkynyl group, a C₂₋₆alkenyl group, a C₁₋₆alkylSO₂O group, a C₃₋₆cycloalkyl group, a C₃₋₆cycloalkyl C₁₋₆alkyl group, a C₃₋₆cycloalkoxy group, nitro, sulfamoyl, a group R^{b}R^{c}N(CH₂)ₚ- in which R^{b} and R^{c} independently represent H, a C₁₋₆alkyl group, a C₁₋₆alkoxycarbonyl group or a C₃₋₆cycloalkyl group or R^{b} and R^{c} together with the nitrogen atom to which they are attached represent a saturated or partially unsaturated 3 to 10 membered heterocyclic ring optionally containing an additional oxygen, nitrogen, S or SO₂ wherein the heterocyclic ring is optionally substituted by one or more of the following: a C₁₋₆alkyl group, hydroxy, a C₁₋₆alkoxycarbonyl group or a group -NR⁵R⁶ in which R⁵ and R⁶ independently represent H, a C₁₋₆alkyl group, a C₁₋₆alkoxycarbonyl group or a C₃₋₆cycloalkyl group or R⁵ and R⁶ together with the nitrogen atom to which they are attached represent a saturated or partially unsaturated 3 to 7 membered heterocyclic ring; and p = 0, 1, 2, 3, 4, 5 or 6, or **R²** represents a C₁₋₆alkoxy group optionally substituted by a group -NR^{b}R^{c} in which R^{b} and **R¹** are as defined above; or **R²** represents a five or six membered heteroaryl ring each of which is optionally substituted by one or more C₁₋₄alkyl groups or by one or more amino groups of formula -NR^{b}R^{c} in which R^{b} and **R¹** are as defined above; or **R²** represents a group (O)ᵤR⁷ in which u is 0 or 1 and R⁷ represents a carbon linked saturated or partially unsaturated 3 to 10 membered heterocyclic group containing one or more N, S or O, wherein the S may be in its oxidised form of SO or SO₂, which is optionally bicyclic including bridged and/or optionally fused to a benzene ring and any ring is optionally substituted by one or more of the following: hydroxy, oxo, carboxy, C₁₋₆alkoxycarbonyl, a C₁₋₆alkoxy group optionally substituted by one or more hydroxy and/or C₁₋₆alkoxy, C₁₋₆alkanoyl, benzoyl, amino, C₁₋₃alkylamino, di(C₁₋₃ alkyl)amino or a C₁₋₆alkyl, C₃₋₆cycloalkyl or C₃₋₆cycloalkylC₁₋₄alkyl wherein each of the last three groups is optionally substituted by one or more hydroxy and/or C₁₋₆alkoxy;
**R³** represents a C₁₋₆alkyl group, a hydroxyC₁₋₆alkyl group, a chloroC₁₋₆alkyl group, a C₁-₄alkoxyC₁₋₄alkyl group, a C₃₋₁₀cycloalkylC₁₋₄alkyl group or a group -(CH₂)_{q}-NR^{f}R^{g} in which q is 0, 1, 2 , 3, 4, 5 or 6 and the alkylene chain is optionally substituted by 1, 2, 3 or 4 C₁₋₄alkyl groups and R^{f} and R^{g} independently represent H, a C₁₋₆alkyl group optionally substituted by one or more fluoro, a C₃₋₁₀cycloalkyl group (optionally substituted by one or more fluoro and/or by or more C₁₋₄alkyl groups), a C₃₋₁₀cycloalkylC₁₋₄alkyl group, a phenylC₁₋₄alkyl group, a group (CH₂)ᵥ-R^{L} wherein v is 0, 1, 2 or 3 and R^{L} is a carbon linked saturated or partially unsaturated 3 to 10 membered heterocyclic group containing one or more N, S or O, wherein the S may be in its oxidised form of SO or SO₂, which is optionally bicyclic including bridged and/or optionally fused to a benzene ring and any ring is optionally substituted by one or more of the following: hydroxy, oxo, carboxy, a C₁₋₆alkoxy group optionally substituted by one or more hydroxy and/or C₁₋₆alkoxy, C₁₋₄alkanoyl, benzoyl, amino, C₁₋₃alkylamino, di(C₁₋₃ alkyl)amino or a C₁₋₆alkyl, C₃₋₁₀cycloalkyl or C₃₋₁₀cycloalkylC₁₋₄alkyl wherein each of the last three groups is optionally substituted by one or more hydroxy and/or C₁₋₆alkoxy;
or R^{f} and R^{g} together with the nitrogen atom to which they are attached represent a saturated or partially unsaturated 3 to 10 membered heterocyclic ring optionally containing an additional oxygen, nitrogen, S or SO₂ wherein the heterocyclic ring is optionally substituted by one or more of the following: fluoro, C₁₋₄alkyl optionally substituted by cyano, C₃₋₆cycloalkyl, hydroxy, C₁₋₄alkanoyl, C₁₋₄alkoxyC₁₋₄alkyl, C₁₋₆alkoxycarbonyl, C₁₋₄alkylsulfonyl or a group -NR^{h}Rⁱ and R^{h} and Rⁱ independently represent H or a C₁₋₄alkyl group;
or **R³** represents a group -(CH₂)ᵣ-A-(CH₂)ₛ-R^{j} in which r is 2 , 3 or 4 s is 2, 3 or 4, A is N(R^{k})-, O, S, SO or SO₂ and either alkylene chain is optionally substituted by 1, 2, 3 or 4 C₁₋₄alkyl groups, and R^{j} is hydroxyl, a C₁₋₄alkoxy group, carboxy, a group -CO₂C₁₋₄alkyl a group -CONR¹²R¹³ in which R¹² and R¹³ independently represent H or a C₁₋₄alkyl group and R^{k} is H, a C₁₋₄alkyl group or a C₃₋₆cycloalkyl group;
or **R³** represents a group -(CH₂)ᵣ-A-(CH₂)ₛ-NR^{m}Rⁿ in which r is 2 or 3, s is 2 or 3, A is N(R^{k})-, O, S, SO or SO₂ and either alkylene chain is optionally substituted by 1, 2, 3 or 4 C₁₋₄alkyl groups, and R^{m} and Rⁿ independently represent H or a C₁₋₄alkyl group, and R^{k} is H, a C₁₋₄alkyl group or a C₃₋₆cycloalkyl group;
or **R³** represents a group (CH₂)ₜ-R^{o} wherein t is 0, 1, 2 or 3 and R^{o} is a carbon linked saturated or partially unsaturated 3 to 10 membered heterocyclic group containing one or more N, S or O, wherein the S may be in its oxidised form of SO or SO₂, which is optionally bicyclic including bridged and/or optionally fused to a benzene ring and any ring is optionally substituted by one or more of the following: hydroxy, oxo, carboxy, C₁₋₆alkoxycarbonyl, a C₁₋₆alkoxy group optionally substituted by one or more hydroxy or C₁₋₆alkoxy, C₁₋₄alkanoyl, benzoyl, amino, C₁₋₃alkylamino, di(C₁₋₃ alkyl)amino or a C₁₋₆alkyl, C₃₋₆cycloalkyl or C₃₋₆cycloalkylC₁₋₄alkyl wherein each of the last three groups is optionally substituted by one or more hydroxy or C₁₋₆alkoxy; or R^{o} represents an aromatic 5 or 6 membered heterocyclic group containing one or more N, S or O, optionally substituted by one or more of the following: halo, a C₁₋₃alkyl group or a C₁₋₃alkoxy group;
or **R³** represents a C₃₋₁₀cycloalkyl group (optionally substituted by one or more groups of formula -NR^{p} R^{q} in which R^{p} and R^{q} independently represent H, C₁₋₄alkyl, C₁₋₆alkoxycarbonyl, C₁₋₄alkanoyl, C₁₋₄alkylsulfonyl or C₁₋₄alkoxyC₁₋₄alkyl group);
wherein any available aliphatic carbon atom in a group **R³** is optionally substituted by hydroxy, a C₁₋₃alkyl or C₁₋₃alkoxy provided that no more than six positions are substituted in this manner;
and any available aromatic carbon atom in a group **R³** is optionally substituted by halo, hydroxy, a C₁₋₃alkyl, or C₁₋₃alkoxy provided that no more than four positions are substituted in this manner;
**R⁴** represents halo, a C₁₋₄alkyl, C₁₋₄alkoxy, nitro, or independently a group R^{a}S(O)ₙ(O)ₒ as defined above, a saturated or partially unsaturated 3 to 10 membered heterocyclic group containing one or more N, S or O, wherein the S may be in its oxidised form of SO or SO₂, which is optionally substituted by one or more of the following: hydroxy, oxo, carboxy, a C₁₋₆alkoxy group optionally substituted by one or more hydroxy or C₁₋₆alkoxy, C₁₋₄alkanoyl, benzoyl, amino, C₁₋₃alkylamino, di(C₁₋₃ alkyl)amino or a C₁₋₆alkyl, C₃₋₆cycloalkyl or C₃₋₆cycloalkylC₁₋₄alkyl wherein each of the last three groups is optionally substituted by one or more hydroxy and/or C₁₋₆alkoxy; or R⁴ represents pyrrolyl or pyrazolyl optionally substituted by one or more C₁₋₆alkyl groups;
and m is 0, 1, 2 or 3.

2. A compound as claimed in claim 1 as represented by formula IA 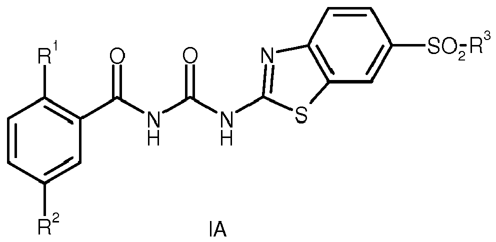 or a pharmaceutically acceptable salt thereof in which
**R¹** represents halo;
**R²** represents a C₂₋₄alkynyl group, a C₁₋₄alkylSO₂O, a C₃₋₆cycloalkyl group, a C₃₋₆cycloalkoxy group, nitro, a group R^{b}R^{c}N(CH₂)ₚ- in which p is 0 or 1 and R^{b} and **R^{c}** together with the nitrogen atom to which they are attached represent a saturated or partially unsaturated 4 to 6 membered heterocyclic ring optionally containing an additional oxygen or nitrogen wherein the heterocyclic ring is optionally substituted by one or more of the following: a C₁₋₄alkyl group or a group -NR^{d}R^{e} in which R^{d} and R^{e} independently represent H or a C₁₋₄alkyl group; a C₁₋₄alkoxy group (optionally substituted by a group NR^{d}R^{e} in which R^{d} and R^{e} independently represent H or a C₁₋₄alkyl group); or **R²** represents pyrrolyl, pyrazolyl, imidazolyl, triazolyl, thiazolyl, pyrimidinyl or pyridinyl each of which is optionally substituted by one or more C₁₋₄alkyl groups;
**R³** represents a C₁₋₄alkyl group or a group-(CH₂)_{q}-NR^{f}R^{g} in which q is 2 or 3 and R^{f} and R^{g} independently represent H, a C₁₋₄alkyl group, a C₃₋₆cycloalkyl group, a C₃₋₆cycloalkyl C₁₋₄alkyl group, or R^{f} and R^{g} together with the nitrogen atom to which they are attached represent a saturated or partially unsaturated 4 to 6 membered heterocyclic ring optionally containing an additional oxygen or nitrogen wherein the heterocyclic ring is optionally substituted by one or more C₁₋₄alkyl groups,
**or R³** represents a group -(CH₂)ᵣ-NH-(CH₂)ₛ-R^{j} in which r is 2 or 3, s is 2 or 3, and R^{j} is a C₁₋₄alkoxy group;
**or R³** represents a carbon linked saturated 4 to 6 membered heterocyclic group containing one N optionally substituted by one or more C₁₋₄alkyl groups.

3. A compound of formula IA 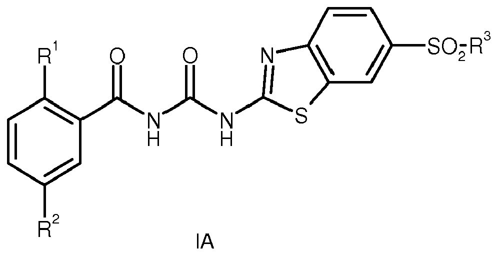 or a pharmaceutically acceptable salt thereof in which
R¹ represents bromo, chloro and iodo;
R² represents cyclopropyl, cyclopentyl, ethoxy, ethynyl, cyclopentyloxy, nitro, pyrrol-1-yl, pyridin-2-yl, pyrimidin-2-yl, pyrazol-1-yl, imidazol-1-yl, thiazol-5-yl, [1,2,3]-triazol-1-yl, [1,2,4]-triazol-1-yl, 1-pyrrolidinyl, 2,5-dihydro-pyrrol-1-yl, morpholin-4-yl, pyrrolidin-1-ylmethyl, 2-(dimethylamino)ethoxy, methylsulfonyloxy, 3-methylpyrazol-1-yl, 5-methylpyrazol-1-yl, 4-methylpiperazin-1-yl or 3-dimethylaminopyrrolidin-1-yl; and
**R³** represents methyl, pyrrolidin-3-yl, (3S)-pyrrolidin-3-yl, (3R)-pyrrolidin-3-yl, 2-(isopropylamino)ethyl, 3-(isopropylamino)propyl, 3-(diethylamino)propyl, 3-(cyclopropyl-methylamino)propyl, 3-(piperazin-1-yl)propyl, 2-(azetidin-1-yl)ethyl, 3-(azetidin-1-yl)propyl, 2-(propan-2-ylamino)ethyl, 3-(propan-2-ylamino)propyl, 2-piperazin-1-ylethyl, 3-(4-methylpiperazin-1-yl)propyl, 2-(2-methoxyethylamino)ethyl, 3-(2-methoxyethylamino)propyl or 1-methyl-4 piperidinyl.

4. A compound as claimed in any previous claim in which **R¹** is chloro.

5. A compound selected from one or more of the following:
2-chloro-N-[[6-(2-morpholin-4-ylethylsulfonyl)benzothiazol-2-yl]carbamoyl]benzamide;
2-chloro-N-[[6-[2-(2-methoxyethylamino)ethylsulfonyl]benzothiazol-2-yl]carbamoyl]-benzamide;
2-chloro-N-[[6-(2-dimethylaminoethylsulfonyl)benzothiazol-2-yl]carbamoyl]benzamide;
2-chloro-N-[[6-(2-methylaminoethylsulfonyl)benzothiazol-2-yl]carbamoyl]benzamide;
2-chloro-N-[[6-[2-(3-hydroxypyrrolidin-1-yl)ethylsulfonyl]benzothiazol-2-yl]carbamoyl]benzamide;
2-chloro-N-[[6-(2-pyrrolidin-1-ylethylsulfonyl)benzothiazol-2-yl]carbamoyl]benzamide;
2-chloro-N-[[6-(2-diethylaminoethylsulfonyl)benzothiazol-2-yl]carbamoyl]benzamide;
2-chloro-N-[[6-(2-piperazin-1-ylethylsulfonyl)benzothiazol-2-yl]carbamoyl]benzamide;
2-chloro-N-[[6-[2-(2-methoxyethyl-methyl-amino)ethylsulfonyl]benzothiazol-2-yl]carbamoyl]benzamide;
2-chloro-N-[[6-[2-(propan-2-ylamino)ethylsulfonyl]benzothiazol-2-yl]carbamoyl]benzamide;
2-chloro-N-[[6-[2-(2-hydroxyethyl-methyl-amino)ethylsulfonyl]benzothiazol-2-yl]carbamoyl]benzamide;
N-[[6-[2-(butan-2-ylamino)ethylsulfonyl]benzothiazol-2-yl]carbamoyl]-2-chlorobenzamide;
N-[[6-[2-(azetidin-1-yl)ethylsulfonyl]benzothiazol-2-yl]carbamoyl]-2-chlorobenzamide;
2-chloro-N-[[6-[2-(2-hydroxyethylamino)ethylsulfonyl]benzothiazol-2-yl]carbamoyl]-benzamide;
2-chloro-N-[[6-[2-(ethyl-(2-methoxyethyl)amino)ethylsulfonyl]benzothiazol-2-yl]carbamoyl]benzamide;
2-chloro-N-[[6-[2-[(3S)-3-fluoropyrrolidin-1-yl]ethylsulfonyl]benzothiazol-2-yl]carbamoyl]benzamide;
2-chloro-N-[[6-[2-(cyclopentylamino)ethylsulfonyl]benzothiazol-2-yl]carbamoyl]benzamide;
2-chloro-N-[[6-[2-(2,5-dimethylpyrrolidin-1-yl)ethylsulfonyl]benzothiazol-2-yl]carbamoyl]benzamide;
2-chloro-N-[[6-[2-(2,6-dimethylmorpholin-4-yl)ethylsulfonyl]benzothiazol-2-yl]carbamoyl]benzamide;
2-chloro-N-[[6-[2-(1-phenylethylamino)ethylsulfonyl]benzothiazol-2-yl]carbamoyl]benzamide;
2-chloro-N-[[6-[2-(3,3-difluoropyrrolidin-1-yl)ethylsulfonyl]benzothiazol-2-yl]carbamoyl]benzamide;
2-chloro-N-[[6-[2-(oxolan-2-ylmethylamino)ethylsulfonyl]benzothiazol-2-yl]carbamoyl]benzamide;
2-chloro-N-[[6-[2-(4-methylpiperazin-1-yl)ethylsulfonyl]benzothiazol-2-yl]carbamoyl]benzamide;
2-chloro-N-[[6-[2-(3,5-dimethylpiperazin-1-yl)ethylsulfonyl]benzothiazol-2-yl]carbamoyl]benzamide;
2-chloro-N-[[6-[2-(cyclobutylamino)ethylsulfonyl]benzothiazol-2-yl]carbamoyl]benzamide;
2-chloro-N-[[6-[2-(methyl-(oxolan-2-ylmethyl)amino)ethylsulfonyl]benzothiazol-2-yl]carbamoyl]benzamide;
2-chloro-N-[[6-[2-(2-methyl-1-piperidyl)ethylsulfonyl]benzothiazol-2-yl]carbamoyl]benzamide;
2-chloro-N-[[6-[2-(2-methylpropylamino)ethylsulfonyl]benzothiazol-2-yl]carbamoyl]benzamide;
2-chloro-N-[[6-[2-(4-ethylpiperazin-1-yl)ethylsulfonyl]benzothiazol-2-yl]carbamoyl]benzamide;
2-chloro-N-[[6-[2-(1,4-oxazepan-4-yl)ethylsulfonyl]benzothiazol-2-yl]carbamoyl]benzamide;
2-chloro-N-[[6-[2-(2-fluoroethylamino)ethylsulfonyl]benzothiazol-2-yl]carbamoyl]benzamide;
2-chloro-N-[[6-[2-[(3R)-3-fluoropyrrolidin-1-yl]ethylsulfonyl]benzothiazol-2-yl]carbamoyl]benzamide;
2-chloro-N-[[6-[2-(1-piperidyl)ethylsulfonyl]benzothiazol-2-yl]carbamoyl]benzamide;
2-chloro-N-[[6-[2-(cyclopropylmethylamino)ethylsulfonyl]benzothiazol-2-yl]carbamoyl]benzamide;
2-chloro-N-[[6-[2-(norboman-2-ylamino)ethylsulfonyl]benzothiazol-2-yl]carbamoyl]benzamide;
2-chloro-N-[[6-[2-[2-(methoxymethyl)pyrrolidin-1-yl]ethylsulfonyl]benzothiazol-2-yl]carbamoyl]benzamide;
2-chloro-N-[[6-[2-[(1S,4S)-3-oxa-6-azabicyclo[2.2.1]hept-6-yl]ethylsulfonyl]benzothiazol-2-yl] carbamoyl]benzamide;
2-chloro-N-[[6-[2-(2-propan-2-yloxyethylamino)ethylsulfonyl]benzothiazol-2-yl]carbamoyl]benzamide;
2-chloro-N-[[6-[2-[(1-methylcyclopropyl)amino]ethylsulfonyl]benzothiazol-2-yl]carbamoyl]benzamide;
2-chloro-N-[[6-(2-methoxyethylsulfonyl)benzothiazol-2-yl]carbamoyl]benzamide;
2-chloro-N-[[6-(2-hydroxyethylsulfonyl)benzothiazol-2-yl]carbamoyl]benzamide;
2-chloro-N-[[6-[2-(2-methoxyethoxy)ethylsulfonyl]benzothiazol-2-yl]carbamoyl]-benzamide;
2-chloro-N-[[6-[2-(2-dimethylaminoethoxy)ethylsulfonyl]benzothiazol-2-yl]carbamoyl]benzamide;
2-chloro-N-[(6-pyrrolidin-3-ylsulfonylbenzothiazol-2-yl)carbamoyl]benzamide;
2-chloro-5-(pyridin-2-yl)-N-(6-(methylsulfonyl)benzothiazol-2-ylcarbamoyl)benzamide;
2-chloro-5-(pyrazol-1-yl)-N-(6-(methylsulfonyl)benzothiazol-2-ylcarbamoyl)benzamide;
2-chloro-5-[1,2,3]triazol-1-yl-N-(6-(methylsulfonyl)benzothiazol-2-ylcarbamoyl)benzamide;
2-chloro-5-(1-pyrrolidinyl)-N-(6-(methylsulfonyl)benzothiazol-2-ylcarbamoyl)benzamide;
2-chloro-5-cyclopropyl-N-(6-(methylsulfonyl)benzothiazol-2-ylcarbamoyl)benzamide;
2-chloro-5-(2,5-dihydro-pyrrol-1-yl)-N-(6-(methylsulfonyl)benzothiazol-2-ylcarbamoyl)benzamide;
2-chloro-5-cyclopentyloxy-N-(6-(methylsulfonyl)benzothiazol-2-ylcarbamoyl)benzamide;
2-chloro-5-cyclopentyl-N-(6-(methylsulfonyl)benzothiazol-2-ylcarbamoyl)benzamide;
2-chloro-5-ethoxy-N-(6-(methylsulfonyl)benzothiazol-2-ylcarbamoyl)benzamide;
2-chloro-N-(6-(methylsulfonyl)benzothiazol-2-ylcarbamoyl)-5- morpholin-4-yl benzamide;
2-chloro-5-(5-methyl-1H-pyrazol-1-yl)-N-(6-(methylsulfonyl)benzothiazol-2-ylcarbamoyl)benzamide;
2-chloro-5-(3-methyl-1H-pyrazol-1-yl)-N-(6-(methylsulfonyl)benzothiazol-2-ylcarbamoyl)benzamide;
2-chloro-N-(6-(methylsulfonyl)benzothiazol-2-ylcarbamoyl)-5-(pyrimidin-2-yl)benzamide;
4-chloro-3-(6-(methylsulfonyl)benzothiazol-2-ylcarbamoylcarbamoyl)phenyl methanesulfonate;
2-chloro-5-(4-methylpiperazin-1-yl)-N-(6-(methylsulfonyl)benzothiazol-2-ylcarbamoyl)benzamide;
2-chloro-5-(3-(dimethylamino)pyrrolidin-1-yl)-N-(6-(methylsulfonyl)benzothiazol-2-ylcarbamoyl)benzamide;
2-chloro-5-ethynyl-N-(6-(methylsulfonyl)benzothiazol-2-ylcarbamoyl)benzamide;
2-chloro-5-pyrrolidin-1-ylmethyl-N-(6-(methylsulfonyl)benzothiazol-2-ylcarbamoyl)benzamide;
2-chloro-5-(2-(dimethylamino)ethoxy)-N-(6-(methylsulfonyl)benzothiazol-2-ylcarbamoyl)benzamide;
2,5-chloro-N-[(6-methylsulfonylbenzothiazol-2-yl)carbamoyl]benzamide;
2-chloro-5-methyl-N-(6-(methylsulfonyl)benzothiazol-2-ylcarbamoyl)benzamide;
2-chloro-N-[(6-methylsulfonylbenzothiazol-2-yl)carbamoyl]-5-pyrrol-1-yl-benzamide;
2-chloro-N-(6-(methylsulfonyl)benzothiazol-2-ylcarbamoyl)-5-(1H-1,2,4-triazol-1-yl)benzamide;
2-chloro-N-(6-(methylsulfonyl)benzothiazol-2-ylcarbamoyl)-5-(thiazol-5-yl)benzamide;
2-chloro-5-(1H-imidazol-1-yl)-N-(6-(methylsulfonyl)benzothiazol-2-ylcarbamoyl)benzamide;
2-chloro-N-(6-(methylsulfonyl)benzothiazol-2-ylcarbamoyl)-5-nitrobenzamide;
2-chloro-N-[[6-(3-diethylaminopropylsulfonyl)benzothiazol-2-yl]carbamoyl]-5-pyrrol-1-yl-benzamide;
2-chloro-N-[[6-[3-(cyclopropylmethylamino)propylsulfonyl]benzothiazol-2-yl]carbamoyl]-5-pyrrol-1-yl-benzamide;
2-chloro-N-[[6-(3-piperazin-1-ylpropylsulfonyl)benzothiazol-2-yl]carbamoyl]-5-pyrrol-1-yl-benzamide;
N-[[6-[3-(azetidin-1-yl)propylsulfonyl]benzothiazol-2-yl]carbamoyl]-2-chloro-5-pyrrol-1-yl-benzamide;
2-chloro-N-[[6-[3-(propan-2-ylamino)propylsulfonyl]benzothiazol-2-yl]carbamoyl]-5-pyrrol-1-yl-benzamide;
2-chloro-N-[[6-[3-(2-methoxyethylamino)propylsulfonyl]benzothiazol-2-yl]carbamoyl]-5-pyrrol-1-yl-benzamide;
2-chloro-N-[[6-[(3S)-pyrrolidin-3-yl]sulfonylbenzothiazol-2-yl]carbamoyl]-5-pyrrol-1-yl-benzamide;
2-chloro-N-[[6-[(3R)-pyrrolidin-3-yl]sulfonylbenzothiazol-2-yl]carbamoyl]-5-pyrrol-1-yl-benzamide;
2-chloro-N-[[6-(2-piperazin-1-ylethylsulfonyl)benzothiazol-2-yl]carbamoyl]-5-pyrrol-1-yl-benzamide;
2-chloro-N-[[6-[2-(2-methoxyethylamino)ethylsulfonyl]benzothiazol-2-yl]carbamoyl]-5-pyrrol-1-yl-benzamide;
N-[[6-[2-(azetidin-1-yl)ethylsulfonyl]benzothiazol-2-yl]carbamoyl]-2-chloro-5-pyrrol-1-yl-benzamide;
2-chloro-N-[[6-[2-(propan-2-ylamino)ethylsulfonyl]benzothiazol-2-yl]carbamoyl]-5-pyrrol-1-yl-benzamide;
2-bromo-N-(6-(2-(isopropylamino)ethylsulfonyl)benzothiazol-2-ylcarbamoyl)-5-(1 H-pyrrol-1-yl)benzamide;
2-iodo-N-(6-(2-(isopropylamino)ethylsulfonyl)benzothiazol-2-ylcarbamoyl)-5-(1 H-pyrrol-1-yl)benzamide;
2-chloro-N-(6-(3-(4-methylpiperazin-1-yl)propylsulfonyl)benzothiazol-2-ylcarbamoyl)-5-(1H-pyrrol-1-yl)benzamide;
2-chloro-N-(6-(3-(4-methylpiperazin-1-yl)propylsulfonyl)benzothiazol-2-ylcarbamoyl)-5-(pyridin-2-yl)benzamide;
2-chloro-N-(6-(3-(4-methylpiperazin-1-yl)propylsulfonyl)benzothiazol-2-ylcarbamoyl)-5-(pyrrolidin-1-yl)benzamide;
2-chloro-N-(6-(3-(4-methylpiperazin-1-yl)propylsulfonyl)benzothiazol-2-ylcarbamoyl)-5-(1H-pyrazol-1-yl)benzamide;
2-chloro-N-(6-(3-(4-methylpiperazin-1-yl)propylsulfonyl)benzothiazol-2-ylcarbamoyl)-5-morpholinobenzamide;
2-chloro-5-(5-methyl-1H-pyrazol-1-yl)-N-(6-(3-(4-methylpiperazin-1-yl)propylsulfonyl)benzothiazol-2-ylcarbamoyl)benzamide;
2-chloro-5-(3-methyl-1H-pyrazol-1-yl)-N-(6-(3-(4-methylpiperazin-1-yl)propylsulfonyl)benzothiazol-2-ylcarbamoyl)benzamide;
4-chloro-3-(6-(3-(4-methylpiperazin-1-yl)propylsulfonyl)benzothiazol-2-ylcarbamoylcarbamoyl)phenyl methanesulfonate;
2-chloro-5-ethoxy-N-(6-(3-(4-methylpiperazin-1-yl)propylsulfonyl)benzothiazol-2-ylcarbamoyl)benzamide;
2-chloro-5-cyclopropyl-N-(6-(3-(4-methylpiperazin-1-yl)propylsulfonyl)benzothiazol-2-ylcarbamoyl)benzamide;
2-chloro-N-(6-(3-(4-methylpiperazin-1-yl)propylsulfonyl)benzothiazol-2-ylcarbamoyl)-5-(1H-1,2,4-triazol-1-yl)benzamide;
2-chloro-N-(6-(3-(4-methylpiperazin-1-yl)propylsulfonyl)benzothiazol-2-ylcarbamoyl)-5-(thiazol-5-yl)benzamide;
2-chloro-N-(6-(3-(diethylamino)propylsulfonyl)benzothiazol-2-ylcarbamoyl)-5-morpholinobenzamide;
2-chloro-N-(6-(3-(diethylamino)propylsulfonyl)benzothiazol-2-ylcarbamoyl)-5-(1H-pyrazol-1-yl)benzamide;
2-chloro-N-(6-(1-methylpiperidin-4-ylsulfonyl)benzothiazol-2-ylcarbamoyl)-5-morpholinobenzamide;
2-chloro-N-(6-(1-methylpiperidin-4-ylsulfonyl)benzothiazol-2-ylcarbamoyl)-5-(1H-pyrazol-1-yl)benzamide;
2,6-dichloro-N-[(6-methylsulfonylbenzothiazol-2-yl)carbamoyl]benzamide;
2-chloro-N-[(6-methylsulfonylbenzothiazol-2-yl)carbamoyl]benzamide;
2-bromo-N-[(6-methylsulfonylbenzothiazol-2-yl)carbamoyl]benzamide;
N-[(6-methylsulfonylbenzothiazol-2-yl)carbamoyl]-2-nitro-benzamide;
N-[(6-methylsulfonylbenzothiazol-2-yl)carbamoyl]-2-phenyl-benzamide;
2-chloro-6-fluoro-N-[(6-methylsulfonylbenzothiazol-2-yl)carbamoyl]benzamide;
2-chloro-4-fluoro-N-[(6-methylsulfonylbenzothiazol-2-yl)carbamoyl]benzamide;
2-fluoro-N-[(6-methylsulfonylbenzothiazol-2-yl)carbamoyl]benzamide;
2-chloro-3-fluoro-N-[(6-methylsulfonylbenzothiazol-2-yl)carbamoyl]benzamide;
2-chloro-3,4-dimethoxy-N-[(6-methylsulfonylbenzothiazol-2-yl)carbamoyl]benzamide;
2,6-difluoro-N-[(6-methylsulfonylbenzothiazol-2-yl)carbamoyl]benzamide;
2-chloro-4-methylsulfonyl-N-[(6-methylsulfonylbenzothiazol-2-yl)carbamoyl]benzamide;
2-Chloro-N-[(6-methylsulfonylbenzothiazol-2-yl)carbamoyl]-4-morpholin-4-yl-benzamide;
2-Chloro-N-[(6-methylsulfonylbenzothiazol-2-yl)carbamoyl]-4-pyrazol-1-yl-benzamide;
2-Chloro-N-[(6-methylsulfonylbenzothiazol-2-yl)carbamoyl]-4-pyrrolidin-1-yl-benzamide;
2-Iodo-N-[(6-methylsulfonylbenzothiazol-2-yl)carbamoyl]benzamide;
2-Chloro-4-(2,5-dimethylpyrrol-1-yl)-N-[(6-methylsulfonylbenzothiazol-2-yl)carbamoyl]benzamide;
N-[[6-[2-(Carbamoylmethoxy)ethylsulfonyl]benzothiazol-2-yl]carbamoyl]-2-chlorobenzamide;
2-Chloro-N-[[6-[2-(2-hydroxyethoxy)ethylsulfonyl]benzothiazol-2-yl]carbamoyl]-benzamide;
2-[2-[2-[(2-Chlorobenzoyl)carbamoylamino]benzothiazol-6-yl]sulfonylethoxy]acetic acid;
2-(4-Fluorophenyl)-4-methoxy-N-[(6-methylsulfonylbenzothiazol-2-yl)carbamoyl]benzamide;
2-(4-Methoxyphenyl)-N-[(6-methylsulfonylbenzothiazol-2-yl)carbamoyl]benzamide;
2-(2-Fluorophenyl)-N-[(6-methylsulfonylbenzothiazol-2-yl)carbamoyl]benzamide;
2-(4-Fluorophenyl)-N-[(6-methylsulfonylbenzothiazol-2-yl)carbamoyl]benzamide;
N-[(6-Methylsulfonylbenzothiazol-2-yl)carbamoyl]-2-phenoxy-benzamide;
2-Methyl-N-[(6-methylsulfonylbenzothiazol-2-yl)carbamoyl]benzamide;
2-Chloro-N-[(6-ethenylsulfonylbenzothiazol-2-yl)carbamoyl]benzamide;
2-Ethylsulfanyl-N-[(6-methylsulfonylbenzothiazol-2-yl)carbamoyl]benzamide;
N-[(6-Methylsulfonylbenzothiazol-2-yl)carbamoyl]-2-(phenoxymethyl)benzamide;
2-Methylsulfanyl-N-[(6-methylsulfonylbenzothiazol-2-yl)carbamoyl]benzamide;
N-[(6-Methylsulfonylbenzothiazol-2-yl)carbamoyl]-2-phenylsulfanyl-benzamide;
N-[(6-Methylsulfonylbenzothiazol-2-yl)carbamoyl]-2-pyrrol-1-yl-benzamide;
2-Ethylsulfonyl-N-[(6-methylsulfonylbenzothiazol-2-yl)carbamoyl]benzamide;
N-[(6-Methylsulfonylbenzothiazol-2-yl)carbamoyl]-2-propan-2-yl-benzamide;
N-[(6-Methylsulfonylbenzothiazol-2-yl)carbamoyl]-2-(trifluoromethylsulfonyloxy)-benzamide;
2-Chloro-N-[(6-methylsulfonylbenzothiazol-2-yl)carbamoyl]-5-sulfamoyl-benzamide;
2-Chloro-N-[[6-(3-piperidylsulfonyl)benzothiazol-2-yl]carbamoyl]benzamide;
2-Chloro-N-[[6-[(1-propan-2-yl-3-piperidyl)sulfonyl]benzothiazol-2-yl]carbamoyl]benzamide;
2-Ethyl-N-[(6-methylsulfonylbenzothiazol-2-yl)carbamoyl]benzamide;
2-Chloro-N-[[6-[(1-ethyl-3-piperidyl)sulfonyl]benzothiazol-2-yl]carbamoyl]benzamide;
2-Chloro-N-[[6-[[1-(cyclopropylmethyl)-3-piperidyl]sulfonyl]benzothiazol-2-yl]carbamoyl]benzamide;
2-Chloro-N-[[6-[1-(cyclopropylmethyl)pyrrolidin-3-yl]sulfonylbenzothiazol-2-yl]carbamoyl]benzamide;
2-Chloro-N-[[6-[3-(propan-2-ylamino)propylsulfonyl]benzothiazol-2-yl]carbamoyl]benzamide;
2-Chloro-N-[[6-(3-morpholin-4-ylpropylsulfonyl)benzothiazol-2-yl]carbamoyl]benzamide;
N-[[6-[3-(Azetidin-1-yl)propylsulfonyl]benzothiazol-2-yl]carbamoyl]-2-chloro-benzamide;
2-Chloro-N-[[6-(3-chloropropylsulfonyl)benzothiazol-2-yl]carbamoyl]benzamide;
2-Chloro-N-[[6-(3-pyrrolidin-1-ylpropylsulfonyl)benzothiazol-2-yl]carbamoyl]benzamide;
2-Chloro-N-[[6-[3-(1,1-dioxo-1,4-thiazinan-4-yl)propylsulfonyl]benzothiazol-2-yl]carbamoyl]benzamide;
2-Chloro-N-[[6-[3-(cyclopropylmethylamino)propylsulfonyl]benzothiazol-2-yl]carbamoyl]benzamide;
2-Chloro-N-[[6-[3-(1-piperidyl)propylsulfonyl]benzothiazol-2-yl]carbamoyl]benzamide;
2-Chloro-N-[[6-(3-diethylaminopropylsulfonyl)benzothiazol-2-yl]carbamoyl]benzamide;
2-Chloro-N-[[6-[3-(3,3-difluoropyrrolidin-1-yl)propylsulfonyl]benzothiazol-2-yl]carbamoyl]benzamide;
2-Chloro-N-[[6-[3-(4-methylpiperazin-1-yl)propylsulfonyl]benzothiazol-2-yl]carbamoyl]benzamide;
2-Chloro-N-[[6-[[(2R)-1-(cyclopropylmethyl)pyrrolidin-2-yl]methylsulfonyl]benzothiazol-2-yl] carbamoyl]benzamide;
2-Chloro-N-[[6-[3-(3-fluoropyrrolidin-1-yl)propylsulfonyl]benzothiazol-2-yl]carbamoyl]benzamide;
2-Chloro-N-[[6-[3-(4,4-difluoro-1-piperidyl)propylsulfonyl]benzothiazol-2-yl]carbamoyl]benzamide;
2-Chloro-N-[[6-[3-(2-hydroxyethylamino)propylsulfonyl]benzothiazol-2-yl]carbamoyl]benzamide;
2-Chloro-N-[[6-[3-(3-hydroxypyrrolidin-1-yl)propylsulfonyl]benzothiazol-2-yl]carbamoyl]benzamide;
5-Bromo-2-chloro-N-[(6-methylsulfonylbenzothiazol-2-yl)carbamoyl]benzamide;
2-Chloro-N-[[6-(1-propan-2-ylpyrrolidin-3-yl)sulfonylbenzothiazol-2-yl]carbamoyl]benzamide;
2-Chloro-N-[[6-(1-ethylpyrrolidin-3-yl)sulfonylbenzothiazol-2-yl]carbamoyl]benzamide;
2-Chloro-N-[[6-[[(2R)-1-ethylpyrrolidin-2-yl]methylsulfonyl]benzothiazol-2-yl]carbamoyl]benzamide;
2-Chloro-N-[[6-[[(2R)-pyrrolidin-2-yl]methylsulfonyl]benzothiazol-2-yl]carbamoyl]benzamide;
2-Chloro-N-[[6-[(1-methyl-3-piperidyl)sulfonyl]benzothiazol-2-yl]carbamoyl]benzamide;
2-Chloro-N-[[6-(1-methylpyrrolidin-3-yl)sulfonylbenzothiazol-2-yl]carbamoyl]benzamide;
2-Chloro-N-[[6-[[(2R)-1-propan-2-ylpyrrolidin-2-yl]methylsulfonyl]benzothiazol-2-yl]carbamoyl]benzamide;
2-Chloro-N-[[6-[[(2R)-1-methylpyrrolidin-2-yl]methylsulfonyl]benzothiazol-2-yl]carbamoyl]benzamide;
N-[(6-Methylsulfonylbenzothiazol-2-yl)carbamoyl]-2-(trifluoromethyl)benzamide;
2-Chloro-N-[[6-[(3S)-pyrrolidin-3-yl]sulfonylbenzothiazol-2-yl]carbamoyl]benzamide;
2-Chloro-N-[[6-[(3R)-pyrrolidin-3-yl]sulfonylbenzothiazol-2-yl]carbamoyl]benzamide;
2-Chloro-N-[[6-[(1-ethyl-4-piperidyl)sulfonyl]benzothiazol-2-yl]carbamoyl]benzamide;
2-Chloro-N-[[6-[(1-propan-2-yl-4-piperidyl)sulfonyl]benzothiazol-2-yl]carbamoyl]benzamide;
2-Chloro-N-[[6-[1-(cyclopropylmethyl)azetidin-3-yl]sulfonylbenzothiazol-2-yl]carbamoyl]benzamide;
2-Chloro-N-[[6-(4-piperidylsulfonyl)benzothiazol-2-yl]carbamoyl]benzamide;
2-Chloro-4,5-difluoro-N-[(6-methylsulfonylbenzothiazol-2-yl)carbamoyl]benzamide;
2-Chloro-N-[[6-(pyridin-2-ylmethylsulfonyl)benzothiazol-2-yl]carbamoyl]benzamide;
2,4-Dichloro-N-[(6-methylsulfonylbenzothiazol-2-yl)carbamoyl]benzamide;
2-Chloro-N-[(6-methylsulfonylbenzothiazol-2-yl)carbamoyl]-4-nitro-benzamide;
2-Chloro-N-[(6-methylsulfonylbenzothiazol-2-yl)carbamoyl]-5-(trifluoromethyl)benzamide;
2-Chloro-N-[[6-[[1-(cyclopropylmethyl)-4-piperidyl]sulfonyl]benzothiazol-2-yl]carbamoyl]benzamide;
N-[[6-(Azetidin-3-ylsulfonyl)benzothiazol-2-yl]carbamoyl]-2-chloro-benzamide;
2-Chloro-N-[[6-[(1-methyl-4-piperidyl)sulfonyl]benzothiazol-2-yl]carbamoyl]benzamide;
2-Chloro-N-[[6-(1-ethylazetidin-3-yl)sulfonylbenzothiazol-2-yl]carbamoyl]benzamide;
2-Chloro-N-[[6-(1-propan-2-ylazetidin-3-yl)sulfonylbenzothiazol-2-yl]carbamoyl]benzamide;
2-Chloro-N-[[6-(pyridin-3-ylmethylsulfonyl)benzothiazol-2-yl]carbamoyl]benzamide;
2-Chloro-N-[[6-[(5-methyl-1,2-oxazol-3-yl)methylsulfonyl]benzothiazol-2-yl]carbamoyl]benzamide;
2-Chloro-N-[[6-(1H-imidazol-2-ylmethylsulfonyl)benzothiazol-2-yl]carbamoyl]benzamide;
2-Chloro-N-[[6-(2-pyridin-2-ylethylsulfonyl)benzothiazol-2-yl]carbamoyl]benzamide;
2-Chloro-N-[[6-[(2-methyl-1,3-thiazol-4-yl)methylsulfonyl]benzothiazol-2-yl]carbamoyl]benzamide;
2-Chloro-N-[[6-(3-methoxypropylsulfonyl)benzothiazol-2-yl]carbamoyl]benzamide;
2-Chloro-N-[[6-(3-imidazol-1-ylpropylsulfonyl)benzothiazol-2-yl]carbamoyl]benzamide;
2-Chloro-6-fluoro-3-methyl-N-[(6-methylsulfonylbenzothiazol-2-yl)carbamoyl]benzamide;
6-Chloro-2-fluoro-3-methyl-N-[(6-methylsulfonylbenzothiazol-2-yl)carbamoyl]benzamide;
2-Chloro-N-[[6-[3-[(3S,5R)-3,5-dimethylpiperazin-1-yl]propylsulfonyl]benzothiazol-2-yl]carbamoyl]benzamide;
2-Chloro-N-[[6-[3-(4-ethylpiperazin-1-yl)propylsulfonyl]benzothiazol-2-yl]carbamoyl]benzamide;
N-[[6-[3-(4-Acetylpiperazin-1-yl)propylsulfonyl]benzothiazol-2-yl]carbamoyl]-2-chlorobenzamide;
2-Chloro-N-[[6-[3-(4-propan-2-ylpiperazin-1-yl)propylsulfonyl]benzothiazol-2-yl]carbamoyl]benzamide;
2-Chloro-N-[[6-[3-[4-(2-methoxyethyl)piperazin-1-yl]propylsulfonyl]benzothiazol-2-yl]carbamoyl]benzamide;
2-Chloro-N-[[6-[3-(4-dimethylamino-1-piperidyl)propylsulfonyl]benzothiazol-2-yl]carbamoyl]benzamide;
2-Chloro-N-[[6-(3-dimethylaminopropylsulfonyl)benzothiazol-2-yl]carbamoyl]benzamide;
2-Chloro-N-[[6-[3-(2-methoxyethylamino)propylsulfonyl]benzothiazol-2-yl]carbamoyl]benzamide;
2-Chloro-N-[[6-[3-(4-methyl-1,4-diazepan-1-yl)propylsulfonyl]benzothiazol-2-yl]carbamoyl]benzamide;
tert-Butyl 4-[3-[2-[(2-chlorobenzoyl)carbamoylamino]benzothiazol-6-yl]sulfonylpropyl]piperazine-1-carboxylate;
2-Chloro-N-[[6-(3-piperazin-1-ylpropylsulfonyl)benzothiazol-2-yl]carbamoyl]benzamide;
2-Chloro-N-[[6-[3-[4-(2-cyanoethyl)piperazin-1-yl]propylsulfonyl]benzothiazol-2-yl]carbamoyl]benzamide;
2-Chloro-N-[[6-[3-(4-methylsulfonylpiperazin-1-yl)propylsulfonyl]benzothiazol-2-yl]carbamoyl]benzamide;
2-Chloro-N-[[6-[3-(3-methylpiperazin-1-yl)propylsulfonyl]benzothiazol-2-yl]carbamoyl]benzamide;
2-Chloro-N-[[6-[4-(4-methylpiperazin-1-yl)butylsulfonyl]benzothiazol-2-yl]carbamoyl]benzamide;
2-Chloro-N-[[6-(4-diethylaminobutylsulfonyl)benzothiazol-2-yl]carbamoyl]benzamide;
2-Chloro-N-[[6-[3-(4-methylpiperazin-1-yl)propylsulfonyl]benzothiazol-2-yl]carbamoyl]-4-(3-methylpyrazol-1-yl)benzamide;
tert-Butyl N-[3-[2-[(2-chlorobenzoyl)carbamoylamino]benzothiazol-6-yl]sulfonylcyclobutyl]carbamate;
N-[[6-(3-Aminocyclobutyl)sulfonylbenzothiazol-2-yl]carbamoyl]-2-chloro-benzamide;
2-Chloro-N-[[6-(3-methylaminocyclobutyl)sulfonylbenzothiazol-2-yl]carbamoyl]benzamide;
2-Chloro-N-[[6-(3-dimethylaminocyclobutyl)sulfonylbenzothiazol-2-yl]carbamoyl]benzamide;
*tert*-Butyl 4-[[2-[(2-chlorobenzoyl)carbamoylamino]benzothiazol-6-yl]sulfonylmethyl]piperidine-1-carboxylate;
tert-Butyl 4-[2-[2-[(2-chlorobenzoyl)carbamoylamino]benzothiazol-6-yl]sulfonylethyl]piperidine-1-carboxylate;
2-Chloro-N-[[6-(4-piperidylmethylsulfonyl)benzothiazol-2-yl]carbamoyl]benzamide;
2-Chloro-5-ethynyl-N-[[6-[3-(4-methylpiperazin-1-yl)propylsulfonyl]benzothiazol-2-yl]carbamoyl]benzamide;
2-Chloro-N-[[6-[2-(4-piperidyl)ethylsulfonyl]benzothiazol-2-yl]carbamoyl]benzamide;
2-Chloro-N-[[6-[3-(2-methoxyethylamino)cyclobutyl]sulfonylbenzothiazol-2-yl]carbamoyl]benzamide;
N-[[6-(3-Acetamidocyclobutyl)sulfonylbenzothiazol-2-yl]carbamoyl]-2-chlorobenzamide;
2-chloro-N-[[6-(isopropylsulfamoyl)-1,3-benzothiazol-2-yl]carbamoyl]benzamide;
2-chloro-N-(6-(1-(isopropylamino)-2-methylpropan-2-ylsulfonyl)benzo[d]thiazol-2-ylcarbamoyl)benzamide;
2-chloro-5-ethyl-N-(6-(3-(4-methylpiperazin-1-yl)propylsulfonyl)benzo[d]thiazol-2-ylcarbamoyl)benzamide;
5-(1-acetylpyrrolidin-3-yloxy)-2-chloro-N-(6-(methylsulfonyl)benzo[d]thiazol-2-ylcarbamoyl)benzamide;
(S)-2-chloro-5-((1-isopropylpiperidin-3-yl)methoxy)-N-(6-(methylsulfonyl)benzo[d]-thiazol-2-ylcarbamoyl)benzamide;
2-chloro-N-[(6-sulfamoyl-1,3-benzothiazol-2-yl)carbamoyl]benzamide;
2-chloro-N-[[6-(methylsulfamoyl)-1,3-benzothiazol-2-yl]carbamoyl]benzamide;
2-chloro-N-[[6-(2-hydroxyethylsulfamoyl)-1,3-benzothiazol-2-yl]carbamoyl]benzamide;
2-chloro-N-[[6-(dimethylsulfamoyl)-1,3-benzothiazol-2-yl]carbamoyl]benzamide;
(R)-2-chloro-5-(3-(dimethylamino)pyrrolidin-1-yl)-N-(6-(methylsulfonyl)benzo[d]thiazol-2-ylcarbamoyl)benzamide;
tert-butyl 4-(4-chloro-3-(6-(methylsulfonyl)benzo[d]thiazol-2-ylcarbamoylcarbamoyl)phenyl)piperazine-1-carboxylate;
(S)-2-chloro-5-(3-(dimethylamino)pyrrolidin-1-yl)-N-(6-(methylsulfonyl)benzo[d]thiazol-2-ylcarbamoyl)benzamide;
2-chloro-5-(4-(dimethylamino)piperidin-1-yl)-N-(6-(methylsulfonyl)benzo[d]thiazol-2-ylcarbamoyl)benzamide;
2-chloro-5-(3,5-dimethyl-1H-pyrazol-1-yl)-N-(6-(3-(4-methylpiperazin-1-yl)propylsulfonyl)benzo[d]thiazol-2-ylcarbamoyl)benzamide;
2-chloro-5-iodo-N-(6-(3-(4-methyl-1,4-diazepan-1-yl)propylsulfonyl)benzo[d]thiazol-2-ylcarbamoyl)benzamide;
2-chloro-5-(3-(diethylamino)pyrrolidin-1-yl)-N-(6-(methylsulfonyl)benzo[d]thiazol-2-ylcarbamoyl)benzamide;
2-chloro-N-(6-(3-(4-methylpiperazin-1-yl)propylsulfonyl)benzo[d]thiazol-2-ylcarbamoyl)-3-(1H-pyrazol-1-yl)benzamide;
2,4-dichloro-N-(6-(3-(4-methylpiperazin-1-yl)propylsulfonyl)benzo[d]thiazol-2-ylcarbamoyl)-5-(1H-pyrazol-1-yl)benzamide;
2-chloro-4-methoxy-N-(6-(3-(4-methylpiperazin-1-yl)propylsulfonyl)benzo[d]thiazol-2-ylcarbamoyl)-5-(1H-pyrazol-1-yl)benzamide;
tert-butyl 4-(4-chloro-3-(6-(methylsulfonyl)benzo[d]thiazol-2-ylcarbamoylcarbamoyl)phenoxy)piperidine-1-carboxylate;
2-chloro-5-(1-methylpiperidin-4-yloxy)-N-(6-(methylsulfonyl)benzo[d]thiazol-2-ylcarbamoyl)benzamide;
2-chloro-N-(6-(2-methyl-1-(pyrrolidin-1-yl)propan-2-ylsulfonyl)benzo[d]thiazol-2-ylcarbamoyl)-5-morpholinobenzamide;
2-chloro-N-(6-(1-(isopropylamino)-2-methylpropan-2-ylsulfonyl)benzo[d]thiazol-2-ylcarbamoyl)-5-morpholinobenzamide;
2-chloro-5-(3-(dimethylamino)pyridin-2-yl)-N-(6-(methylsulfonyl)benzo[d]thiazol-2-ylcarbamoyl)benzamide;
tert-butyl 3-(4-chloro-3-(6-(methylsulfonyl)benzo[d]thiazol-2-ylcarbamoylcarbamoyl)phenoxy)pyrrolidine-1-carboxylate;
2-chloro-N-(6-(methylsulfonyl)benzo[d]thiazol-2-ylcarbamoyl)-5-(pyrrolidin-3-yloxy)benzamide;
2-chloro-5-(1-methylpyrrolidin-3-yloxy)-N-(6-(methylsulfonyl)benzo[d]thiazol-2-ylcarbamoyl)benzamide;
2-chloro-5-(6-(dimethylamino)pyridin-2-yl)-N-(6-(methylsulfonyl)benzo[d]thiazol-2-ylcarbamoyl)benzamide;
tert-butyl 3-(4-chloro-3-(6-(methylsulfonyl)benzo[d]thiazol-2-ylcarbamoylcarbamoyl)phenoxy)azetidine-1-carboxylat;e
5-(azetidin-3-yloxy)-2-chloro-N-(6-(methylsulfonyl)benzo[d]thiazol-2-ylcarbamoyl)benzamide;
2-chloro-4-fluoro-N-(6-(1-methylpiperidin-4-ylsulfonyl)benzo[d]thiazol-2-ylcarbamoyl)-5-(1H-pyrazol-1-yl)benzamide;
2-chloro-4-methoxy-N-(6-(1-methylpiperidin-4-ylsulfonyl)benzo[d]thiazol-2-ylcarbamoyl)-5-morpholinobenzamide;
2-chloro-5-ethoxy-4-methoxy-N-(6-(1-methylpiperidin-4-ylsulfonyl)benzo[d]thiazol-2-ylcarbamoyl)benzamide;
2-chloro-5-(1-methylazetidin-3-yloxy)-N-(6-(methylsulfonyl)benzo[d]thiazol-2-ylcarbamoyl)benzamide;
2,4-dichloro-N-(6-(1-methylpiperidin-4-ylsulfonyl)benzo[d]thiazol-2-ylcarbamoyl)-5-morpholinobenzamide;
2,4-dichloro-N-(6-(1-methylpiperidin-4-ylsulfonyl)benzo[d]thiazol-2-ylcarbamoyl)-5-(1H-pyrazol-1-yl)benzamide;
2-chloro-4-methyl-N-(6-(1-methylpiperidin-4-ylsulfonyl)benzo[d]thiazol-2-ylcarbamoyl)-5-(1H-pyrazol-1-yl)benzamide;
2-chloro-4-ethoxy-N-(6-(1-methylpiperidin-4-ylsulfonyl)benzo[d]thiazol-2-ylcarbamoyl)-5-(1H-pyrazol-1-yl)benzamide;
tert-butyl 4-(2-(3-(2-chloro-4-methoxy-5-(1H-pyrazol-1-yl)benzoyl)ureido)benzo[d]-thiazol-6-ylsulfonyl)piperidine-1-carboxylate;
2-chloro-4-methoxy-N-(6-(piperidin-4-ylsulfonyl)benzo[d]thiazol-2-ylcarbamoyl)-5-(1H-pyrazol-1-yl)benzamide;
2-chloro-4-methoxy-N-(6-(1-methylpiperidin-4-ylsulfonyl)benzo[d]thiazol-2-ylcarbamoyl)-5-(1H-pyrazol-1-yl)benzamide;
2-chloro-4-methoxy-5-(5-methyl-1H-pyrazol-1-yl)-N-(6-(1-methylpiperidin-4-ylsulfonyl)benzo[d]thiazol-2-ylcarbamoyl)benzamide;
2-chloro-5-((dimethylamino)methyl)-N-(6-(methylsulfonyl)benzo[d]thiazol-2-ylcarbamoyl)benzamide;
2-chloro-4-isopropoxy-N-(6-(1-methylpiperidin-4-ylsulfonyl)benzo[d]thiazol-2-ylcarbamoyl)-5-(1H-pyrazol-1-yl)benzamide;
2-chloro-5-(3-dimethylamino-1-piperidyl)-N-[(6-methylsulfonyl-1,3-benzothiazol-2-yl)carbamoyl]benzamide;
2-Chloro-5-(3-dimethylamino-1-piperidyl)-N-[(6-methylsulfonyl-1,3-benzothiazol-2-yl)carbamoyl]benzamide; or
2-Chloro-5-(3-dimethylaminocyclobutoxy)-N-[(6-methylsulfonyl-1,3-benzothiazol-2-yl)carbamoyl]benzamide
or a pharmaceutically acceptable salt thereof.

6. A compound selected from one or more of the following:
2-Chloro-5-(3-(dimethylamino)pyrrolidin-1-yl)-N-(6-(methylsulfonyl)benzothiazol-2-ylcarbamoyl)benzamide;
2-Chloro-N-(6-(3-(4-methylpiperazin-1-yl)propylsulfonyl)benzothiazol-2-ylcarbamoyl)-5-(pyrrolidin-1-yl)benzamide;
2-Chloro-N-(6-(3-(4-methylpiperazin-1-yl)propylsulfonyl)benzothiazol-2-ylcarbamoyl)-5-morpholinobenzamide;
2-Chloro-5-(5-methyl-1H-pyrazol-1-yl)-N-(6-(3-(4-methylpiperazin-1-yl)propylsulfonyl)benzothiazol-2-ylcarbamoyl)benzamide;
2-Chloro-5-ethoxy-N-(6-(3-(4-methylpiperazin-1-yl)propylsulfonyl)benzothiazol-2-ylcarbamoyl)benzamide;
2-Chloro-N-(6-(1-methylpiperidin-4-ylsulfonyl)benzothiazol-2-ylcarbamoyl)-5-morpholinobenzamide;
2-Chloro-5-ethynyl-N-[[6-[3-(4-methylpiperazin-1-yl)propylsulfonyl]benzothiazol-2-yl]carbamoyl]benzamide;
2-chloro-5-ethyl-N-(6-(3-(4-methylpiperazin-1-yl)propylsulfonyl)benzo[d]thiazol-2-ylcarbamoyl)benzamide;
2-chloro-N-(6-(2-methyl-1-(pyrrolidin-1-yl)propan-2-ylsulfonyl)benzo[d]thiazol-2-ylcarbamoyl)-5-morpholinobenzamide;
2-chloro-4-fluoro-N-(6-(1-methylpiperidin-4-ylsulfonyl)benzo[d]thiazol-2-ylcarbamoyl)-5-(1H-pyrazol-1-yl)benzamide;
2-chloro-4-methoxy-N-(6-(1-methylpiperidin-4-ylsulfonyl)benzo[d]thiazol-2-ylcarbamoyl)-5-morpholinobenzamide;
or a pharmaceutically acceptable salt thereof.

7. The use of a compound according to any one of claims 1 to 6 as a medicament.

8. A compound according to any one of claims 1 to 6 for the treatment of obesity or being overweight, for the prevention of weight gain, for the modulation of appetite and/or satiety, eating disorders, for the treatment of diabetes, for the treatment of metabolic syndrome, for the treatment of the Prader-Willi syndrome, for the treatment of cachexia resulting from cancer or congestive heart failure, for the treatment of wasting due to ageing or AIDS or chronic liver failure or chronic obstructive pulmonary disease.

9. A compound according to any one of claims 1 to 6 for the treatment of obesity or being overweight, prevention of weight gain, for modulation of appetite and/or satiety, eating disorders and the treatment of diabetes mellitus.

10. A compound according to any one of claims 2 to 4 or claim 6 for use in the treatment of obesity or type 2 diabetes.

11. A pharmaceutical formulation comprising a compound according to any one of claims 1 to 6 or pharmaceutically acceptable salt thereof, in admixture with pharmaceutically acceptable adjuvants, diluents and/or carriers.

12. A compound according to any one of claims 1 to 6 in combination with another therapeutic agent that is useful in the treatment of obesity and/or diabetes.

13. A process to prepare a compound according to any one of claims 1 to 6 comprising:
a) reacting a benzamide of formula (II): 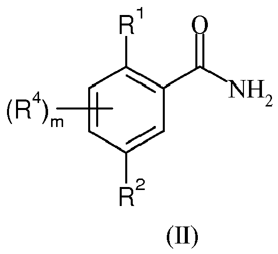 in which R¹, R², R⁴ and m are as previously defined with an amine of formula (III) 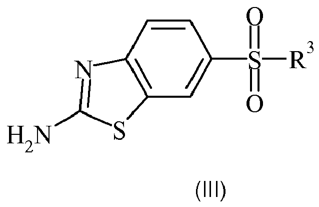 in which R³ is as previously defined in the presence of oxalyl chloride, optionally in the presence of an inert solvent, optionally in the presence of a base, optionally in the presence of a Lewis acid, at a temperature in the range of 80 - 150 °C to give a compound of formula (I); or
b) reacting a compound of formula (IV): 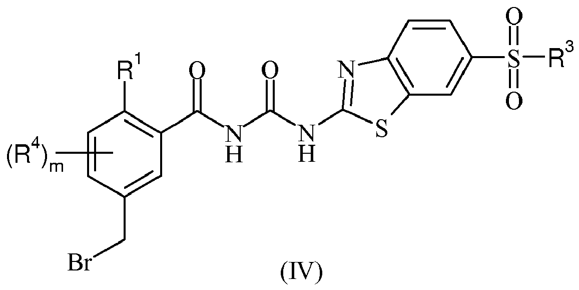 in which R¹, R³, R⁴ and m are as previously defined with an amine, optionally in the presence of an inert solvent, at a temperature in the range between ambient temperature and the boiling point of the solvent to give a compound of formula (I); or
c) reacting a compound of formula (V): 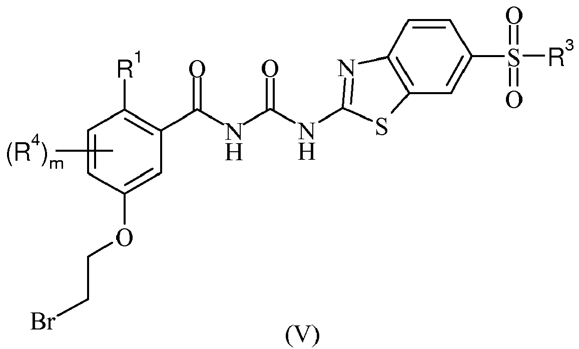 in which R¹, R³, R⁴ and m are as previously defined with an amine, optionally in the presence of an inert solvent, at a temperature in the range of 80 - 150°C to give a compound of formula (I); or
d) reacting a carbamate of formula (VI): 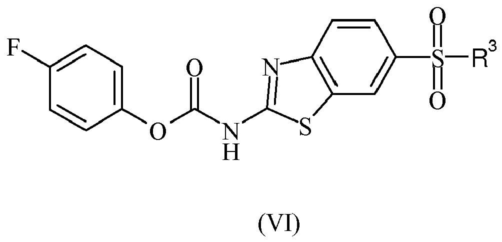 in which R³ is as previously defined with a benzamide of formula (II), optionally in the presence of an inert solvent, in the presence of a base, at a temperature in the range between ambient temperature and 150°C to give a compound of formula (I); or
e) reacting a compound of formula (VII): 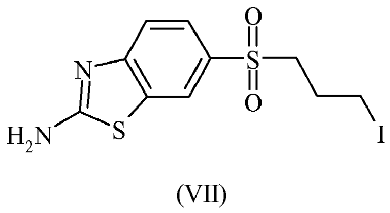 with a benzamide of formula (II) in the presence of oxalyl chloride and then an amine, optionally in the presence of an inert solvent, at a temperature in the range of 50 - 150°C to give a compound of formula (I); or
f) reacting a compound of formula (VIII): 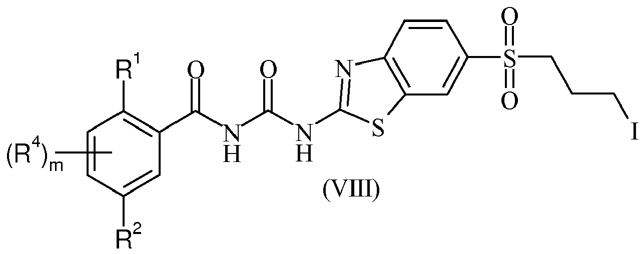 in which R¹, R², R⁴ and m are as previously defined with an amine, optionally in the presence of an inert solvent, at a temperature in the range between ambient temperature and 150 °C to give a compound of formula (I); or
g) reacting a compound of formula (IX): 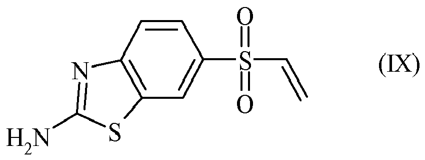 with a benzamide of formula (II) in the presence of oxalyl chloride and then an amine, optionally in the presence of an inert solvent, at a temperature in the range of 80 - 150 °C to give a compound of formula (I); or
h) reacting a compound of formula (X): 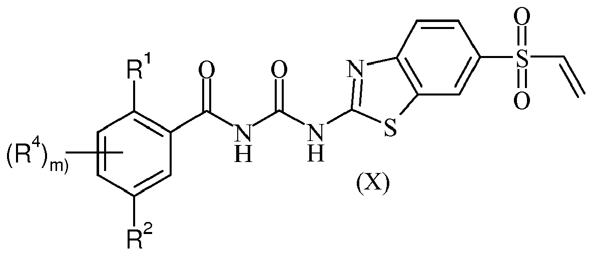 in which R¹, R², R⁴ and m are as previously defined with an amine of formula (XI) 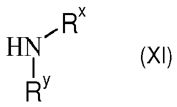 in which R^{x} and R^{y} are as previously defined optionally in the presence of an inert solvent, at a temperature in the range of 80 - 150 °C to give a compound of formula (I) I in which R³ represents a group -(CH₂)₂-NR^{x}R^{y}, and R¹, R², R⁴, m, R^{x} and R^{y} are as previously defined; or
i) reacting a compound of formula (X) with an alcohol or an alkoxide salt thereof, optionally in the presence of an inert solvent, in the presence of a base when the alcohol is used, at a temperature in the range between 0 °C and the boiling point of the solvent to give a compound of formula (I); or
j) reacting a compound of formula (X) with a base or a hydrolysing agent, optionally in the presence of an inert solvent, at a temperature in the range between ambient temperature and 150 °C to give a compound of formula (I); or
k) reacting a compound of formula (XII): 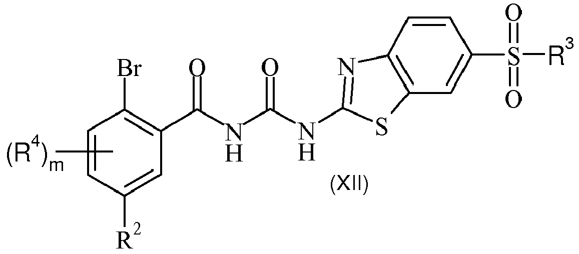 in which R², R³, R⁴ and m are as previously defined with copper(I) iodide, and a ligand, optionally in the presence of an additive, optionally in the presence of an inert solvent, at a temperature in the range of 80 - 150 °C to give a compound of formula (I).
